# EUROPEAN PATENT APPLICATION

(11) **EP 4 835 076 A1**
(43) Date of publication of application: **04.11.2026**
(21) Application number: 24913148.3
(22) Date of filing: 27.12.2024
(51) Int. Cl.: C12N 15/113, A61K 31/713, A61K 48/00, C07H 21/02

(54) **DOUBLE-STRANDED RNA CONTAINING CARBAMOYLETHYL MODIFICATION**

(30) Priority: 28.12.2023 JP 2023222881
(71) Applicant: Nissan Chemical Corporation, Tokyo 103-6119 (JP)
(72) Inventor: IRIYAMA Yusuke, Funabashi-shi, Chiba 274-8507 (JP); NAKAO Moemi, Funabashi-shi, Chiba 274-8507 (JP); NISHIGUCHI Yuna, Funabashi-shi, Chiba 274-8507 (JP); ISHIKAWA Ryutaro, Shiraoka-shi, Saitama 349-0294 (JP)
(74) Representative: dompatent
(86) International application number: PCT/JP2024/046495
(87) International publication number: WO 2025/143262

(57) **Abstract**

An object of the present invention is to provide a chemically modified siRNA in which metabolic stability is further enhanced while activity is maintained.

The present invention provides a double-stranded RNA capable of inhibiting expression of a target RNA, in which the double-stranded RNA comprises a sense strand and an antisense strand, the antisense strand has 14 to 40 nucleotides and has complementarity to the target RNA sufficient to mediate RNA interference, the sense strand has 14 to 40 nucleotides and has complementarity to the antisense strand, and the double-stranded RNA comprises at least one 2'-O-XCE nucleotide.

## Description

### Technical Field

The present invention relates to a double-stranded RNA that contains a carbamoylethyl modification (a 2'-O-XCE nucleotide) and is capable of inhibiting expression of a target RNA, a pharmaceutical composition including the double-stranded RNA, and a method for controlling expression of the target RNA using the double-stranded RNA.

### Background Art

As a method for inhibiting expression of a target gene in a cell, a tissue, or an individual, there is a technique in which a double-stranded RNA called a small interfering RNA (siRNA) is introduced into the cell, the tissue, or the individual. By introduction of a double-stranded RNA, a target RNA having homology to the sequence is degraded, and expression of a target gene is inhibited. This effect is called "RNA interference" or "RNAi". The siRNA is complementary to a target sequence on a transcript, and after entering a cell, the siRNA is loaded into an RNA-induced silencing complex (RISC). In this loading process, a sense strand is removed, and an antisense strand remains in the RISC, where the antisense strand binds to a complementary site thereof on the target RNA. The RNA that has undergone binding is then cleaved by nuclease activity of RISC, and then can be further degraded by a cellular nuclease (for example, refer to Non Patent Literature 1).

The siRNA composed of natural RNA nucleotides is rapidly degraded even when administered in vivo, and does not easily reach a target organ. Therefore, for therapeutic use, it is necessary to use a chemically modified siRNA. Usually, in order to enhance metabolic stability of siRNA, 2'-fluoro (2'-F) nucleotides and 2'-O-methyl (2'-O-Me) nucleotides are used as chemically modified nucleic acid units (a modified nucleoside or a modified nucleotide formed by phosphorylation thereof) (for example, refer to Non Patent Literature 2 and Non Patent Literature 3).

As a modification form of an oxygen atom at position 2' of a sugar moiety of a ribonucleotide, a methylcarbamoylethyl nucleotide (2'-O-MCE nucleotide) has been reported (for example, refer to Patent Literature 1 and Non Patent Literature 4). Furthermore, it has been reported that high nuclease resistance is obtained by introducing an amino group or a heterocyclic group to a nitrogen atom of a carbamoylethyl group via an alkyl group (2'-O-R-ECE nucleotide) (for example, refer to Patent Literature 2 and Non Patent Literature 5). These nucleotides, such as a 2'-O-MCE nucleotide and a 2'-O-R-ECE nucleotide, in which an oxygen atom at position 2' of a sugar moiety is modified via a carbamoylethyl group, are collectively referred to as 2'-O-XCE nucleotides.

### Citation List

### Patent Literature

Patent Literature 1: WO 2007/102581 A
Patent Literature 2: WO 2017/142054 A

### Non Patent Literature

Non Patent Literature 1: Nature, 1998, 391, pp 806-811
Non Patent Literature 2: Nature Reviews Drug Discovery volume, 2019, 18, pp 421-446
Non Patent Literature 3: Molecular Therapy, 2018, 26, pp 708-717
Non Patent Literature 4: The Journal of Organic Chemistry, 2011, 76, pp 3042-3053
Non Patent Literature 5: Organic & Biomolecular Chemistry, 2019, 17, pp 4835-4842

### Summary of Invention

### Technical Problem

In a chemically modified siRNA, there is a demand for a novel technique in which metabolic stability is further enhanced while maintaining activity, or in which activity and persistence are improved.

An object of the present invention is to provide a chemically modified siRNA in which metabolic stability is further enhanced while maintaining activity, or in which activity and persistence are improved.

### Solution to Problem

The present inventors have found that, in a chemically modified siRNA, by partially substituting with a 2'-O-XCE nucleotide, metabolic stability is further enhanced while maintaining activity, or activity and persistence are improved, and have thereby completed the present invention. That is, the present invention includes the following aspects.
1. A double-stranded RNA capable of inhibiting expression of a target RNA, wherein
   the double-stranded RNA comprises a sense strand and an antisense strand,
   the antisense strand has 14 to 40 nucleotides and has complementarity to the target RNA sufficient to mediate RNA interference,
   the sense strand has 14 to 40 nucleotides and has complementarity to the antisense strand, and
   the double-stranded RNA comprises at least one 2'-O-XCE nucleotide.
2. The double-stranded RNA according to 1., wherein the antisense strand includes at least one 2'-O-XCE nucleotide, or the sense strand includes at least one 2'-O-XCE nucleotide.
2-2. The double-stranded RNA according to 1., wherein the antisense strand includes at least one 2'-O-XCE nucleotide.
2-3. The double-stranded RNA according to 1., wherein the sense strand includes at least one 2'-O-XCE nucleotide.
2-4. The double-stranded RNA according to 1., wherein the antisense strand and the sense strand each independently include at least one 2'-O-XCE nucleotide.
3. The double-stranded RNA according to any one of 1. to 2-4., wherein the double-stranded RNA comprises at least one 2'-O-XCE nucleotide and at least one 2'-fluoro nucleotide.
4. The double-stranded RNA according to any one of 1. to 3., wherein the double-stranded RNA comprises at least one 2'-O-XCE nucleotide and at least one 2'-O-Me nucleotide.
4-2. The double-stranded RNA according to any one of 1. to 4., wherein the nucleotides constituting the double-stranded RNA are selected from a 2'-O-XCE nucleotide, a 2'-O-Me nucleotide, and a 2'-fluoro nucleotide.
5. The double-stranded RNA according to any one of 1. to 4-2., wherein the double-stranded RNA comprises at least one 2'-O-XCE nucleotide and at least one deoxyribonucleotide.
5-2. The double-stranded RNA according to any one of 1. to 5., wherein the nucleotides constituting the double-stranded RNA are selected from a 2'-O-XCE nucleotide, a 2'-O-Me nucleotide, a 2'-fluoro nucleotide, and a deoxyribonucleotide.
6. The double-stranded RNA according to any one of 1. to 5-2., wherein the antisense strand includes a 2'-O-XCE nucleotide at least one of positions 1, 3, 4, 5, 6, 7, 8, 9, 10, 11, 12, 13, 15, 16, 17, 18, 19, 20, 21, 22, and 23 as counted from a 5' end of the antisense strand hybridizing portion.
6-2. The double-stranded RNA according to any one of 1. to 6., wherein the antisense strand includes a 2'-fluoro nucleotide or a deoxyribonucleotide at position 2 as counted from a 5' end of the antisense strand hybridizing portion.
6-3. The double-stranded RNA according to any one of 1. to 6-2., wherein the antisense strand includes 1 to 10 (preferably 1 to 9, more preferably 1, 2, 3, or 4) 2'-O-XCE nucleotides.
6-4. The double-stranded RNA according to any one of 1. to 6-3., wherein the antisense strand includes a 2'-O-XCE nucleotide at least one of positions 1, 3, 4, 5, 6, 7, 8, 9, and 10 as counted from a 5' end of the antisense strand hybridizing portion.
6-5. The double-stranded RNA according to any one of 1. to 6-4., wherein the antisense strand includes a 2'-O-XCE nucleotide at position 1 as counted from a 5' end of the antisense strand hybridizing portion.
6-6. The double-stranded RNA according to any one of 1. to 6-5., wherein the antisense strand includes a 2'-O-XCE nucleotide at position 3 as counted from a 5' end of the antisense strand hybridizing portion.
6-7. The double-stranded RNA according to any one of 1. to 6-6., wherein the antisense strand includes a 2'-O-XCE nucleotide at position 4 as counted from a 5' end of the antisense strand hybridizing portion.
6-8. The double-stranded RNA according to any one of 1. to 6-7., wherein the antisense strand includes a 2'-O-XCE nucleotide at position 5 as counted from a 5' end of the antisense strand hybridizing portion.
6-9. The double-stranded RNA according to any one of 1. to 6-8., wherein the antisense strand includes a 2'-O-XCE nucleotide at position 6 as counted from a 5' end of the antisense strand hybridizing portion.
6-10. The double-stranded RNA according to any one of 1. to 6-9., wherein the antisense strand includes a 2'-O-XCE nucleotide at position 7 as counted from a 5' end of the antisense strand hybridizing portion.
6-11. The double-stranded RNA according to any one of 1. to 6-10., wherein the antisense strand includes a 2'-O-XCE nucleotide at position 8 as counted from a 5' end of the antisense strand hybridizing portion.
6-12. The double-stranded RNA according to any one of 1. to 6-11., wherein the antisense strand includes a 2'-O-XCE nucleotide at position 9 as counted from a 5' end of the antisense strand hybridizing portion.
6-13. The double-stranded RNA according to any one of 1. to 6-12., wherein the antisense strand includes a 2'-O-XCE nucleotide at position 10 as counted from a 5' end of the antisense strand hybridizing portion.
7. The double-stranded RNA according to any one of 1. to 6-13., wherein the sense strand includes a 2'-O-XCE nucleotide at least one of positions 1, 2, 3, 4, 5, 6, 7, 8, 9, 10, 14, 15, 16, 17, 18, 19, 20, 21, 22, and 23 as counted from a 3' end of the sense strand hybridizing portion.
7-2. The double-stranded RNA according to any one of 1. to 7., wherein the sense strand includes a 2'-O-Me nucleotide, a 2'-fluoro nucleotide, or a deoxyribonucleotide each independently at positions 11, 12, and 13 as counted from a 3' end of the sense strand hybridizing portion.
7-3. The double-stranded RNA according to any one of 1. to 7-2., wherein the sense strand includes 2 to 10 (preferably 2 to 4, more preferably 2 or 4) 2'-O-XCE nucleotides.
7-4. The double-stranded RNA according to any one of 1. to 7-3., wherein the sense strand includes a 2'-O-XCE nucleotide at least one of positions 1, 2, 20, and 21 as counted from a 3' end of the sense strand hybridizing portion.
7-5. The double-stranded RNA according to any one of 1. to 7-4., wherein the sense strand includes a 2'-O-XCE nucleotide at position 1 as counted from a 3' end of the sense strand hybridizing portion.
7-6. The double-stranded RNA according to any one of 1. to 7-5., wherein the sense strand includes a 2'-O-XCE nucleotide at position 21 as counted from a 3' end of the sense strand hybridizing portion.
7-7. The double-stranded RNA according to any one of 1. to 7-6., wherein the sense strand includes a 2'-O-XCE nucleotide at position 2 as counted from a 3' end of the sense strand hybridizing portion.
7-8. The double-stranded RNA according to any one of 1. to 7-7., wherein the sense strand includes a 2'-O-XCE nucleotide at position 20 as counted from a 3' end of the sense strand hybridizing portion.
7-9. The double-stranded RNA according to any one of 1. to 7-8., wherein the sense strand includes a 2'-O-XCE nucleotide at position 1 as counted from a 5' end of the sense strand hybridizing portion.
7-10. The double-stranded RNA according to any one of 1. to 7-9., wherein the sense strand includes a 2'-O-XCE nucleotide at position 2 as counted from a 5' end of the sense strand hybridizing portion.
7-11. The double-stranded RNA according to any one of 1. to 7-10., wherein the sense strand includes a 2'-O-XCE nucleotide at position 1 as counted from a 5' end of the sense strand.
7-12. The double-stranded RNA according to any one of 1. to 7-11., wherein the sense strand includes a 2'-O-XCE nucleotide at position 2 as counted from a 5' end of the sense strand.
7-13. The double-stranded RNA according to any one of 1. to 7-12., wherein the sense strand includes a 2'-O-XCE nucleotide at position 1 as counted from a 3' end of the sense strand.
7-14. The double-stranded RNA according to any one of 1. to 7-13., wherein the sense strand includes a 2'-O-XCE nucleotide at position 2 as counted from a 3' end of the sense strand.
8. The double-stranded RNA according to any one of 1. to 7-13., wherein the 2'-O-XCE nucleotide is a nucleotide including a partial structure represented by Formula (I) below: 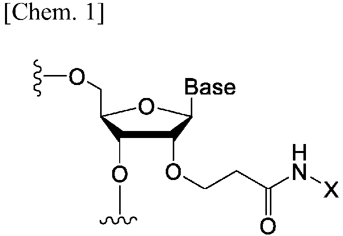
   {in which Base is a purin-9-yl group, a 2-oxo-pyrimidin-1-yl group, or a 2-thioxo-pyrimidin-1-yl group (the purin-9-yl group, the 2-oxo-pyrimidin-1-yl group, and the 2-thioxo-pyrimidin-1-yl group are each independently unsubstituted or substituted with one or more substituents solely or differently selected from the group consisting of a halogen atom, a C1-6 alkyl group, an amino group, a protected amino group, a hydroxy group, a protected hydroxy group, a sulfanyl group, and a protected sulfanyl group),
   X is a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group), or
   a group represented by Formula (Ia) below: 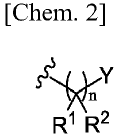
   [in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
   Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group) or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with the nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
   a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
   n is an integer of 1 to 3, and when n is 2 or 3, two or three R¹ and R² may be the same or different, respectively]}.
9. The double-stranded RNA according to 8., wherein the X is a C1-6 alkyl group or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group).
10. The double-stranded RNA according to 8. or 9., wherein the X is a methyl group.
11. The double-stranded RNA according to 8., wherein the X is a group represented by the following Formula (Ia): 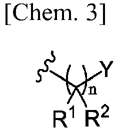
   [in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
   Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with a nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
   a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
   n is an integer of 1 to 3, and when n is 2 or 3, each of the two or three R¹ and R² may be the same or different].
12. The double-stranded RNA according to 11., wherein the R¹ and R² are hydrogen atoms.
13. The double-stranded RNA according to 11. or 12., wherein
   the Y is NR³R⁴, and the R³ and R⁴ together with a nitrogen atom, to which they are bonded, form a ring including 4 to 6 methylene groups among 4- to 8-membered nitrogen-containing non-aromatic heterocyclic rings, or
   Y is a C2-9 aromatic heterocyclic group.
14. The double-stranded RNA according to any one of 11. to 13., wherein the Y is NR³R⁴, and the R³ and R⁴ together with a nitrogen atom, to which they are bonded, form morpholine.
15. The double-stranded RNA according to any one of 11. to 13., wherein the Y is a pyridyl group, an imidazolyl group, or a benzimidazolyl group.
16. The double-stranded RNA according to any one of 11. to 15., wherein n is 2.
17. The double-stranded RNA according to any one of 1. to 16., wherein the antisense strand includes one, two, three, four, five, or six 2'-fluoro nucleotides.
17-2. The double-stranded RNA according to any one of 1. to 17., wherein the antisense strand includes a 2'-fluoro nucleotide at least one position selected from positions 2, 6, 8, 9, 14, and 16 as counted from a 5' end of the antisense strand hybridizing portion.
17-3. The double-stranded RNA according to any one of 1. to 17-2., wherein the antisense strand includes a 2'-fluoro nucleotide at position 2 as counted from a 5' end of the antisense strand hybridizing portion.
17-4. The double-stranded RNA according to any one of 1. to 17-3., wherein the antisense strand includes a 2'-fluoro nucleotide at position 14 as counted from a 5' end of the antisense strand hybridizing portion.
17-5. The double-stranded RNA according to any one of 1. to 17-4., wherein the antisense strand includes a 2'-fluoro nucleotide at position 16 as counted from a 5' end of the antisense strand hybridizing portion.
17-6. The double-stranded RNA according to any one of 1. to 17-5., wherein the antisense strand includes a 2'-fluoro nucleotide at position 6 as counted from a 5' end of the antisense strand hybridizing portion.
18. The double-stranded RNA according to any one of 1. to 17-6., wherein the sense strand includes one, two, three, four, or five 2'-fluoro nucleotides.
18-2. The double-stranded RNA according to any one of 1. to 18., wherein the sense strand includes a 2'-fluoro nucleotide at least one position selected from positions 11, 12, 13, and 15 as counted from a 3' end of the sense strand hybridizing portion.
18-3. The double-stranded RNA according to any one of 1. to 18-2., wherein the sense strand includes a 2'-fluoro nucleotide at position 15 as counted from a 3' end of the sense strand hybridizing portion.
18-4. The double-stranded RNA according to any one of 1. to 18-3., wherein the sense strand includes 2'-fluoro nucleotides at positions 11, 12, and 13 as counted from a 3' end of the sense strand hybridizing portion.
18-5. The double-stranded RNA according to any one of 1. to 18-3., wherein the sense strand includes a deoxyribonucleotide at position 11 and 2'-fluoro nucleotides at positions 12 and 13 as counted from a 3' end of the sense strand hybridizing portion.
18-6. The double-stranded RNA according to any one of 1. to 18-3., wherein the sense strand includes a deoxyribonucleotide at position 12 and 2'-fluoro nucleotides at positions 11 and 13 as counted from a 3' end of the sense strand hybridizing portion.
18-7. The double-stranded RNA according to any one of 1. to 18-3., wherein the sense strand includes a deoxyribonucleotide at position 13 and 2'-fluoro nucleotides at positions 11 and 12 as counted from a 3' end of the sense strand hybridizing portion.
18-8. The double-stranded RNA according to any one of 1. to 18-3., wherein the sense strand includes a 2'-O-Me nucleotide at position 11 and 2'-fluoro nucleotides at positions 12 and 13 as counted from a 3' end of the sense strand hybridizing portion.
18-9. The double-stranded RNA according to any one of 1. to 18-3., wherein the sense strand includes a 2'-O-Me nucleotide at position 12 and 2'-fluoro nucleotides at positions 11 and 13 as counted from a 3' end of the sense strand hybridizing portion.
18-10. The double-stranded RNA according to any one of 1. to 18-3., wherein the sense strand includes a 2'-O-Me nucleotide at position 13 and 2'-fluoro nucleotides at positions 11 and 12 as counted from a 3' end of the sense strand hybridizing portion.
19. The double-stranded RNA according to any one of 1. to 18-10., wherein the sense strand includes seven to eighteen 2'-O-Me nucleotides.
19-2. The double-stranded RNA according to any one of 1. to 19., wherein the sense strand includes thirteen, fourteen, fifteen, sixteen, seventeen, or eighteen 2'-O-Me nucleotides.
20. The double-stranded RNA according to any one of 1. to 19-2., wherein the antisense strand includes thirteen to twenty 2'-O-Me nucleotides.
20-2. The double-stranded RNA according to any one of 1. to 20., wherein the antisense strand includes thirteen to eighteen 2'-O-Me nucleotides.
21. The double-stranded RNA according to any one of 1. to 20-2., wherein the sense strand includes 0 to 6 deoxyribonucleotides.
21-2. The double-stranded RNA according to any one of 1. to 21., wherein the sense strand includes 0 or 1 deoxyribonucleotide.
22. The double-stranded RNA according to any one of 1. to 21-2., wherein the antisense strand includes 0 to 6 deoxyribonucleotides.
22-2. The double-stranded RNA according to any one of 1. to 22., wherein the antisense strand includes deoxyribonucleotides at one to six positions (preferably two to six, or three to five) selected from positions 2, 5, 7, 12, 14, and 16 as counted from a 5' end of the antisense strand hybridizing portion.
22-3. The double-stranded RNA according to any one of 1. to 22-2., wherein the antisense strand includes deoxyribonucleotides at one to four positions (preferably two, three or four) selected from positions 2, 5, 7, and 12 as counted from a 5' end of the antisense strand hybridizing portion.
22-4. The double-stranded RNA according to any one of 1. to 22-3., wherein the antisense strand includes a deoxyribonucleotide at one or two positions selected from positions 5 and 7 as counted from a 5' end of the antisense strand hybridizing portion.
22-5. The double-stranded RNA according to any one of 1. to 22., wherein the antisense strand does not contain a deoxyribonucleotide.
23. The double-stranded RNA according to any one of 1. to 22-5., wherein the double-stranded RNA comprises at least one phosphorothioate bond.
23-2. The double-stranded RNA according to any one of 1. to 23., wherein the bonds linking the nucleotides constituting the double-stranded RNA are each independently a phosphodiester bond or a modified phosphodiester bond (preferably a phosphorothioate bond).
24. The double-stranded RNA according to any one of 1. to 23-2., wherein the sense strand includes 1, 2, 3, or 4 phosphorothioate bonds.
25. The double-stranded RNA according to any one of 1. to 24., wherein the sense strand includes a phosphorothioate bond at least one position selected from the group consisting of between nucleotide positions 1 and 2 and between nucleotide positions 2 and 3 as counted from a 5' end of the sense strand, and between nucleotide positions 1 and 2, and between nucleotide positions 2 and 3 as counted from a 3' end.
25-2. The double-stranded RNA according to any one of 1. to 25., wherein the sense strand includes phosphorothioate bonds between nucleotide positions 1 and 2 and between nucleotide positions 2 and 3 as counted from a 5' end of the sense strand, and between nucleotide positions 1 and 2 and between nucleotide positions 2 and 3 as counted from a 3' end.
26. The double-stranded RNA according to any one of 1. to 25-2., wherein the antisense strand includes 1, 2, 3, or 4 phosphorothioate bonds.
27. The double-stranded RNA according to any one of 1. to 26., wherein the antisense strand includes phosphorothioate bonds between nucleotide positions 1 and 2 and between nucleotide positions 2 and 3 as counted from a 5' end of the antisense strand, and between nucleotide positions 1 and 2 and between nucleotide positions 2 and 3 as counted from a 3' end.
28. The double-stranded RNA according to any one of 1. to 27., wherein the antisense strand includes a 5'-phosphonate group at a 5' end of the antisense strand.
29. The double-stranded RNA according to 28., wherein the 5'-phosphonate group is a 5'-vinylphosphonate (VP) group.
30. The double-stranded RNA according to 28. or 29., wherein the antisense strand includes a 5'-vinylphosphonate (VP) modified 2'-O-Me nucleotide at position 1 as counted from a 5' end of the antisense strand.
31. The double-stranded RNA according to 28. or 29., wherein the antisense strand includes a 5'-vinylphosphonate (VP) modified 2'-O-XCE nucleotide at position 1 as counted from a 5' end of the antisense strand.
32. The double-stranded RNA according to 28., wherein the 5'-phosphonate group is a 5'-cyclopropylphosphonate (CPP) group.
33. The double-stranded RNA according to 28. or 32., wherein the antisense strand includes a 5'-cyclopropylphosphonate (CPP) modified 2'-O-Me nucleotide at position 1 as counted from a 5' end of the antisense strand.
34. The double-stranded RNA according to 28. or 32., wherein the antisense strand includes a 5'-cyclopropylphosphonate (CPP) modified 2'-O-XCE nucleotide at position 1 as counted from a 5' end of the antisense strand.
35. The double-stranded RNA according to 28., wherein the 5'-phosphonate group is a 5'-ethylphosphonate (EP) group.
36. The double-stranded RNA according to 28. or 35., wherein the antisense strand includes a 5'-ethylphosphonate (EP) modified 2'-O-Me nucleotide at position 1 as counted from a 5' end of the antisense strand.
37. The double-stranded RNA according to 28. or 35., wherein the antisense strand includes a 5'-ethylphosphonate (EP) modified 2'-O-XCE nucleotide at position 1 as counted from a 5' end of the antisense strand.
38. The double-stranded RNA according to any one of 1. to 37., wherein the sense strand and the antisense strand independently have a length of 19 to 25 (preferably, 19 to 23) nucleotides.
38-2. The double-stranded RNA according to any one of 1. to 38., wherein the sense strand has a length of 19 to 21 nucleotides, and the antisense strand has a length of 21 to 23 nucleotides.
39. The double-stranded RNA according to any one of 1. to 38-2., wherein the sense strand has a length of 21 nucleotides.
40. The double-stranded RNA according to any one of 1. to 39., wherein the antisense strand has a length of 23 nucleotides.
41. The double-stranded RNA according to any one of 1. to 40., wherein the sense strand has a length of 21 nucleotides, and the antisense strand has a length of 23 nucleotides.
42. The double-stranded RNA according to any one of 1. to 41., wherein the antisense strand includes an overhang at a 3' end of the antisense strand.
42-2. The double-stranded RNA according to 42., wherein the overhang at a 3' end of the antisense strand has a length of 2 nucleotides.
43. The double-stranded RNA according to any one of 1. to 42-2., wherein the antisense strand includes a blunt end at a 5' end of the antisense strand.
44. The double-stranded RNA according to any one of 1. to 43., further comprising a group derived from a functional molecule having at least one function selected from the group consisting of a labeling function, a purification function, and a function of delivery to a target site.
45. The double-stranded RNA according to 44., wherein the functional molecule is selected from the group consisting of a sugar, a lipid, a peptide, and a protein, and derivatives thereof.
46. The double-stranded RNA according to 44. or 45., wherein the functional molecule is selected from the group consisting of cholesterol, vitamins, steroids, C5-30 saturated fatty acids, C5-30 unsaturated fatty acids, C5-30 alkyl groups, and C5-30 alkenyl groups.
46-2. The double-stranded RNA according to 46., wherein the functional molecule is a lipid selected from the group consisting of cholesterol, a vitamin, a steroid, C5-30 saturated fatty acids, and C5-30 unsaturated fatty acids.
46-3. The double-stranded RNA according to 46., wherein the group derived from the functional molecule is a C5-30 alkyl group (preferably, a C14-24 alkyl group, a C16-22 alkyl group, and a hexadecyl group).
47. The double-stranded RNA according to 44. or 45., wherein the functional molecule is a peptide or protein selected from the group consisting of a ligand of a receptor and an antibody.
48. The double-stranded RNA according to 44. or 45., wherein the functional molecule is a sugar derivative that interacts with an asialoglycoprotein receptor.
48-2. The double-stranded RNA according to 48., wherein a group derived from the functional molecule is a ligand group represented by Formula below: 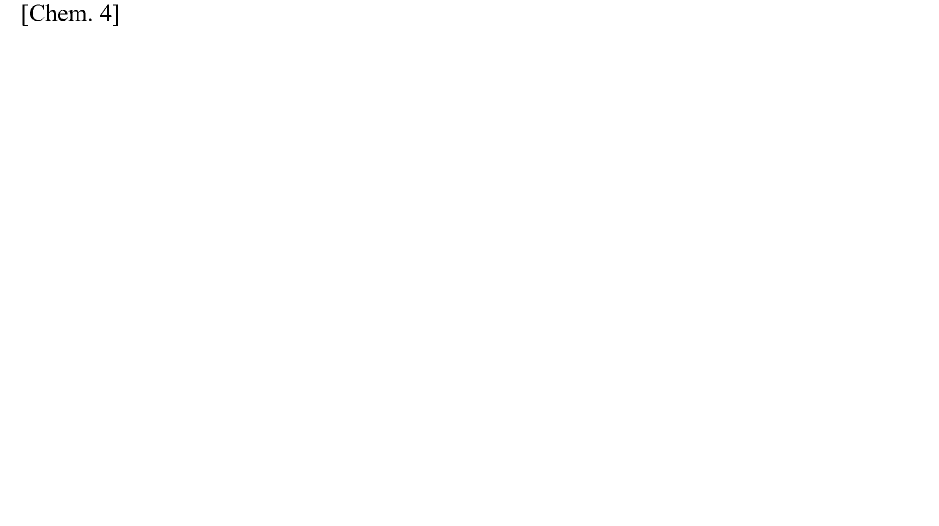 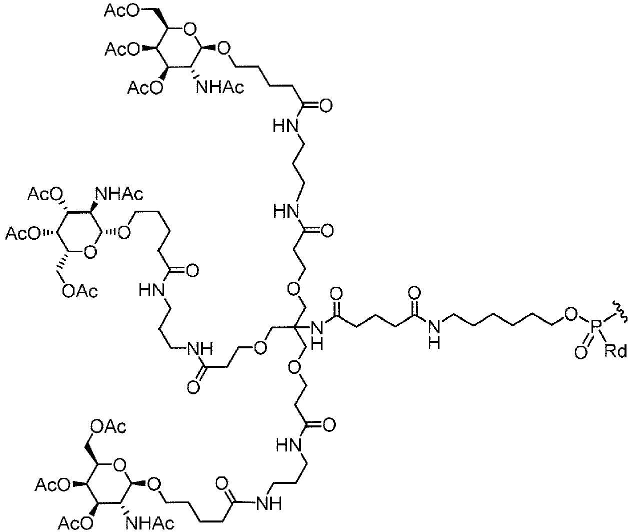 (in which, Rd is a hydroxy group or a thiol group (preferably a thiol group)).
48-3. The double-stranded RNA according to 48., wherein a group derived from the functional molecule is a ligand group represented by Formula below: 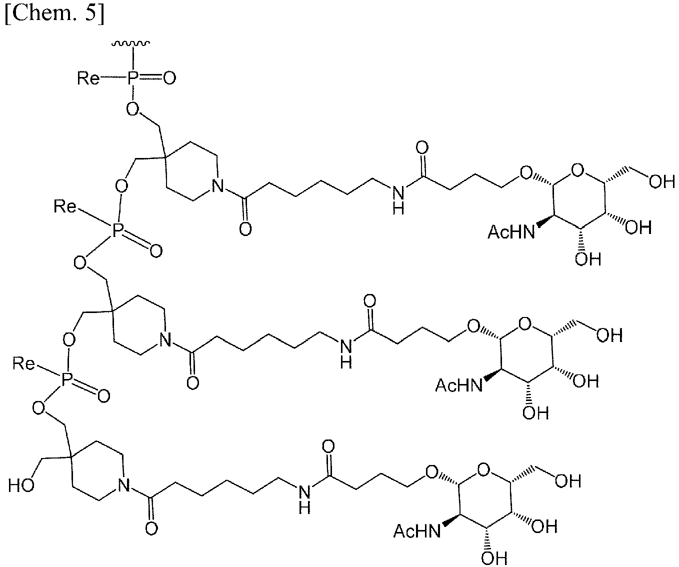 (in which, Re are each independently a hydroxy group or a thiol group (preferably a thiol group)).
48-4. The double-stranded RNA according to any one of 44. to 48-3., wherein the functional molecule is bonded to a 5' end or a 3' end of the sense strand or the antisense strand.
48-5. The double-stranded RNA according to any one of 44. to 48-3., wherein the functional molecule is bonded to a 3' end of the sense strand.
48-6. The double-stranded RNA according to any one of 44. to 48-3., wherein the functional molecule is bonded to an internal nucleotide (preferably, the oxygen atom at position 2' of the internal nucleotide) of the sense strand.
48-7. The double-stranded RNA according to any one of 48. to 48-3., wherein the functional molecule is bonded to an internal nucleotide (preferably, the oxygen atom at position 2' of the internal nucleotide) of the antisense strand.
49. A pharmaceutical composition comprising the double-stranded RNA according to any one of 1. to 48-7. and a pharmacologically acceptable carrier.
50. A method for controlling a function of a target RNA, comprising a step of bringing the double-stranded RNA according to any one of 1. to 49. into contact with a cell.
51. A method for controlling a function of a target RNA in a mammal, comprising a step of administering the pharmaceutical composition according to 49. to the mammal.
52. A method for producing the double-stranded RNA according to any one of 1. to 48-7. using a 2'-O-XCE nucleotide.
53. A compound represented by Formula (II) below, or a salt thereof: 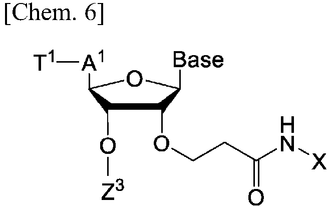
   {in which Base is a purin-9-yl group, a 2-oxo-pyrimidin-1-yl group, or a 2-thioxo-pyrimidin-1-yl group (the purin-9-yl group, the 2-oxo-pyrimidin-1-yl group, and the 2-thioxo-pyrimidin-1-yl group are each independently unsubstituted or substituted with one or more substituents solely or differently selected from the group consisting of a halogen atom, a C1-6 alkyl group, an amino group, a protected amino group, a hydroxy group, a protected hydroxy group, a sulfanyl group, and a protected sulfanyl group),
   Z³ is a hydrogen atom, a hydroxy-protecting group, or a phosphorus-containing group,
   X is a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group), or
   a group represented by Formula (IIa) below: 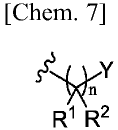
   [in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
   Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group) or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with the nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
   a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
   n is an integer of 1 to 3, and when n is 2 or 3, two or three R¹ and R² may be the same or different, respectively],
   T¹ is a group represented by Formula (IIb) below: 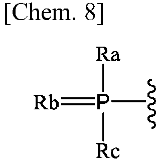
   [Ra and Rc are each independently selected from a hydroxy group, a protected hydroxy group, a thiol group, a protected thiol group, an amino group, a protected amino group, a C1-6 alkyl group, a C2-6 alkenyl group, and a C1-6 alkoxy group (the hydroxy group, the thiol group, the amino group, the C1-6 alkyl group, the C2-6 alkenyl group, and the C1-6 alkoxy group are each independently unsubstituted or substituted with one or more substituents solely or differently selected from the group consisting of a halogen atom, a C1-6 alkyl group, a C1-6 alkoxy group, and a cyano group), and
   Rb is an oxygen atom or a sulfur atom], and
   A¹ is a group selected from Formula (IIc) below: 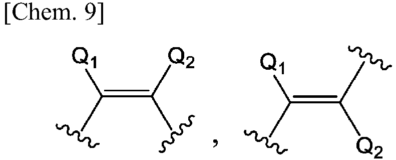
   [Q₁ and Q₂ are each independently a hydrogen atom, a halogen atom, a C1-C6 alkyl group, a C1-6 alkoxy group, a C2-6 alkenyl group, a C2-6 alkynyl group, or an amino group (the C1-C6 alkyl group, the C1-6 alkoxy group, the C2-6 alkenyl group, the C2-6 alkynyl group, and the amino group are each independently unsubstituted or substituted with one or more substituents solely or differently selected from the group consisting of a halogen atom, a C1-6 alkyl group, a C1-6 alkoxy group, and a cyano group)]}.
54. The compound according to 53., or a salt thereof, wherein the X is a methyl group, and Q₁ and Q₂ are hydrogen atoms.
55. The compound according to 53. or 54., or a salt thereof, wherein the R¹ and R² are hydrogen atoms, the Y is NR³R⁴, the R³ and R⁴ together with the nitrogen atom, to which they are bonded, form morpholine, and Q₁ and Q₂ are hydrogen atoms.
56. The compound according to any one of 53. to 55., or a salt thereof, wherein the phosphorus-containing group is a cyanoethoxy(diisopropylamino)phosphino group or a hydroxyphosphinyl group.
57. A compound represented by Formula (III) below, or a salt thereof: 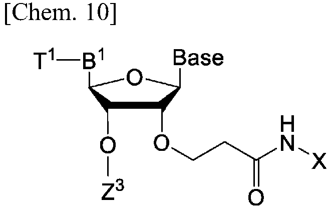
   {in which Base is a purin-9-yl group, a 2-oxo-pyrimidin-1-yl group, or a 2-thioxo-pyrimidin-1-yl group (the purin-9-yl group, the 2-oxo-pyrimidin-1-yl group, and the 2-thioxo-pyrimidin-1-yl group are each independently unsubstituted or substituted with one or more substituents solely or differently selected from the group consisting of a halogen atom, a C1-6 alkyl group, an amino group, a protected amino group, a hydroxy group, a protected hydroxy group, a sulfanyl group, and a protected sulfanyl group),
   Z³ is a hydrogen atom, a hydroxy-protecting group, or a phosphorus-containing group,
   X is a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group), or
   a group represented by Formula (IIIa) below: 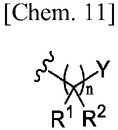
   [in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
   Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group) or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with the nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
   a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
   n is an integer of 1 to 3, and when n is 2 or 3, two or three R¹ and R² may be the same or different, respectively],
   T¹ is a group represented by Formula (IIIb) below: 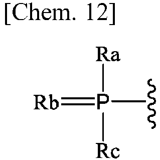
   [Ra and Rc are each independently selected from a hydroxy group, a protected hydroxy group, a thiol group, a protected thiol group, an amino group, a protected amino group, a C1-6 alkyl group, a C2-6 alkenyl group, and a C1-6 alkoxy group (the hydroxy group, the thiol group, the amino group, the C1-6 alkyl group, the C2-6 alkenyl group, and the C1-6 alkoxy group are each independently unsubstituted or substituted with one or more substituents solely or differently selected from the group consisting of a halogen atom, a C1-6 alkyl group, a C1-6 alkoxy group, and a cyano group), and
   Rb is an oxygen atom or a sulfur atom], and
   B¹ is a group represented by Formula (IIIc) below: 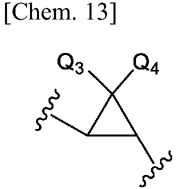
   [Q₃ and Q₄ are each independently a hydrogen atom, a halogen atom, a C1-C6 alkyl group, a C1-6 alkoxy group, a C2-6 alkenyl group, a C2-6 alkynyl group, or an amino group (the C1-C6 alkyl group, the C1-6 alkoxy group, the C2-6 alkenyl group, the C2-6 alkynyl group, and the amino group are each independently unsubstituted or substituted with one or more substituents solely or differently selected from the group consisting of a halogen atom, a C1-6 alkyl group, a C1-6 alkoxy group, and a cyano group)]}.
58. The compound according to 57., or a salt thereof, wherein the X is a methyl group, and Q₃ and Q₄ are hydrogen atoms.
59. The compound according to 57. or 58., or a salt thereof, wherein the R¹ and R² are hydrogen atoms, the Y is NR³R⁴, the R³ and R⁴ together with the nitrogen atom, to which they are bonded, form morpholine, and Q₃ and Q₄ are hydrogen atoms.
60. The compound according to any one of 57. to 59., or a salt thereof, wherein the phosphorus-containing group is a cyanoethoxy(diisopropylamino)phosphino group or a hydroxyphosphinyl group.
61. A compound represented by Formula (IV) below, or a salt thereof: 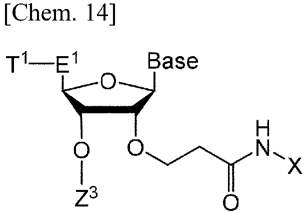
   {in which Base is a purin-9-yl group, a 2-oxo-pyrimidin-1-yl group, or a 2-thioxo-pyrimidin-1-yl group (the purin-9-yl group, the 2-oxo-pyrimidin-1-yl group, and the 2-thioxo-pyrimidin-1-yl group are each independently unsubstituted or substituted with one or more substituents solely or differently selected from the group consisting of a halogen atom, a C1-6 alkyl group, an amino group, a protected amino group, a hydroxy group, a protected hydroxy group, a sulfanyl group, and a protected sulfanyl group),
   Z³ is a hydrogen atom, a hydroxy-protecting group, or a phosphorus-containing group,
   X is a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group), or
   Formula (IVa) below: 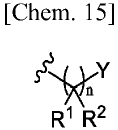
   [in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
   Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group) or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with the nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
   a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
   n is an integer of 1 to 3, and when n is 2 or 3, two or three R¹ and R² may be the same or different, respectively],
   T¹ is a group represented by Formula (IVb) below: 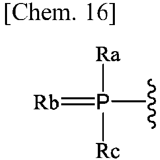
   [Ra and Rc are each independently selected from a hydroxy group, a protected hydroxy group, a thiol group, a protected thiol group, an amino group, a protected amino group, a C1-6 alkyl group, a C2-6 alkenyl group, and a C1-6 alkoxy group (the hydroxy group, the thiol group, the amino group, the C1-6 alkyl group, the C2-6 alkenyl group, and the C1-6 alkoxy group are each independently unsubstituted or substituted with one or more substituents solely or differently selected from the group consisting of a halogen atom, a C1-6 alkyl group, a C1-6 alkoxy group, and a cyano group), and
   Rb is an oxygen atom or a sulfur atom], and
   E¹ is a group represented by Formula (IVc) below: 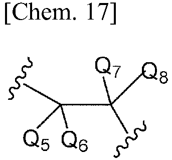
   [Q₅ to Q₈ are each independently a hydrogen atom, a halogen atom, a C1-C6 alkyl group, a C1-6 alkoxy group, a C2-6 alkenyl group, a C2-6 alkynyl group, or an amino group (the C1-C6 alkyl group, the C1-6 alkoxy group, the C2-6 alkenyl group, the C2-6 alkynyl group, and the amino group are each independently unsubstituted or substituted with one or more substituents solely or differently selected from the group consisting of a halogen atom, a C1-6 alkyl group, a C1-6 alkoxy group, and a cyano group)]}.
62. The compound according to 61., or a salt thereof, wherein the X is a methyl group, and Q₅ to Q₈ are hydrogen atoms.
63. The compound according to 61. or 62., or a salt thereof, wherein the R¹ and R² are hydrogen atoms, the Y is NR³R⁴, the R³ and R⁴ together with the nitrogen atom, to which they are bonded, form morpholine, and Q₅ to Q₈ are hydrogen atoms.
64. The compound according to any one of 61. to 63., or a salt thereof, wherein the phosphorus-containing group is a cyanoethoxy(diisopropylamino)phosphino group or a hydroxyphosphinyl group.

### Advantageous Effects of Invention

The present invention provides a chemically modified siRNA in which metabolic stability is further enhanced while maintaining activity, or in which activity and persistence are improved.

The double-stranded RNA of the present invention can effectively control the expression of a target RNA, and is useful as a nucleic acid drug.

### Brief Description of Drawings

Fig. 1 shows an ORTEP diagram of a single-crystal X-ray analysis of isomer A of Compound 13.
Fig. 2 shows expression levels of a rat Sod1 gene for each siRNA (si-0001 to si-0014) measured by quantitative real-time PCR in Evaluation Example 1.
Fig. 3 shows expression levels of the rat Sod1 gene for each siRNA (si-0001 and si-0015 to si-0027) measured by quantitative real-time PCR in Evaluation Example 1.
Fig. 4 shows expression levels of the rat Sod1 gene for each siRNA (si-0001, and si-0028 to si-0040) measured by quantitative real-time PCR in Evaluation Example 1.
Fig. 5 shows expression levels of the rat Sod1 gene for each siRNA (si-0001, and si-0041 to si-0053) measured by quantitative real-time PCR in Evaluation Example 1.
Fig. 6 shows expression levels of the rat Sod1 gene for each siRNA (si-0001, and si-0054 to si-0066) measured by quantitative real-time PCR in Evaluation Example 1.
Fig. 7 shows expression levels of the rat Sod1 gene for each siRNA (si-0067 to si-0080) measured by quantitative real-time PCR in Evaluation Example 1.
Fig. 8 shows expression levels of the rat Sod1 gene for each siRNA (si-0067, and si-0081 to si-0093) measured by quantitative real-time PCR in Evaluation Example 1.
Fig. 9 shows expression levels of the rat Sod1 gene for each siRNA (si-0067, and si-0094 to si-0106) measured by quantitative real-time PCR in Evaluation Example 1.
Fig. 10 shows expression levels of the rat Sod1 gene for each siRNA (si-0067, and si-0107 to si-0119) measured by quantitative real-time PCR in Evaluation Example 1.
Fig. 11 shows expression levels of the rat Sod1 gene for each siRNA (si-0067, and si-0120 to si-0132) measured by quantitative real-time PCR in Evaluation Example 1.
Fig. 12 shows expression levels of the rat Sod1 gene for each siRNA (si-0001, and si-0133 to si-0138) measured by quantitative real-time PCR in Evaluation Example 1.
Fig. 13 shows expression levels of the rat Sod1 gene for each siRNA (si-0001, and si-0139 to si-0145) measured by quantitative real-time PCR in Evaluation Example 1.
Fig. 14 shows expression levels of the rat Sod1 gene for each siRNA (si-0001, and si-0146 to si-0157) measured by quantitative real-time PCR in Evaluation Example 2.
Fig. 15 shows expression levels of the rat Sod1 gene for each siRNA (si-0001, and si-0158 to si-0161) measured by quantitative real-time PCR in Evaluation Example 2.
Fig. 16 shows expression levels of the rat Sod1 gene for each siRNA (si-0001, and si-0162 to si-0169) measured by quantitative real-time PCR in Evaluation Example 2.
Fig. 17 shows expression levels of the rat Sod1 gene for each siRNA (si-0001, and si-0170 to si-0182) measured by quantitative real-time PCR in Evaluation Example 2.
Fig. 18 shows expression levels of the rat Sod1 gene for each siRNA (si-0001, and si-0183 to si-0190) measured by quantitative real-time PCR in Evaluation Example 2.
Fig. 19 shows expression levels of the rat Sod1 gene for each siRNA (si-0001, and si-0191 to si-0197) measured by quantitative real-time PCR in Evaluation Example 2.
Fig. 20 shows expression levels of the rat Sod1 gene for each siRNA (si-0001, and si-0198 to si-0208) measured by quantitative real-time PCR in Evaluation Example 2.
Fig. 21 shows expression levels of the rat Sod1 gene for each siRNA (si-0001, and si-0209 to si-0211) measured by quantitative real-time PCR in Evaluation Example 3.
Fig. 22 shows expression levels of the rat Sod1 gene for each siRNA (si-0001, and si-0212 to si-0223) measured by quantitative real-time PCR in Evaluation Example 3.
Fig. 23 shows expression levels of a mouse Sod1 gene in the lung for each siRNA (si-0224 to si-0227) measured by quantitative real-time PCR in Evaluation Example 6.
Fig. 24 shows expression levels of the mouse Sod1 gene in the lung for each siRNA (si-0224 to si-0225) measured by quantitative real-time PCR in Evaluation Example 7.
Fig. 25 shows expression levels of the mouse Sod1 gene in the cerebrum for each siRNA (si-0224 to si-0226, and si-0228) measured by quantitative real-time PCR in Evaluation Example 8.
Fig. 26 shows expression levels of the mouse Sod1 gene in the hippocampus for each siRNA (si-0224 to si-0226, and si-0228) measured by quantitative real-time PCR in Evaluation Example 8.
Fig. 27 shows expression levels of the mouse Sod1 gene in the cerebellum for each siRNA (si-0224 to si-0226, and si-0228) measured by quantitative real-time PCR in Evaluation Example 8.
Fig. 28 shows expression levels of the mouse Sod1 gene in the medulla oblongata for each siRNA (si-0224 to si-0226, and si-0228) measured by quantitative real-time PCR in Evaluation Example 8.
Fig. 29 shows expression levels of a mouse Ttr gene for each siRNA (si-0229 to si-0241) measured by quantitative real-time PCR in Evaluation Example 9.
Fig. 30 shows expression levels of the mouse Ttr gene for each siRNA (si-0229, and si-0242 to si-0250) measured by quantitative real-time PCR in Evaluation Example 9.
Fig. 31 shows expression levels of the mouse Ttr gene for each siRNA (si-0229, and si-0251 to si-0259) measured by quantitative real-time PCR in Evaluation Example 9.
Fig. 32 shows expression levels of a mouse FXII gene for each siRNA (si-0260 to si-0270) measured by quantitative real-time PCR in Evaluation Example 10.
Fig. 33 shows expression levels of the mouse FXII gene for each siRNA (si-0260, and si-0271 to si-0281) measured by quantitative real-time PCR in Evaluation Example 10.
Fig. 34 shows expression levels of the mouse FXII gene for each siRNA (si-0260, and si-0282 to si-0289) measured by quantitative real-time PCR in Evaluation Example 10.
Fig. 35 shows expression level of TTR in the mouse serum of each siRNA (si-0290 to si-0297) measured by ELISA in Evaluation Example 11.
Fig. 36 shows expression levels of the mouse Ttr gene for each siRNA (si-0298 to si-0305) measured by quantitative real-time PCR in Evaluation Example 12.
Fig. 37 shows expression levels of the mouse Ttr gene for each siRNA (si-0306 to si-0312) measured by quantitative real-time PCR in Evaluation Example 12.
Fig. 38 shows expression levels of the mouse Ttr gene for each siRNA (si-0313 to si-0321) measured by quantitative real-time PCR in Evaluation Example 13.
Fig. 39 shows expression levels of the mouse Ttr gene for each siRNA (si-0322 to si-0329) measured by quantitative real-time PCR in Evaluation Example 13.
Fig. 40 shows expression levels of the mouse Ttr gene for each siRNA (si-0330 to si-0343) measured by quantitative real-time PCR in Evaluation Example 14.
Fig. 41 shows expression levels of the mouse Ttr gene for each siRNA (si-0298, and si-0344 to si-0349) measured by quantitative real-time PCR in Evaluation Example 15.
Fig. 42 shows expression level of TTR in the mouse serum of each siRNA (si-0350 to si-0351) measured by ELISA in Evaluation Example 16.
Fig. 43 shows expression levels of the rat Sod1 gene for each siRNA (si-0209, si-0216, and si-0352 to si-0362) measured by quantitative real-time PCR in Evaluation Example 17.
Fig. 44 shows expression levels of the rat Sod1 gene for each siRNA (si-0209, si-0216, and si-0363 to si-0373) measured by quantitative real-time PCR in Evaluation Example 17.
Fig. 45 shows expression levels of the rat Sod1 gene for each siRNA (si-0209, si-0216, and si-0374 to si-0383) measured by quantitative real-time PCR in Evaluation Example 18.
Fig. 46 shows expression levels of the rat Sod1 gene for each siRNA (si-0209, si-0216, and si-0384 to si-0394) measured by quantitative real-time PCR in Evaluation Example 18.
Fig. 47 shows expression levels of the mouse Ttr gene for each siRNA (si-0251, si-0253, and si-0395 to si-0403) measured by quantitative real-time PCR in Evaluation Example 19.
Fig. 48 shows expression levels of the mouse Ttr gene for each siRNA (si-0251, si-0253, and si-0404 to si-0416) measured by quantitative real-time PCR in Evaluation Example 19.
Fig. 49 shows expression levels of the mouse Ttr gene for each siRNA (si-0251, and si-0411 to si-0412) measured by quantitative real-time PCR in Evaluation Example 19.
Fig. 50 shows expression levels of the mouse FXII gene for each siRNA (si-0282, and si-0417 to si-0419) measured by quantitative real-time PCR in Evaluation Example 20.
Fig. 51 shows expression levels of the mouse FXII gene for each siRNA (si-0282, si-0284, and si-0420 to si-0425) measured by quantitative real-time PCR in Evaluation Example 20.
Fig. 52 shows expression levels of the mouse Ttr gene for each siRNA (si-0298, si-0347, and si-0426 to si-0429) measured by quantitative real-time PCR in Evaluation Example 21.
Fig. 53 shows expression levels of the mouse Ttr gene for each siRNA (si-0298, and si-0430 to si-0436) measured by quantitative real-time PCR in Evaluation Example 21.
Fig. 54 shows expression levels of the mouse Ttr gene for each siRNA (si-0251, and si-0437 to si-0448) measured by quantitative real-time PCR in Evaluation Example 22.
Fig. 55 shows expression levels of the mouse Ttr gene for each siRNA (si-0251, and si-0449 to si-0453) measured by quantitative real-time PCR in Evaluation Example 23.
Fig. 56 shows expression levels of the mouse Ttr gene for each siRNA (si-0251, and si-0454 to si-0460) measured by quantitative real-time PCR in Evaluation Example 24.
Fig. 57 shows expression levels of the mouse Ttr gene for each siRNA (si-0251, and si-0461 to si-0467) measured by quantitative real-time PCR in Evaluation Example 25.
Fig. 58 shows expression levels of the mouse Ttr gene for each siRNA (si-0298, and si-0468 to si-0470) measured by quantitative real-time PCR in Evaluation Example 26.
Fig. 59 shows expression levels of the rat Sod1 gene for each siRNA (si-0209, si-0216, si-0220, and si-0471 to si-0480) measured by quantitative real-time PCR in Evaluation Example 27.
Fig. 60 shows expression levels of the rat Sod1 gene for each siRNA (si-0209, and si-0481 to si-0486) measured by quantitative real-time PCR in Evaluation Example 29.
Fig. 61 shows expression level of TTR in the mouse serum for each siRNA (si-0488, si-00489, and si-0497 to si-0499) measured by ELISA in Evaluation Example 30.
Fig. 62 shows expression level of TTR in the mouse serum for each siRNA (si-0488, si-00489, si-0493 to si-0495, and si-0500) measured by ELISA in Evaluation Example 30.
Fig. 63 shows expression level of TTR in the mouse serum for each siRNA (si-0488, si-00489, si-0490 to si-0492, and si-0496) measured by ELISA in Evaluation Example 30.
Fig. 64 shows nuclease resistance of oligonucleotides measured in Evaluation Example 31.
Fig. 65 shows nuclease resistance of oligonucleotides measured in Evaluation Example 32.

### Description of Embodiments

Terms used in the present specification are used in the meanings commonly used in the art unless otherwise specified. Hereinafter, each term used in the present specification will be described. Note that, in the present specification, in both a case where each term is used alone and a case where each term is used together with another term, each term has the same meaning unless otherwise specified. It is understood that both the above summary and the following detailed description are exemplary and explanatory only and do not limit the present invention as claimed. In the present specification, the use of a singular form shall include a plural form unless otherwise specifically stated.

"n-" means normal, "i-" means iso, "s-" means secondary, "t-" means tertiary, "o-" means ortho, "m-" means meta, and "p-" means para. "Ph" means phenyl, "Me" means methyl, "Bu" means butyl, "Ac" means acetyl, and "DMTr" means dimethoxytrityl.

"Halogen atom" means a fluorine atom, a chlorine atom, a bromine atom, or an iodine atom.

"C1-6 alkyl group" means a monovalent group of a linear or branched saturated aliphatic hydrocarbon having 1 to 6 carbon atoms. Examples of the C1-6 alkyl group include a methyl group, an ethyl group, an n-propyl group, an isopropyl group, an n-butyl group, an isobutyl group, an s-butyl group, a t-butyl group, an n-pentyl group, an isopentyl group, a neopentyl group, an n-hexyl group, an isohexyl group, and the like. Similarly, "C1-4 alkyl group" means a monovalent group of a linear or branched saturated aliphatic hydrocarbon having 1 to 4 carbon atoms. Similarly, "C1-3 alkyl group" means a monovalent group of a linear or branched saturated aliphatic hydrocarbon having 1 to 3 carbon atoms. Similarly, "C5-30 alkyl group" means a monovalent group of a linear or branched saturated aliphatic hydrocarbon having 5 to 30 carbon atoms. Examples of the C5-30 alkyl group include an n-pentyl group, an n-hexyl group, an isohexyl group, an n-heptyl group, a decyl group, a tetradecyl group, a hexadecyl group, an octadecyl group, an icosyl group, a docosyl group, a tetracosyl group, a triacontyl group, and the like. Similarly, "C14-24 alkyl group" means a monovalent group of a linear or branched saturated aliphatic hydrocarbon having 14 to 24 carbon atoms. Similarly, "C16-22 alkyl group" means a monovalent group of a linear or branched saturated aliphatic hydrocarbon having 16 to 22 carbon atoms.

"Halo C1-6 alkyl group" means a group in which at least one hydrogen atom at any position of the "C1-6 alkyl group" is substituted with the "halogen atom".

"C2-6 alkenyl group" means a monovalent group of a linear or branched unsaturated aliphatic hydrocarbon having 2 to 6 carbon atoms and including at least one carbon-carbon double bond. Examples of the C2-6 alkenyl group include a vinyl group, an allyl group, a propenyl group, an isopropenyl group, a butenyl group, an isobutenyl group, a butadienyl group, a 3-methyl-2-butenyl group, a pentenyl group, an isopentenyl group, a pentadienyl group, a hexenyl group, an isohexenyl group, a hexadienyl group, and the like. Similarly, "C5-30 alkenyl group" means a monovalent group of a linear or branched unsaturated aliphatic hydrocarbon having 5 to 30 carbon atoms and including at least one carbon-carbon double bond. Examples of the C5-30 alkenyl group include a pentenyl group, an isopentenyl group, a pentadienyl group, a hexenyl group, an isohexenyl group, a hexadienyl group, a decenyl group, a tetradecenyl group, a hexadecenyl group, an octadecenyl group, an icosenyl group, a docosenyl group, a tetracosenyl group, a triacontenyl group, and the like.

"C1-6 alkoxy group" means a group in which the "C1-6 alkyl group" is bonded to an oxy (-O-) group. Examples of the C1-6 alkoxy group include a methoxy group, an ethoxy group, an n-propoxy group, an isopropoxy group, an n-butoxy group, a t-butoxy group, an isobutoxy group, an s-butoxy group, an n-pentyloxy group, an isopentyloxy group, an n-hexyloxy group, and the like.

"C2-6 alkenyloxy group" means a group in which the "C2-6 alkenyl group" is bonded to an oxy (-O-) group. Examples of the C2-6 alkenyloxy group include a vinyloxy group, an allyloxy group, a propenyloxy group, an isopropenyloxy group, a butenyloxy group, an isobutenyloxy group, a butadienyloxy group, a 3-methyl-2-butenyloxy group, a pentenyloxy group, an isopentenyloxy group, a pentadienyloxy group, a hexenyloxy group, an isohexenyloxy group, a hexadienyloxy group, and the like.

"C1-6 alkylcarbonyl group" means a group in which the "C1-6 alkyl group" is bonded to a carbonyl (-C(=O)-) group.

"C1-6 alkoxycarbonyl group" means a group in which the "C1-6 alkoxy group" is bonded to a carbonyl (-C(=O)-) group, and "C2-6 alkenyloxycarbonyl group" means a group in which the "C2-6 alkenyloxy group" is bonded to a carbonyl (-C(=O)-) group.

"C2-20 alkylene group" means a divalent group of a linear or branched saturated aliphatic hydrocarbon having 2 to 20 carbon atoms.

"C8-12 alkylene group" means, of the "C2-20 alkylene group", a divalent group of a linear or branched saturated aliphatic hydrocarbon having 8 to 12 carbon atoms.

"C2-6 alkylene group" means, of the "C2-20 alkylene group", a divalent group of a linear or branched saturated aliphatic hydrocarbon having 2 to 6 carbon atoms, and examples thereof include an ethylene (ethanediyl) group, a propylene group, a propane-1,3-diyl (trimethylene) group, a propane-2,2-diyl (isopropylidene) group, a 2,2-dimethyl-propane-1,3-diyl group, a hexane-1,6-diyl (hexamethylene) group, a 3-methylbutane-1,2-diyl group, and the like.

"C2-20 alkenylene group" means a divalent group of a linear or branched unsaturated aliphatic hydrocarbon having 2 to 20 carbon atoms and including at least one carbon-carbon double bond.

"C1-6 alkylamino group" includes a mono-C1-6 alkylamino group and a di-C1-6 alkylamino group. "Mono-C1-6 alkylamino group" means a group in which one "C1-6 alkyl group" is bonded to an amino group. Examples of the mono-C1-6 alkylamino group include a methylamino group, an ethylamino group, an n-propylamino group, an isopropylamino group, an n-butylamino group, an n-pentylamino group, an n-hexylamino group, and the like. "Di-C1-6 alkylamino group" means a group in which two "C1-6 alkyl groups" are bonded to an amino group. The two alkyl groups may be the same or different. Examples of the di-C1-6 alkylamino group include a dimethylamino group, a diethylamino group, an N,N-diisopropylamino group, an N-methyl-N-ethylamino group, an N-isopropyl-N-methylamino group, an N-n-butyl-N-methylamino group, an N-tert-butyl-N-methylamino group, an N-methyl-N-n-pentylamino group, an N-n-hexyl-N-methylamino group, an N-isopropyl-N-ethylamino group, and the like.

"C1-6 alkylaminocarbonyl group" means a group in which the "C1-6 alkylamino group" is bonded to a carbonyl group.

"C1-6 alkylcarbonyloxy group" means a group in which the "C1-6 alkylcarbonyl group" is bonded to an oxy group.

"C1-6 alkylcarbonylamino group" means a group in which one "C1-6 alkylcarbonyl group" is bonded to an amino group.

"C1-6 alkoxycarbonylamino group" means a group in which one "C1-6 alkoxycarbonyl group" is bonded to an amino group.

"Aryl group" means a monovalent group obtained by removing one hydrogen atom at any position from a monocyclic or bicyclic aromatic hydrocarbon in which atoms constituting the ring are all carbon atoms, and specific examples thereof include a "C6-10 aryl group" such as a phenyl group or a naphthyl group.

"Aralkyl group" means a monovalent group in which a hydrogen atom at any position of the "C1-6 alkyl group" is replaced with the "C6-10 aryl group". "C7-10 aralkyl group" means a monovalent group in which a hydrogen atom at any position of the "C1-4 alkyl group" is replaced with a phenyl group.

"3- to 11-membered nitrogen-containing non-aromatic heterocyclic ring" means a monocyclic, fused polycyclic (in the fused polycyclic system, a non-aromatic ring may be fused to a non-aromatic ring or an aromatic ring), bridged cyclic, or spirocyclic non-aromatic heterocyclic compound containing at least one nitrogen atom and having 3 to 11 atoms constituting the ring, and examples thereof include azetidine, pyrrolidine, 2-oxopyrrolidine, piperidine, 3-oxopiperidine, piperazine, morpholine, thiomorpholine, homomorpholine, homopiperazine, and the like.

"C2-9 aromatic heterocyclic group" means a monovalent group obtained by removing one hydrogen atom at any position from an aromatic monocyclic or fused ring compound having one or more identical or different heteroatoms optionally selected from an oxygen atom, a sulfur atom, and a nitrogen atom in the ring and having 2 to 9 carbon atoms constituting the ring. Examples of the C2-9 aromatic heterocycle include a purinyl group, a pyrimidinyl group, a thienyl group, a furyl group, an isobenzofuranyl group, a pyrrolyl group, an imidazolyl group, a pyrazolyl group, a thiazolyl group, an isothiazolyl group, an isoxazolyl group, an oxadiazolyl group, a pyridyl group, a pyridazinyl group, a pyrazinyl group, an indolizinyl group, an indolyl group, an isoindolyl group, an isoquinolyl group, a quinolyl group, a naphthyridinyl group, a quinoxalinyl group, a quinazolinyl group, a pteridinyl group, a benzofuranyl group, a benzothienyl group, a benzimidazolyl group, and the like.

"5- to 10-membered heterocyclic group" means a monovalent group obtained by removing one hydrogen atom at any position from a monocyclic or fused polycyclic aromatic heterocyclic compound having 5 to 10 atoms constituting the ring and containing 1 to 5 heteroatoms (the heteroatom means a nitrogen atom, an oxygen atom, or a sulfur atom, and two or more heteroatoms may be the same or different) in the atoms constituting the ring.

Examples of the monocyclic "5- to 10-membered heterocyclic group" include a 2-thienyl group, a 3-thienyl group, a 2-furyl group, a 3-furyl group, a 2-pyranyl group, a 3-pyranyl group, a 4-pyranyl group, a 1-pyrrolyl group, a 2-pyrrolyl group, a 3-pyrrolyl group, a 1-imidazolyl group, a 2-imidazolyl group, a 4-imidazolyl group, a 1-pyrazolyl group, a 3-pyrazolyl group, a 4-pyrazolyl group, a 5-pyrazolyl group, a 2-thiazolyl group, a 4-thiazolyl group, a 5-thiazolyl group, a 3-isothiazolyl group, a 4-isothiazolyl group, a 5-isothiazolyl group, a 1,2,4-triazol-1-yl group, a 1,2,4-triazol-3-yl group, a 1,2,4-triazol-5-yl group, a 1,2,3-triazol-1-yl group, a 1,2,3-triazol-4-yl group, a 1,2,3-triazol-5-yl group, a 2-oxazolyl group, a 4-oxazolyl group, a 5-oxazolyl group, a 3-isoxazolyl group, a 4-isoxazolyl group, a 5-isoxazolyl group, a 2-pyridyl group, a 3-pyridyl group, a 4-pyridyl group, a 2-pyrazinyl group, a 2-pyrimidinyl group, a 4-pyrimidinyl group, a 5-pyrimidinyl group, a 3-pyridazinyl group, a 4-pyridazinyl group, a 1,3,4-oxadiazol-2-yl group, a 1,3,4-thiadiazol-2-yl group, a 1,2,4-oxadiazol-3-yl group, a 1,2,4-oxadiazol-5-yl group, a 1,2,4-thiadiazol-3-yl group, a 1,2,4-thiadiazol-5-yl group, a 1,2,5-oxadiazol-3-yl group, a 1,2,5-thiadiazol-3-yl group, and the like.

Examples of the fused polycyclic "5- to 10-membered heterocyclic group" include a 2-benzofuranyl group, a 3-benzofuranyl group, a 4-benzofuranyl group, a 5-benzofuranyl group, a 6-benzofuranyl group, a 7-benzofuranyl group, a 1-isobenzofuranyl group, a 4-isobenzofuranyl group, a 5-isobenzofuranyl group, a 2-benzothienyl group, a 3-benzothienyl group, a 4-benzothienyl group, a 5-benzothienyl group, a 6-benzothienyl group, a 7-benzothienyl group, a 1-isobenzothienyl group, a 4-isobenzothienyl group, a 5-isobenzothienyl group, a 2-benzothiazolyl group, a 4-benzothiazolyl group, a 5-benzothiazolyl group, a 6-benzothiazolyl group, a 7-benzothiazolyl group, a 2-chromenyl group, a 3-chromenyl group, a 4-chromenyl group, a 5-chromenyl group, a 6-chromenyl group, a 7-chromenyl group, a 8-chromenyl group, a 1-indolizinyl group, a 2-indolizinyl group, a 3-indolizinyl group, a 5-indolizinyl group, a 6-indolizinyl group, a 7-indolizinyl group, a 8-indolizinyl group, a 1-isoindolyl group, a 2-isoindolyl group, a 4-isoindolyl group, a 5-isoindolyl group, a 1-indolyl group, a 2-indolyl group, a 3-indolyl group, a 4-indolyl group, a 5-indolyl group, a 6-indolyl group, a 7-indolyl group, a 1-indazolyl group, a 2-indazolyl group, a 4-indazolyl group, a 5-indazolyl group, a 6-indazolyl group, a 7-indazolyl group, a 2-purinyl group, a 6-purinyl group, a 7-purinyl group, a 8-purinyl group, a 2-quinolyl group, a 3-quinolyl group, a 4-quinolyl group, a 5-quinolyl group, a 6-quinolyl group, a 7-quinolyl group, a 8-quinolyl group, a 1-isoquinolyl group, a 3-isoquinolyl group, a 4-isoquinolyl group, a 5-isoquinolyl group, a 6-isoquinolyl group, a 7-isoquinolyl group, a 8-isoquinolyl group, a 1-phthalazinyl group, a 5-phthalazinyl group, a 6-phthalazinyl group, a 2,7-naphthyridin-1-yl group, a 2,7-naphthyridin-3-yl group, a 2,7-naphthyridin-4-yl group, a 2,6-naphthyridin-1-yl group, a 2,6-naphthyridin-3-yl group, a 2,6-naphthyridin-4-yl group, a 1,8-naphthyridin-2-yl group, a 1,8-naphthyridin-3-yl group, a 1,8-naphthyridin-4-yl group, a 1,7-naphthyridin-2-yl group, a 1,7-naphthyridin-3-yl group, a 1,7-naphthyridin-4-yl group, a 1,7-naphthyridin-5-yl group, a 1,7-naphthyridin-6-yl group, a 1,7-naphthyridin-8-yl group, a 1,6-naphthyridin-2-yl group, a 1,6-naphthyridin-3-yl group, a 1,6-naphthyridin-4-yl group, a 1,6-naphthyridin-5-yl group, a 1,6-naphthyridin-7-yl group, a 1,6-naphthyridin-8-yl group, a 1,5-naphthyridin-2-yl group, a 1,5-naphthyridin-3-yl group, a 1,5-naphthyridin-4-yl group, a 2-quinoxalinyl group, a 5-quinoxalinyl group, a 6-quinoxalinyl group, a 2-quinazolinyl group, a 4-quinazolinyl group, a 5-quinazolinyl group, a 6-quinazolinyl group, a 7-quinazolinyl group, a 8-quinazolinyl group, a 3-cinnolinyl group, a 4-cinnolinyl group, a 5-cinnolinyl group, a 6-cinnolinyl group, a 7-cinnolinyl group, a 8-cinnolinyl group, a 2-pteridinyl group, a 4-pteridinyl group, a 6-pteridinyl group, a 7-pteridinyl group, and the like.

"Oxo" refers to a group (=O) obtained by substitution with an oxygen atom via a double bond. When a carbon atom is substituted with oxo, the oxo forms carbonyl together with the carbon atom.

"Thioxo" refers to a group (=S) obtained by substitution with a sulfur atom via a double bond. When a carbon atom is substituted with thioxo, the thioxo forms thiocarbonyl together with the carbon atom.

"Protected hydroxy group", "protected amino group", and "protected sulfanyl group" mean a hydroxy group protected with a hydroxy protecting group, an amino group protected with an amino protecting group, and a sulfanyl group protected with a sulfanyl protecting group, respectively, and the hydroxy protecting group, the amino protecting group, and the sulfanyl protecting group are not particularly limited as long as they are stable in synthesizing an oligonucleotide, and examples thereof include protecting groups described in Protective Groups in Organic Synthesis, 4th edition, written by T. W. Greene, P. G. M. Wuts, John Wiley & Sons Inc. (2006), and the like, which are well known to a person skilled in the art.

Examples of the "hydroxy protecting group" include an ether-based protecting group such as C1-6 alkyl (examples thereof include methyl, ethyl, t-butyl, and the like) or triarylmethyl (examples thereof include triphenylmethyl (trityl), monomethoxytrityl, dimethoxytrityl (DMTr), trimethoxytrityl, and the like); an acetal-based protecting group such as methoxymethyl, methylthiomethyl, methoxyethyl, benzyloxymethyl, 2-tetrahydropyranyl, or ethoxyethyl; an acyl-based protecting group such as acyl (examples thereof include formyl, acetyl, pivaloyl, benzoyl, and the like); a silyl-based protecting group such as tri(C1-6 alkyl)silyl (examples thereof include trimethylsilyl, triethylsilyl, triisopropylsilyl, t-butyldimethylsilyl, dimethylisopropylsilyl, and the like), (C1-6 alkyl)diarylsilyl (examples thereof include t-butyldiphenylsilyl, diphenylmethylsilyl, and the like), triarylsilyl (examples thereof include triphenylsilyl, and the like), tribenzylsilyl, or [(triisopropylsilyl)oxy]methyl (Tom group); 1-(4-chlorophenyl)-4-ethoxypiperidin-4-yl (Cpep group), 9-phenylxanthen-9-yl (Pixyl group), 9-(p-methoxyphenyl)xanthen-9-yl (MOX group), acyloxymethyl (examples thereof include methyl substituted with a C1-6 alkylcarbonyloxy group, specifically pivaloyloxymethyl, acetyloxymethyl, and the like), and the like.

Examples of the "amino protecting group" include an amide-based protecting group such as acyl (examples thereof include formyl, acetyl, propionyl, pivaloyl (Pv), tigloyl, and the like), haloacyl (examples thereof include fluoroacetyl, difluoroacetyl, trifluoroacetyl, chloroacetyl, dichloroacetyl, trichloroacetyl, and the like), or arylcarbonyl (examples thereof include benzoyl, p-bromobenzoyl, p-nitrobenzoyl, 2,4-dinitrobenzoyl, and the like); a carbamate-based protecting group such as C1-6 alkoxycarbonyl (examples thereof include methoxycarbonyl, ethoxycarbonyl, n-propoxycarbonyl, i-propoxycarbonyl, n-butoxycarbonyl, i-butoxycarbonyl, t-butoxycarbonyl (Boc), t-amyloxycarbonyl, and the like, preferably Boc, and the like), C2-6 alkenyloxycarbonyl (examples thereof include vinyloxycarbonyl (Voc), allyloxycarbonyl (Alloc), and the like), tri(C1-3 alkyl)silylethoxycarbonyl (examples thereof include 2-(trimethylsilyl)ethoxycarbonyl (Teoc), and the like), halo C1-6 alkoxycarbonyl (examples thereof include 2,2,2-trichloroethoxycarbonyl (Troc), and the like), or aryloxycarbonyl (examples thereof include benzyloxycarbonyl (Z or Cbz), p-nitrobenzyloxycarbonyl, p-methoxybenzyloxycarbonyl (Moz), and the like); a sulfonamide-based protecting group such as alkylsulfonyl (examples thereof include methanesulfonyl (Ms), ethanesulfonyl, and the like) or arylsulfonyl (examples thereof include benzenesulfonyl, p-toluenesulfonyl (Ts), p-chlorobenzenesulfonyl, p-methoxybenzenesulfonyl (MBS), m-nitrobenzenesulfonyl, o-nitrobenzenesulfonyl, p-nitrobenzenesulfonyl, 2,4-nitrobenzenesulfonyl, 2,6-dimethoxy-4-methylbenzenesulfonyl (iMds), 2,6-dimethyl-4-methoxybenzenesulfonyl (Mds), 2,4,6-trimethoxybenzenesulfonyl (Mtb), 2,3,5,6-tetramethyl-4-methoxybenzenesulfonyl (Mte), 2,3,6-trimethyl-4-methoxybenzenesulfonyl (Mtr), 2,4,6-trimethylbenzenesulfonyl (Mts), pentamethylbenzenesulfonyl (Pme), and the like), and the like.

Examples of the "sulfanyl protecting group" can include an ether-based protecting group such as benzyl, p-methoxybenzyl, p-acetoxybenzyl, p-nitrobenzyl, 4-picolyl, 9-fluorenylmethyl, or di- or triarylmethyl (examples thereof include diphenylmethyl, triphenylmethyl (trityl), bis(4-methoxyphenyl)methyl, and the like); a thioacetal-based protecting group such as methoxymethyl, isobutoxymethyl, benzylthiomethyl, or 2-tetrahydropyranyl; a thioester-based protecting group such as acyl (examples thereof include acetyl, benzoyl, and the like), and the like. The sulfanyl group may be referred to as a thiol group in the present specification.

Regarding protection and deprotection of the "hydroxy protecting group", the "amino protecting group", and the "sulfanyl protecting group" in the present invention, reference can also be made to Protective Groups in Organic Synthesis, 4th edition, written by T. W. Greene, P. G. M. Wuts, John Wiley & Sons Inc. (2006), and the like.

The phosphorus-containing group is a group containing a phosphorus atom, and means a group useful for forming an internucleoside bond including a phosphodiester structure or a phosphorothioate structure. As the phosphorus-containing group, those known in the art can be used, and examples thereof include a group derived from phosphoramidite, a group derived from H-phosphonate, a group derived from phosphoric acid diester, a group derived from phosphoric acid triester, and the like.

Specific examples thereof include groups represented by any of the following Formulae (Z³-1) to (Z³-3) (provided that the bonding of the phosphorus atom in the formula to the sugar moiety of the nucleotide is via an oxygen atom).
[Chem. 18]

Formula (Z³-1): -P(OR^{X1})(NR^{X2}₂)

In the formula, R^{X1} and R^{X2} are each independently a C1-6 alkyl group, and the alkyl group is unsubstituted or substituted with one or more substituents solely or differently selected from the group consisting of a halogen atom, a C1-6 alkoxy group, a cyano group, a C6-10 aryl group, and a C2-9 aromatic heterocyclic group.
[Chem. 19]

Formula (Z³-2): -P(=R^{X3})(OR^{X4})₂

In the formula, R^{X3} is an oxygen atom or a sulfur atom, R^{X4} are each independently a hydrogen atom, a hydroxy protecting group, a C1-6 alkyl group, or a C6-10 aryl group, the alkyl group is unsubstituted or substituted with one or more substituents solely or differently selected from the group consisting of a halogen atom, a C1-6 alkoxy group, a cyano group, a C6-10 aryl group, and a C2-9 aromatic heterocyclic group, and the aryl group is unsubstituted or substituted with one or more substituents solely or differently selected from the group consisting of a halogen atom, a C1-6 alkoxy group, a C1-6 alkyl group, and a cyano group.
[Chem. 20]

Formula (Z³=3): =P(=R^{X5})H(OR^{X6})

In the formula, R^{X5} is an oxygen atom or a sulfur atom. R^{X6} is a hydrogen atom, a hydroxy protecting group, or a C6-10 aryl group.

The phosphorus-containing group is preferably any of the groups represented by Formulae (Z³-4) to (Z³-6).
[Chem. 21]

Formula(Z³-4): -P(OR^{X1})(NR^{X2}₂)

In the formula, R^{X1} is a C1-6 alkyl group or a C1-6 alkyl group substituted with a cyano group and R^{X2} is a C1-6 alkyl group.

[Chem. 22] Formula(Z³-5):-P(=O)(OH)₂ Formula(Z³-6):-P(=O)H(OH)

The phosphorus-containing group is more preferably a cyanoethoxy(diisopropylamino)phosphino group (a group represented by formula: - P(OC₂H₄CN)(N(i-Pr)₂)) or a hydroxyphosphinyl group (a group represented by formula: -P(=O)H(OH)).

"Phosphonate group" means a monovalent group containing one phosphorus-carbon single bond, one phosphorus-oxygen double bond, and two phosphorus-oxygen single bonds.

"5'-phosphonate group" means the phosphonate group located at position 5' of the oligonucleotide. Preferably, the 5' phosphonate group is located at position 5' of the 5' end of the oligonucleotide. As the bond between the nucleotide and the phosphonate group, preferably, the oxygen atom at position 5' of the nucleotide is replaced with a carbon atom, and the carbon atom is bonded to a phosphorus atom. In the present specification, the phosphonate group is included in a modified phosphate group.

"RNA interference" means that a target RNA having homology to the sequence is degraded by introduction of a double-stranded RNA, and expression of the target RNA is inhibited.

"Mediate RNA interference" is understood to mean having an ability to degrade a target RNA in a sequence-specific manner by introduction of a double-stranded RNA and to inhibit expression of the target RNA.

"siRNA" is a double-stranded RNA that causes the RNA interference, in which an antisense strand capable of hybridizing with a target RNA is hybridized with a sense strand. The antisense strand and the sense strand have a length of, for example, 14 to 40 nucleotides or 14 to 20 nucleotides, and preferably have a length of 19 to 23 nucleotides. The antisense strand is complementary to the target RNA and can be understood as a strand mainly composed of optionally modified RNA, and the sense strand is complementary to a part or all of the antisense strand and can be understood as a strand mainly composed of optionally modified RNA. The mechanism of action of RNA interference has not been clarified, but is considered as follows. After entering the cell, the antisense strand is loaded onto the RNA-induced silencing complex (RISC). In this loading process, a sense strand is removed, and an antisense strand remains in the RISC, where the antisense strand binds to a complementary site thereof on the target RNA. The RNA that has undergone binding is then cleaved by nuclease activity of RISC, and then can be further degraded by a cellular nuclease.

"Target RNA" means mRNA, an mRNA precursor, or ncRNA, and includes mRNA transcribed from genomic DNA encoding a target gene, mRNA not subjected to base modification, an mRNA precursor and ncRNA not subjected to splicing, and the like. "Target RNA" whose expression is inhibited by RNA interference is not particularly limited, and examples thereof include RNAs related to genes whose expression is upregulated in various diseases. "Target RNA" may be any RNA synthesized by DNA-dependent RNA polymerase, preferably mRNA or an mRNA precursor. Mammalian mRNA is more preferable, and human mRNA is still more preferable.

"Hybridize" means an action of forming a double strand between oligonucleotides or groups derived from oligonucleotides including sequences complementary to each other, and a phenomenon that oligonucleotides or groups derived from oligonucleotides including sequences complementary to each other form a double strand. The antisense strand and the sense strand hybridize with each other.

"Sequence portion" means a partial structure of an oligonucleotide chain. For example, a sequence portion including a nucleotide is a partial structure of a region including the nucleotide in an oligonucleotide chain.

"Hybridizing portion" refers to a complementary sequence portion that forms the double strand. "Sense strand hybridizing portion" refers to a region of a sense strand that hybridizes with the antisense strand. "Antisense strand hybridizing portion" refers to a region of an antisense strand that hybridizes with the sense strand. The sense strand included in the double-stranded RNA of the present invention includes a sense strand hybridizing portion, and the antisense strand includes an antisense strand hybridizing portion. The sense strand hybridizing portion and the antisense strand hybridizing portion are complementary to each other to form a double strand.

"Complementary" or "having complementarity" means that two nucleobases can form a Watson-Crick base pair (natural base pair) or a non-Watson-Crick base pair (Hoogsteen base pair, and the like) via a hydrogen bond. Two oligonucleotides or groups derived from oligonucleotides can "hybridize" with each other when their sequences are complementary to each other. In order for two oligonucleotides or groups derived from oligonucleotides to hybridize with each other, they need not be completely complementary to each other, but complementarity for two oligonucleotides or groups derived from oligonucleotides to hybridize with each other is preferably 70% or more, more preferably 80% or more, and still more preferably 90% or more (for example, 95% or more, 96% or more, 97% or more, 98% or more, 99% or more, or 100%). Complementarity between sequences can be determined by using a computer program that automatically identifies a partial sequence of an oligonucleotide. For example, OligoAnalyzer is one type of such software and is provided by Integrated DNA Technologies, Inc. This program can also be used on a website. A person skilled in the art can easily determine conditions (temperature, salt concentration, and the like) under which two oligonucleotides or groups derived from oligonucleotides can hybridize. In addition, a person skilled in the art can easily design an antisense strand complementary to a target RNA, for example, by using the BLAST program or the like based on information of a nucleotide sequence of the target RNA. For the BLAST program, Proceedings of the National Academy of Sciences of the United States of America, 1990, 87, pp 2264-2268, Proceedings of the National Academy of Sciences of the United States of America, 1993, 90, pp 5873-5877, Journal of Molecular Biology, 1990, 215, pp 403-410, and the like can be referred to.

"Complementarity with a target RNA sufficient to mediate RNA interference" is not particularly limited as long as it is sufficient to mediate RNA interference, but is, for example, preferably 70% or more, more preferably 80% or more, and still more preferably 90% or more (for example, 95% or more, 96% or more, 97% or more, 98% or more, 99% or more, or 100%).

"Nucleotide" means a molecule that can be a constituent unit of a nucleic acid (oligonucleotide), and usually has a base and/or sugar as a constituent element. The nucleotide is composed of, for example, a sugar, a base, and a phosphate. The sugar, base and phosphate may be modified. The nucleotide includes a ribonucleotide, a deoxyribonucleotide, and a sugar-modified nucleotide, as described later. In addition, a nucleotide includes a molecule composed of a sugar and a phosphate without having a base, as well as their modified forms.

"Nucleoside" means a molecule that can be a constituent unit of a nucleic acid (oligonucleotide), and usually has a base as a constituent element. The nucleoside is composed of, for example, a sugar and a base, and represents a partial structure that does not include a phosphate or a modified phosphate in the "nucleotide". "Nucleoside structure" refers to a structural unit linked to an adjacent nucleoside in an oligonucleotide via a phosphodiester bond or a modified phosphodiester bond. In the present specification, the nucleoside structure is used to represent a partial structure that does not include a phosphodiester bond or a modified phosphodiester bond.

"Oligonucleotide" means a molecule having a structure in which one or more of the nucleotides are polymerized. When an "oligonucleotide" is composed of one nucleotide, the oligonucleotide can be said to be a "nucleotide".

Nucleotides included in the double-stranded RNA of the present invention are each independently linked to each other by a phosphodiester bond or a modified phosphodiester bond described later. In the double-stranded RNA of the present invention, the nucleotide at the 3' end of each oligonucleotide strand preferably has a hydroxy group, a phosphate group, or a modified phosphate group at its 3' position, more preferably has a hydroxy group, and usually has a hydroxy group. The nucleotide at the 5' end of each oligonucleotide strand included in the double-stranded RNA preferably has a hydroxy group, a phosphate group, or a modified phosphate group at its 5' position.

"Internucleotide bond" means a group or a bond that forms a covalent bond between adjacent nucleosides in an oligonucleotide. The internucleotide bond includes a phosphodiester bond or a modified phosphodiester bond (for example, a phosphorothioate bond). The internucleotide bond may be referred to as an internucleoside bond.

"Group derived from an oligonucleotide" means a group obtained by removing a hydrogen atom, a hydroxy group, and the like from a hydroxy group of at least one of the 3' end and the 5' end of the oligonucleotide, and is linked to another group (for example, a group derived from another oligonucleotide) through a covalent bond indirectly by forming a phosphodiester bond or a modified phosphodiester bond. The hydroxy group at the 3' end or the 5' end includes a hydroxy group of a phosphate group. For example, a group obtained by removing a hydrogen atom from a hydroxy group at the 3' end of the oligonucleotide and a group obtained by removing a hydroxy group from a phosphate group at the 5' end of another oligonucleotide form a phosphodiester bond or a modified phosphodiester bond.

Similarly, "group derived from" a certain molecule means a group obtained by removing a hydrogen atom, a hydroxy group, or the like from the molecule.

"Nucleotide sequence" means a base sequence of nucleotides constituting an oligonucleotide.

In the present specification, the term "blunt end" means that the ends of double-stranded RNA are paired without either protruding.

In the present specification, "overhang" means that one strand of the ends of double-stranded RNA has a protrusion. The overhang region means the protrusion. The overhang region can be of any length, but preferably has a length of 1 to 50 nucleotides, more preferably has a length of 1 to 30 nucleotides, still more preferably has a length of 1 to 15 nucleotides, and most preferably has a length of 2 to 6 nucleotides. The protrusion is also referred to as a single-stranded overhang because the overhang does not form a double strand as the double-stranded RNA in the present specification.

"Deoxyribonucleotide" means a molecule in which the sugar is 2-deoxyribose, a base is bonded to a carbon atom at position 1 of 2-deoxyribose, and a phosphate group is present at position 3 or position 5. The deoxyribonucleotide in the present invention may be a naturally occurring deoxyribonucleotide or a deoxyribonucleotide in which a base moiety or a phosphodiester bond moiety of a naturally occurring deoxyribonucleotide is modified. A plurality of modifications of a base moiety and modifications of a phosphodiester bonding site may be made to one deoxyribonucleotide in combination. The modified deoxyribonucleotide is described, for example, in Journal of Medicinal Chemistry, 2016, 59, pp 9645-9667, Medicinal Chemistry Communication, 2014, 5, pp 1454-1471, Future Medicinal Chemistry, 2011, 3, pp 339-365, RNA, 2023, 29, pp 423-433, and the like.

When the "deoxyribonucleotide" constitutes each oligonucleotide strand included in the double-stranded RNA of the present invention, usually, position 3' of the deoxyribonucleotide is linked to another nucleotide or the like by a phosphodiester bond or a modified phosphodiester bond (for example, a phosphorothioate bond), and position 5' of the deoxyribonucleotide is linked to another nucleotide or the like by a phosphodiester bond or a modified phosphodiester bond (for example, a phosphorothioate bond). The deoxyribonucleotide at the 3' end of each oligonucleotide strand included in the double-stranded RNA of the present invention has preferably a hydroxy group, a phosphate group, or a modified phosphate group at its position 3', and position 5' is as described above. The deoxyribonucleotide at the 5' end of each oligonucleotide strand included in the double-stranded RNA has preferably a hydroxy group, a phosphate group, or a modified phosphate group at its 5' position, and position 3' is as described above.

"Ribonucleotide" means a molecule in which the sugar is ribose, a base is bonded to a carbon atom at position 1 of ribose, and a phosphate group is present at position 2, position 3, or position 5. The ribonucleotide in the present invention may be a naturally occurring ribonucleotide or a ribonucleotide in which a base moiety or a phosphodiester bond moiety of a naturally occurring ribonucleotide is modified. A plurality of modifications of a base moiety and modifications of a phosphodiester bonding site may be made to one ribonucleotide in combination. The modified ribonucleotide is described, for example, in Journal of Medicinal Chemistry, 2016, 59, pp 9645-9667, Medicinal Chemistry Communication, 2014, 5, pp 1454-1471, Future Medicinal Chemistry, 2011, 3, pp 339-365, RNA, 2023, 29, pp 423-433, and the like.

When the "ribonucleotide" constitutes each oligonucleotide strand included in the double-stranded RNA of the present invention, typically, position 3' of the ribonucleotide is linked to another nucleotide by a phosphodiester bond or a modified phosphodiester bond (for example, a phosphorothioate bond), and position 5' of the ribonucleotide is linked to another nucleotide or the like by a phosphodiester bond or a modified phosphodiester bond (for example, a phosphorothioate bond). The ribonucleotide at the 3' end of each oligonucleotide strand included in the double-stranded RNA of the present invention has preferably a hydroxy group, a phosphate group, or a modified phosphate group at its position 3', and position 5' is as described above. The ribonucleotide at the 5' end of each oligonucleotide strand included in the double-stranded RNA has preferably a hydroxy group, a phosphate group, or a modified phosphate group at its 5' position, and position 3' is as described above.

The "modified sugar" encompasses:
(Z1) molecules in which ribose or 2-deoxyribose is partially substituted with one or more substituents;
(Z2) pentoses or hexoses different from ribose and 2-deoxyribose (for example, hexitol, threose, and the like); and
(Z3) molecules in which the entire ribose or 2-deoxyribose, or the tetrahydrofuran ring thereof, is replaced with a 5- to 7-membered saturated or unsaturated ring (for example, cyclohexane, cyclohexene, morpholine, and the like).

The modified sugar includes a "2-modified sugar", a "2-4-bridged sugar", and a "5-modified sugar" described later.

Examples of the modified sugar and the sugar-modified nucleotide described later include sugars, sugar-modified nucleotides, and the like disclosed in JP H10-304889 A, WO 2005/021570 A, JP H10-195098 A, JP 2002-521310 A, WO 2007/143315 A, WO 2008/043753 A, WO 2008/029619 A, WO 2008/049085 A, WO 2017/142054 A, and the like. In addition, the modified sugar and the sugar-modified nucleotide are also disclosed in Journal of Medicinal Chemistry, 2016, 59, pp 9645-9667, Medicinal Chemistry Communication, 2014, 5, pp 1454-1471, Future Medicinal Chemistry, 2011, 3, pp 339-365, RNA, 2023, 29, pp 423-433, WO 2018/155450 A, and the like.

Examples of a modified sugar that is partially substituted with one substituent include ribose or 2-deoxyribose in which any position of a sugar moiety is substituted with one or more (preferably one or two) substituents of the following (i) or (ii).
(i) C1-6 alkyl group. Here, when substitution with two or more C1-6 alkyl groups is contained, the two or more C1-6 alkyl groups may form a 3- to 6-membered ring together.
(ii) A C1-6 alkyl group substituted with at least one selected from the group consisting of a halogen atom, a C1-6 alkoxy group, a halo C1-6 alkoxy group, a mono- or di-C1-6 alkylamino group, a 5- to 10-membered heterocyclic group, a carboxy group, a carbamoyl group, and an N-substituted carbamoyl group.

Here, examples of the N-substituted carbamoyl group include an N-methyl-carbamoyl group and an N-ethyl-carbamoyl group, and the methyl group and the ethyl group of the N-methyl-carbamoyl group and the N-ethyl-carbamoyl group may be substituted with a 5- to 10-membered heterocyclic group or a mono- or di-C₁₋₆ alkylamino group. Specific examples of the N-substituted carbamoyl group include an N-methylcarbamoyl group, an N-ethylcarbamoyl group, an N-dimethylaminoethyl-carbamoyl group, an N-(morpholinoethyl)carbamoyl group, an N-(2-pyridylethyl)carbamoyl group, an N-((benzimidazol-1-yl)ethyl)carbamoyl group, and the like.

The "sugar-modified nucleotide" encompasses, in addition to a molecule having a "modified sugar" in place of a sugar moiety of a deoxyribonucleotide or ribonucleotide, a molecule having an acyclic structure in place of the sugar moiety. For example, a sugar-modified nucleotide encompasses a "2'-modified nucleotide", a "2'-4'-bridged nucleotide", a "5'-modified nucleotide", and an "acyclic nucleotide" described later. When the modified sugar is (Z3) as defined above, the sugar-modified nucleotide also includes a molecule in which a modified sugar and a nucleobase are bonded to each other via a methylene chain or the like.

"2-modified sugar" means a non-bridged sugar in which an oxygen atom or a carbon atom at position 2 of ribose is modified, and includes "2-O-Me", "2-O-MOE", "2-O-MCE", "2-O-NMA", "2-O-AP", "2-fluoro", "2-O-DMAECE", "2-O-MorECE", "2-O-PyECE", and "2-O-BimECE". Position 4 of the "2-modified sugar" is preferably unmodified.

"2'-modified nucleotide" means a molecule in which a base is bonded to a carbon atom at position 1 (position 1 of ribose before modification) of the "2-modified sugar" and which has a phosphate group at position 3 or position 5, and examples thereof include "2'-O-Me nucleotide", "2'-O-MOE nucleotide", "2'-O-MCE nucleotide", "2'-O-NMA nucleotide", "2'-O-AP nucleotide", "2'-F nucleotide", "2'-O-DMAECE nucleotide", "2'-O-MorECE nucleotide", "2'-O-PyECE nucleotide", and "2'-O-BimECE nucleotide". Position 5' of the 2'-modified nucleotide may or may not be modified.

"2-O-Me" (also referred to as 2-O-methyl) means a sugar in which a hydroxy group at position 2 of ribose is replaced with a methoxy group.

"2'-O-Me nucleotide" (also referred to as 2'-O-methyl nucleotide) means a molecule in which a base is bonded to a carbon atom at position 1 (position 1 of ribose before modification) of "2-O-Me", and a phosphate group is present at position 3 or position 5.

"2-O-MOE" (also referred to as 2-O-methoxyethyl) means a sugar in which a hydroxy group at position 2 of ribose is replaced with a 2-methoxyethyloxy group.

"2'-O-MOE nucleotide" (also referred to as 2'-O-methoxyethyl nucleotide) means a molecule in which a base is bonded to a carbon atom at position 1 (position 1 of ribose before modification) of "2-O-MOE", and a phosphate group is present at position 3 or position 5.

"2-O-MCE" (also referred to as 2-O-methylcarbamoylethyl) means a sugar in which a hydroxy group at position 2 of ribose is replaced with a methylcarbamoylethyloxy group.

"2'-O-MCE nucleotide" (also referred to as 2'-O-methylcarbamoylethyl nucleotide) means a molecule in which a base is bonded to a carbon atom at position 1 (position 1 of ribose before modification) of "2-O-MCE", and a phosphate group is present at position 3 or position 5.

"2-O-NMA" means a sugar in which a hydroxy group at position 2 of ribose is replaced with a [2-(methylamino)-2-oxoethyl]oxy group.

"2'-O-NMA nucleotide" means a molecule in which a base is bonded to a carbon atom at position 1 (position 1 of ribose before modification) of "2-O-NMA", and a phosphate group is present at position 3 or position 5.

"2-O-AP" means a sugar in which a hydroxy group at position 2 of ribose is replaced with a 3-aminopropyloxy group.

"2'-O-AP nucleotide" means a molecule in which a base is bonded to a carbon atom at position 1 (position 1 of ribose before modification) of "2-O-AP", and a phosphate group is present at position 3 or position 5.

"2-fluoro" means a sugar in which a hydroxy group at position 2 of ribose is replaced with a fluorine atom.

"2'-fluoro nucleotide" means a molecule in which a base is bonded to a carbon atom at position 1 (position 1 of ribose before modification) of "2-fluoro", and a phosphate group is present at position 3 or position 5.

"2-O-DMAECE", "2-O-MorECE", "2-O-PyECE", and "2-O-BimECE" are sugars in which a hydroxy group at position 2 of ribose is replaced with a structure shown by the following DMAECE, MorECE, PyECE, and BimECE, respectively. In the following structures, a wavy line indicates a bonding position to a carbon atom to which the hydroxy group at position 2 of ribose is bonded.

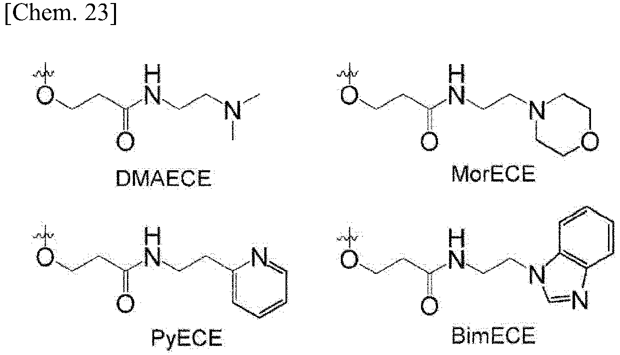

"2'-O-DMAECE nucleotide", "2'-O-MorECE nucleotide", "2'-O-PyECE nucleotide", and "2'-O-BimECE nucleotide" mean molecules in which a base is bonded to a carbon atom at position 1 (position 1 of ribose before modification) of "2-O-DMAECE", "2-O-MorECE", "2-O-PyECE", and "2-O-BimECE", respectively, and a phosphate group is present at position 3 or position 5.

"2'-O-XCE nucleotide" means a nucleotide in which the hydroxy group at position 2' of a ribonucleotide is modified with a substituted or unsubstituted carbamoylethyl group.

In some embodiments of various aspects disclosed in the present specification, the 2'-O-XCE nucleotide is a nucleotide including a nucleoside structure represented by Formula (I) below: 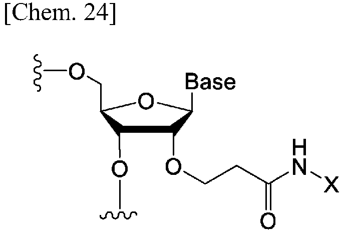
{in which Base is a purin-9-yl group, a 2-oxo-pyrimidin-1-yl group, or a 2-thioxo-pyrimidin-1-yl group (the purin-9-yl group, the 2-oxo-pyrimidin-1-yl group, and the 2-thioxo-pyrimidin-1-yl group are each independently unsubstituted or substituted with one or more substituents solely or differently selected from the group consisting of a halogen atom, a C1-6 alkyl group, an amino group, a protected amino group, a hydroxy group, a protected hydroxy group, a sulfanyl group, and a protected sulfanyl group),
X is a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group), or
a group represented by Formula (Ia) below: 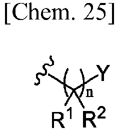
[in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group) or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with the nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
n is an integer of 1 to 3, and when n is 2 or 3, two or three R¹ and R² may be the same or different, respectively]}.

Note that the wavy lines in Formula (I) are understood as a bonding position for an internucleotide bond with an adjacent nucleotide, a bonding position with a functional molecule or a linker thereof, a bonding position with a hydrogen atom, a hydroxy protecting group, or a phosphorus-containing group, or the like, and the wavy line in Formula (Ia) indicates the bonding position for a bond with the nitrogen atom in Formula (I).

In some embodiments, X represents a hydrogen atom, a C1-6 alkyl group, or a C2-6 alkenyl group. Here, the C1-6 alkyl group and the C2-6 alkenyl group are each independently unsubstituted or substituted with one or more substituents solely or differently selected from the group consisting of a halogen atom, a C1-6 alkoxy group, and a cyano group.

X is preferably a hydrogen atom or a C1-6 alkyl group, more preferably a C1-3 alkyl group, and still more preferably a methyl group.

In some embodiments, X is a group represented by the following Formula (Ia): 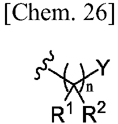
[in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with a nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
n is an integer of 1 to 3, and when n is 2 or 3, two or three R¹ and R² may be the same or different, respectively].

R¹ and R² each independently represent a hydrogen atom, a halogen atom, a cyano group, a C1-6 alkyl group, or a C2-6 alkenyl group. Here, the C1-6 alkyl group and the C2-6 alkenyl group are unsubstituted or substituted with one or more substituents solely or differently selected from the group consisting of a halogen atom, a C1-6 alkoxy group, and a cyano group. Here, when n is 2 or 3, each R¹ may be the same or different, and each R² may be the same or different.

R¹ is preferably a hydrogen atom or a C1-3 alkyl group, and more preferably a hydrogen atom. R² is preferably a hydrogen atom or a C1-3 alkyl group, and more preferably a hydrogen atom.

When Y is NR³R⁴, R³ and R⁴ each independently represent a hydrogen atom, a C1-6 alkyl group, a C2-6 alkenyl group, or a C7-10 aralkyl group. Here, the C1-6 alkyl group and the C2-6 alkenyl group are unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group, and the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group.

R³ is preferably a hydrogen atom or a C1-3 alkyl group, and more preferably a methyl group.

R⁴ is preferably a hydrogen atom or a C1-3 alkyl group, and more preferably a methyl group.

Here, NR³R⁴ may be expressed as N(R³)R⁴, and means one in which R³ and R⁴ are bonded to the nitrogen atom.

As another embodiment, R³ and R⁴ together with a nitrogen atom, to which they are bonded, may form a 3- to 11-membered nitrogen-containing non-aromatic heterocyclic ring. Here, the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group.

The 3- to 11-membered nitrogen-containing non-aromatic heterocyclic ring formed by R³ and R⁴ together with a nitrogen atom, to which they are bonded, is preferably a ring including 4 to 6 methylene groups among 4- to 8-membered nitrogen-containing non-aromatic heterocyclic rings, and examples thereof include piperidine, pyrrolidine, morpholine, thiomorpholine, homopiperidine, homomorpholine, and the like. More preferably, the ring is a ring including an oxygen atom or a sulfur atom as an atom constituting the ring, and examples thereof include morpholine, thiomorpholine, homomorpholine, and the like. Morpholine is particularly preferable. In addition, the 4- to 8-membered nitrogen-containing non-aromatic heterocyclic ring is preferably unsubstituted.

As another embodiment, Y may be a C2-9 aromatic heterocyclic group. A pyridyl group, an imidazolyl group, or a benzimidazolyl group is preferable; 2-pyridyl, imidazole-1-yl, or (benzimidazol)-1-yl is more preferable; and 2-pyridyl or (benzimidazol)-1-yl is particularly preferable.

n is the number of repeating unit structures and is an integer from 1 to 3, preferably 2.

The 2'-O-XCE nucleotide may contain the nucleoside structure represented by Formula (I) at the 3' end or the 5' end of the oligonucleotide. When the 2'-O-XCE nucleotide is included at the 3' end, for example, the 2'-O-XCE nucleotide has a structure represented by the following Formula (I-1): 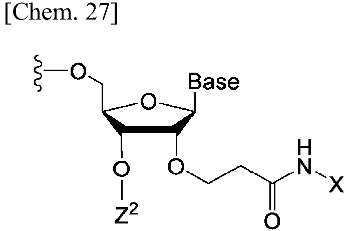 (in Formula (I-1), X and Base have the same meaning as X and Base in Formula (I), and Z² is a hydrogen atom, a hydroxy protecting group, or a phosphorus-containing group, and is preferably a hydrogen atom).

The wavy line in Formula (I-1) is understood as a bonding position for an internucleotide bond with an adjacent nucleotide, a bonding position with a functional molecule or a linker thereof, or a bonding position with a hydrogen atom, a hydroxy protecting group, or a phosphorus-containing group.

In addition, when the 2'-O-XCE nucleotide is included at the 5' end, the 2'-O-XCE nucleotide is represented, for example, by the following Formula (I-2): 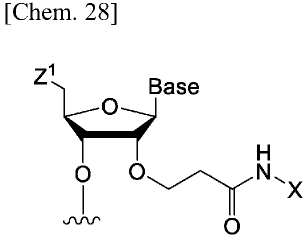 (in Formula (I-2), X and Base have the same meaning as X and Base in Formula (I), and Z¹ is a hydroxy group, a phosphate group, or a modified phosphate group).

The wavy line in Formula (I-2) is understood as a bonding position for an internucleotide bond with an adjacent nucleotide, a bonding position with a functional molecule or a linker thereof, or a bonding position with a hydrogen atom, a hydroxy protecting group, or a phosphorus-containing group.

Exemplary phosphate groups or modified phosphate groups include those compatible with RISC-mediated gene silencing. Examples of the phosphate group or modified phosphate group preferably include a monophosphate ((HO)₂(O)P-O-), a diphosphate ((HO)₂(O)P-O-P(HO)(O)-O-), a triphosphate ((HO)₂(O)P-O-(HO)(O)P-OP(HO)(O)-O-), a monothiophosphate (phosphorothioate: (HO)₂(S)P-O-), a monodithiophosphate (dithiophosphate: (HO)(HS)(S)P-O-), a phosphorothiolate ((HO)₂(O)P-S-), any further combination of monophosphate, diphosphate, and triphosphate in which oxygen/sulfur are substituted (for example, α-thio triphosphate, γ-thio triphosphate, and the like), a phosphoramidate ((HO)₂(O)P-NH-, (HO)(NH₂)(O)P-O-), a 5'-alkylphosphonate (alkylene phosphate (for example, (HO)₂(O)P-CH₂-5'(methylene phosphate), (HO)₂(O)P-CH₂CH₂-5'-(ethylene phosphate), R'P(OH)(O)-O-5'-(R' = alkyl, for example methyl, ethyl, isopropyl, propyl, and the like)), a 5'-cycloalkylphosphonate ((HO)₂(O)P-C₃H₄-5'-(cyclopropylene phosphate)), a 5'-alkenylphosphonate (the alkenyl is, for example, vinyl ((OH)₂(O)P-CH=CH-5'-), substituted vinyl), a 5'-alkoxyalkylphosphonate-(R"P(OH)(O)-O-5' (R" = alkoxyalkyl, for example methoxymethyl, ethoxymethyl, and the like)), and the like.

In some embodiments, Z¹ is preferably a hydroxy group. In some other embodiments, Z¹ is preferably a monophosphate ((HO)₂(O)P-O-).

In some embodiments, when the 2'-O-XCE nucleotide is included at the 5' end, a structure represented by the following Formula (II-1): 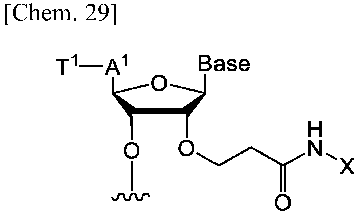
[in Formula (II-1), X and Base have the same meaning as X and Base in Formula (II),
T¹ is a group of the following Formula (IIb): 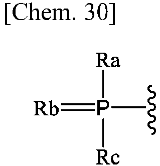
[Ra and Rc are each independently selected from a hydroxy group, a protected hydroxy group, a thiol group, a protected thiol group, an amino group, a protected amino group, a C1-6 alkyl group, a C2-6 alkenyl group, or a C1-6 alkoxy group (the hydroxy group, the thiol group, the amino group, the C1-6 alkyl group, the C2-6 alkenyl group, and the C1-6 alkoxy group are each independently unsubstituted or substituted with one or more substituents solely or differently selected from the group consisting of a halogen atom, a C1-6 alkyl group, a C1-6 alkoxy group, and a cyano group), and
Rb is an oxygen atom or a sulfur atom], and
A¹ is a group selected from the following Formula (IIc): 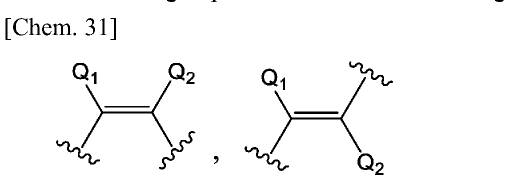
[Q₁ and Q₂ are each independently a hydrogen atom, a halogen atom, a C1-C6 alkyl group, a C1-6 alkoxy group, a C2-6 alkenyl group, a C2-6 alkynyl group, or an amino group (the C1-C6 alkyl group, the C1-6 alkoxy group, the C2-6 alkenyl group, the C2-6 alkynyl group, and the amino group are each independently unsubstituted or substituted with one or more substituents solely or differently selected from the group consisting of a halogen atom, a C1-6 alkyl group, a C1-6 alkoxy group, and a cyano group)] is obtained.

The wavy line in Formula (II-1) is understood as a bonding position for an internucleotide bond with an adjacent nucleotide, a bonding position with a functional molecule or a linker thereof, or a bonding position with a hydrogen atom, a hydroxy protecting group, or a phosphorus-containing group, and the wavy line in Formula (IIb) indicates a bonding position with A¹ in Formula (II-1), and the wavy lines in Formula (IIc) respectively indicate a bonding position with T¹ in Formula (II-1) and a bonding position with a sugar carbon atom.

In some embodiments, Ra and Rc are preferably hydroxy groups, and Rb is preferably an oxygen atom.

In some other embodiments, Q₁ and Q₂ are preferably hydrogen atoms.

In some embodiments, when the nucleoside structure represented by Formula (I) is included at the 5' end of an oligonucleotide, a structure represented by Formula (II-2): 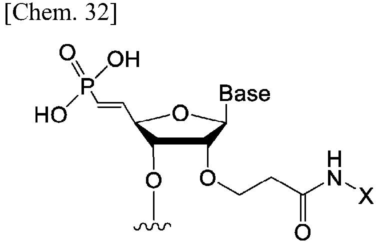 (in Formula (II-2), X and Base have the same meaning as X and Base in Formula (II)) (5'-vinylphosphonate (VP) modified 2'-O-XCE nucleotide) is preferable.

The wavy line in Formula (II-2) is understood as a bonding position for an internucleotide bond with an adjacent nucleotide, a bonding position with a functional molecule or a linker thereof, or a bonding position with a hydrogen atom, a hydroxy protecting group, or a phosphorus-containing group.

In some embodiments, when the 2'-O-XCE nucleotide is included at the 5' end, the 2'-O-XCE nucleotide has a structure represented by the following Formula (III-1): 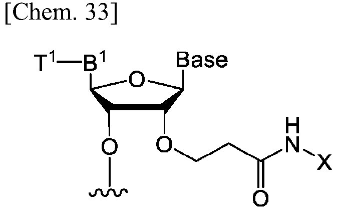
{in Formula (III-I), T¹, X, and Base are the same as T¹, X, and Base in Formula (III), and
B¹ is a group represented by the following Formula (IIIc): 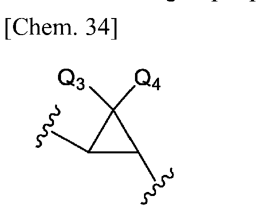
[Q₃ and Q₄ each independently represent a hydrogen atom, a halogen atom, a C1 to C6 alkyl group, a C1-6 alkoxy group, a C2-6 alkenyl group, a C2-6 alkynyl group, or an amino group (the C1 to C6 alkyl group, the C1-6 alkoxy group, the C2-6 alkenyl group, the C2-6 alkynyl group, and the amino group are each independently unsubstituted or substituted with one or more substituents solely or differently selected from the group consisting of a halogen atom, a C1-6 alkyl group, a C1-6 alkoxy group, and a cyano group)]}.

The wavy line in Formula (III-1) is understood as a bonding position for an internucleotide bond with an adjacent nucleotide, a bonding position with a functional molecule or a linker thereof, or a bonding position with a hydrogen atom, a hydroxy protecting group or a phosphorus-containing group, or the like, and the wavy lines in Formula (IIIc) indicate a bonding position with T¹ of Formula (III-1) and a bonding position with a sugar carbon atom, respectively.

In some other embodiments, Q₃ and Q₄ are preferably hydrogen atoms.

In some embodiments, when the nucleoside structure represented by Formula (I) is included at the 5' end of an oligonucleotide, a structure (5'-cyclopropanephosphonate (CPP) modified 2'-O-XCE nucleotide) represented by Formula (III-2): 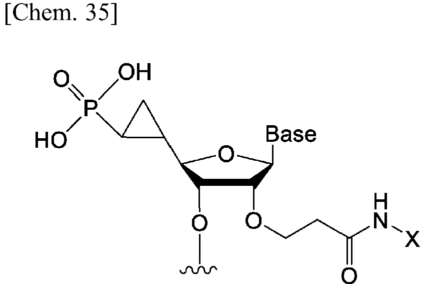 (in Formula (III-2), X and Base have the same meaning as X and Base in Formula (III)) is preferable.

The wavy line in Formula (III-2) is understood as a bonding position for an internucleotide bond with an adjacent nucleotide, a bonding position with a functional molecule or a linker thereof, or a bonding position with a hydrogen atom, a hydroxy protecting group or a phosphorus-containing group, or the like, and the wavy line in Formula (IIIc) indicates a bonding position with T¹ of Formula (III-2) and a bonding position with a sugar carbon atom, respectively.

In some embodiments, when the 2'-O-XCE nucleotide is included at the 5' end, the 2'-O-XCE nucleotide has a structure represented by the following Formula (IV-1): 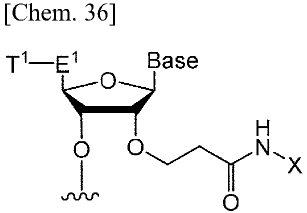
{in Formula (IV-1), T¹, X, and Base are the same as T¹, X, and Base in Formula (IV), and
E¹ is a group represented by the following Formula (IVc): 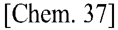 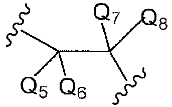
[Q₅ to Q₈ each independently represent a hydrogen atom, a halogen atom, a C1 to C6 alkyl group, a C1-6 alkoxy group, a C2-6 alkenyl group, a C2-6 alkynyl group, or an amino group (the C1 to C6 alkyl group, the C1-6 alkoxy group, the C2-6 alkenyl group, the C2-6 alkynyl group, and the amino group are each independently unsubstituted or substituted with one or more substituents solely or differently selected from the group consisting of a halogen atom, a C1-6 alkyl group, a C1-6 alkoxy group, and a cyano group)]}.

The wavy line in Formula (IV-1) is understood as a bonding position for an internucleotide bond with an adjacent nucleotide, a bonding position with a functional molecule or a linker thereof, or a bonding position with a hydrogen atom, a hydroxy protecting group or a phosphorus-containing group, or the like, and the wavy line in Formula (IIIc) indicates a bonding position with T¹ of Formula (IV-1) and a bonding position with a sugar carbon atom, respectively.

In some other embodiments, Q₅ to Q₈ are preferably hydrogen atoms.

In some embodiments, when the nucleoside structure represented by Formula (I) is included at the 5' end of an oligonucleotide, a structure represented by Formula (IV-2): 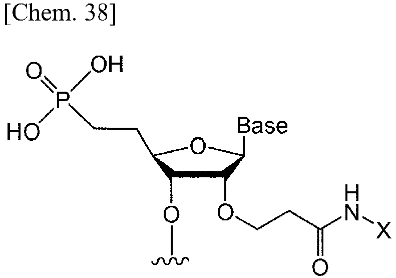 (in Formula (IV-2), X and Base have the same meaning as X and Base in Formula (IV)) (5'-ethylphosphonate (EP) modified 2'-O-XCE nucleotide) is preferable.

The wavy line in Formula (IV-2) is understood as a bonding position for an internucleotide bond with an adjacent nucleotide, a bonding position with a functional molecule or a linker thereof, or a bonding position with a hydrogen atom, a hydroxy protecting group, or a phosphorus-containing group.

In some embodiments, in T¹ of Formulae (II-1), (III-1), and (IV-1) above, Rb is preferably an oxygen atom, Ra and Rc are each independently preferably a hydroxy group or a protected hydroxy group, more preferably a protected hydroxy group, still more preferably an acyloxymethyl group (for example, a methyl group substituted with a C1-6 alkylcarbonyloxy group) or an alkyl group (for example, a C1-6 alkyl group), and particularly preferably a pivaloyloxymethyl group or an ethyl group.

The suitable structure of X in Formulae (II-1), (III-1), and (IV-1) is the same as the suitable corresponding structure encompassed in the double-stranded RNA agent.

The hydroxy protecting group of Z³ in Formulae (II-1), (III-1), and (IV-1) is preferably a silyl group, and particularly preferably a tert-butyldimethylsilyl group.

Isomers may be present in the compound represented by Formulae (I) to (IV-2). In that case, the compound of the present embodiment is not limited to a specific isomer, and includes all possible isomers (for example, keto-enol isomers, imine-enamine isomers, diastereoisomers, optical isomers, rotational isomers, and the like), racemates, and mixtures thereof.

"2'-modified nucleotide" contains a "2'-O-XCE nucleotide", and the "2'-O-R-XCE nucleotide" contains a "2'-O-MCE nucleotide", "2'-O-DMAECE nucleotide", a "2'-O-MorECE nucleotide", a "2'-O-PyECE nucleotide", and a "2'-O-BimECE nucleotide".

"2-4-bridged sugar" means a sugar in which a bridging unit is introduced at two positions, namely, position 2 and position 4 in ribose. Examples of the bridging unit include a C2-6 alkylene group (the alkylene group is unsubstituted or substituted with one or more substituents selected from the group consisting of a halogen atom, an oxo group, and a thioxo group, and one or two methylene groups of the alkylene group are not replaced or independently replaced with a group selected from the group consisting of -O-, -NR1- (R1 represents a hydrogen atom, a C1-6 alkyl group, or a halo C1-6 alkyl group), and -S-). The position 5 of the "2-4-bridged sugar" may or may not be modified, but is preferably unmodified.

"2'-4'-bridged nucleotide" (2',4'-BNA) means a molecule in which a base is bonded to a carbon atom at position 1 (position 1 of ribose or 2-deoxyribose before modification) of the "2-4-bridged sugar", and a phosphate group is present at position 3 or position 5. Examples thereof include β-D-methyleneoxy (4'-CH₂-O-2') BNA or α-L-methyleneoxy (4'-CH₂-O-2') BNA also referred to as LNA (locked nucleic acid (registered trademark)), ethyleneoxy (4'-(CH₂)₂-O-2') BNA also referred to as ENA, 4β-D-thio (4'-CH₂-S-2') BNA, aminooxy (4'-CH₂-O-N(R¹¹)-2') BNA (R¹¹ is a hydrogen atom or methyl), oxyamino (4'-CH₂-N(R¹²)-O-2') BNA (R¹² is a hydrogen atom or methyl) also referred to as 2',4'-BNANC, 2',4'-BNACOC, 3'-amino-2',4'-BNA, 5'-methyl BNA, (4'-CH(CH₃)-O-2') BNA also referred to as cEt, (4'-CH(CH₂OCH₃)-O-2') BNA also referred to as cMOE-BNA, amide-based BNA (4'-C(=O)-N(R¹³)-2') BNA (R¹³ is a hydrogen atom or methyl) also referred to as AmNA, (4'-C(spiro-cyclopropyl)-O-2') BNA also referred to as scpBNA, (4'-CH₂-N(R¹⁴)-2') BNA (R¹⁴ is C(=NHR¹⁵+)NHR¹⁶, and R¹⁵ and R¹⁶ are each independently a hydrogen atom, methyl, ethyl, isopropyl, or t-butyl) also referred to as GuNA, (4'-CH₂-N(R¹⁷)-2') BNA (R¹⁷ is a hydrogen atom or methyl) also referred to as amino LNA, other BNAs known to a person skilled in the art, and the like.

"5-modified sugar" means a non-bridged sugar in which the oxygen atom or carbon atom at position 5 of the ribose skeleton is modified, and includes "5-CP", "5-methyl", and "5-aminopropyl". Position 2 and position 4 of the "5-modified sugar" are preferably unmodified.

"5'-modified nucleotide" means a molecule in which a base is bonded to a carbon atom at position 1 (position 1 of 2-deoxyribose before modification) of the "5-modified sugar", and a nucleobase is present in a phosphate group at position 2, position 3, or position 5, and includes, for example, "5'-CP nucleotide", "5'-methyl nucleotide", and "5'-aminopropyl nucleotide". Position 2' of the 5'-modified nucleotide may or may not be modified. Positions 2' and 4' of the 5'-modified nucleotide may or may not be bridged, but it is preferable that they are not bridged.

"5-CP" is a sugar in which position 5 of ribose skeleton is substituted with two methyl groups, and the two methyl groups, together with position 5 of ribose skeleton, form a cyclopropane ring.

"5'-CP nucleotide" is a molecule in which the sugar is the "5-CP", a base is bonded to a carbon atom at its position 1 (position 1 of the parent 2-deoxyribose), and a phosphate group is present at position 3 or position 5, and can be represented by the following structural formula. In the formula, Base is a nucleobase. The wavy line is understood as a bonding position for an internucleotide bond with an adjacent nucleotide, a bonding position with a functional molecule or a linker thereof, or a bonding position with a hydrogen atom, a hydroxy protecting group, or a phosphorus-containing group.

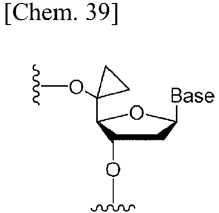

"5-methyl" is a sugar in which position 5 of ribose skeleton is substituted with a methyl group.

"5-aminopropyl" is a sugar in which position 5 of ribose skeleton is substituted with a 3-aminopropyl group.

"5'-Methyl nucleotide" and "5'-aminopropyl nucleotide" mean a molecule in which a base is bonded to a carbon atom at position 1 (position 1 of the parent 2-deoxyribose) of "5-methyl" and "5-aminopropyl", respectively, and a phosphate group is present at position 3 or position 5.

"5-Vinyl" is a sugar in which the methylene group at position 5 of ribose is replaced with an ethene-1,2-diyl group (vinyl group).

"5-CP" is a sugar in which the methylene group at position 5 of ribose is replaced with a cyclopropane-1,1-diyl group (cyclopropyl group).

The vinylphosphonate (VP) modified nucleotide, cyclopropanephosphonate (CPP) modified nucleotide, and ethylphosphonate (EP) modified nucleotide, which are described later, are also contained in the 5'-modified nucleotides.

In the "deoxyribonucleotide", "ribonucleotide", "2'-modified nucleotide", "2'-4'-bridged nucleotide", "5'-modified nucleotide", and the like, examples of a bond between a carbon atom at position 1' and a base include an α-glycosidic bond and a β-glycosidic bond, but the bond is usually a β-glycosidic bond. Therefore, β-D-methyleneoxy BNA is usually used as LNA.

When the "sugar-modified nucleotide" constitutes each oligonucleotide strand included in the double-stranded RNA of the present invention, for example, position 3' of the sugar-modified nucleotide is linked to another nucleotide or the like by a phosphodiester bond or a modified phosphodiester bond (for example, a phosphorothioate bond), and position 5' of the sugar-modified nucleotide is linked to another nucleotide or the like by a phosphodiester bond or a modified phosphodiester bond (for example, a phosphorothioate bond). The sugar-modified nucleotide at the 3' end of each oligonucleotide strand included in the double-stranded RNA of the present invention preferably has, for example, at its position 3', a hydroxy group, a phosphate group, or a modified phosphate group, and position 5' is as described above. The sugar-modified nucleotide at the 5' end of each oligonucleotide strand included in the double-stranded RNA of the present invention preferably has, for example, at its position 5', a hydroxy group, a phosphate group, or a modified phosphate group, and position 3' is as described above.

Examples of modification of a phosphodiester bond moiety in the deoxyribonucleotide, ribonucleotide, and sugar-modified nucleotide include phosphorothioation, methylphosphonation (including chiral-methylphosphonation), methylthiophosphonation, phosphorodithioation, phosphoroamidation, phosphorodiamidation, phosphoramidothioation, boranophosphation, phosphorylguanidination, mesylamidation, and the like. In addition, examples of modification of a phosphodiester bond moiety in the nucleotide are disclosed in Journal of Medicinal Chemistry, 2016, 59, pp 9645-9667, Medicinal Chemistry Communication, 2014, 5, pp 1454-1471, Future Medicinal Chemistry, 2011, 3, pp 339-365, RNA, 2023, 29, pp 423-433, and the like, and these can be used for a phosphodiester bond moiety in the deoxyribonucleotide, ribonucleotide, and sugar-modified nucleotide.

"Bridged nucleotide" is a sugar-modified nucleotide in which a bridging unit is introduced at two positions in a sugar moiety, and examples thereof include a 2'-4'-bridged nucleotide, and the like.

"Acyclic nucleotide" refers to any nucleotide having an acyclic structure in place of a ribonucleotide or deoxyribonucleotide sugar. For example, in an acyclic nucleotide, any of the bonds between ribose carbons (for example, C1'-C2', C2'-C3', C3'-C4', C4'-O4', or C1'-O4') are absent, and/or at least one of the ribose carbon atoms or oxygen atoms (for example, C1', C2', C3', C4', or O4') is independently or in combination absent from the nucleotide. In some embodiments, the acyclic nucleotide is represented by the following Formula (V): 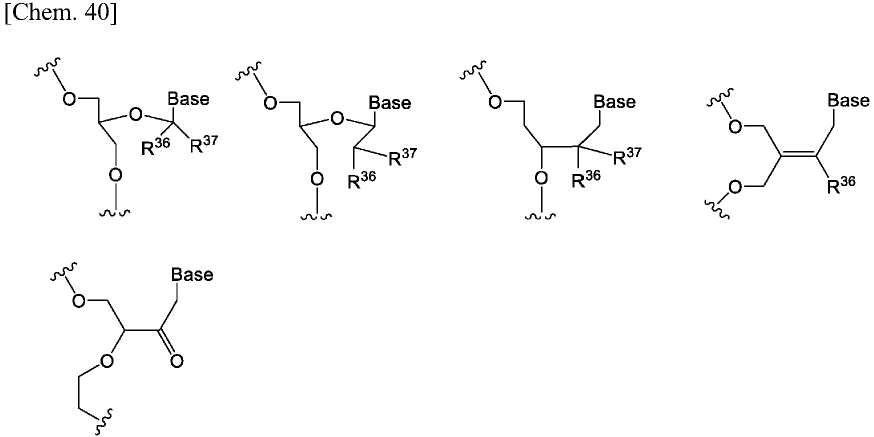 (in Formula (V), Base has the same meaning as Base in Formula (I), and R³⁶ and R³⁷ are independently a hydrogen atom, halogen, a hydroxy group, a C1-6 alkoxy group, or a C1-6 alkyl group).

"UNA" is referred to as an unlocked acyclic nucleic acid, and is a type of acyclic nucleotide herein. In UNA, any one of the sugar bonds in a ribonucleotide or a deoxyribonucleotide is removed to form an unlocked "sugar" residue. In one example, UNA encompasses monomers in which the bond between C1' and C4' (that is, the covalent carbon-oxygen-carbon bond between the C1' and C4' carbons) has been removed. In another example, UNA encompasses monomers from which the C2'-C3' bond of a sugar (that is, the covalent carbon-carbon bond between the C2' and C3' carbons) can be removed (Tetrahedron Letters, 1985, 26 (17), p. 2059 and Mol. Biosyst., 2009, 10, p. 1039) (These are incorporated by reference herein in their entirety.). The acyclic structure provides backbone flexibility without affecting Watson-Crick pairing. UNAs can be linked by a 2'-5' or 3'-5' bond.

"GNA" is commonly referred to as a glycol nucleic acid and is a type of acyclic nucleotide herein. GNAs are composed of repeating units of glycerol linked by phosphodiester bonds or modified phosphodiester bonds. The "backbone" differs in composition, but is similar to DNA or RNA (deoxyribonucleotide or ribonucleotide). A typical structure thereof is represented by the following Formula (VI): 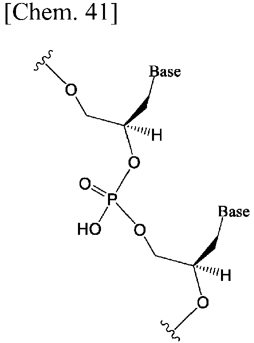 (in Formula (VI), Base has the same meaning as Base in Formula (I)).

The sugar-modified nucleotide is not limited to those exemplified herein. A large number of sugar-modified nucleotides are known in the art, and for example, the sugar-modified nucleotides described in US 8299039 to Tachas et al. (in particular columns 17 to 22), Journal of Medicinal Chemistry, 2016, 59, pp 9645-9667, Medicinal Chemistry Communication, 2014, 5, pp 1454-1471, Future Medicinal Chemistry, 2011, 3, pp 339-365, or the like can also be used as embodiments of the present invention.

Nucleobase is generally a base component constituting a nucleic acid, and includes, as natural nucleobases, purine bases: adenine (A) and guanine (G), and pyrimidine bases: thymine (T), cytosine (C), and uracil (U). For the base moiety of a deoxyribonucleotide, a ribonucleotide, and a sugar-modified nucleotide used herein, a natural nucleobase and a modified nucleobase thereof can be used. The modified nucleobase can form a base pair with any nucleobase (preferably, a base complementary to a nucleobase before modification) (that is, can form a hydrogen bond). Typically, the modified nucleobase includes 5-methylcytosine (5-me-C), 5-hydroxymethylcytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH3) uracil and cytosine and other alkynyl derivatives of pyrimidine bases, 6-azouracil, cytosine and thymine, 5-uracil (pseudouracil), 4-thiouracil, 8-halo, 8-amino, 8-thiol, 8-thioalkyl, 8-hydroxy, and other 8-substituted adenine and guanine, 5-halo, in particular 5-bromo, 5-trifluoromethyl and other 5-substituted uracil and cytosine, 7-methylguanine and 7-methyladenine, 2-F-adenine, 2-amino-adenine, 8-azaguanine and 8-azaadenine, 7-deazaguanine and 7-deazaadenine, 3-deazaguanine and 3-deazaadenine. Further modified nucleobases include tricyclic pyrimidines such as phenoxazine cytidine (1H-pyrimido[5,4-b][1,4]benzoxazin-2(3H)-one), phenothiazine cytidine (1H-pyrimido[5,4-b][1,4]benzothiazin-2(3H)-one), G-clamps such as substituted phenoxazine cytidine (for example, 9-(2-aminoethoxy)-H-pyrimido[5,4-b][1,4]benzoxazin-2(3H)-one), carbazole cytidine (2H-pyrimido[4,5-b]indol-2-one), and pyridoindole cytidine (H-pyrido[3',2':4,5]pyrrolo[2,3-d]pyrimidin-2-one). The modified nucleobase may include one in which a purine or pyrimidine base is replaced with another heterocyclic ring, for example, 7-deazaadenine, 7-deazaguanosine, 2-aminopyridine, and 2-pyridone. Examples of modification of a base moiety in the nucleotide are disclosed in Journal of Medicinal Chemistry, 2016, 59, pp 9645-9667, Medicinal Chemistry Communication, 2014, 5, pp 1454-1471, Future Medicinal Chemistry, 2011, 3, pp 339-365, WO 2007/090071 A, and the like, and these can be used for a base moiety in the deoxyribonucleotide, ribonucleotide, and sugar-modified nucleotide. The amino and hydroxy of the base moiety may each independently be protected.

The base moiety in the deoxyribonucleotide, ribonucleotide, and sugar-modified nucleotide is preferably at least one selected from the group consisting of adenine (A), guanine (G), thymine (T), cytosine (C), uracil (U), and 5-methylcytosine (5-me-C).

"Thermally destabilizing modification" is a modification to a double-stranded RNA that results in a double-stranded RNA with an overall lower melting temperature (Tm) (preferably, Tm which is 1, 2, 3 or 4 degrees lower) than the Tm of a double-stranded RNA without such one or more modifications. "Thermally destabilizing modification" may include, but is not limited to, abasic modifications, mismatches with opposing nucleotides in the opposite strand, 2'-deoxy modifications, acyclic modifications, and the like (for example, an unlocked nucleic acid (UNA) or a glycol nucleic acid (GNA)).

"Thermally destabilizing sugar modification" refers to a thermally destabilizing modification using a sugar-modified nucleotide, and may include the 2'-deoxy modification, acyclic modification, sugar modification, and the like.

"Abasic nucleotide" is a nucleotide having no nucleobase.

Abasic modification can be made by using an abasic nucleotide.

Nucleoside structures used as exemplary abasic modifications include, but are not limited to, the following Formula (VII): 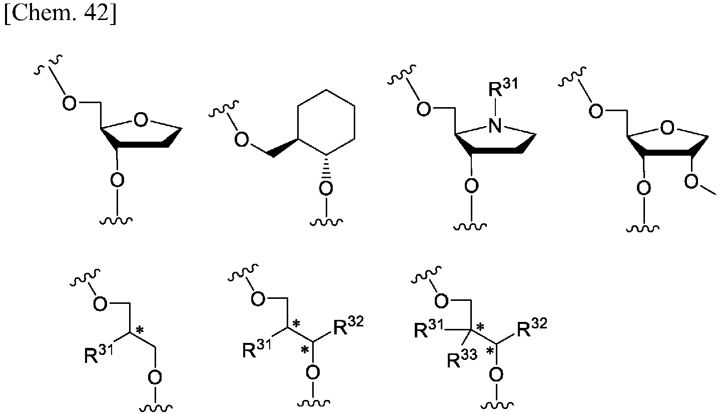 (in Formula (VII), R³¹ is a hydrogen atom, a methyl group, an ethyl group, or a methoxy group, R³² is a hydrogen atom, a methyl group, an ethyl group, or a methoxy group, R³³ is a hydrogen atom, a methyl group, an ethyl group, or a methoxy group, and * represents that the stereocenter is any one of R and S or a racemate).

Nucleoside structures used as exemplary thermally destabilizing sugar modifications include, but are not limited to, the following Formula (VIII): 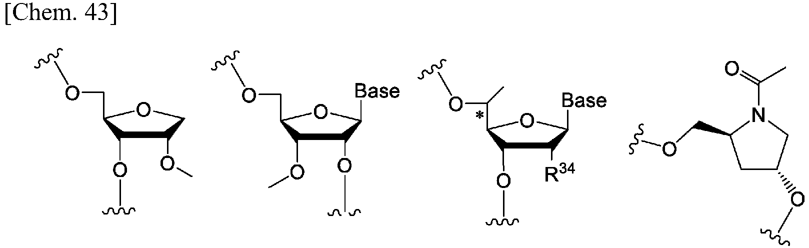 (in Formula (VIII), Base is as defined for Base in Formula (I); R³⁴ is fluorine or a methoxy group, and * represents that the stereocenter is any one of R and S or a racemate).

Examples of the nucleoside structure used as a further thermally destabilizing sugar modification include, but are not limited to, the following Formula (IX): 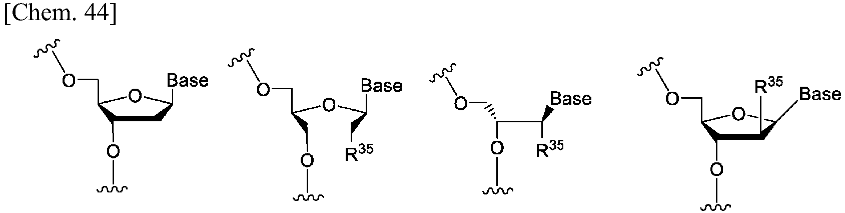
(in Formula (IX), Base has the same meaning as Base in Formula (I), and
R³⁵ is a hydrogen atom, a hydroxy group, or a C1-6 alkoxy group).

In some embodiments, the nucleoside structure used as the thermally destabilizing sugar modification may be a structure of the following Formula (X): 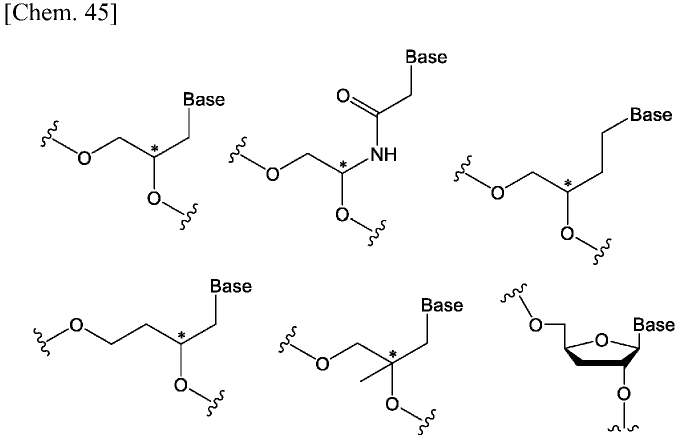 (in Formula (X), Base has the same meaning as Base in Formula (I), and * represents that the stereocenter is any one of R and S or a racemate).

Note that the wavy lines in Formulae (V) to (X) are understood as a bonding position for an internucleotide bond with an adjacent nucleotide, a bonding position with a functional molecule or a linker thereof, a bonding position with a hydrogen atom, a hydroxy protecting group, or a phosphorus-containing group, or the like, respectively.

The thermally destabilizing modification of a double-stranded RNA molecule can be a mismatch (that is, a non-complementary base pair) between a thermally destabilizing nucleotide within a dsRNA double strand and an opposing nucleotide of the opposite strand. Exemplary mismatched base pairs include G:G, G:A, G:U, G:T, A:A, A:C, C:C, C:U, C:T, U:U, T:T, U:T, or combinations thereof. Other mismatched base pairing known in the art is also suitable for the present invention. Mismatches can appear between any nucleotides, whether naturally occurring or modified, that is, mismatched base pairing can appear between nucleic acid bases from each nucleotide irrespective of modifications on the ribose sugar of the nucleotide.

In some embodiments, thermally destabilizing modifications in the seed region of the antisense strand include nucleotides that exhibit impaired Watson-Crick base pairing with complementary bases on the target RNA. Exemplary nucleotides in which Watson-Crick base pairing with a complementary base on the target RNA is impaired include, but are not limited to, nucleotides containing a nucleobase independently selected from the following Formula (XI):

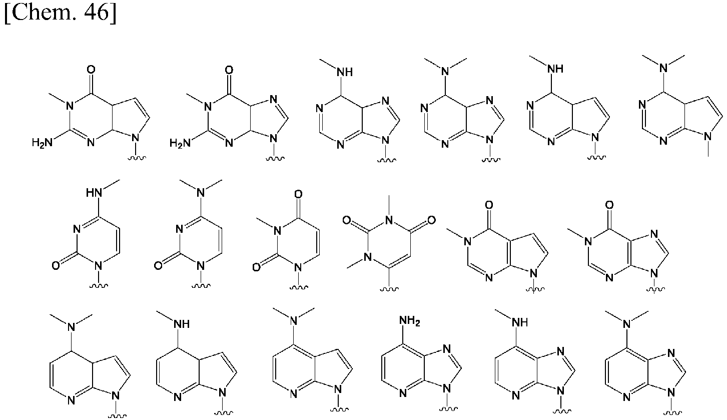

Further examples of abasic nucleotides, acyclic nucleotide modifications (including UNA and GNA), and mismatch modifications are described in detail in WO 2011/133876 A, which is incorporated herein by reference in its entirety.

Thermally destabilizing modifications can also include universal nucleobase and phosphodiester bond modifications that have reduced or eliminated the ability to form hydrogen bonds with opposing bases.

In some embodiments, thermally destabilizing modifications include nucleotides with non-standard bases, such as, but not limited to, nucleobase modifications that have impaired or completely abolished ability to form hydrogen bonds with bases of the opposite strand. These nucleobase modifications have been evaluated in terms of destabilization of the central region of the dsRNA double strand as described in WO 2010/0011895 A (incorporated herein by reference in its entirety). Exemplary such nucleobase modifications include those of Formula (XII):

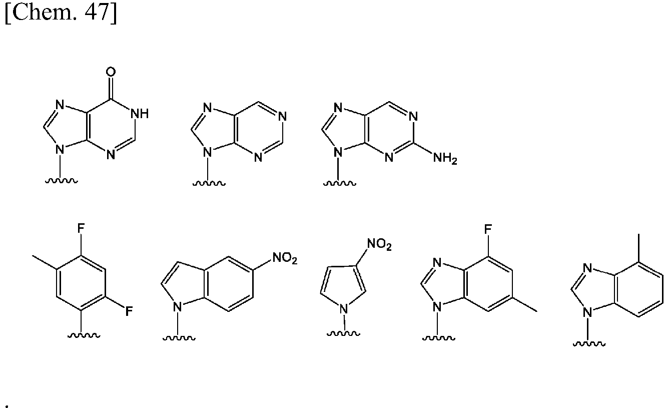

As such, some of the thermally destabilizing modifications may be included in the modified nucleobase.

Note that wavy lines in Formulae (XI) to (XII) are understood as a bonding position of a nucleotide with a sugar carbon atom, or the like, respectively.

In some embodiments, one or more thermally destabilizing modifications of the following Formula (XIII): 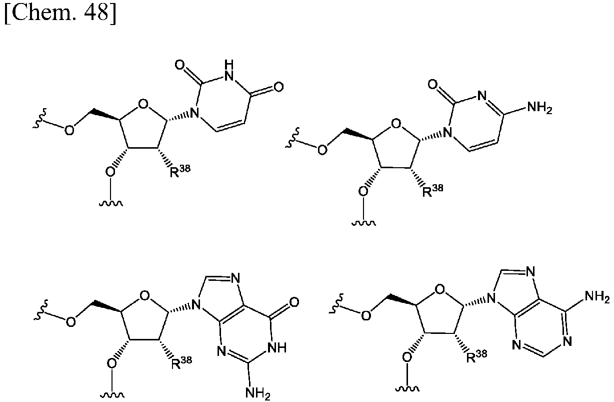
(in the formula, R³⁸ is a hydrogen atom, a hydroxy group, a C1-6 alkoxy group, fluorine, an amino group, an N-methylamino group, or an N,N'-dimethylamino group)
are included.

Note that the wavy lines in Formula (XIII) are understood as a bonding position for an internucleotide bond with an adjacent nucleotide, a bonding position with a functional molecule or a linker thereof, a bonding position with a hydrogen atom, a hydroxy protecting group, or a phosphorus-containing group, or the like.

Exemplary modifications of the phosphodiester bond known to decrease the thermal stability of a dsRNA double strand compared to a natural phosphodiester bond include, but are not limited to, the following Formula (XIV): 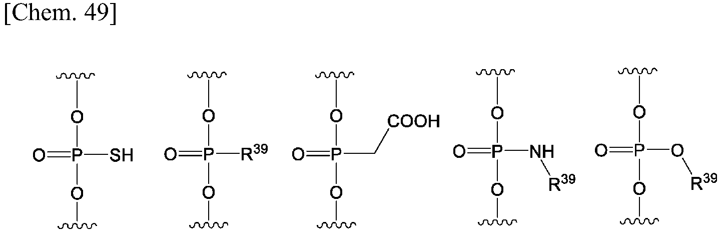 (in the formula, R³⁹ is a C1-6 alkyl group, and examples of the C1-6 alkyl group include methyl, ethyl, propyl, isopropyl, butyl, pentyl, and hexyl).

Note that wavy lines in Formula (XIV) are understood as a bonding position of a nucleotide with a sugar carbon atom, or the like, respectively.

As such, some of the thermally destabilizing modifications may be included in the modified phosphodiester bonds.

In some embodiments, the thermally destabilizing modification is selected from Formula (XV): 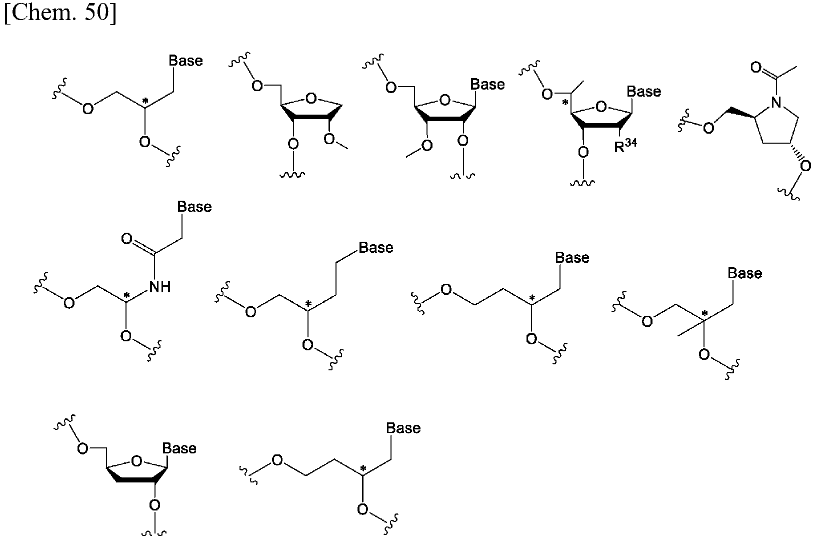 (in Formula (XV), Base has the same meaning as Base in Formula (I), and R³⁴ has the same meaning as R³⁴ in Formula (VIII)).

The wavy line in Formula (XV) is understood as a bonding position for an internucleotide bond with an adjacent nucleotide, a bonding position with a functional molecule or a linker thereof, or a bonding position with a hydrogen atom, a hydroxy protecting group, or a phosphorus-containing group.

When the "thermally destabilizing modification" constitutes each oligonucleotide strand included in the double-stranded RNA of the present invention, for example, position 3' of the thermally destabilizing modification is linked to another nucleotide or the like by a phosphodiester bond or a modified phosphodiester bond (for example, a phosphorothioate bond), and position 5' of the thermally destabilizing modification is linked to another nucleotide or the like by a phosphodiester bond or a modified phosphodiester bond (for example, a phosphorothioate bond). The thermally destabilizing modification at the 3' end of each oligonucleotide strand included in the double-stranded RNA of the present invention preferably has, for example, at its position 3', a hydroxy group, a phosphate group, or a modified phosphate group, and position 5' is as described above. The thermally destabilizing modification at the 5' end of each oligonucleotide strand included in the double-stranded RNA preferably has, for example, at its position 5', a hydroxy group, a phosphate group, or a modified phosphate group, and position 3' is as described above.

Some of the "thermally destabilizing modifications" may be included in the above-described "sugar-modified nucleotides".

Next, the double-stranded RNA of the present invention will be described.

Hereinafter, in the present specification, the double-stranded RNA may be referred to as "dsRNA".

In one aspect, the present invention provides a dsRNA, and the dsRNA is capable of inhibiting expression of a target RNA, the dsRNA including a sense strand (also referred to as a passenger strand) and an antisense strand (also referred to as a guide strand), in which the antisense strand has sufficient complementarity with a target sequence to mediate RNA interference, and the dsRNA comprises at least one 2'-O-XCE nucleotide.

Each strand of the dsRNA independently has a length of 14 to 40 nucleotides. For example, each strand independently has a length of 14 to 40, 17 to 37, 25 to 37, 27 to 30, 17 to 23, 17 to 21, 17 to 19, 19 to 25, 19 to 23, 19 to 21, 21 to 25, or 21 to 23 nucleotides. Without limitation, the sense strand and antisense strand can be equal or unequal in length. In some embodiments, the antisense strand is longer than the sense strand, for example, by a length of 1, 2, 3, 4, or 5 nucleotides.

In some embodiments, the antisense strand has a length of 18 to 35 nucleotides. In some embodiments, the antisense strand has a length of 21 to 25, 19 to 25, 19 to 21, or 21 to 23 nucleotides. In some specific embodiments, the antisense strand has a length of 23 nucleotides. In some specific embodiments, the antisense strand has a length of 22 nucleotides. In some other specific embodiments, the antisense strand has a length of 21 nucleotides.

Similarly to the antisense strand, in some embodiments, the sense strand has a length of 18 to 35 nucleotides. In some embodiments, the sense strand has a length of 21 to 25, 19 to 25, 19 to 21, or 21 to 23 nucleotides. In some specific embodiments, the sense strand has a length of 21 nucleotides. In some specific embodiments, the sense strand has a length of 20 nucleotides. In some specific embodiments, the sense strand has a length of 19 nucleotides.

In some specific embodiments, the sense strand has a length of 21 nucleotides, and the antisense strand has a length of 23 nucleotides. In some specific embodiments, the sense strand has a length of 20 nucleotides, and the antisense strand has a length of 23 nucleotides. In some specific embodiments, the sense strand has a length of 19 nucleotides, and the antisense strand has a length of 23 nucleotides. In some specific embodiments, the sense strand has a length of 20 nucleotides, and the antisense strand has a length of 22 nucleotides. In some specific embodiments, the sense strand has a length of 21 nucleotides, and the antisense strand has a length of 21 nucleotides. In some specific embodiments, the sense strand has a length of 19 nucleotides, and the antisense strand has a length of 21 nucleotides.

The double-stranded RNA has a hybridizing portion. Generally, the hybridizing portion has a length of 12 to 40 nucleotide base pairs. For example, dsRNA has a hybridizing portion having a length of 12 to 25 nucleotide pairs. In some embodiments, the dsRNA has a hybridizing portion having a length of 18, 19, 20, 21, 22, 22, 23, 24, or 25 nucleotide base pairs. In some specific embodiments, the dsRNA has a hybridizing portion having a length of 19, 20, 21, or 22 nucleotide base pairs. In further certain specific embodiments, the dsRNA has a hybridizing portion having a length of 21 nucleotide base pairs.

The 2'-O-XCE nucleotide may be present anywhere in the dsRNA. For example, the 2'-O-XCE nucleotide may be present in the sense strand of dsRNA. In general, 2'-O-XCE nucleotides may be present anywhere in the antisense strand. For example, the 2'-O-XCE nucleotide can be at the 5' end of the sense strand, at the 3' end of the sense strand, and/or at an internal position of the sense strand.

In some embodiments, the 2'-O-XCE nucleotide is present at the 5' end of the sense strand. In some embodiments, the 2'-O-XCE nucleotide is present at the 3' end of the sense strand.

In some embodiments, the 2'-O-XCE nucleotides may be present at the internal position of the sense strand. For example, the 2'-O-XCE nucleotide may be present at position 1, 2, 3, 4, 5, 6, 7, 8, 9, 10, 11, 12, 13, 14, 15, 16, 17, 18, 19, 20, 21, 22, 23, 24, or 25 as counted from the 5' end of the sense strand. In some embodiments, the 2'-O-XCE nucleotide may be present within the range of the 5' region (that is, positions 2 to 8 as counted from the 5' end of the sense strand). In some embodiments, the 2'-O-XCE nucleotide may be present in the central region of the sense strand.

In some embodiments, the 2'-O-XCE nucleotide may be present in the sense strand at a position opposite the seed region of the antisense strand (that is, positions 2 to 8 as counted from the 5' end of the antisense strand hybridizing portion).

In some embodiments, the sense strand includes at least 1, for example, 1, 2, 3, 4, 5, 6, 7, 8, 9, 10, 11, 12, 13, 14, 15, 16, 17, 18, 19, 20, 21, 22, 23, 24, or more 2'-O-XCE nucleotides. In some embodiments, the sense strand includes at least one 2'-O-XCE nucleotide. In some embodiments, the sense strand preferably includes at least one 2'-O-XCE nucleotide at positions 1, 2, 3, 4, 5, 6, 7, 8, 9, 10, 14, 15, 16, 17, 18, 19, 20, 21, 22, and 23 as counted from the 3' end of the sense strand hybridizing portion.

In some embodiments, the sense strand preferably includes the 2'-O-XCE nucleotide at least one of positions 1, 2, 3, 4, 5, 6, 7, 8, 9, 10, 14, 15, 16, 17, 18, 19, 20, and 21 as counted from the 3' end of the sense strand hybridizing portion. In some embodiments, the sense strand includes 1 to 10 (more preferably 2 to 10, still more preferably 2 to 4, even more preferably 2 or 4) 2'-O-XCE nucleotides. In some embodiments, the sense strand preferably includes the 2'-O-XCE nucleotide at least one of positions 1, 2, 20, and 21 as counted from the 3' end of the sense strand hybridizing portion. In some embodiments, the sense strand preferably includes the 2'-O-XCE nucleotide at least one of positions 1 and 21 as counted from the 3' end of the sense strand hybridizing portion. In some embodiments, the sense strand includes at least two 2'-O-XCE nucleotides. In some embodiments, the sense strand preferably includes the 2'-O-XCE nucleotide at least two of positions 1, 2, 3, 4, 5, 6, 7, 8, 9, 10, 14, 15, 16, 17, 18, 19, 20, and 21 as counted from the 3' end of the sense strand hybridizing portion. In some embodiments, the sense strand preferably includes the 2'-O-XCE nucleotide at least two of positions 1, 2, 20, and 21 as counted from the 3' end of the sense strand hybridizing portion. In some embodiments, the sense strand preferably includes the 2'-O-XCE nucleotides at positions 1 and 21 as counted from the 3' end of the sense strand hybridizing portion. In some embodiments, the sense strand includes at least three 2'-O-XCE nucleotides. In some embodiments, the sense strand preferably includes the 2'-O-XCE nucleotide at least three of positions 1, 2, 3, 4, 5, 6, 7, 8, 9, 10, 14, 15, 16, 17, 18, 19, 20, and 21 as counted from the 3' end of the sense strand hybridizing portion. In some embodiments, the sense strand preferably includes the 2'-O-XCE nucleotide at least three of positions 1, 2, 20, and 21 as counted from the 3' end of the sense strand hybridizing portion. In some embodiments, the sense strand includes at least four 2'-O-XCE nucleotides. In some embodiments, the sense strand preferably includes the 2'-O-XCE nucleotide at least four of positions 1, 2, 3, 4, 5, 6, 7, 8, 9, 10, 14, 15, 16, 17, 18, 19, 20, and 21 as counted from the 3' end of the sense strand hybridizing portion. In some embodiments, the sense strand preferably includes the 2'-O-XCE nucleotides at positions 1, 2, 20, and 21 as counted from the 3' end of the sense strand hybridizing portion.

In some embodiments, the sense strand preferably includes the 2'-O-XCE nucleotide at least one of positions 1 and 2 as counted from the 5' end of the sense strand, and more preferably includes the 2'-O-XCE nucleotides at positions 1 and 2 as counted from the 5' end of the sense strand. In some embodiments, the sense strand preferably includes the 2'-O-XCE nucleotide at least one of positions 1 and 2 as counted from the 3' end of the sense strand, and more preferably includes the 2'-O-XCE nucleotides at positions 1 and 2 as counted from the 3' end of the sense strand. In some embodiments, the sense strand preferably includes the 2'-O-XCE nucleotide at least one of positions 1 and 2 as counted from the 5' end and positions 1 and 2 as counted from the 3' end of the sense strand, and more preferably includes the 2'-O-XCE nucleotides at all of positions 1 and 2 as counted from the 5' end of the sense strand and positions 1 and 2 as counted from the 3' end of the sense strand.

In some embodiments, the sense strand preferably includes the 2'-O-XCE nucleotide at position 1 as counted from the 5' end of the sense strand. In some embodiments, the sense strand preferably includes the 2'-O-XCE nucleotide at position 1 as counted from the 3' end of the sense strand. In some embodiments, the sense strand preferably includes the 2'-O-XCE nucleotide at position 1 as counted from the 5' end of the sense strand and at position 1 as counted from the 3' end of the sense strand.

In some embodiments, the sense strand includes at least two 2'-O-XCE nucleotides. When two or more 2'-O-XCE nucleotides are present, they may be adjacent to each other. Thus, in some embodiments, the sense strand includes at least 2, for example, 3, 4, 5, 6, 7, 8, 9, 10, 11, 12, 13, 14, 15, 16, 17, 18, 19, 20 or more 2'-O-XCE nucleotides adjacent to each other.

In some embodiments, the sense strand includes three 2'-O-XCE nucleotides adjacent to each other.

In some embodiments, the 2'-O-XCE nucleotide is present in the antisense strand. In general, 2'-O-XCE nucleotides may be present anywhere in the antisense strand. For example, the 2'-O-XCE nucleotide can be at the 5' end of the antisense strand, at the 3' end of the antisense strand, and/or at an internal position of the antisense strand.

In some embodiments, the 2'-O-XCE nucleotide is present at the 5' end of the antisense strand. In some embodiments, the 2'-O-XCE nucleotide is present at the 3' end of the antisense strand.

In some embodiments, the 2'-O-XCE nucleotide may be present at the internal position of the antisense strand. For example, the 2'-O-XCE nucleotide may be present at position 1, 2, 3, 4, 5, 6, 7, 8, 9, 10, 11, 12, 13, 14, 15, 16, 17, 18, 19, 20, 21, 22, 23, 24, or 25 as counted from the 5' end of the antisense strand hybridizing portion. In some embodiments, the 2'-O-XCE nucleotides may be present within the range of seed region (that is, positions 2 to 8 as counted from the 5' end of the antisense strand hybridizing portion). Positions 2 to 8 as counted from the 5' end of the antisense strand hybridizing portion are referred to as seed regions. The seed region first recognizes the target RNA. When the thermally destabilizing modifications are added to the seed region, off-target action can be suppressed. For example, the antisense strand includes the 2'-O-XCE nucleotide at least one of positions 3 to 8 as counted from the 5' end of the antisense strand hybridizing portion. In some embodiments, the 2'-O-XCE nucleotide may be present in the central region of the antisense strand.

In some embodiments, the antisense strand includes at least 1, for example, 1, 2, 3, 4, 5, 6, 7, 8, 9, 10, 11, 12, 13, 14, 15, 16, 17, 18, 19, 20, 21, 22, 23, 24, or more 2'-O-XCE nucleotides. In some embodiments, the antisense strand includes at least one 2'-O-XCE nucleotide. In some embodiments, the antisense strand includes 1 to 10 (preferably 1 to 9, and more preferably 1, 2, 3 or 4) 2'-O-XCE nucleotides. In some embodiments, the antisense strand preferably includes the 2'-O-XCE nucleotide at least one of positions 1, 3, 4, 5, 6, 7, 8, 9, 10, 11, 12, 13, 15, 16, 17, 18, 19, 20, 21, 22, and 23 as counted from the 5' end of the antisense strand hybridizing portion. In some embodiments, the antisense strand preferably includes the 2'-O-XCE nucleotide at least one of positions 1, 3, 4, 5, 6, 7, 8, 9, and 10 as counted from the 5' end of the antisense strand hybridizing portion. In some embodiments, the antisense strand preferably includes the 2'-O-XCE nucleotide at least one of positions 1, 5, and 10 as counted from the 5' end of the antisense strand hybridizing portion. In some embodiments, the antisense strand includes at least two 2'-O-XCE nucleotides. In some embodiments, the antisense strand preferably includes the 2'-O-XCE nucleotides at least two of positions 1, 3, 4, 5, 6, 7, 8, 9, 10, 11, 12, 13, 15, 16, 17, 18, 19, 20, 21, 22, and 23 as counted from the 5' end of the antisense strand hybridizing portion. In some embodiments, the antisense strand preferably includes the 2'-O-XCE nucleotides at least two of positions 1, 3, 4, 5, 6, 7, 8, 9, and 10 as counted from the 5' end of the antisense strand hybridizing portion. In some embodiments, the antisense strand preferably includes the 2'-O-XCE nucleotides at least two of positions 1, 5, and 10 as counted from the 5' end of the antisense strand hybridizing portion. In some embodiments, the antisense strand preferably includes the 2'-O-XCE nucleotides at positions 1 and 3 as counted from the 5' end of the antisense strand hybridizing portion. In some embodiments, the antisense strand preferably includes the 2'-O-XCE nucleotides at positions 1 and 4 as counted from the 5' end of the antisense strand hybridizing portion. In some embodiments, the antisense strand preferably includes the 2'-O-XCE nucleotides at positions 1 and 5 as counted from the 5' end of the antisense strand hybridizing portion. In some embodiments, the antisense strand preferably includes the 2'-O-XCE nucleotides at positions 1 and 6 as counted from the 5' end of the antisense strand hybridizing portion. In some embodiments, the antisense strand preferably includes the 2'-O-XCE nucleotides at positions 1 and 7 as counted from the 5' end of the antisense strand hybridizing portion. In some embodiments, the antisense strand preferably includes the 2'-O-XCE nucleotides at positions 1 and 8 as counted from the 5' end of the antisense strand hybridizing portion. In some embodiments, the antisense strand preferably includes the 2'-O-XCE nucleotides at positions 1 and 9 as counted from the 5' end of the antisense strand hybridizing portion. In some embodiments, the antisense strand preferably includes the 2'-O-XCE nucleotides at positions 1 and 10 as counted from the 5' end of the antisense strand hybridizing portion. In some embodiments, the antisense strand preferably includes the 2'-O-XCE nucleotides at positions 5 and 10 as counted from the 5' end of the antisense strand hybridizing portion. In some embodiments, the antisense strand includes at least three 2'-O-XCE nucleotides. In some embodiments, the antisense strand preferably includes the 2'-O-XCE nucleotides at least three of positions 1, 3, 4, 5, 6, 7, 8, 9, 10, 11, 12, 13, 15, 16, 17, 18, 19, 20, 21, 22, and 23 as counted from the 5' end of the antisense strand hybridizing portion. In some embodiments, the antisense strand more preferably includes the 2'-O-XCE nucleotides at least three of positions 1, 3, 4, 5, 6, 7, 8, 9, and 10 as counted from the 5' end of the antisense strand hybridizing portion. In some embodiments, the antisense strand particularly preferably includes the 2'-O-XCE nucleotides at positions 1, 5, and 10 as counted from the 5' end of the antisense strand hybridizing portion.

In some embodiments, the antisense strand includes at least two or more 2'-O-XCE nucleotides. When two or more 2'-O-XCE nucleotides are present, they may be adjacent to each other. Thus, in some embodiments, the antisense strand includes at least 2, for example, 3, 4, 5, 6, 7, 8, 9, 10, 11, 12, 13, 14, 15, 16, 17, 18, 19, 20 or more 2'-O-XCE nucleotides adjacent to each other.

In some embodiments, the antisense strand includes three 2'-O-XCE nucleotides adjacent to each other.

In some embodiments, the 2'-O-XCE nucleotide is a 2'-O-MCE.

In some embodiments, the 2'-O-XCE nucleotide is 2'-O-DMAECE.

In some embodiments, the 2'-O-XCE nucleotide is 2'-O-MorECE.

In some embodiments, the 2'-O-XCE nucleotide is 2'-O-PyECE.

In some embodiments, the 2'-O-XCE nucleotide is 2'-O-BimECE.

When the dsRNA comprises at least two or more 2'-O-XCE nucleotides, the 2'-O-XCE nucleotides may be identical or different.

The double-stranded RNA described in the present specification may include one or more sugar-modified nucleotides. It is noted that the double-stranded RNA comprises one or more sugar-modified nucleotides in addition to one or more 2'-O-XCE nucleotides.

In some embodiments, the dsRNA may include a 2'-fluoro nucleotide. For example, the dsRNA may include at least 1, for example, 2, 3, 4, 5, 6, 7, 8, 9, 10, 11, 12, 13, 14, 15 or more 2'-fluoro nucleotides. Without limitation, 2'-fluoro nucleotides may all be present in one strand. In some embodiments, both the sense strand and antisense strand include at least two 2'-fluoro nucleotides. 2'-Fluoro nucleotide may appear on any nucleotide of the sense or antisense strand. For example, a 2'-fluoro nucleotide may appear on all nucleotides on the sense and/or antisense strand; each 2'-fluoro nucleotide can appear in an alternating pattern on the sense or antisense strand; or both the sense strand and antisense strand contain 2'-fluoro nucleotides in an alternating pattern. The alternating pattern of 2'-fluoro nucleotides on the sense strand may be the same or different from that on the antisense strand, and the alternating pattern of 2'-fluoro nucleotides on the sense strand may be shifted relative to the alternating pattern of 2'-fluoro nucleotides on the antisense strand.

The antisense strand of the dsRNA may include at least one (for example, 1, 2, 3, 4, 5, 6, 7, 8, 9, 10 or more) 2'-fluoro nucleotide. In some embodiments, the antisense strand includes one, two, three, four, five, or six 2'-fluoro nucleotides. Without limitation, the 2'-fluoro nucleotide of the antisense strand may be present at any position. In some embodiments, the antisense strand includes at least one 2'-fluoro nucleotide. For example, the antisense strand includes a 2'-fluoro nucleotide at least one position selected from positions 2, 6, 8, 9, 14 and 16 as counted from the 5' end of the antisense strand hybridizing portion. Preferably, the antisense strand includes a 2'-fluoro nucleotide at least one position selected from position 2 or position 14 as counted from the 5' end of the antisense strand hybridizing portion. In some embodiments, the antisense strand includes at least two 2'-fluoro nucleotides. For example, the antisense strand includes a 2'-fluoro nucleotide at least two positions selected from positions 2, 6, 8, 9, 14 and 16 as counted from the 5' end of the antisense strand hybridizing portion. Preferably, the antisense strand includes 2'-fluoro nucleotides at positions 2 and 14 as counted from the 5' end of the antisense strand hybridizing portion. In some embodiments, the antisense strand includes at least three 2'-fluoro nucleotides. For example, the antisense strand includes 2'-fluoro nucleotides at least three positions selected from positions 2, 6, 8, 9, 14 and 16 as counted from the 5' end of the antisense strand hybridizing portion. Preferably, the antisense strand includes 2'-fluoro nucleotides at positions 2, 14 and 16 as counted from the 5' end of the antisense strand hybridizing portion. In some other embodiments, the antisense strand includes at least four 2'-fluoro nucleotides. For example, the antisense strand includes 2'-fluoro nucleotides at least four positions selected from 2, 6, 8, 9, 14 and 16 as counted from the 5' end of the antisense strand hybridizing portion. Preferably, the antisense strand includes 2'-fluoro nucleotides at positions 2, 6, 14 and 16 as counted from the 5' end of the antisense strand hybridizing portion. In some other embodiments, the antisense strand includes at least five 2'-fluoro nucleotides. For example, the antisense strand includes 2'-fluoro nucleotides at least five positions selected from 2, 6, 8, 9, 14 and 16 as counted from the 5' end of the antisense strand hybridizing portion. Preferably, the antisense strand includes 2'-fluoro nucleotides at positions 2, 6, 9, 14 and 16 as counted from the 5' end of the antisense strand hybridizing portion. In some other embodiments, the antisense strand includes at least six 2'-fluoro nucleotides. For example, the antisense strand includes 2'-fluoro nucleotides at positions 2, 6, 8, 9, 14 and 16 as counted from the 5' end of the antisense strand hybridizing portion.

The sense strand of the dsRNA may include at least one (for example, 1, 2, 3, 4, 5, 6, 7, 8, 9, 10 or more) 2'-fluoro nucleotide. In some embodiments, the sense strand includes one, two, three, four, or five 2'-fluoro nucleotides. Without limitation, the 2'-fluoro nucleotide of the sense strand may be present at any position. In some embodiments, the sense strand includes at least one 2'-fluoro nucleotide. For example, the sense strand includes a 2'-fluoro nucleotide in one position selected from positions 11, 12, 13, or 15 as counted from the 3' end of the sense strand hybridizing portion. In some embodiments, the sense strand includes at least two 2'-fluoro nucleotides. For example, the sense strand includes a 2'-fluoro nucleotide at two positions selected from positions 11, 12, 13, or 15 as counted from the 3' end of the sense strand hybridizing portion. In some embodiments, the sense strand includes at least three 2'-fluoro nucleotides. For example, the sense strand includes 2'-fluoro nucleotides at three positions selected from positions 11, 12, 13, or 15 as counted from the 3' end of the sense strand hybridizing portion. In some other embodiments, the sense strand includes at least four 2'-fluoro nucleotides. For example, the sense strand includes 2'-fluoro nucleotides at positions 11, 12, 13, and 15 as counted from the 3' end of the sense strand hybridizing portion.

In some embodiments, the sense strand includes a 2'-fluoro nucleotide at least one of the positions opposite or complementary to position 11, 12, or 13 as counted from the 5' end of the antisense strand hybridizing portion. In some embodiments, the sense strand includes a 2'-fluoro nucleotide at least one position opposite or complementary to position 11, 12, or 15 of the antisense strand as counted from the 5' end of the antisense strand hybridizing portion. In some other embodiments, the sense strand includes a 2'-fluoro nucleotide at least one of the positions opposite or complementary to position 11, 12, 13, or 15 of the antisense strand as counted from the 5' end of the antisense strand hybridizing portion.

In some embodiments, the sense strand includes 2'-fluoro nucleotides at positions 11 and 12 starting from the 3' end of the sense strand hybridizing portion, and the antisense strand includes 2'-fluoro nucleotides at positions 2 and 14 starting from the 5' end of the antisense strand hybridizing portion. In some embodiments, the sense strand includes 2'-fluoro nucleotides at positions 11 and 13 starting from the 3' end of the sense strand hybridizing portion, and the antisense strand includes 2'-fluoro nucleotides at positions 2 and 14 starting from the 5' end of the antisense strand hybridizing portion. In some embodiments, the sense strand includes 2'-fluoro nucleotides at positions 11 and 13 starting from the 3' end of the sense strand hybridizing portion, and the antisense strand includes 2'-fluoro nucleotides at positions 2 and 14 starting from the 5' end of the antisense strand hybridizing portion. In some embodiments, the sense strand includes 2'-fluoro nucleotides at positions 11, 12, and 13 as counted from the 3' end of the sense strand hybridizing portion, and the antisense strand includes 2'-fluoro nucleotides at positions 2 and 14 as counted from the 5' end of the antisense strand hybridizing portion. In some other embodiments, the sense strand includes 2'-fluoro nucleotides at positions 11, 13, and 15 as counted from the 3' end of the sense strand hybridizing portion, and the antisense strand includes 2'-fluoro nucleotides at positions 2, 14, and 16 as counted from the 5' end of the antisense strand hybridizing portion. In some other embodiments, the sense strand includes 2'-fluoro nucleotides at positions 11, 13, and 15 as counted from the 3' end of the sense strand hybridizing portion, and the antisense strand includes 2'-fluoro nucleotides at positions 2, 6, 9, 14, and 16 as counted from the 5' end of the antisense strand hybridizing portion. In still some other embodiments, the sense strand includes 2'-fluoro nucleotides at least positions 11, 13, and 15 from the 3' end of the sense strand hybridizing portion, and the antisense strand includes 2'-fluoro nucleotides at least positions 2, 6, 8, 9, 14, and 16 from the 5' end of the antisense strand hybridizing portion.

In some embodiments, the sense strand includes 2'-fluoro nucleotides at positions 11, 12, and 15 as counted from the 3' end of the sense strand hybridizing portion, and the antisense strand includes 2'-fluoro nucleotides at positions 2 and 14 as counted from the 5' end of the antisense strand hybridizing portion. In some embodiments, the sense strand includes 2'-fluoro nucleotides at positions 11, 13, and 15 as counted from the 3' end of the sense strand hybridizing portion, and the antisense strand includes 2'-fluoro nucleotides at positions 2 and 14 as counted from the 5' end of the antisense strand hybridizing portion. In some embodiments, the sense strand includes 2'-fluoro nucleotides at positions 11, 13, and 15 as counted from the 3' end of the sense strand hybridizing portion, and the antisense strand includes 2'-fluoro nucleotides at positions 2 and 14 as counted from the 5' end of the antisense strand hybridizing portion. In some embodiments, the sense strand includes 2'-fluoro nucleotides at positions 11, 12, 13, and 15 as counted from the 3' end of the sense strand hybridizing portion, and the antisense strand includes 2'-fluoro nucleotides at positions 2 and 14 as counted from the 5' end of the antisense strand hybridizing portion. In some embodiments, the sense strand includes 2'-fluoro nucleotides at positions 11, 12, and 15 as counted from the 3' end of the sense strand hybridizing portion, and the antisense strand includes 2'-fluoro nucleotides at positions 2, 14, and 16 as counted from the 5' end of the antisense strand hybridizing portion. In some embodiments, the sense strand includes 2'-fluoro nucleotides at positions 11, 13, and 15 as counted from the 3' end of the sense strand hybridizing portion, and the antisense strand includes 2'-fluoro nucleotides at positions 2, 14, and 16 as counted from the 5' end of the antisense strand hybridizing portion. In some embodiments, the sense strand includes 2'-fluoro nucleotides at positions 11, 13, and 15 as counted from the 3' end of the sense strand hybridizing portion, and the antisense strand includes 2'-fluoro nucleotides at positions 2, 14, and 16 as counted from the 5' end of the antisense strand hybridizing portion. In some embodiments, the sense strand includes 2'-fluoro nucleotides at positions 11, 12, 13, and 15 as counted from the 3' end of the sense strand hybridizing portion, and the antisense strand includes 2'-fluoro nucleotides at positions 2, 14, and 16 as counted from the 5' end of the antisense strand hybridizing portion. In some embodiments, the sense strand includes 2'-fluoro nucleotides at positions 11, 12, 13, and 15 as counted from the 3' end of the sense strand hybridizing portion, and the antisense strand includes 2'-fluoro nucleotides at positions 2, 14, and 16 as counted from the 5' end of the antisense strand hybridizing portion. In some other embodiments, the sense strand includes 2'-fluoro nucleotides at positions 11, 12, 13, and 15 as counted from the 3' end of the sense strand hybridizing portion, and the antisense strand includes 2'-fluoro nucleotides at positions 2, 6, 9, 14, and 16 as counted from the 5' end of the antisense strand hybridizing portion. In still some other embodiments, the sense strand includes 2'-fluoro nucleotides at positions 11, 12, 13, and 15 as counted from the 3' end of the sense strand hybridizing portion, and the antisense strand includes 2'-fluoro nucleotides at positions 2, 6, 8, 9, 14, and 16 as counted from the 5' end of the antisense strand hybridizing portion.

In some embodiments, the antisense strand does not include the 2'-fluoro nucleotides at positions 3 to 9 as counted from the 5' end of the antisense strand hybridizing portion.

The dsRNA may include at least 1, for example, 1, 2, 3, 4, 5, 6, 7, 8, 9, 10, 11, 12, 13, 14, 15, 16, 17, 18, 19, 20 or more 2'-O-Me nucleotides. Without limitation, the 2'-O-Me nucleotides may all be present in one strand. In some embodiments, both the sense strand and antisense strand include at least one 2'-O-Me nucleotide. The 2'-O-Me nucleotide may appear on any nucleotide of the sense or antisense strand. For example, a 2'-O-Me nucleotide may appear on all nucleotides on the sense and/or antisense strand; 2'-O-Me nucleotides may appear in an alternating pattern on the sense or antisense strand; or both the sense strand and antisense strand contain 2'-O-Me nucleotides in an alternating pattern. The alternating pattern of 2'-O-Me nucleotides on the sense strand can be the same as or different from the antisense strand, and the alternating pattern of 2'-O-Me nucleotides on the sense strand can be shifted relative to the alternating pattern of 2'-O-Me nucleotides on the antisense strand.

The antisense strand of the dsRNA may include at least 1, for example, 2, 3, 4, 5, 6, 7, 8, 9, 10, 11, 12, 13, 14, 15, 16, 17 or more 2'-O-Me nucleotides. Without limitation, the 2'-O-Me nucleotide of the antisense strand may be at any position. In some embodiments, the antisense strand includes at least three 2'-O-Me nucleotides.

In some embodiments, the antisense strand does not include the 2'-O-Me nucleotide at positions 2 and 14 as counted from the 5' end of the antisense strand hybridizing portion. For example, the antisense strand does not include 2'-O-Me nucleotides at positions 2, 14, and 16 as counted from the 5' end of the antisense strand hybridizing portion. In some other embodiments, the antisense strand does not include 2'-O-Me nucleotides at positions 2, 6, 14, and 16 as counted from the 5' end of the antisense strand hybridizing portion. In some other embodiments, the antisense strand does not include 2'-O-Me nucleotides at positions 2, 6, 9, 14, and 16 as counted from the 5' end of the antisense strand hybridizing portion. In still some other embodiments, the antisense strand does not include 2'-O-Me nucleotides at positions 2, 6, 8, 9, 14, and 16 as counted from the 5' end of the antisense strand hybridizing portion.

The sense strand of the dsRNA may include at least 1, for example, 2, 3, 4, 5, 6, 7, 8, 9, 10, 11, 12, 13, 14, 15, 16, or more 2'-O-Me nucleotides. Without limitation, the 2'-O-Me nucleotide of the sense strand may be present at any position. In some embodiments, the sense strand does not include 2'-O-Me nucleotides at positions 11, 12, and 13 as counted from the 3' end of the sense strand hybridizing portion. In some other embodiments, the sense strand does not include 2'-O-Me nucleotides at positions 11, 12, and 15 as counted from the 3' end of the sense strand hybridizing portion. In some other embodiments, the sense strand does not include 2'-O-Me nucleotides at positions 11, 12, 13, and 15 as counted from the 3' end of the sense strand hybridizing portion. In some embodiments, the sense strand includes a 2'-O-Me nucleotide at least one position selected from positions 11, 12, and 13 as counted from the 3' end of the sense strand hybridizing portion. In some embodiments, the sense strand includes a 2'-O-Me nucleotide at position 11 as counted from the 3' end of the sense strand hybridizing portion. In some embodiments, the sense strand includes a 2'-O-Me nucleotide at position 12 as counted from the 3' end of the sense strand hybridizing portion. In some embodiments, the sense strand includes a 2'-O-Me nucleotide at position 13 as counted from the 3' end of the sense strand hybridizing portion.

The dsRNA may additionally include 2'-modified nucleotides except 2'-O-XCE nucleotides, 2'-fluoro nucleotides and 2'-O-Me nucleotides. For example, the dsRNA may include at least 1, for example, 1, 2, 3, 4, 5, 6, 7, 8, 9, 10, 11, 12, 13, 14, 15, 16, 17, 18, 19, 20 or more 2'-modified nucleotides. Without limitation, the 2'-modified nucleotides may all be present in one strand. In some embodiments, both the sense strand and antisense strand include at least one 2'-modified nucleotides. The 2'-modified nucleotide may appear on any nucleotide of the sense or antisense strand.

For example, a 2'-modified nucleotide may appear on all nucleotides on the sense and/or antisense strand; each 2'-modified nucleotide may appear in an alternating pattern on the sense or antisense strand; or both the sense strand and antisense strand include 2'-modified nucleotides in an alternating pattern. The alternating pattern of a 2'-modified nucleotide on the sense strand can be the same as or different from the antisense strand, and the alternating pattern of 2' modified nucleotides on the sense strand can be shifted relative to the alternating pattern of a 2'-modified nucleotide on the antisense strand.

The antisense strand of the dsRNA may include at least 1, for example, 2, 3, 4, 5, 6, 7, 8, 9, 10, or more 2'-modified nucleotides. Without limitation, the 2'-modified nucleotide of the antisense strand may be present at any position.

The sense strand of the dsRNA may include at least 1, for example, 2, 3, 4, 5, 6, 7, 8, 9, 10, or more 2'-modified nucleotides. Without limitation, the 2'-modified nucleotide of the sense strand may be present at any position. In some embodiments, the sense strand includes at least 1, for example, 2, 3, 4, 5, 6, 7, 8, 9, 10, or more 2'-modified nucleotides, and the antisense strand does not include 2'-fluoro nucleotides at positions 3 to 9 as counted from the 5' end of the antisense strand hybridizing portion.

The dsRNA may include 2'-4'-bridged nucleotides. For example, the dsRNA may include at least 1, for example, 1, 2, 3, 4, 5, 6, 7, 8, 9, 10, 11, 12, 13, 14, 15, 16, 17, 18, 19, 20 or more 2'-4'-bridged nucleotides. Without limitation, 2'-4'-bridged nucleotides may all be present in one strand. In some embodiments, both the sense strand and antisense strand include at least one 2'-4'-bridged nucleotide. The 2'-4'-bridged nucleotide may appear on any nucleotide of the sense or antisense strand. For example, a 2'-4'-bridged nucleotide may appear on all nucleotides on the sense and/or antisense strands; each 2'-4'-bridged nucleotide may appear in an alternating pattern on the sense or antisense strand; or both the sense strand and antisense strand contain 2'-4'-bridged nucleotides in an alternating pattern. The alternating pattern of 2'-4'-bridged nucleotides on the sense strand may be the same as or different from that on the antisense strand, and the alternating pattern of 2'-4'-bridged nucleotides on the sense strand may be shifted relative to the alternating pattern of 2'-4'-bridged nucleotides on the antisense strand.

The antisense strand of the dsRNA may include at least 1, for example, 2, 3, 4, 5, 6, 7, 8, 9, 10, or more 2'-4'-bridged nucleotides. Without limitation, the 2'-4'-bridged nucleotide of the antisense strand may be present at any position.

The sense strand of the dsRNA may include at least 1, for example, 2, 3, 4, 5, 6, 7, 8, 9, 10, or more 2'-4'-bridged nucleotides. Without limitation, the 2'-4'-bridged nucleotide of the sense strand may be present at any position. In some embodiments, the sense strand includes at least 1, for example, 2, 3, 4, 5, 6, 7, 8, 9, 10, or more 2'-4'-bridged nucleotides, and the antisense strand does not include 2'-fluoro nucleotides at positions 3 to 9 as counted from the 5' end of the antisense strand hybridizing portion.

In some embodiments, the dsRNA may include deoxyribonucleotides. For example, the dsRNA may include at least 1, for example, 2, 3, 4, 5, 6, 7, 8, 9, 10, 11, 12, 13, 14, 15 or more deoxyribonucleotides. Without limitation, deoxyribonucleotides may all be present in one strand. In some embodiments, both the sense strand and antisense strand include at least two deoxyribonucleotides. Deoxyribonucleotides may appear on any nucleotide of the sense or antisense strand. For example, deoxyribonucleotides may appear on all nucleotides on the sense and/or antisense strands; each deoxyribonucleotide can appear in an alternating pattern on the sense or antisense strand; or both sense strand and antisense strand contain deoxyribonucleotides in alternating patterns. The alternating pattern of deoxyribonucleotides on the sense strand may be the same as or different from that on the antisense strand, and the alternating pattern of deoxyribonucleotides on the sense strand may be shifted relative to the alternating pattern of deoxyribonucleotides on the antisense strand.

The antisense strand of the dsRNA may include at least one (for example, 1, 2, 3, 4, 5, 6, 7, 8, 9, 10 or more) deoxyribonucleotide. In some embodiments, the antisense strand includes 1, 2, 3, 4, 5, or 6 deoxyribonucleotides. Without limitation, the deoxyribonucleotide of the antisense strand may be present at any position. In some embodiments, the antisense strand includes at least one deoxyribonucleotide.

For example, the antisense strand includes a deoxyribonucleotide at least one position (for example 1 to 6, preferably 2 to 6, 3 to 5) selected from positions 2, 5, 6, 7, 8, 9, 12, 14, and 16 as counted from the 5' end of the antisense strand hybridizing portion. Preferably, the antisense strand includes a deoxyribonucleotide at least one position (for example 1 to 6, preferably 2 to 6, 3 to 5) selected from positions 2, 5, 7, 12, 14 and 16 as counted from the 5' end of the antisense strand hybridizing portion. In some embodiments, the antisense strand includes at least two deoxyribonucleotides. For example, the antisense strand includes deoxyribonucleotides at least two positions selected from positions 2, 5, 6, 7, 8, 9, 12, 14 and 16 as counted from the 5' end of the antisense strand hybridizing portion. Preferably, the antisense strand includes deoxyribonucleotides at least two positions selected from positions 2, 5, 7, 12, 14 and 16 as counted from the 5' end of the antisense strand hybridizing portion. In some embodiments, the antisense strand includes at least three deoxyribonucleotides. For example, the antisense strand includes deoxyribonucleotides at least three positions selected from positions 2, 5, 6, 7, 8, 9, 12, 14 and 16 as counted from the 5' end of the antisense strand hybridizing portion. Preferably, the antisense strand includes deoxyribonucleotides at least three positions selected from positions 2, 5, 7, 12, 14 and 16 as counted from the 5' end of the antisense strand hybridizing portion. In some other embodiments, the antisense strand includes at least four deoxyribonucleotides. For example, the antisense strand includes deoxyribonucleotides at least four positions selected from positions 2, 5, 6, 7, 8, 9, 12, 14 and 16 as counted from the 5' end of the antisense strand hybridizing portion. Preferably, the antisense strand includes deoxyribonucleotides at least four positions selected from positions 2, 5, 7, 12, 14 and 16 as counted from the 5' end of the antisense strand hybridizing portion. In some other embodiments, the antisense strand includes at least 5 deoxyribonucleotides. For example, the antisense strand includes deoxyribonucleotides at least five positions selected from positions 2, 5, 6, 7, 8, 9, 12, 14 and 16 as counted from the 5' end of the antisense strand hybridizing portion. Preferably, the antisense strand includes deoxyribonucleotides at least five positions selected from positions 2, 5, 7, 12, 14 and 16 as counted from the 5' end of the antisense strand hybridizing portion. In some other embodiments, the antisense strand includes at least 6 deoxyribonucleotides. For example, the antisense strand includes deoxyribonucleotides at least six positions selected from positions 2, 5, 6, 7, 8, 9, 12, 14 and 16 as counted from the 5' end of the antisense strand hybridizing portion. Preferably, the antisense strand includes deoxyribonucleotides at positions 2, 5, 7, 12, 14 and 16 as counted from the 5' end of the antisense strand hybridizing portion.

In some other embodiments, deoxyribonucleotides are contained at 1 to 4 (preferably 2, 3, or 4) positions selected from positions 2, 5, 7, and 12. In some other embodiments, one or two selected from positions 5 and 7 include a deoxyribonucleotide. In some other embodiments, the antisense strand may not include deoxyribonucleotides.

The sense strand of the dsRNA may include at least one (for example, 1, 2, 3, 4, 5, 6, 7, 8, 9, 10 or more) deoxyribonucleotide. In some embodiments, the sense strand includes 1, 2, 3, 4, or 5 deoxyribonucleotides. Without limitation, the deoxyribonucleotide of the sense strand may be present at any position. In some embodiments, the sense strand includes at least one deoxyribonucleotide. For example, the sense strand includes a deoxyribonucleotide at one position selected from positions 11, 12, 13, or 15 as counted from the 3' end of the sense strand hybridizing portion. In some embodiments, the sense strand includes a deoxyribonucleotide at position 11 as counted from the 3' end of the sense strand hybridizing portion. In some embodiments, the sense strand includes a deoxyribonucleotide at position 12 as counted from the 3' end of the sense strand hybridizing portion. In some embodiments, the sense strand includes a deoxyribonucleotide at position 13 as counted from the 3' end of the sense strand hybridizing portion. In some embodiments, the sense strand includes at least two deoxyribonucleotides. For example, the sense strand includes deoxyribonucleotides at two positions selected from positions 11, 12, 13, or 15 as counted from the 3' end of the sense strand hybridizing portion. In some embodiments, the antisense strand includes at least three deoxyribonucleotides. For example, the sense strand includes deoxyribonucleotides at three positions selected from positions 11, 12, 13, or 15 as counted from the 3' end of the sense strand hybridizing portion. In some other embodiments, the sense strand includes at least four deoxyribonucleotides. For example, the sense strand includes deoxyribonucleotides at positions 11, 12, 13, and 15 as counted from the 3' end of the sense strand hybridizing portion.

As another embodiment, the sense strand may not contain deoxyribonucleotides.

In some embodiments, the sense strand includes a deoxyribonucleotide at least one position of the positions opposite or complementary to position 11, 12, or 13 as counted from the 5' end of the antisense strand hybridizing portion. In some embodiments, the sense strand includes a deoxyribonucleotide at least one position opposite or complementary to position 11, 12, or 15 of the antisense strand as counted from the 5' end of the antisense strand hybridizing portion. In some other embodiments, the sense strand includes a deoxyribonucleotide at least one of the positions opposite or complementary to position 11, 12, 13, or 15 of the antisense strand as counted from the 5' end of the antisense strand hybridizing portion.

In some embodiments, the sense strand includes deoxyribonucleotides at positions 11, 12, and 13 as counted from the 3' end of the sense strand hybridizing portion, and the antisense strand includes deoxyribonucleotides at positions 2 and 14 as counted from the 5' end of the antisense strand hybridizing portion. In some other embodiments, the sense strand includes deoxyribonucleotides at positions 11, 13, and 15 as counted from the 3' end of the sense strand hybridizing portion, and the antisense strand includes deoxyribonucleotides at positions 2, 14, and 16 as counted from the 5' end of the antisense strand hybridizing portion. In some other embodiments, the sense strand includes deoxyribonucleotides at positions 11, 13, and 15 as counted from the 3' end of the sense strand hybridizing portion, and the antisense strand includes deoxyribonucleotides at positions 2, 6, 9, 14, and 16 as counted from the 5' end of the antisense strand hybridizing portion. In still some other embodiments, the sense strand includes deoxyribonucleotides at least positions 11, 13, and 15 from the 3' end of the sense strand hybridizing portion, and the antisense strand includes deoxyribonucleotides at least positions 2, 6, 8, 9, 14, and 16 from the 5' end of the antisense strand hybridizing portion.

In some embodiments, the sense strand includes deoxyribonucleotides at positions 11, 12, 13, and 15 as counted from the 3' end of the sense strand hybridizing portion, and the antisense strand includes deoxyribonucleotides at positions 2, 14, and 16 as counted from the 5' end of the antisense strand hybridizing portion. In some other embodiments, the sense strand includes deoxyribonucleotides at positions 11, 12, 13, and 15 as counted from the 3' end of the sense strand hybridizing portion, and the antisense strand includes deoxyribonucleotides at positions 2, 6, 9, 14, and 16 as counted from the 5' end of the antisense strand hybridizing portion. In still some other embodiments, the sense strand includes deoxyribonucleotides at positions 11, 12, 13, and 15 as counted from the 3' end of the sense strand hybridizing portion, and the antisense strand includes deoxyribonucleotides at positions 2, 6, 8, 9, 14, and 16 as counted from the 5' end of the antisense strand hybridizing portion.

In some embodiments, the antisense strand does not include deoxyribonucleotides at positions 3 to 9 as counted from the 5' end of the antisense strand hybridizing portion.

In some embodiments, the dsRNA comprises one or more overhang regions (that is, single-stranded regions) and/or capping groups of the dsRNA at the 3' or 5' end or both ends of the strand. Without limitation, the overhang can have a length of 1 to 10, 1 to 6, 1 to 5, 1 to 4, 1 to 3, 2 to 6, 2 to 5, 2 to 4, 2 to 3, or 1 to 2 nucleotides. An overhang can be the result of one strand being longer than the other or the result of two strands of the same length being misaligned. The overhang may form a mismatch with the target sequence, or it may be complementary to the target sequence or other sequences. The sense strand and antisense strand can be linked, for example, by forming hairpins with additional nucleotides or by other non-nucleotide linkers. Without limitation, the overhang may be present at the 3' end of the sense strand, the antisense strand, or both strands.

In some embodiments, the dsRNA comprises a single overhang. For example, the dsRNA has a single overhang, and this overhang has a length of at least 2, 3, 4, 5, 6, 7, 8, 9, or 10 nucleotides. In some embodiments, the overhang is at the 3' end of the antisense strand. In some specific embodiments, the dsRNA comprises a 2 nucleotide overhang at the 3' end of the antisense strand. In some specific embodiments, the dsRNA comprises a 3 nucleotide overhang at the 3' end of the antisense strand. In some specific embodiments, the dsRNA comprises a 4 nucleotide overhang at the 3' end of the antisense strand.

The dsRNA may also have blunt ends. For example, one end of the dsRNA is blunt and the other end has an overhang. Without limitation, blunt ends can be located at the 5' end of the antisense strand (or the 3' end of the sense strand) or vice versa. In general, the antisense strand of dsRNA has a nucleotide overhang at the 3' end and a blunt 5' end. For example, a dsRNA having a blunt end at the 5' end of the antisense strand and a 3' end overhang of the antisense strand asymmetrically acts advantageously in the process of loading the guide strand into the RISC. In some embodiments, the dsRNA has a 2 nucleotide overhang at the 3' end of the antisense strand and a blunt end at the 5' end of the antisense strand. In some embodiments, the dsRNA has a 3 nucleotide overhang at the 3' end of the antisense strand and a blunt end at the 5' end of the antisense strand. In some embodiments, the dsRNA has a 4 nucleotide overhang at the 3' end of the antisense strand and a blunt end at the 5' end of the antisense strand.

In some other embodiments, the dsRNA has two blunt ends, that is, at both ends of the dsRNA.

Nucleotides in the overhang region of dsRNA can each independently be modified or unmodified nucleotides such as, but not limited to, sugar-modified nucleotides, deoxyribonucleotides, ribonucleotides, and any combination thereof. In some other embodiments, examples include 2'-O-XCE nucleotides, 2'-fluoro nucleotides, 2'-O-methyl nucleotides, deoxyribonucleotides, ribonucleotides, 2'-O-methoxyethyl nucleotides, and any combination thereof, and the like. For example, TT (or UU) as the base sequence may be an overhang sequence for any one end of any one chain. The 5'- or 3'-overhang of the sense, antisense, or both strands of dsRNA can be phosphorylated. In some embodiments, the overhang region contains two nucleotides with a phosphorothioate bond therebetween, in which the two nucleotides of the overhang region can be the same or different.

The dsRNA may include at least 1, for example, 2, 3, 4, 5, 6, 7, 8, 9, 10 or more modified phosphodiester bonds. In some other embodiments, phosphorothioate bonds may be included. Modified phosphodiester bonds can occur on any nucleotide at any position of a sense strand, an antisense strand, or both strands. For example, modified phosphodiester bonds may occur on all nucleotides on the sense and/or antisense strands, each modified phosphodiester bond may occur in an alternating pattern on the sense or antisense strands, or the sense strand or antisense strand include both modified phosphodiester bonds in an alternating pattern. The alternating pattern of modified phosphodiester bonds on the sense strand may be the same as or different from the antisense strand, and the alternating pattern of modified phosphodiester bonds on the sense strand may have a change relative to the alternating pattern of modified phosphodiester bonds on the antisense strand.

In some embodiments, the dsRNA may or may not include modified phosphodiester bonds within the overhang region, but preferably includes modified phosphodiester bonds. In some other embodiments, the dsRNA may include a phosphorothioate bond within the overhang region. For example, an overhang region includes two nucleotides with a modified phosphodiester bond between the two nucleotides. Modified phosphodiester bonds can also be provided to link an overhang nucleotide with a paired nucleotide at the end of the hybridizing portion. For example, at least 2, 3, 4, or all overhang nucleotides may be linked through modified phosphodiester bonds, and optionally additional modified phosphodiester bonds may be present that link an overhang nucleotide with a paired nucleotide adjacent to the overhang nucleotide. For example, there may be at least two modified phosphodiester bonds between the three nucleotides at the ends, in which two of the three nucleotides are overhang nucleotides and the third is a paired nucleotide adjacent to the overhang nucleotide. Preferably, these three nucleotides at the ends may be present at the 3' end of the antisense strand.

In some embodiments, the dsRNA comprises one or more modified phosphodiester bonds within positions 1 to 10 of the end positions of the sense and/or antisense strand. For example, at least 2, 3, 4, 5, 6, 7, 8, 9, or 10 nucleotides include phosphodiester bonds modified at one or both ends of the sense and/or antisense strand.

In some embodiments, the dsRNA comprises 1 to 5 modified phosphodiester bonds within positions 1 to 5 as counted from the 5' end of the sense strand, 1 to 5 modified phosphodiester bonds within positions 1 to 5 as counted from the 3' end of the sense strand, and 1 to 5 modified phosphodiester bonds within positions 1 to 5 as counted from the 5' end of the antisense strand and 1 to 5 modified phosphodiester bonds within positions 1 to 5 as counted from the 3' end of the antisense strand.

In some embodiments, the dsRNA comprises 1 to 5 phosphorothioate bonds within positions 1 to 5 as counted from the 5' end of the sense strand, 1 to 5 modified phosphorothioate bonds within positions 1 to 5 as counted from the 3' end of the sense strand, and 1 to 5 phosphorothioate bonds within positions 1 to 5 as counted from the 5' end of the antisense strand and 1 to 5 phosphorothioate bonds within positions 1 to 5 as counted from the 3' end of the antisense strand.

In some embodiments, the dsRNA comprises two phosphorothioate bonds within positions 1 to 5 as counted from the 5' end of the sense strand, one modified phosphorothioate bond within positions 1 to 5 as counted from the 3' end of the sense strand, and one phosphorothioate bond within positions 1 to 5 as counted from the 5' end of the antisense strand, and two phosphorothioate bonds within positions 1 to 5 as counted from the 3' end of the antisense strand.

In some embodiments, the dsRNA comprises two phosphorothioate bonds within positions 1 to 5 as counted from the 5' end of the sense strand, two modified phosphorothioate bonds within positions 1 to 5 as counted from the 3' end of the sense strand, and one phosphorothioate bond within positions 1 to 5 as counted from the 5' end of the antisense strand and two phosphorothioate bonds within positions 1 to 5 as counted from the 3' end of the antisense strand.

In some embodiments, the dsRNA comprises two phosphorothioate bonds within positions 1 to 5 as counted from the 5' end of the sense strand, two modified phosphorothioate bonds within positions 1 to 5 as counted from the 3' end of the sense strand, and one phosphorothioate bond within positions 1 to 5 as counted from the 5' end of the antisense strand and one phosphorothioate bond within positions 1 to 5 as counted from the 3' end of the antisense strand.

In some embodiments, the dsRNA comprises one phosphorothioate bond within positions 1 to 5 as counted from the 5' end of the sense strand, one modified phosphorothioate bond within positions 1 to 5 as counted from the 3' end of the sense strand, and two phosphorothioate bonds within positions 1 to 5 as counted from the 5' end of the antisense strand, and two phosphorothioate bonds within positions 1 to 5 as counted from the 3' end of the antisense strand.

In some embodiments, the dsRNA comprises two phosphorothioate bonds within positions 1 to 5 as counted from the 5' end of the sense strand, two modified phosphorothioate bonds within positions 1 to 5 as counted from the 3' end of the sense strand, and two phosphorothioate bonds within positions 1 to 5 as counted from the 5' end of the antisense strand, and two phosphorothioate bonds within positions 1 to 5 as counted from the 3' end of the antisense strand.

In some embodiments, the dsRNA comprises one phosphorothioate bond within positions 1 to 5 as counted from the 5' end of the sense strand, two phosphorothioate bonds at positions 1 and 2 as counted from the 5' end of the antisense strand, and one phosphorothioate bond within positions 1 to 5 as counted from the 3' end of the antisense strand.

In some embodiments, the dsRNA comprises two phosphorothioate bonds within positions 1 to 5 as counted from the 5' end of the sense strand, two phosphorothioate bonds at positions 1 and 2 as counted from the 5' end of the antisense strand, and two phosphorothioate bonds within positions 1 to 5 as counted from the 3' end of the antisense strand.

In some embodiments, the dsRNA comprises two phosphorothioate bonds within positions 1 to 5 as counted from the 5' end of the sense strand, one phosphorothioate bond within positions 1 to 5 as counted from the 3' end of the sense strand, two phosphorothioate bonds within positions 1 and 2 as counted from the 5' end of the antisense strand, and one phosphorothioate bond within positions 1 to 5 as counted from the 3' end of the antisense strand.

In some embodiments, the dsRNA comprises two phosphorothioate bonds within positions 1 to 5 as counted from the 5' end of the sense strand, one phosphorothioate bond within positions 1 to 5 as counted from the 3' end of the sense strand, two phosphorothioate bonds within positions 1 and 2 as counted from the 5' end of the antisense strand, and two phosphorothioate bonds within positions 1 to 5 as counted from the 3' end of the antisense strand.

In some embodiments, the dsRNA comprises two phosphorothioate bonds at positions 1 and 2 as counted from the 5' end of the sense strand, two phosphorothioate bonds at positions 1 and 2 as counted from the 3' end of the sense strand, one phosphorothioate bond at position 1 as counted from the 5' end of the antisense strand, and one phosphorothioate bond at position 1 as counted from the 3' end of the antisense strand.

In some embodiments, the dsRNA comprises one phosphorothioate bond at position 1 as counted from the 5' end of the sense strand, one phosphorothioate bond at position 1 as counted from the 3' end of the sense strand, two phosphorothioate bonds at positions 1 and 2 as counted from the 5' end of the antisense strand, and two phosphorothioate bonds at positions 1 and 2 as counted from the 3' end of the antisense strand.

In some exemplary dsRNAs, the sense strand may include 0, 1, 2, 3, or 4 phosphorothioate bonds. For example, the sense strand includes phosphorothioate bonds between nucleotide positions 1 and 2 and between nucleotide positions 2 and 3 from the 5' end of the sense strand.

In some exemplary dsRNAs, the antisense strand may include 1, 2, 3, or 4 phosphorothioate bonds. For example, the sense strand includes phosphorothioate bonds between nucleotide positions 1 and 2 and between nucleotide positions 2 and 3 from the 3' end of the sense strand. In a further example, the antisense strand includes phosphorothioate bonds between nucleotide positions 1 and 2 and between nucleotide positions 2 and 3 as counted from the 5' end of the antisense strand, and between nucleotide positions 1 and 2 and between nucleotide positions 2 and 3 as counted from the 3' end of the antisense strand.

In some embodiments, the sense strand includes phosphorothioate bonds between nucleotide positions 1 and 2 and between nucleotide positions 2 and 3 as counted from the 5' end of the sense strand, and the antisense strand includes phosphorothioate bonds between nucleotide positions 1 and 2 and between nucleotide positions 2 and 3 as counted from the 3' end of the antisense strand. For example, the sense strand includes phosphorothioate bonds between nucleotide positions 1 and 2 and between nucleotide positions 2 and 3 as counted from the 5' end of the sense strand, and the antisense strand includes phosphorothioate bonds between nucleotide positions 1 and 2 and between nucleotide positions 2 and 3 as counted from the 5' end of the antisense strand, and between nucleotide positions 1 and 2 and between nucleotide positions 2 and 3 as counted from the 3' end of the antisense strand.

In some embodiments, the sense strand and/or antisense strand of the dsRNA can be 5' phosphorylated or include a phosphate modification form at the 5' end. Exemplary phosphate groups or modified phosphate groups include those compatible with RISC-mediated gene silencing. For example, suitable phosphate groups or modified phosphate groups include 5'-monophosphate ((HO)₂(O)P-O-5'), 5'-diphosphate ((HO)₂(O)P-O-P(HO)(O)-O-5'), 5'-triphosphate ((HO)₂(O)P-O-(HO)(O)P-O-P(HO)(O)-O-5'), 5'-monothiophosphate (phosphorothioate; (HO)₂(S)P-O-5'), 5'-monodithiophosphate (dithiophosphate; (HO)(HS)(S)P-O-5'), 5'-phosphorothiolate ((HO)₂(O)P-S-5'), any further combinations of mono-, di-, and triphosphates in which oxygen/sulfur is substituted (for example, 5'-α-thiotriphosphate, 5'-γ-thiotriphosphate, etc.), 5'-phosphoramidate ((HO)₂(O)P-NH-5', (HO)(NH₂)(O)P-O-5'), 5'-alkylphosphonate (alkylene phosphate (for example, (HO)₂(O)P-CH₂-5' (methylene phosphate), (HO)₂(O)P-CH₂CH₂-5' (ethylene phosphate)), R'P(OH)(O)-O-5'- (R' = alkyl, for example methyl, ethyl, isopropyl, propyl, and the like)), 5'-cycloalkylphosphonate ((HO)₂(O)P-C₃H₄-5' (cyclopropylene phosphate)), 5'-alkenylphosphonate (examples of the alkenyl include vinyl ((OH)₂(O)P-CH=CH-5'-), substituted vinyl), and 5'-alkoxyalkylphosphonate (R"P(OH)(O)-O-5'- (R" = alkoxyalkyl, for example methoxymethyl, ethoxymethyl, etc.)). The modification may be located within the antisense strand of the dsRNA. For example, the antisense strand may include a 5' phosphonate group at the 5' end and may include a 5'-vinylphosphonate-modified nucleotide, a 5'-ethylphosphonate-modified nucleotide, and a 5'-cyclopropanephosphonate-modified nucleotide.

In some embodiments, the nucleotide at the 5' end of the antisense strand includes a 5'-E-vinylphosphonate. For example, a structure represented by Formula (II-3): 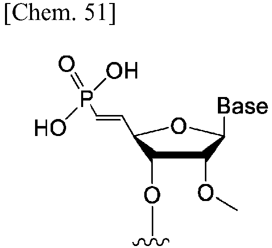 (in Formula (II-3), Base has the same meaning as X and Base in Formula (II)) (5'-vinylphosphonate (VP) modified 2'-O-Me nucleotide) is preferable as the 5'-E-vinylphosphonate.

In some other embodiments, a structure represented by the following Formula (II-2): 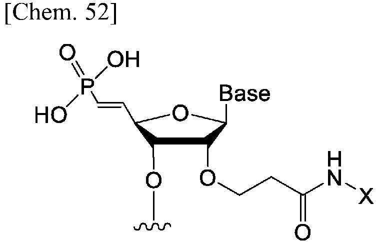 (in Formula (II-2), X and Base have the same meaning as X and Base in Formula (II)) (5'-vinylphosphonate (VP) modified 2'-O-XCE nucleotide) is preferable.

In some embodiments, a structure represented by the following Formula (III-3): 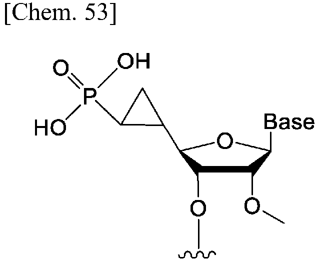 (in Formula (III-3), Base has the same meaning as X and Base in Formula (III)) (5'-cyclopropanephosphonate (CPP) modified 2'-O-Me nucleotide) is preferable.

In some other embodiments, a structure represented by Formula (III-2): 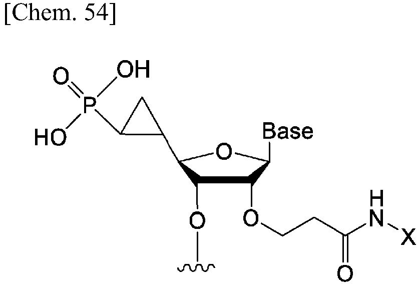 (in Formula (III-2), X and Base have the same meaning as X and Base in Formula (III)) (5'-cyclopropanephosphonate (CPP) modified 2'-O-XCE nucleotide) is preferable.

In some other embodiments, a structure represented by Formula (IV-3): 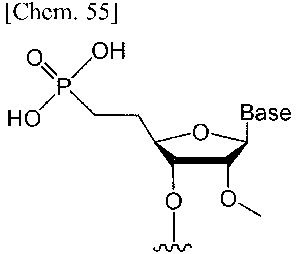 (in Formula (IV-3), Base has the same meaning as X and Base in Formula (IV)) (5'-ethylphosphonate (EP) modified 2'-O-Me nucleotide) is preferable.

In some other embodiments, a structure represented by Formula (IV-2): 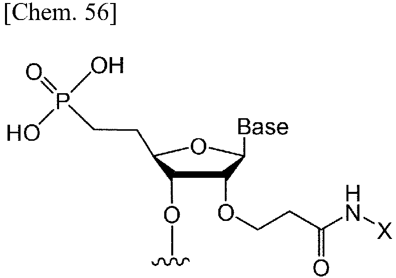 (in Formula (IV-2), X and Base have the same meaning as X and Base in Formula (IV)) (5'-ethylphosphonate (EP) modified 2'-O-XCE nucleotide) is preferable.

The wavy lines in Formulae (II-2) to (IV-3) are understood as a bonding position for an internucleotide bond with an adjacent nucleotide, a bonding position with a functional molecule or a linker thereof, or a bonding position with a hydrogen atom, a hydroxy protecting group, or a phosphorus-containing group.

In some embodiments, the sense strand includes a 5'-morpholino modification, a 5'-dimethylamino modification, a 5'-deoxy modification, an inverted abasic modification, or an inverted abasic locked nucleic acid modification at the 5' end.

Nucleoside structures used as exemplary 5'-morpholino modifications include, but are not limited to, those represented by the following Formula (XVI): 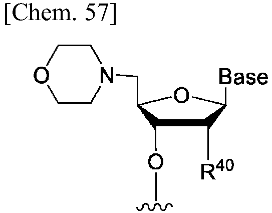
(in Formula (XVI), Base has the same meaning as Base in Formula (I), and
R⁴⁰ is a hydrogen atom, a hydroxy group, a C1-6 alkoxy group, fluorine, an amino group, an N-methylamino group, an N,N'-dimethylamino group, a 2-methoxyethoxy group (O-MOE), a 2-(N-methylcarbamoyl)ethoxy group (O-MCE), DMAECE, MorECE, PyECE, and BimECE).

Nucleoside structures used as exemplary 5'-dimethylamino modifications include, but are not limited to, those represented by the following Formula (XVII): 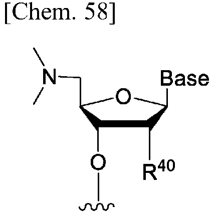
(in Formula (XVII), Base has the same meaning as Base in Formula (I), and
R⁴⁰ has the same meaning as R⁴⁰ in Formula (XVI)).

Nucleoside structures used as exemplary 5'-deoxy modifications include, but are not limited to, those represented by the following Formula (XVIII): 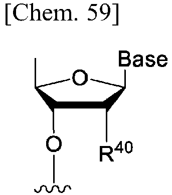 (in Formula (XVIII), Base has the same meaning as Base in Formula (I), and R⁴⁰ has the same meaning as R⁴⁰ in Formula (XVI)).

Nucleoside structures used as exemplary inverted abasic modifications include, but are not limited to, those represented by the following Formula (XIX): 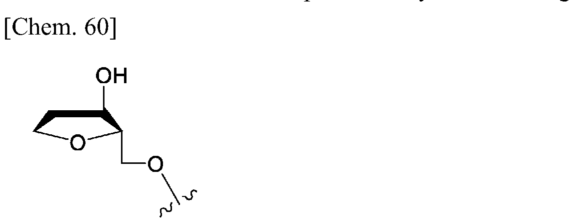 (in the formula, the wavy line indicates a bonding position for bonding to position 5' of an adjacent nucleotide).

Nucleoside structures used as exemplary inverted abasic locked nucleic acid modifications include, but are not limited to, those represented by the following Formula (XX): 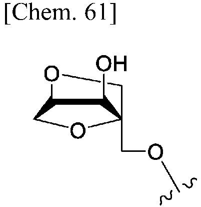 (in the formula, the wavy line indicates a bonding position for bonding to position 5' of an adjacent nucleotide).

In general, dsRNA has a melting temperature (Tm) in the range of approximately 40°C to approximately 80°C. For example, the dsRNA has a melting temperature with the lower end of the range from approximately 40°C, 45°C, 50°C, 55°C, 60°C, or 65°C and the upper end of the range from approximately 70°C, 75°C, or 80°C. In some embodiments, the dsRNA has a melting temperature in the range of approximately 55°C to approximately 70°C, or in the range of approximately 60°C to approximately 75°C. In some embodiments, the dsRNA has a melting temperature in the range of approximately 57°C to approximately 67°C. In some specific embodiments, the dsRNA has a melting temperature in the range of approximately 60°C to approximately 67°C. In some further embodiments, the dsRNA has a melting temperature in the range of approximately 62°C to approximately 66°C.

For example, dsRNA having a melting temperature of at least 60°C has increased efficacy in vivo and in vitro. Thus, in some embodiments, the dsRNA has a melting temperature of at least 60°C.

For example, thermally destabilizing modifications at positions 2-9 as counted from the 5' end of the antisense strand hybridizing portion can reduce or inhibit off-target gene silencing. Thus, in some embodiments, the antisense strand includes at least one (for example, 1, 2, 3, 4, 5 or more) thermally destabilizing modification of the double strand within the range of 9 nucleotide positions as counted from the 5' end of the antisense strand hybridizing portion. The term "one or more thermally destabilizing modification(s)" includes one or more modifications that would result in a dsRNA having a melting temperature (Tm) (preferably a Tm lower by 1, 2, 3 or 4 degrees) that is generally lower than the Tm of a dsRNA without such one or more modifications.

In some embodiments, the thermally destabilizing modification is located at position 2, 3, 4, 5, 6, 7, 8, or 9, or preferably at position 4, 5, 6, 7, or 8 as counted from the 5' end of the antisense strand hybridizing portion. In some embodiments, the thermally destabilizing modification is located at position 2, 3, 4, 5, or 9 as counted from the 5' end of the antisense strand hybridizing portion. In some other embodiments, the thermally destabilizing modification is located at position 6, 7, or 8 as counted from the 5' end of the antisense strand hybridizing portion. In some specific embodiments, the thermally destabilizing modification is located at position 7 as counted from the 5' end of the antisense strand hybridizing portion.

In some embodiments, the antisense strand includes a 2'-modified nucleotide or a 2'-4'-bridged nucleotide at least one position adjacent to the thermally destabilizing modification. For example, a 2'-modified nucleotide or 2'-4'-bridged nucleotide can be a nucleotide at the 5' end or 3' end of the thermally destabilizing modification, that is, at position -1 or position +1 from the position of the thermally destabilizing modification. In some embodiments, the antisense strand includes a 2'-modified nucleotide or 2'-4'-bridged nucleotide at each of the 5' end and 3' end of the thermally destabilizing modification, that is, at position -1 and position +1 from the position of the destabilizing modification.

In some embodiments, the antisense strand includes at least two modified nucleotides each independently selected from a 2'-modified nucleotide or a 2'-4'-bridged nucleotide at the 3' end of the thermally destabilizing modification, that is, at position +1 and position +2 from the position of the thermally destabilizing modification.

In some embodiments, the sense strand does not include a 2'-modified nucleotide or a 2'-4'-bridged nucleotide at a position opposite or complementary to the thermally destabilizing modification of the double strand in the antisense strand.

In some embodiments, the antisense strand includes a 2'-fluoro nucleotide at least one position adjacent to the thermally destabilizing modification. For example, a 2'-fluoro nucleotide can be a nucleotide at the 5' end or 3' end of the thermally destabilizing modification, that is, at position -1 or position +1 from the position of the thermally destabilizing modification. In some embodiments, the antisense strand includes a 2'-fluoro nucleotide at each of the 5' end and 3' end of the thermally destabilizing modification, that is, at position -1 and position +1 from the position of the destabilizing modification.

In some embodiments, the antisense strand includes at least two 2'-fluoro nucleotides at the 3' end of the thermally destabilizing modification, that is, at position +1 and position +2 from the position of the thermally destabilizing modification.

In some embodiments, the sense strand does not include a 2'-fluoro nucleotide at a position opposite or complementary to the thermally destabilizing modification of the double strand in the antisense strand.

In some embodiments, it may be possible to include specific bases in the overhangs or to include modified nucleotides or nucleotide alternatives in single stranded overhangs, such as 5' or 3' overhangs or both. For example, it may be desirable to include a purine nucleotide in the overhang. In some embodiments, all or a portion of the bases in the 3' or 5' overhang can be modified, for example, with the modifications described in the present specification. Modifications may include, for example, the use of 2'-modified nucleotides, such as 2'-O-XCE nucleotide, 2'-fluoro nucleotides or 2'-O-Me nucleotide, and modified phosphate groups, such as phosphorothioate bonds. The overhang need not be homologous to the target sequence.

In some embodiments, each residue of the sense strand and antisense strand is independently modified with a 2'-modification (2'-O-MOE, 2'-O-Me, 2'-fluoro, or the like), a 2'-4'-bridged modification (such as an LNA), or 2'-deoxy. Each strand may individually include two or more modifications. In some embodiments, each residue of the sense strand and antisense strand is independently modified with 2'-O-Me or 2'-fluoro. It is to be understood that, in addition to one or more 2'-O-XCE nucleotides, these modifications may be present in the dsRNA.

In some embodiments, in addition to one or more 2'-O-XCE nucleotides, at least one (preferably two) different modified nucleotide may be present on the sense strand and/or antisense strand. Each of these modifications can be a deoxyribonucleotide, a 2'-O-Me nucleotide, a 2'-fluoro nucleotide, an acyclic nucleotide, and the like. In some embodiments, the sense strand and antisense strand each include one or two different modified nucleotides selected from 2'-O-Me nucleotides, 2'-fluoro nucleotides, and/or 2'-deoxyribonucleotides. In some embodiments, the sense strand and antisense strand are independently modified with a 2'-O-Me nucleotide, a deoxyribonucleotide, a 2'-fluoro nucleotide, a 2'-O-N-methylacetamide (2'-O-NMA) nucleotide, a 2'-O-dimethylaminoethoxyethyl (2'-O-DMAEOE) nucleotide, a 2'-O-aminopropyl (2'-O-AP) nucleotide, or a 2'-ara-F nucleotide (2'-Fluoroarabinonucleotide, also referred to as 2'-F-ANA). Again, it is to be understood that in this case, in addition to one or more 2'-O-XCE nucleotides, these modifications are present in the dsRNA.

In some embodiments, the dsRNA may include an alternating pattern of modifications. The term "alternating pattern" as used in the present specification refers to a modified pattern in which one strand has one or more modifications, each modified nucleotide has a repeating unit that appears alternately, and the number of repeats is two or more. The nucleotides appearing in the alternating pattern may appear every other position to every third position. For example, when A, B, and C each represent one type of modification to a nucleotide, alternating patterns with a repeat number of 2 include "ABAB", "AABBAABB", "AABAAB", "AAABAAAB", "AAABBBAAABBB", "ABCABC", and the like. The alternating pattern in each strand may be "ABABABABABAB...", "AABBAABBAABB...", "AABAABAABAAB...", "AAABAAABAAAB...", "AAABBBAAABBB...", or "ABCABCABCABC..." or the like.

The types of modifications included in the alternating pattern may be the same or different. For example, where A, B, C, and D each represent one type of modification on a nucleotide, the alternating pattern, that is, modification of every other nucleotide, may be the same, but each of the sense strand and antisense strand may be selected from several possibilities of modifications within the range of alternating patterns, such as "ABAB...", "ACAC...", "BDBD...", or "CDCD...".

In some embodiments, the dsRNA comprises an alternating pattern on the sense strand shifted relative to an alternating pattern on the antisense strand. This shift may be such that the modified group of nucleotides in the sense strand corresponds to the otherwise modified group of nucleotides in the antisense strand and vice versa. For example, when the sense strand pairs with the antisense strand in a dsRNA double strand, the alternating pattern of the sense strand may begin with "ABABAB" at the 5'-3' within the range of the hybridizing portion, and the alternating pattern of the antisense strand may begin with "BABABA" at the 3'-5'. As another example, within the range of the hybridizing portion, an alternating pattern of sense strands can begin at 5'-3' of the strand with "AABBAABB" and an alternating pattern of antisense strands can begin at 3'-5' of the strand with "BBAABBAA", thus there is a complete or partial shift in the modification pattern between the sense strand and antisense strand.

In some embodiments, the dsRNA comprises one or more mismatches with the target RNA, which are within the range of the double strand, or a combination thereof. Mismatches may appear in an overhang region or a hybridizing portion. Base pairs can be ranked based on their tendency to promote dissociation or melting (for example, with respect to the free energy of association or dissociation of a particular pairing, the simplest technique is to examine pairs on individual base pairs, but nearest-neighbor analysis or a similar analysis can also be used). In terms of promoting dissociation, A:U is preferable to G:C, G:U is preferable to G:C, and I:C is preferable to G:C (I = inosine). Mismatches, such as non-standard or non-canonical pairings (as described in other parts in the present specification), are preferred over standard (A:T, A:U, G:C) pairings; and pairings including a universal base are preferred over standard pairings.

In some embodiments, the dsRNA comprises at least one of the first 1, 2, 3, 4, or 5 base pairs (this may be independently selected from the group of A: U, G: U, and I: C) as counted from the 5' end of the antisense strand hybridizing portion, and a mismatch pair, such as a non-standard or non-canonical pairing or a pairing including a universal base, to promote dissociation of the antisense strand at the 5' end of the double strand.

In some embodiments, the nucleotide at position 1 as counted from the 5' end of the antisense strand hybridizing portion is selected from the group consisting of A, dA, dU, U, and dT. Instead, at least one of the first 1, 2 or 3 base pairs as counted from the 5' end of the antisense strand hybridizing portion is an AU base pair. For example, the first base pair counted from the 5' end of the antisense strand hybridizing portion is an AU base pair.

For example, the introduction of a 4'- and/or 5'-modified nucleotide at the 3' end of a phosphodiester bond, a phosphorothioate bond, and/or a phosphorodithioate bond of a dinucleotide at any position of a single-stranded or double-stranded oligonucleotide can exert a steric effect on the internucleotide bond and thus protect or stabilize it from nucleases.

In some embodiments, a 5'-modified nucleotide is introduced at the 3' end of a dinucleotide at any position in the dsRNA. For example, a 5'-alkyl nucleotide can be introduced at the 3' end of a dinucleotide at any position of the dsRNA.

In some embodiments, a 4'-modified nucleotide is introduced at the 3' end of a dinucleotide at any position in the dsRNA. For example, a 4'-alkyl nucleotide can be introduced at the 3' end of a dinucleotide at any position of the dsRNA. Alternatively, a 4'-O-alkyl nucleotide may be introduced at the 3' end of a dinucleotide at any position in the single-stranded or double-stranded siRNA. Exemplary 4'-O-alkyl nucleotides are 4'-O-methyl nucleotides. 4'-O-methyl can be either racemic or a chirally pure R or S isomer.

In some embodiments, a 5'-alkyl nucleotide is introduced at any position on the sense strand or antisense strand of the dsRNA, and such modifications maintain or improve the potency of the dsRNA. The 5'-alkyl may be either racemic or a chirally pure R or S isomer. Exemplary 5'-alkyl nucleotides are 5'-methyl nucleotides. 5'-methyl can be either racemic or a chirally pure R or S isomer. Another exemplary 5'-alkyl nucleotide is a 5'-CP nucleotide.

In some embodiments, a 4'-alkyl nucleotide is introduced at any position on the sense strand or antisense strand of the dsRNA, and such modifications maintain or improve the potency of the dsRNA. The 4'-alkyl may be either racemic or a chirally pure R or S isomer. Exemplary 4'-alkyl nucleotides are 4'-methyl nucleotides. 4'-methyl can be either racemic or a chirally pure R or S isomer.

In some embodiments, a 4'-O-alkyl nucleotide is introduced at any position on the sense strand or antisense strand of the dsRNA, and such modifications maintain or improve the potency of the dsRNA. The 5'-alkyl may be either racemic or a chirally pure R or S isomer. Exemplary 4'-O-alkyl nucleotides are 4'-O-methyl nucleotides. 4'-O-methyl can be either racemic or a chirally pure R or S isomer.

In some embodiments, the dsRNA may include a 2'-5' bond (containing 2'-H, 2'-OH, and 2'-O-Me, and containing P=O or P=S). For example, a 2'-5' bonding modification can be used to promote nuclease resistance or inhibit the sense strand from binding to the antisense strand, or can be used at the 5' end of the sense strand to avoid activation of the sense strand by RISC. In some embodiments, the sense strand includes a 2'-5' bond between nucleotides at position 1 and position 2 as counted from the 5' end.

In some embodiments, a functional molecule may be directly or indirectly bonded to the dsRNA. The bond between the functional molecule and the sense strand and/or antisense strand of the dsRNA may be direct or indirect via another substance. However, the oligonucleotide and the functional molecule are preferably bonded to each other through a covalent bond, an ionic bond, or a hydrogen bond. From the viewpoint of high stability of the bond, more preferably, they are directly bonded via a covalent bond or bonded via a linker (linking group) through a covalent bond.

When the functional molecule is bonded to the sense strand and/or antisense strand of dsRNA through a covalent bond, the functional molecule is preferably directly or indirectly bonded to the 3' end or the 5' end of the sense strand and/or antisense strand of dsRNA. In some embodiments, the functional molecule is preferably directly or indirectly bonded to the internal nucleotides (for example, a sugar moiety, a base moiety, or an internucleotide bond moiety) of the sense strand and/or antisense strand of the dsRNA. The bonding between the linker or functional molecule and the end nucleotide or internal nucleotide of the sense strand and/or antisense strand of the dsRNA is selected depending on the functional molecule.

The linker or the functional molecule and the end nucleotide or the internal nucleotide of the sense strand and/or antisense strand of the dsRNA are preferably linked to each other by a phosphodiester bond or a modified phosphodiester bond, and more preferably linked to each other by a phosphodiester bond. In some embodiments, the linker or functional molecule and the end nucleotide of the sense strand and/or antisense strand of the dsRNA are preferably linked by a modified phosphodiester bond, and more preferably linked by a phosphorothioate bond. The linker or the functional molecule may be directly linked to an oxygen atom at position 3' of a nucleotide at the 3' end, or an oxygen atom at position 5' of a nucleotide at the 5' end of the sense strand and/or antisense strand of the dsRNA. In some embodiments, the linker or functional molecule may be directly linked to the oxygen atom at position 2' of the end nucleotide or internal nucleotide of the sense strand and/or antisense strand of the dsRNA.

The structure of "functional molecule" is not particularly limited, and bonding of the functional molecule to the dsRNA imparts a desired function to the dsRNA. Examples of the desired function include a labeling function, a purification function, and a function of delivery to a target site. Examples of a molecule that imparts a labeling function include compounds such as a fluorescent protein, luciferase, and the like. Examples of a molecule that imparts a purification function include compounds such as biotin, avidin, His tag peptide, GST tag peptide, FLAG tag peptide, and the like.

In addition, a molecule having a function of delivering the dsRNA to a target site is preferably bonded as the functional molecule from the viewpoint of highly specifically and efficiently delivering the dsRNA to a target site (for example, target cell) with extremely effectively controlling expression of the target RNA by the dsRNA. For the molecule having the function of delivery, for example, European Journal of Pharmaceutics and Biopharmaceutics, 2016, 107, pp 321-340, Advanced Drug Delivery Reviews, 2016, 104, pp 78-92, Expert Opinion on Drug Delivery, 2014, 11, pp 791-822, and the like can be referred to.

Examples of a molecule that imparts a function of delivery to the target RNA include a lipid and a sugar from the viewpoint that the dsRNA can be efficiently delivered with high specificity to the liver, central tissue, lung, and the like. Examples of such a lipid include: cholesterol; fatty acid; a fat-soluble vitamin such as vitamin E (tocopherols or tocotrienols), vitamin A, vitamin D, or vitamin K; an intermediate metabolite such as acylcarnitine or acyl-CoA; a glycolipid; glyceride; long-chain hydrocarbons; derivatives thereof; and the like. Among them, cholesterol and vitamin E (tocopherols or tocotrienols) are preferable from the viewpoint of higher safety. Among them, tocopherols are more preferable, tocopherol is still more preferable, and α-tocopherol is particularly preferable. Examples of the sugar include a sugar derivative having an interaction with an asialoglycoprotein receptor.

In some other embodiments, a structure including a lipid represented by the following Formula (XXI): 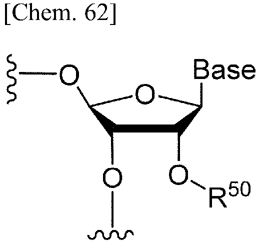 (in Formula (XXI), Base has the same meaning as Base in Formula (I), and R⁵⁰ is a C5-30 alkyl group or a C5-30 alkenyl group) is preferably included in the sense strand and/or antisense strand of the dsRNA, and more preferably included in the sense strand. In some other embodiments, at least one position selected from positions 14 to 21 as counted from the 3' end of the sense strand hybridizing portion preferably includes Formula (XXI), and more preferably, includes Formula (XXI) at position 16. In some other embodiments, R⁵⁰ is preferably a C14-24 alkyl group, and R⁵⁰ is more preferably a C16-22 alkyl group.

"Asialoglycoprotein receptor" is present on the surface of liver cells, and functions to recognize the galactose residues of asialoglycoproteins, take the molecules into the cells, and then decompose them. The "sugar derivative having an interaction with an asialoglycoprotein receptor" is preferably a compound that has a structure similar to that of a galactose residue and is taken into a cell by an interaction with an asialoglycoprotein receptor, and examples thereof include a GalNAc (N-acetylgalactosamine) derivative, a galactose derivative, and a lactose derivative.

In some other embodiments, as "a sugar derivative having an interaction with an asialoglycoprotein receptor", a GalNAc structure represented by the following Formula (i): 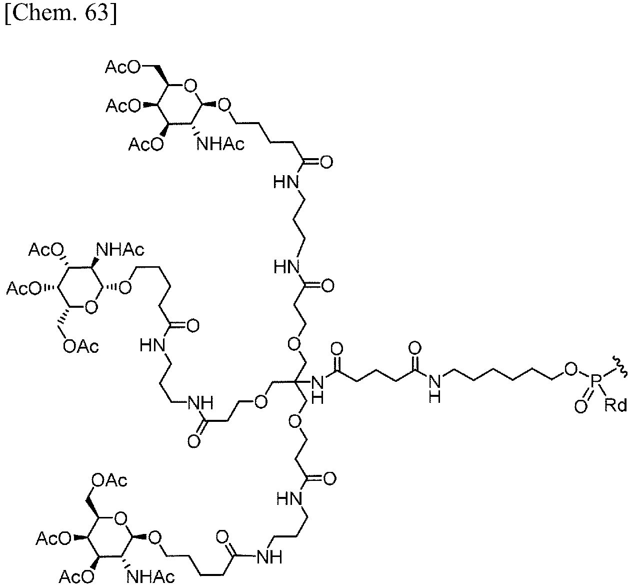 (in the formula, Rd is a hydroxy group or a thiol group, and preferably a thiol group) is preferably included in the sense strand and/or antisense strand of the dsRNA, and more preferably included in the sense strand. In some other embodiments, the structure is preferably directly attached to the 3' or 5' end of the sense strand of the dsRNA, and more preferably directly attached to the 5' end of the sense strand.

In some other embodiments, as "a sugar derivative having an interaction with an asialoglycoprotein receptor", a GalNAc structure represented by the following Formula (ii): 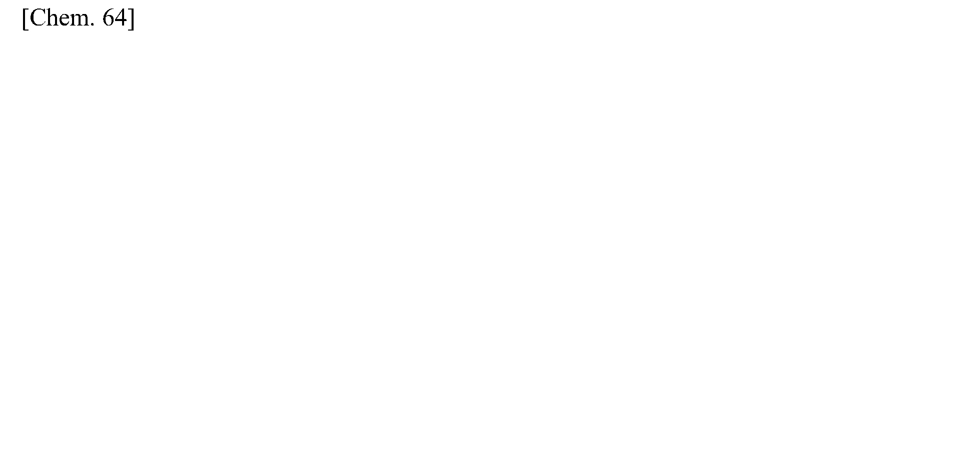 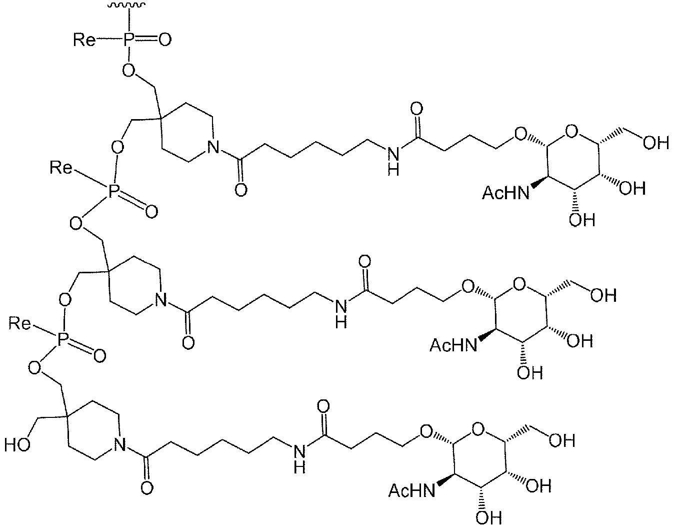
(in the formula, Re are each independently a hydroxy group or a thiol group, and preferably a thiol group)
is preferably included in the sense strand and/or antisense strand of the dsRNA, and more preferably included in the sense strand. In some other embodiments, the molecule is preferably directly attached to the 3' or 5' end of the sense strand of the dsRNA, and more preferably directly attached to the 3' end of the sense strand.

In addition, from the viewpoint that the dsRNA of the present invention can be delivered highly specifically and efficiently to the brain, examples of the "functional molecule" include a sugar (for example, glucose, sucrose, or the like). In addition, from the viewpoint that the dsRNA can be delivered highly specifically and efficiently to an organ by interacting with various proteins on a surface of a cell of the organ, examples of the "functional molecule" include a ligand of a receptor, an antibody, and a peptide or a protein of fragments thereof.

The linker via which the functional molecule is bonded to the sense strand and/or antisense strand of dsRNA is not particularly limited, provided that the functional molecule can exert its function as part of the dsRNA. Accordingly, any linker capable of stably linking the functional molecule to the dsRNA may be used. Examples of the linker include a group derived from an oligonucleotide consisting of 1 to 20 nucleotides, a group derived from a polypeptide consisting of 2 to 20 amino acids, an alkylene consisting of 2 to 20 carbon atoms, an alkenylene consisting of 2 to 20 carbon atoms, and the like. The group derived from an oligonucleotide consisting of 2 to 20 nucleotides is a group obtained by removing hydroxy, a hydrogen atom, and the like from an oligonucleotide consisting of 2 to 20 nucleotides. For the group derived from an oligonucleotide consisting of 1 to 20 nucleotides, for example, WO 2017/053995 A can be referred to. WO 2017/053995 A describes, for example, a 3-base linker having a TCA motif, a 1- to 5-base linker having no TCA motif, and the like. The group derived from a polypeptide consisting of 2 to 20 amino acids is a group obtained by removing hydroxy, a hydrogen atom, amino, and the like from a polypeptide consisting of 2 to 20 amino acids.

The linker is preferably a C2-20 alkylene or a C2-20 alkenylene (the methylenes included in the alkylene and the alkenylene are each independently unsubstituted or substituted with 1 or 2 substituents selected from the group consisting of a halogen atom, hydroxy, protected hydroxy, oxo, and thioxo. In addition, the methylenes of the alkylene and the alkenylene are each independently not replaced or replaced with -O-, -NR^{B}- (R^{B} is a hydrogen atom, C1-6 alkyl or halo C1-6 alkyl), -S-, - S(=O)-, or -S(=O)₂-). Here, by combining the substitution and the replacement, the linker may include a group represented by -C(=O)-O-, -O-C(=O)-NR²³- (R²³ represents a hydrogen atom, C1-6 alkyl, or halo C1-6 alkyl), -C(=O)-NR²³- (R²³ represents a hydrogen atom, C1-6 alkyl, or halo C1-6 alkyl), -C(=S)-NR²³- (R²³ represents a hydrogen atom, C1-6 alkyl, or halo C1-6 alkyl), -NR²³-C(=O)-NR²³- (R²³ each independently represents a hydrogen atom, C1-6 alkyl, or halo C1-6 alkyl), or the like.

For example, a linker structure of -[C2-6 alkylene]-[C(=O)-NR²³- (R²³ represents a hydrogen atom or C1-6 alkyl)]-[C2-6 alkylene]-[C(=O)]- can be used. Here, preferably the left-hand [C2-6 alkylene] is bonded to the functional molecule and the right-hand [C(=O)] is bonded to the oligonucleotide.

The linker is more preferably a C2-20 alkylene (the methylenes of the alkylene are each independently not replaced or replaced with -O-. The non-replaced methylenes are each independently unsubstituted or substituted with hydroxy or protected hydroxy), more preferably a C8-12 alkylene (the methylenes of the alkylene are each independently not replaced or replaced with -O-. The non-replaced methylenes are each independently unsubstituted or substituted with hydroxy).

As another embodiment, the linker is more preferably a C2-20 alkylene (the methylenes of the alkylene are each independently not replaced or replaced with -O- or - NR^{B}- (R^{B} is a hydrogen atom or C1-6 alkyl). The non-replaced methylenes are each independently unsubstituted or substituted with oxo), more preferably a C8-12 alkylene (the methylenes of the alkylene are each independently not replaced or replaced with - O- or -NR^{B}- (R^{B} is a hydrogen atom or C1-6 alkyl). The unsubstituted methylenes are each independently unsubstituted or substituted with oxo).

To the 5' end or a 3' end of the sense strand and/or antisense strand of dsRNA, 1 to 5 (preferably 1 to 3, particularly preferably 3) 3- to 11-membered nitrogen-containing non-aromatic heterocyclic-diyldimethanol structures may be linked by a phosphodiester bond or a phosphorothioate bond, and the linker may be linked to the 3-to 11-membered nitrogen-containing non-aromatic heterocyclic ring. In this case, the number of functional molecules bonded via the linker is also preferably 1 to 5, more preferably 1 to 3, and particularly preferably 3. The 3- to 11-membered nitrogen-containing non-aromatic heterocyclic ring is preferably pyrrolidine or piperidine. The 3- to 11-membered nitrogen-containing non-aromatic heterocyclic-diyldimethanol is particularly preferably piperidine-4,4-diylmethanol.

The protecting group of the "protected hydroxy" is not particularly limited as long as it is stable during bonding of the functional molecule to the oligonucleotide. Examples of the protecting group include, but are not particularly limited, any protecting group described in Protective Groups in Organic Synthesis, 4th edition, written by T. W. Greene, P. G. M. Wuts, John Wiley & Sons Inc. (2006), and the like. Specifically, as described in the explanation of terms, preferable examples include benzoyl, trimethylsilyl, triethylsilyl, triisopropylsilyl, t-butyldimethylsilyl, triphenylmethyl, monomethoxytrityl, dimethoxytrityl, trimethoxytrityl, 9-phenylxanthen-9-yl, or 9-(p-methoxyphenyl)xanthen-9-yl; more preferably, monomethoxytrityl, dimethoxytrityl, or trimethoxytrityl; and still more preferably, dimethoxytrityl.

It can be bonded to a multimer.

In dsRNA, a functional molecule can be bonded to one or both strands. In some embodiments, the dsRNA has a functional molecule bonded to the sense strand. In other embodiments, the dsRNA has a functional molecule bonded to the antisense strand.

In some embodiments, the functional molecule can be bonded to a nucleobase, sugar moiety, or internucleoside bond of a sense and/or antisense strand of dsRNA. The bonding to a purine nucleobase or derivative thereof can occur at any position, including endocyclic and exocyclic atoms. In some embodiments, it is bonded to position 2, position 6, position 7, or position 8 of a purine nucleobase. The bonding to a pyrimidine nucleobase or derivative thereof may also occur at any position. In some embodiments, it can be bonded to position 2, position 5, and position 6 of a pyrimidine nucleobase. The bonding of a nucleotide to a sugar moiety can occur at any carbon atom. Examples of carbon atoms of sugar moieties that may be bonded include 2', 3', and 5' carbon atoms. The position 1' may also be bonded, for example, in an abasic residue. The internucleoside bond may also have a conjugate moiety. In a phosphorus-containing bond (for example, phosphodiester, phosphorothioate, dithiophosphoric acid, phosphoramic acid, and the like), it can be bonded directly to a phosphorus atom or to an O, N, or S atom bonded to a phosphorus atom. In an amine or amide-containing internucleoside bond (for example, PNA), it can be bonded to a nitrogen atom or adjacent carbon atom of the amine or amide.

In some embodiments, the functional molecule is bonded to the sense strand. As described in the present specification, a functional molecule can be bonded to the 3' end, the 5' end, or an internal position of the sense strand. In some embodiments, the functional molecule is bonded to the 3' end of the sense strand. In addition, the functional molecule can be bonded to a nucleobase, sugar moiety, or internucleotide bond of the sense strand.

The dsRNA comprises those existing via tautomerism thereof or geometric isomerism thereof, and also includes those existing as a mixture thereof or a mixture of the respective isomers. In addition, when an asymmetric center exists, or when an asymmetric center is generated as a result of isomerization, the dsRNA also includes those existing as respective optical isomers and mixtures thereof in any ratio. Further, in the case of a compound having two or more asymmetric centers, diastereomers due to the respective optical isomerism also exist. The present invention also includes those including all these types at any ratio. In addition, an optically active form can be obtained by a well-known method for this purpose.

For example, when the dsRNA of the present invention includes a modified phosphodiester bond (for example, a phosphorothioate bond) and the phosphorus atom is an asymmetric atom, both forms of an oligonucleotide in which the stereochemistry of the phosphorus atom is controlled and an oligonucleotide in which the stereochemistry of the phosphorus atom is not controlled are included in the scope of the present invention.

The dsRNA or a pharmaceutically acceptable salt thereof of the present invention can exist in any crystalline form depending on producing conditions, and can exist in any hydrate, but these crystalline forms, hydrates, and mixtures thereof are also included in the scope of the present invention. In addition, the dsRNA or a pharmaceutically acceptable salt thereof of the present invention may be present as a solvate containing an organic solvent such as acetone, ethanol, 1-propanol, or 2-propanol, but all of these forms are included in the scope of the present invention.

The dsRNA of the present invention can be optionally converted into a pharmaceutically acceptable salt or released from the produced salt. Examples of the pharmaceutically acceptable salt of the dsRNA include salts with an alkali metal (lithium, sodium, potassium, or the like), an alkaline earth metal (magnesium, calcium, or the like), ammonium, an organic base (triethylamine, trimethylamine, or the like), an amino acid (glycine, lysine, glutamic acid, or the like), an inorganic acid (hydrochloric acid, hydrobromic acid, phosphoric acid, sulfuric acid, or the like), and an organic acid (acetic acid, citric acid, maleic acid, fumaric acid, tartaric acid, benzenesulfonic acid, methanesulfonic acid, p-toluenesulfonic acid, or the like).

In particular, a partial structure represented by -P(=O)(OH)- may be converted into an anionic partial structure represented by -P(=O)(O-)- to form a salt with an alkali metal (lithium, sodium, potassium, or the like), an alkaline earth metal (magnesium, calcium, or the like), ammonium, or the like. A partial structure represented by - P(=O)(SH)- that forms a phosphorothioate bond may be converted into an anionic partial structure represented by -P(=O)(S-)- to form a salt with an alkali metal, an alkaline earth metal, ammonium, or the like similarly. The same applies to other modified phosphodiester bonds.

The dsRNA of the present invention can be prepared by appropriately selecting a method known to a person skilled in the art. For example, a person skilled in the art can synthesize the dsRNA of the present invention by designing a nucleotide sequence of the dsRNA based on information of a nucleotide sequence of a target RNA, and using a commercially available automatic nucleic acid synthesizer (manufactured by Applied Biosystems, manufactured by Beckman, manufactured by GeneDesign, Inc., or the like). In addition, it can also be synthesized by a reaction using an enzyme. Examples of the enzyme include polymerase, ligase, restriction enzyme, and the like, but are not limited thereto. That is, the method for producing dsRNA according to the present embodiment can include a step of extending a nucleotide chain at the 3' end or the 5' end of the sense strand and/or the antisense strand.

Many methods for bonding a functional molecule and the sense strand and/or antisense strand of the dsRNA to each other are well known in the art. For example, European Journal of Pharmaceutics and Biopharmaceutics, 2016, 107, pp 321-340, Advanced Drug Delivery Reviews, 2016, 104, pp 78-92, Expert Opinion on Drug Delivery, 2014, 11, pp 791-822, and the like can be referred to. For example, a functional molecule and a linker are bonded to each other by a known method, then the bonded product is converted into an amidite form with an amidite reagent or into an H-phosphonate form with an H-phosphonate reagent, and can be bonded to an oligonucleotide.

By purifying the obtained oligonucleotide by reverse phase column chromatography or the like, a sense strand and an antisense strand of dsRNA can be prepared. The dsRNA can be prepared by mixing the sense strand and the antisense strand in an equivalent amount and annealing.

The dsRNA of the present invention can effectively control the expression of the target RNA. Therefore, the present invention can provide a composition containing the dsRNA of the present invention as an active ingredient, for example, for controlling expression of a target RNA by an RNA interference effect. In particular, the dsRNA of the present invention can obtain a high drug efficacy by administration at a low concentration, and a pharmaceutical composition for treating, preventing, or ameliorating a disease associated with enhanced expression of a target RNA such as a metabolic disease, a tumor, or an infection can also be provided in some embodiments.

Compositions including the dsRNA of the present invention can be formulated by known pharmaceutical methods. For example, the composition can be used enterally (for example, orally) or parenterally as a capsule, a tablet, a pill, a liquid, a powder, a granule, a fine granule, a film-coated agent, a pellet, a troche, a sublingual agent, a chewing agent, a buccal agent, a paste, a syrup, a suspension agent, an elixir agent, an emulsion, a coating agent, an ointment, a plaster, a cataplasm, a transdermal absorption formulation, a lotion, an inhalation agent, an aerosol agent, an injection, a suppository, or the like.

In the formulation, the composition can be appropriately combined with a pharmacologically acceptable carrier or a carrier acceptable as food/drink, specifically, sterile water, physiological saline, vegetable oil, a solvent, a base, an emulsifier, a suspension agent, a surfactant, a pH adjuster, a stabilizer, a flavoring agent, a fragrance, an excipient, a vehicle, a preservative, a binder, a diluent, an isotonizing agent, a pain-relieving agent, an extender, a disintegrant, a buffer, a coating agent, a lubricant, a coloring agent, a sweetening agent, a thickening agent, a corrigent, a solubilizing agent, other additives, or the like.

The form of administration of the composition containing the dsRNA of the present invention is not particularly limited, and examples thereof include enteral (oral and the like) or parenteral administration. More preferable examples thereof include intravenous administration, intraarterial administration, intraperitoneal administration, subcutaneous administration, intradermal administration, airway administration, rectal administration, intramuscular administration, intrathecal administration, intracerebroventricular administration, nasal administration, intravitreal administration, and administration by infusion.

The disease that can be treated, prevented, or ameliorated by a nucleic acid drug using the dsRNA of the present invention is not particularly limited, and examples thereof include a metabolic disease, a circulatory disease, a tumor, an infection, an ocular disease, an inflammatory disease, an autoimmune disease, or a disease caused by expression of a gene such as a genetic rare disease. More specific examples thereof include hypercholesterolemia, hypertriglyceridemia, spinal muscular atrophy, muscular dystrophy (Duchenne muscular dystrophy, myotonic dystrophy, congenital muscular dystrophy (Fukuyama congenital muscular dystrophy, Ullrich congenital muscular dystrophy, merosin-deficient congenital muscular dystrophy, integrin deficiency, Walker-Warburg syndrome, or the like), Becker muscular dystrophy, limb-girdle muscular dystrophy, Miyoshi muscular dystrophy, facioscapulohumeral muscular dystrophy, or the like), Huntington's disease, Alzheimer's disease, transthyretin amyloidosis, familial amyloid cardiomyopathy, multiple sclerosis, Crohn's disease, inflammatory bowel disease, acromegaly, type 2 diabetes, chronic nephropathy, RS virus infection, Ebola hemorrhagic fever, Marburg fever, HIV, influenza, hepatitis B, hepatitis C, liver cirrhosis, chronic heart failure, myocardial fibrosis, atrial fibrillation, prostate cancer, melanoma, breast cancer, pancreatic cancer, colorectal cancer, renal cell carcinoma, bile duct cancer, cervical cancer, liver cancer, lung cancer, leukemia, non-Hodgkin lymphoma, atopic dermatitis, glaucoma, age-related macular degeneration, and the like. The gene that causes the disease can be set as the target RNA according to the type of the disease, and the expression control sequence (for example, antisense sequence) can be appropriately set according to the sequence of the target RNA.

In addition to primates such as humans, diseases of various other mammals can be treated, prevented, or ameliorated by the composition including the double-stranded RNA of the present invention or a prodrug thereof. For example, diseases of mammalian species including but not limited to a cow, a sheep, a goat, a horse, a dog, a cat, a guinea pig, or a bovine, an ovine, an equine, a canine, a feline, and a rodent species such as a mouse can be treated. In addition, the composition including the double-stranded RNA can also be applied to other species such as birds (for example, chicken).

When the composition including the dsRNA of the present invention is administered to or taken by an animal including a human, the dose or intake is appropriately selected according to the age, body weight, symptom, and health condition of the subject, the type of the composition (pharmaceutical, food/drink, or the like), and the like, and the dose or intake is preferably 0.0001 mg/kg/day to 100 mg/kg/day.

The dsRNA of the present invention has high metabolic stability as compared with conventional siRNAs while effectively controlling the expression of the target RNA. Therefore, it is possible to provide a method for administering the dsRNA of the present invention to an animal including a human and controlling expression of a target RNA for a longer period of time by an RNA interference effect. In addition, it is also possible to provide a method for treating, preventing, and ameliorating various diseases accompanied by enhanced expression of a target RNA or the like, including administering the composition including dsRNA of the present invention to an animal including human.

Examples of a preferable method using the dsRNA of the present invention include those described below.

A method for controlling a function of a target RNA, including a step of bringing the dsRNA of the present invention into contact with a cell.

A method for controlling a function of a target RNA in a mammal, including a step of administering the pharmaceutical composition including the dsRNA of the present invention to the mammal.

Use of the dsRNA of the present invention for controlling the function of a target RNA in a mammal.

Use of the dsRNA of the present invention for producing a medicament for controlling the function of a target RNA in a mammal.

A method for controlling expression of a target RNA, including a step of bringing the dsRNA of the present invention into contact with a cell.

A method for controlling the expression of a target RNA in a mammal, including a step of administering the pharmaceutical composition including the dsRNA of the present invention to the mammal.

Use of the dsRNA of the present invention for controlling the expression of a target RNA in a mammal.

Use of the dsRNA of the present invention for producing a medicament for controlling the expression of a target RNA in a mammal.

The mammal is preferably a human.

The route of administration is preferably enteral. As another embodiment, the route of administration is non-enteral.

2'-O-XCE nucleotides according to embodiments of the present invention can be produced with reference to WO 2007/102581, The Journal of Organic Chemistry, 2011, 76, pp 3042-3053, WO2017/142054, Organic & Biomolecular Chemistry, 2019, 17, pp 4835-4842, and the like.

Some embodiments of the present invention include the following 5'-vinylphosphonate (VP) modified 2'-O-XCE nucleotides, 5'-cyclopropanephosphonate (CPP) modified 2'-O-XCE nucleotides, and 5'-ethylphosphonate (EP) modified 2'-O-XCE nucleotides.
1. A compound represented by Formula (II) below, or a salt thereof: 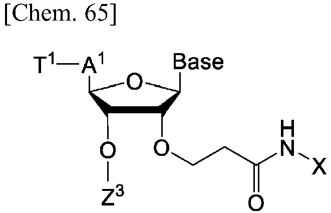
   {in which Base is a purin-9-yl group, a 2-oxo-pyrimidin-1-yl group, or a 2-thioxo-pyrimidin-1-yl group (the purin-9-yl group, the 2-oxo-pyrimidin-1-yl group, and the 2-thioxo-pyrimidin-1-yl group are each independently unsubstituted or substituted with one or more substituents solely or differently selected from the group consisting of a halogen atom, a C1-6 alkyl group, an amino group, a protected amino group, a hydroxy group, a protected hydroxy group, a sulfanyl group, and a protected sulfanyl group),
   Z³ is a hydrogen atom, a hydroxy-protecting group, or a phosphorus-containing group,
   X is a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group), or
   Formula (IIa) below: 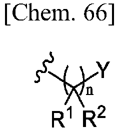
   [in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
   Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)) or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with the nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
   a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
   n is an integer of 1 to 3, and when n is 2 or 3, two or three R¹ and R² may be the same or different, respectively],
   T¹ is a group represented by Formula (IIb) below: 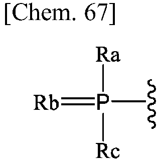
   [Ra and Rc are each independently selected from a hydroxy group, a protected hydroxy group, a thiol group, a protected thiol group, an amino group, a protected amino group, a C1-6 alkyl group, a C2-6 alkenyl group, and a C1-6 alkoxy group (the hydroxy group, the thiol group, the amino group, the C1-6 alkyl group, the C2-6 alkenyl group, and the C1-6 alkoxy group are each independently unsubstituted or substituted with one or more substituents solely or differently selected from the group consisting of a halogen atom, a C1-6 alkyl group, a C1-6 alkoxy group, and a cyano group), and
   Rb is an oxygen atom or a sulfur atom], and
   A¹ is a group represented by Formula (IIc) below: 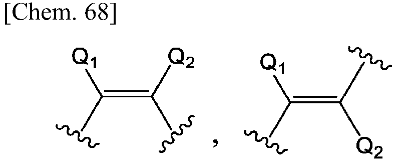
   [Q₁ and Q₂ are each independently a hydrogen atom, a halogen atom, a C1-C6 alkyl group, a C1-6 alkoxy group, a C2-6 alkenyl group, a C2-6 alkynyl group, or an amino group (the C1-C6 alkyl group, the C1-6 alkoxy group, the C2-6 alkenyl group, the C2-6 alkynyl group, and the amino group are each independently unsubstituted or substituted with one or more substituents solely or differently selected from the group consisting of a halogen atom, a C1-6 alkyl group, a C1-6 alkoxy group, and a cyano group)]}.
2. A compound represented by Formula (III) below, or a salt thereof: 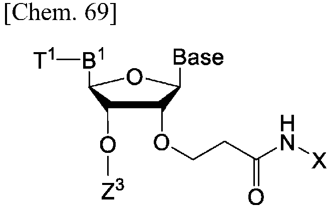
   {in which Base is a purin-9-yl group, a 2-oxo-pyrimidin-1-yl group, or a 2-thioxo-pyrimidin-1-yl group (the purin-9-yl group, the 2-oxo-pyrimidin-1-yl group, and the 2-thioxo-pyrimidin-1-yl group are each independently unsubstituted or substituted with one or more substituents solely or differently selected from the group consisting of a halogen atom, a C1-6 alkyl group, an amino group, a protected amino group, a hydroxy group, a protected hydroxy group, a sulfanyl group, and a protected sulfanyl group),
   Z³ is a hydrogen atom, a hydroxy-protecting group, or a phosphorus-containing group,
   X is a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group), or
   a group represented by Formula (IIIa) below: 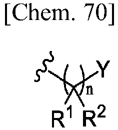
   [in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
   Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)) or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with the nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
   a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
   n is an integer of 1 to 3, and when n is 2 or 3, two or three R¹ and R² may be the same or different, respectively],
   T¹ is a group represented by Formula (IIIb) below: 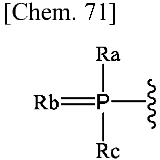
   [Ra and Rc are each independently selected from a hydroxy group, a protected hydroxy group, a thiol group, a protected thiol group, an amino group, a protected amino group, a C1-6 alkyl group, a C2-6 alkenyl group, and a C1-6 alkoxy group (the hydroxy group, the thiol group, the amino group, the C1-6 alkyl group, the C2-6 alkenyl group, and the C1-6 alkoxy group are each independently unsubstituted or substituted with one or more substituents solely or differently selected from the group consisting of a halogen atom, a C1-6 alkyl group, a C1-6 alkoxy group, and a cyano group), and
   Rb is an oxygen atom or a sulfur atom], and
   B¹ is a group represented by Formula (IIIc) below: 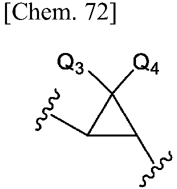
   [Q₃ and Q₄ are each independently a hydrogen atom, a halogen atom, a C1-C6 alkyl group, a C1-6 alkoxy group, a C2-6 alkenyl group, a C2-6 alkynyl group, or an amino group (the C1-C6 alkyl group, the C1-6 alkoxy group, the C2-6 alkenyl group, the C2-6 alkynyl group, and the amino group are each independently unsubstituted or substituted with one or more substituents solely or differently selected from the group consisting of a halogen atom, a C1-6 alkyl group, a C1-6 alkoxy group, and a cyano group)]}.
3. A compound represented by Formula (IV) below, or a salt thereof: 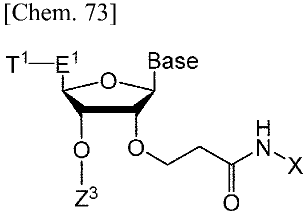
   {in which Base is a purin-9-yl group, a 2-oxo-pyrimidin-1-yl group, or a 2-thioxo-pyrimidin-1-yl group (the purin-9-yl group, the 2-oxo-pyrimidin-1-yl group, and the 2-thioxo-pyrimidin-1-yl group are each independently unsubstituted or substituted with one or more substituents solely or differently selected from the group consisting of a halogen atom, a C1-6 alkyl group, an amino group, a protected amino group, a hydroxy group, a protected hydroxy group, a sulfanyl group, and a protected sulfanyl group),
   Z³ is a hydrogen atom, a hydroxy-protecting group, or a phosphorus-containing group,
   X is a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group), or
   a group represented by Formula (IVa) below: 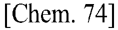 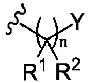
   [in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
   Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)) or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with the nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
   a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
   n is an integer of 1 to 3, and when n is 2 or 3, two or three R¹ and R² may be the same or different, respectively],
   T¹ is a group represented by Formula (IVb) below: 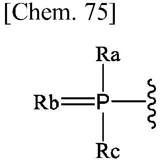
   [Ra and Rc are each independently selected from a hydroxy group, a protected hydroxy group, a thiol group, a protected thiol group, an amino group, a protected amino group, a C1-6 alkyl group, a C2-6 alkenyl group, and a C1-6 alkoxy group (the hydroxy group, the thiol group, the amino group, the C1-6 alkyl group, the C2-6 alkenyl group, and the C1-6 alkoxy group are each independently unsubstituted or substituted with one or more substituents solely or differently selected from the group consisting of a halogen atom, a C1-6 alkyl group, a C1-6 alkoxy group, and a cyano group), and
   Rb is an oxygen atom or a sulfur atom], and
   E¹ is a group represented by Formula (IVc) below: 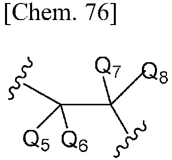
   [Q₅ to Q₈ are each independently a hydrogen atom, a halogen atom, a C1-C6 alkyl group, a C1-6 alkoxy group, a C2-6 alkenyl group, a C2-6 alkynyl group, or an amino group (the C1-C6 alkyl group, the C1-6 alkoxy group, the C2-6 alkenyl group, the C2-6 alkynyl group, and the amino group are each independently unsubstituted or substituted with one or more substituents solely or differently selected from the group consisting of a halogen atom, a C1-6 alkyl group, a C1-6 alkoxy group, and a cyano group)]}.

The 5'-vinylphosphonate (VP) modified 2'-O-XCE nucleotide, the 5'-cyclopropanephosphonate (CPP) modified 2'-O-XCE nucleotide, and the 5'-ethylphosphonate (EP) modified 2'-O-XCE nucleotide according to the present embodiment can be produced by the following method, but the following production method shows an example of a general production method, and does not limit the method for producing the nucleotide and the like according to the present embodiment.

The 5'-vinylphosphonate (VP) modified 2'-O-XCE nucleotide (3'-amidite form) can be synthesized, for example, by a method according to the method described in J. Med. Chem. 2018, 61, 734-744. Specifically, it can be produced by protecting the hydroxy group at position 3' of a 2'-O-XCE nucleotide (for example, with a tert-butyldimethylsilyl group or the like), then converting the hydroxy group at position 5' into an aldehyde (for example, using Dess-Martin reagent, and the like) by an oxidation reaction, then converting the aldehyde into an olefin by a Horner-Wadsworth-Emmons reaction or the like using tetrakis[(pivaloyloxy)methyl] methylenediphosphonate, then deprotecting the hydroxy protecting group at the 3' position, and further amiditing the resultant with an amiditing reagent.

The 5'-cyclopropanephosphonate (CPP) modified 2'-O-XCE nucleotide (3'-amidite form) can be synthesized, for example, by a method according to the method described in Chem. Commun., 2021, 57, 6808. Specifically, it can be produced by protecting the hydroxy group at position 3' of a 2'-O-XCE nucleotide (for example, with a tert-butyldimethylsilyl group or the like), then converting the hydroxy group at position 5' into an aldehyde (for example, using Dess-Martin reagent, and the like) by an oxidation reaction, then converting the aldehyde into an olefin via a Wittig reaction or the like using O,O-diethyl P-[(triphenylphosphoranylidene)methyl]phosphonothioate, then converting the olefin into a cyclopropanated form via a Corey-Chaykovsky reaction or the like, then deprotecting the hydroxy protecting group at the 3' position, then converting the thiophosphonate into a phosphonate via an oxidation reaction (for example, using Oxone or the like), and further amiditing the resultant with an amiditing reagent."

The 5'-ethylphosphonate (EP) modified 2'-O-XCE nucleotide (3'-amidite form) can be produced by reducing an olefin of a synthesis precursor of a 5'-vinylphosphonate (VP) modified 2'-O-XCE nucleotide (3'-amidite form) by hydrogenation reaction or the like, and further amiditing the resultant with an amidite reagent.

The oligonucleotide containing a 5'-vinylphosphonate (VP) modified 2'-O-XCE nucleotide, a 5'-cyclopropanephosphonate (CPP) modified 2'-O-XCE nucleotide, and a 5'-ethylphosphonate (EP) modified 2'-O-XCE nucleotide at the 5' end according to the present embodiment can be produced by solid phase synthesis or the like using each 3'-amidite described above, a commercially available phosphoramidite reagent necessary for producing an oligonucleotide analog having a desired nucleotide sequence, and the like using an automatic nucleic acid synthesizer (for example, nS-8II (manufactured by GeneDesign Inc.)).

The oligonucleotide including the 5'-vinylphosphonate (VP) modified 2'-O-XCE nucleotide and the 5'-ethylphosphonate (EP) modified 2'-O-XCE nucleotide at the 5' end can be produced by treating the solid phase carrier after the reaction using the above-described automatic nucleic acid synthesizer with an amine (for example, ammonia, methylamine, diethylamine, and the like).

The oligonucleotide containing a 5'-cyclopropanephosphonate (CPP) modified 2'-O-XCE nucleotide at the 5' end can be produced by treating the solid phase carrier after the reaction using the above-described automatic nucleic acid synthesizer with, for example, a mixed solution of trimethylsilyl iodide, pyridine, and acetonitrile, and then treating the solid phase carrier with an amine (for example, ammonia, methylamine, diethylamine, and the like).

### Examples

Hereinafter, the present invention will be described more specifically based on Examples, but the following Examples do not limit the scope of the present invention. In addition, "NMR" means nuclear magnetic resonance, "LC/MS" means high performance liquid chromatography/mass spectrometry, "(v/v)" means (volume/volume), and "(w/v)" means (mass/volume).

When ¹H-NMR data are described, they represent chemical shift δ (unit: ppm) (split pattern, integral value) of a signal measured at 400 MHz (JNM-ECZ400; manufactured by JEOL Ltd.) or JNM-ECX300; manufactured by JEOL Ltd.) using tetramethylsilane as an internal standard. Chemical shift δ (unit: ppm) (split pattern, integral value) of a signal with tetramethylsilane as an internal standard is shown. "s" means a singlet, "d" means a doublet, "t" means a triplet, "dd" means a doublet of doublets, "m" means a multiplet, "br" means a broad, "br s" means a broad singlet, "J" means a coupling constant, "CDCl₃" means deuterated chloroform, "DMSO-D₆" means deuterated dimethyl sulfoxide, and "ACETONITRILE-D₃" means deuterated acetonitrile.

When ³¹P-NMR data are described, they represent chemical shift δ (unit: ppm) of a signal measured at 162 MHz (JNM-ECZ400; manufactured by JEOL Ltd.) using phosphoric acid as an internal standard.

LC/MS was measured using an ESI (electrospray ionization) method using any one of the following conditions 1 to 4 unless otherwise specified. "ESI⁺" means an ESI positive ion mode and "ESI⁻" means an ESI negative ion mode. Hereinafter, the mixing ratio of the two solutions described in the LC/MS conditions is a volume ratio, and the content of formic acid is expressed in volume%.

### LC/MS condition 1:

Apparatus: Waters ACQUITY UPLC H CLASS/QDa Detector
Column: Waters ACQUITY UPLC BEH C18 (1.7 µm, 2.1 × 50 mm)
Column temperature: 40°C

### Solvent:

Liquid A: 0.1% aqueous formic acid solution
Liquid B: 0.1% formic acid-acetonitrile solution

### Gradient conditions:

Measurement was started at a flow rate of 0.6 mL/min and a mixing ratio of the liquid A and the liquid B of 90/10, and then the mixing ratio of the liquid A and the liquid B was linearly changed to 10/90 in 3 minutes.

Thereafter, the mixing ratio of the liquid A and the liquid B was fixed to 10/90 for 0.7 minutes.

Thereafter, the mixing ratio of liquid A to liquid B and the flow rate were linearly changed to 90/10 and 0.8 mL/min, respectively, over 0.1 minutes.

Thereafter, the mixing ratio of the liquid A and the liquid B was fixed at 90/10 for 1 minute.

Detection wavelength: PDA (190-400nm)

### LC/MS condition 2:

Apparatus: Waters ACQUITY UPLC H CLASS/SQ Detector 2
Column: Waters ACQUITY UPLC BEH C18 (1.7 µm, 2.1 × 50 mm)
Column temperature: 40°C

### Solvent:

Liquid A: 0.1% aqueous formic acid solution
Liquid B: 0.1% formic acid-acetonitrile solution

### Gradient conditions:

Measurement was started at a flow rate of 0.6 mL/min and a mixing ratio of the liquid A and the liquid B of 90/10, and then the mixing ratio of the liquid A and the liquid B was linearly changed to 10/90 in 3 minutes.

Thereafter, the mixing ratio of the liquid A and the liquid B was fixed to 10/90 for 0.7 minutes.

Thereafter, the mixing ratio of liquid A to liquid B and the flow rate were linearly changed to 90/10 and 0.8 mL/min, respectively, over 0.1 minutes.

Thereafter, the mixing ratio of the liquid A and the liquid B was fixed at 90/10 for 1 minute.

Detection wavelength: PDA (190-400 nm)

### LC/MS condition 3:

Apparatus: Waters ACQUITY UPLC H CLASS/SQ Detector 2
Column: Waters ACQUITY UPLC BEH C18 (1.7 µm, 2.1 × 50 mm)
Column temperature: 40°C

### Solvent:

Liquid A: 10 mmol/L ammonium bicarbonate aqueous solution
Liquid B: 10 mmol/L ammonium bicarbonate-acetonitrile solution (containing 10% of the total solution amount of water)

### Gradient conditions:

Measurement was started at a flow rate of 0.6 mL/min and a mixing ratio of the liquid A and the liquid B of 91/9, and then the mixing ratio of the liquid A and the liquid B was linearly changed to 19/81 in 3 minutes.

Thereafter, the mixing ratio of the liquid A and the liquid B was fixed to 19/81 for 0.7 minutes.

Thereafter, the mixing ratio of liquid A to liquid B and the flow rate were linearly changed to 91/9 and 0.8 mL/min, respectively, over 0.1 minutes.

Thereafter, the mixing ratio of the liquid A and the liquid B was fixed at 91/9 for 1 minute.

Detection wavelength: PDA (190-400 nm)

### LC/MS condition 4:

Apparatus: Waters ACQUITY UPLC H CLASS/SQ Detector 2
Column: Waters ACQUITY UPLC BEH C18 (1.7 µm, 2.1 × 50 mm)
Column temperature: 40°C

### Solvent:

Liquid A: 10 mmol/L ammonium bicarbonate aqueous solution
Liquid B: 10 mmol/L ammonium bicarbonate-acetonitrile solution (containing 10% of the total solution amount of water)

### Gradient conditions:

Measurement was started at a flow rate of 0.6 mL/min and a mixing ratio of the liquid A and the liquid B of 82/18, and then the mixing ratio of the liquid A and the liquid B was linearly changed to 10/90 in 2.5 minutes.

Thereafter, the mixing ratio of the liquid A and the liquid B was fixed at 19/81 for 1.2 minutes.

Thereafter, the mixing ratio of liquid A to liquid B and the flow rate were linearly changed to 82/18 and 0.8 mL/min, respectively, over 0.1 minutes.

Thereafter, the mixing ratio of the liquid A and the liquid B was fixed at 82/18 for 1 minute.

Detection wavelength: PDA (190-400 nm)

For purification by silica gel column chromatography, Purif-Pack (registered trademark)-EX (SI-50 µm) manufactured by Shoko Science Co., Ltd. was used unless otherwise specified.

For purification by silica gel reverse phase column chromatography, either Xbridge PREP C18 5 µm 19 × 100 mm or Xselect CSH Prep C18 5 µm 19 × 100 mm was used unless otherwise specified.

The contents and addition amounts of formic acid and triethylamine to the solvent used for silica gel column chromatography were expressed in volume%.

Purification by supercritical fluid chromatography was performed under the following conditions.
Apparatus: Waters Prep 80q
Column: DAICEL CHIRALPAK IG (20 mmφ × 250 mm, 5 µm)
Column temperature: 40°C
Mobile phase: carbon dioxide/methanol = 50/50,
Flow rate: 15 mL/min

The single crystal X-ray structural analysis was performed using a single crystal X-ray diffractometer XtaLAB Synergy R, DW system, Hypix (manufactured by Rigaku Corporation), and the measurement was performed according to the instruction manual.

### [Production Example 1]

### Synthesis of Compound 8

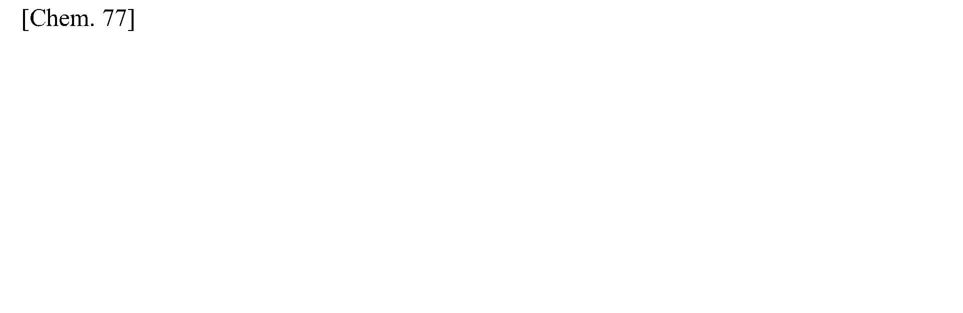

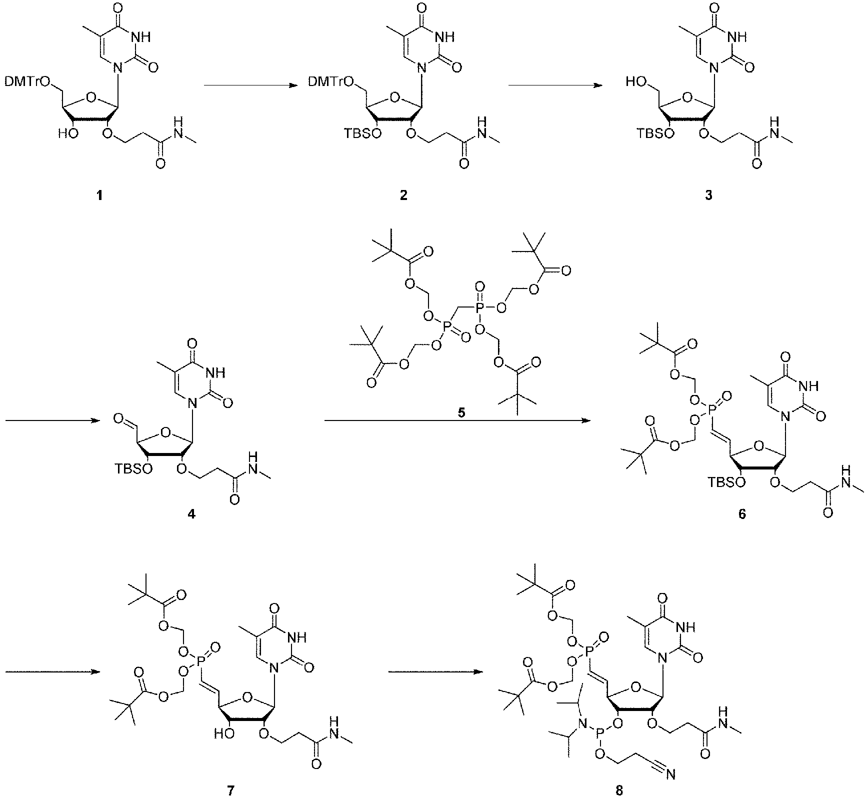

### Step (1): Synthesis of Compound 2

Imidazole (947.7 mg, 13.9 mmol) was added to a mixture of Compound 1 (synthesized according to the method described in WO 2023/054708 A) (3.00 g, 4.64 mmol) and dehydrated N,N-dimethylformamide (9.30 mL), and the mixture was stirred at room temperature for dissolution. tert-butyldimethylchlorosilane (1.40 g, 9.28 mmol) was added to this mixed solution, and the mixture was stirred at room temperature overnight (approximately 16 to 20 hours) under an argon atmosphere. A saturated aqueous sodium bicarbonate solution was added to a reaction mixture, and the mixture was extracted with ethyl acetate. The obtained organic layer was washed with water and a saturated aqueous sodium chloride solution, then dried over sodium sulfate, filtered, and then concentrated under reduced pressure. The obtained residue was purified by silica gel column chromatography (hexane/ethyl acetate) to obtain the desired compound 2 (3.47 g, yield 98%) as a white solid.
LC/MS: Condition 1
LC/MS(ESI-) m/z; 758.1 [M-H]⁻
¹H-NMR (CDCl₃) δ: 8.20 (1H, br s), 7.78-7.77 (1H, m), 7.40-7.38 (2H, m), 7.32-7.28 (7H, m), 6.85-6.83 (4H, m), 6.35-6.34 (1H, m), 5.95 (1H, d, J = 3.3 Hz), 4.34-4.32 (1H, m), 4.10-4.07 (1H, m), 3.95-3.89 (3H, m), 3.80 (6H, s), 3.66 (1H, dd, J = 11.0, 2.2 Hz), 3.26 (1H, dd, J = 11.0, 2.6 Hz), 2.78 (3H, d, J = 4.8 Hz), 2.52-2.48 (2H, m), 1.37-1.37 (3H, m), 0.80 (9H, s), 0.04 (3H, s), -0.07 (3H, s).

### Step (2): Synthesis of Compound 3

1-Dodecanethiol (2.17 mL, 9.13 mmol) was added to a mixture of Compound 2 (3.47 g, 4.57 mmol) and methylene chloride (44.0 mL), then trifluoroacetic acid (0.525 mL, 6.85 mmol) was added thereto, and the mixture was stirred at room temperature for 15 minutes under an argon atmosphere. The saturated aqueous sodium bicarbonate solution was added to the reaction mixture until the pH changed to 8 to 9, and the mixture was extracted with methylene chloride. The obtained organic layer was washed with a saturated aqueous sodium chloride solution, then dried over sodium sulfate, filtered, and then concentrated under reduced pressure. The obtained residue was purified by silica gel column chromatography (hexane/ethyl acetate → ethyl acetate/methanol) to obtain the desired Compound 3 (1.88 g, 90%) as a white solid.
LC/MS: Condition 1
LC/MS(ESI+) m/z; 458.4 [M+H]⁺
¹H-NMR (CDCl₃) δ: 8.44 (1H, br s), 7.55-7.55 (1H, m), 6.22-6.20 (1H, m), 5.70 (1H, d, J = 4.0 Hz), 4.37 (1H, t, J = 5.1 Hz), 4.08-4.03 (2H, m), 4.02-3.98 (1H, m), 3.92-3.90 (1H, m), 3.85-3.74 (2H, m), 2.78 (3H, d, J = 4.8 Hz), 2.54-2.52 (1H, m), 2.48-2.46 (2H, m), 1.93 (3H, d, J = 1.1 Hz), 0.92 (9H, s), 0.12 (6H, s).

### Step (3): Synthesis of Compound 6

1,1,1-Triacetoxy-1,1-dihydro-1,2-benziodoxol-3-(1H)-one (Dess-Martin periodinane) (648.8 mg, 1.5 mmol) was added to a mixture of Compound 3 (500.0 mg, 1.1 mmol) and methylene chloride (25.0 mL) at 0°C. The mixture was stirred at 0°C for 1 hour, and then further stirred at room temperature for 1 hour. After the reaction, a mixed solution of a 10% (w/v) aqueous sodium thiosulfate solution (10.8 mL) and a saturated aqueous sodium bicarbonate solution (10.8 mL) was added at 0°C, and the mixture was extracted with ethyl acetate. The obtained organic layer was dried over sodium sulfate, filtered, and then concentrated under reduced pressure to obtain a crude product containing the desired Compound 4. Subsequently, tetrahydrofuran (2.5 mL) was added to sodium hydride (55 wt% liquid paraffin, 182.1 mg, 3.8 mmol), and Compound 5 was added dropwise thereto at -78°C under an argon atmosphere, then the mixture was stirred at -78°C for 15 minutes. Subsequently, a tetrahydrofuran solution (6.9 mL) of a crude product containing Compound 4 was added dropwise to this reaction solution, and the mixture was stirred at -78°C for 1 hour, then heated to 0°C and stirred for 1 hour, then further heated to room temperature and stirred for 4 hours. After reaction, a saturated aqueous ammonium chloride solution was added, and the mixture was extracted with ethyl acetate. The obtained organic layer was washed with a saturated aqueous sodium chloride solution, then dried over sodium sulfate, filtered, and then concentrated under reduced pressure. The obtained residue was purified by silica gel column chromatography (hexane/ethyl acetate → ethyl acetate/methanol) to obtain the desired Compound 6 (713.0 mg, 2 step yield 86%) as a colorless and transparent oil.
LC/MS: Condition 1
LC/MS(ESI-) m/z; 760.4 [M-H]⁻
¹H-NMR (400 MHz, CDCl₃) δ: 0.10 (s, 6H), 0.88-0.94 (m, 9H), 1.21-1.23 (m, 18H), 1.94-1.98 (m, 3H), 2.45-2.49 (m, 2H), 2.79-2.82 (m, 3H), 3.81-3.86 (m, 1H), 3.89-3.95 (m, 2H), 4.06-4.10 (m, 1H), 4.44-4.48 (m, 1H), 5.63-5.72 (m, 4H), 5.82 (d, J = 3.3 Hz, 1H), 6.07-6.09 (m, 1H), 6.81-6.92 (m, 1H), 7.12-7.12 (m, 1H), 8.01 (s, 1H).
³¹P-NMR (162 MHz, CDCl₃) δ: 14.3, 16.9.

### Step (4): Synthesis of Compound 7

A mixed solution of formic acid/water (35.7 mL, 1/1 (v/v)) was added to Compound 6 (713.0 g, 0.94 mmol), and the mixture was stirred at room temperature for 8 hours. Then, a mixed solution of formic acid/water (35.7 mL, 1/1 (v/v)) was added to the reaction solution, and the mixture was stirred at room temperature for 13 hours. Methanol was added to the reaction mixture, and the mixture was stirred and then concentrated under reduced pressure. The obtained residue was purified by silica gel column chromatography (chloroform/methanol) to obtain a crude product containing the desired Compound 7. The crude product was purified by silica gel reverse phase column chromatography (0.1% trifluoroacetic acid-acetonitrile/0.1% trifluoroacetic acid aqueous solution) to obtain the desired Compound 7 (400.1 mg, yield 66%) as a colorless transparent oil.
LC/MS: Condition 4
LC/MS(ESI+) m/z; 648.7 [M+H]⁺
¹H NMR (400 MHz, DMSO-D₆) δ: 0.97-1.17 (m, 18H), 2.06 (s, 3H), 2.31-2.36 (m, 2H), 2.54 (d, 3H), 3.64-3.79 (m, 3H), 4.10-4.19 (m, 2H), 4.30-4.33 (m, 1H), 5.58-5.62 (m, 4H), 5.78 (d, 1H), 6.02-6.12 (m, 1H), 6.77-6.89 (m, 1H), 7.44-7.44 (m, 1H), 7.84-7.85 (m, 1H), 11.40 (s, 1H)
³¹P-NMR (162 MHz, DMSO-D₆) δ: 17.9

### Step (5): Synthesis of Compound 8

4,5-dicyanoimidazole (80.3 mg, 0.68 mmol) and 2-cyanoethyl N,N,N',N'-tetraisopropylphosphorodiamidite (235.2 µL, 0.74 mmol) were added to a mixture of Compound 7 (400.1 mg, 0.62 mmol) and acetonitrile (8.0 mL). The reaction mixture was then stirred at room temperature for 2 hours. A 1.5% aqueous potassium hydrogen carbonate solution (8.0 mL) was added to the reaction mixture, and the mixture was stirred. Then, hexane (2.0 mL) and ethyl acetate (2.0 mL) were added thereto, the mixture was stirred for 10 minutes, and then an aqueous layer was removed. Next, a 1.5% aqueous potassium hydrogen carbonate solution (8.0 mL) was added to the organic layer, and the mixture was stirred for 10 minutes, and then the aqueous layer was removed. This step was performed once more, water and a saturated aqueous sodium chloride solution were added to the organic layer, and the mixture was stirred. Then, the aqueous layer was removed, and the organic layer was concentrated under reduced pressure. The obtained residue was purified by silica gel column chromatography (ethyl acetate containing 0.15% triethylamine/methanol) to obtain the desired Compound 8 (197.7 mg, yield 38%) as a white solid.
LC/MS: Condition 3
LC/MS(ESI-) m/z; 846.3, 846.2 [M-H]⁻
¹H-NMR (400 MHz, ACETONITRILE-D₃) δ: 1.17-1.23 (m, 30H), 1.82-1.83 (m, 2H), 2.07-2.10 (m, 3H), 2.28-2.36 (m, 2H), 2.62 (t, 3H), 2.66-2.76 (m, 2H), 3.59-3.89 (m, 4H), 4.09-4.13 (m, 1H), 4.24-4.37 (m, 1H), 4.51-4.58 (m, 1H), 5.57-5.65 (m, 4H), 5.82-5.84 (m, 1H), 6.04-6.14 (m, 1H), 6.32-6.35 (m, 1H), 6.78-7.00 (m, 1H), 7.16-7.17 (m, 1H).
³¹P-NMR (162 MHz, ACETONITRILE-D₃) δ: 150.4, 150.3, 17.3, 16.9.

### [Production Example 2]

### Synthesis of Compound 14

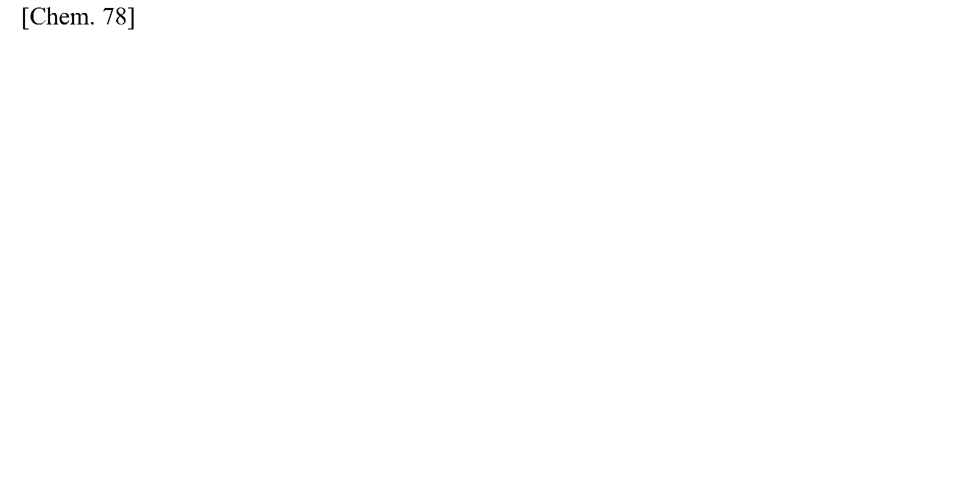

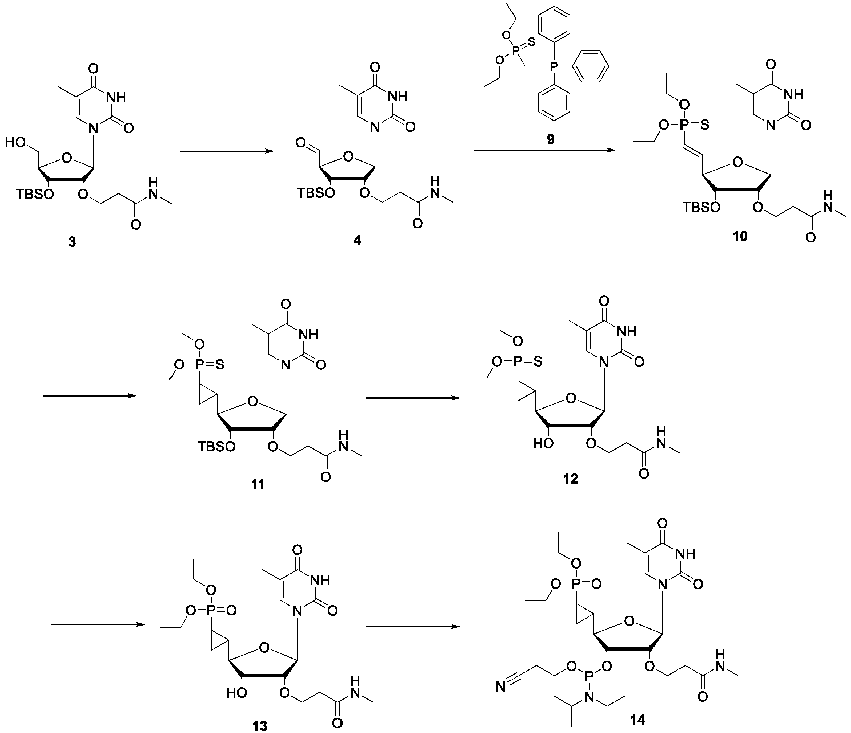

### Step (1): Synthesis of Compound 10

A mixture of Compound 3 (1.06 g, 2.32 mmol) and methylene chloride (46.5 mL) was cooled to 0°C, and 1,1,1-triacetoxy-1,1-dihydro-1,2-benziodoxol-3-(1H)-one (Dess-Martin periodinane) (1.18 g, 2.79 mmol) and saturated sodium bicarbonate (19.5 mg, 0.23 mmol) were added thereto. The mixture was stirred at 0°C for 1 hour under an argon atmosphere, and then stirred at room temperature for 30 minutes. A mixed solution of a saturated aqueous sodium bicarbonate solution and a saturated aqueous sodium thiosulfate solution (40.0 mL, 1/1 (v/v)) was added to the reaction mixture, and the mixture was extracted with ethyl acetate. The organic layer was washed with a saturated aqueous sodium chloride solution, then dried over sodium sulfate, filtered, and then concentrated under reduced pressure to obtain a crude product (1.08 g) of the desired Compound 4 as a white solid. This crude product (1.07 mmol) was dissolved in dehydrated tetrahydrofuran (8.0 mL), and a tetrahydrofuran solution (6.0 mL) of Compound 9 (1.19 g, 2.79 mmol; synthesized according to the method described in Chem. Commun., 2021, 57, 6808) was added dropwise at 0°C under an argon atmosphere, then the temperature was raised to room temperature, and the mixture was stirred overnight (approximately 16 to 20 hours). The reaction mixture was concentrated under reduced pressure, and the obtained residue was purified by silica gel column chromatography (hexane/ethyl acetate → ethyl acetate/methanol) to obtain the desired Compound 10 (976.7 mg, 2 step yield 69%) as a white solid.
LC/MS: Condition 1
LC/MS(ESI+) m/z; 606.2 [M+H]⁺
¹H-NMR (CDCl₃) δ: 8.78 (1H, br s), 7.14-7.13 (1H, m), 6.86-6.78 (1H, m), 6.28-6.22 (2H, m), 5.82 (1H, d, J = 2.5 Hz), 4.55-4.49 (1H, m), 4.19-3.93 (6H, m), 3.88-3.86 (2H, m), 2.80-2.79 (3H, m), 2.53-2.45 (2H, m), 1.96-1.96 (3H, m), 1.32 (6H, td, J = 7.2, 1.6 Hz), 0.92 (9H, s), 0.12 (6H, d, J = 3.7 Hz).
³¹P-NMR (162 MHz, CDCl₃) δ: 84.4

### Step (2): Synthesis of Compound 12

Sodium hydride (55 wt% liquid paraffin, 112.9 mg, 4.7 mmol) was added to a mixture of trimethylsulfoxonium iodide (1.04 g, 4.7 mmol) and dehydrated dimethyl sulfoxide (4.0 mL) under an argon atmosphere, and the mixture was stirred at room temperature for 30 minutes. This suspension was added dropwise to a dimethyl sulfoxide solution (3 mL) of Compound 10 (949.8 mg, 1.57 mmol). After completion of the dropwise addition, the mixture was stirred at room temperature for 90 minutes, and then heated and stirred at 40°C for 6 hours. The reaction mixture was cooled to 0°C, ice water was added thereto, and the mixture was extracted with ethyl acetate. The organic layer was washed with water and a saturated aqueous sodium chloride solution, then dried over sodium sulfate, filtered, and then concentrated under reduced pressure to obtain a crude product of the desired Compound 11 as a white solid. Subsequently, a mixed solution of formic acid/water (10.08 mL, 1/1 (v/v)) was added to the crude product of Compound 11 (937.1 mg), and the mixture was stirred at room temperature overnight (approximately 16 to 20 hours). The reaction mixture was concentrated under reduced pressure, and the obtained residue was purified by silica gel column chromatography (chloroform/methanol) to obtain isomer A (455.0 mg, LC/MS (Condition 1) RT = 1.69 min, white solid, yield 60%) and isomer B (204.5 mg, LC/MS (Condition 1) RT = 1.74 min, white solid, yield 27%) of the desired Compound 12, respectively.
Isomer A
LC/MS: Condition 1
LC/MS(ESI+) m/z; 506.3 [M+H]⁺
¹H-NMR (CDCl₃) δ: 8.64 (1H, br s), 7.23-7.22 (1H, m), 5.88-5.86 (2H, m), 4.23-4.21 (1H, m), 4.19-4.03 (4H, m), 4.00-3.97 (2H, m), 3.86-3.80 (1H, m), 3.51-3.48 (1H, m), 2.82 (3H, d, J = 4.5 Hz), 2.60-2.50 (1H, m), 2.45-2.40 (1H, m), 1.96-1.96 (3H, m), 1.66-1.51 (2H, m), 1.36-1.24 (7H, m), 1.23-1.15 (1H, m), 1.11-1.00 (1H, m).
³¹P-NMR (162 MHz, CDCl₃) δ 101.4
Isomer B
LC/MS: Condition 1
LC/MS(ESI+) m/z; 506.3 [M+H]⁺
¹H-NMR (CDCl₃) δ: 8.74 (1H, s), 7.16 (1H, d, J = 1.2 Hz), 5.98-5.96 (1H, m), 5.77 (1H, d, J = 3.7 Hz), 4.25-3.94 (7H, m), 3.88-3.82 (1H, m), 3.54-3.47 (1H, m), 2.82 (3H, d, J = 4.9 Hz), 2.60-2.53 (1H, m), 2.45-2.40 (1H, m), 1.96-1.96 (3H, m), 1.65-1.50 (1H, m), 1.36-1.29 (7H, m), 1.28-1.18 (1H, m), 1.04-0.98 (1H, m).
³¹P-NMR (162 MHz, CDCl₃) δ: 101.0

### Step (3): Synthesis of Isomer A of Compound 13

Compound 12(Isomer A: 455.0 mg, 0.9 mmol) was dissolved in a mixed solution of tetrahydrofuran/water (6.5 mL, 1/1 (v/v)), OXONE (registered trademark), a monopersulfated compound (553.3 mg, 1.8 mmol) was added, and the mixture was stirred at room temperature for 5 hours. After ethanol was added to the reaction mixture, the mixture was filtered, and the resulting filtrate was concentrated under reduced pressure. The residue was purified by silica gel reverse phase column chromatography (0.1% formic acid-acetonitrile/0.1% aqueous formic acid) to give the desired isomer A of Compound 13 (102.25 mg, LC/MS (Condition 1) RT = 1.14 min, yield 23%) as a white solid.
LC/MS: Condition 1
LC/MS(ESI+) m/z; 490.2 [M+H]⁺
¹H-NMR (CDCl₃) δ: 9.43 (1H, br s), 7.22-7.22 (1H, m), 6.32-6.31 (1H, m), 5.82 (1H, d, J = 4.5 Hz), 4.70 (1H, br s), 4.24-4.22 (1H, m), 4.16-4.05 (4H, m), 4.00-3.97 (2H, m), 3.85-3.79 (1H, m), 3.43-3.40 (1H, m), 2.80 (3H, d, J = 4.5 Hz), 2.62-2.39 (2H, m), 1.93 (3H, s), 1.71-1.57 (1H, m), 1.34-1.29 (6H, m), 1.24-1.18 (1H, m), 1.09-0.92 (2H, m).
³¹P-NMR (162 MHz, CDCl₃) δ: 29.6

### Step (3): Synthesis of Isomer A of Compound 13 (Another Method)

Another production example of isomer A of Compound 13 is shown. Compound 12 (isomer A: 485.3 mg, 0.96 mmol) was dissolved in dichloromethane (33 mL), 3-chloroperoxybenzoic acid (containing approximately 30 mass% water) (1.08 g, 1.63 mmol) was added at 0°C, then the temperature was raised to room temperature, and the mixture was stirred for 15 minutes. Thereafter, a mixed solution (1/1 (v/v)) of a 30% (w/v) aqueous sodium thiosulfate solution and a saturated aqueous sodium bicarbonate solution was added to the reaction mixture, and the mixture was stirred at room temperature for 5 minutes. The mixed solution was concentrated under reduced pressure, ethanol and methanol were added to the obtained residue, and the precipitated solid was filtered, and the filtrate was collected in a round-bottom flask. Ethanol was added to the collected solid, and the mixture was filtered. The filtrates obtained by the first and second filtrations were combined and concentrated under reduced pressure. The obtained residue was purified by silica gel reverse phase column chromatography (0.1% formic acid-acetonitrile/0.1% aqueous formic acid) to give the desired isomer A of Compound 13 (241.2 mg, yield 51%) as a white solid.

1 mg of the isomer A of Compound 13 obtained in the step (3) was diluted with 50 µL of acetone and allowed to stand at 25°C for 24 hours to obtain a crystal.

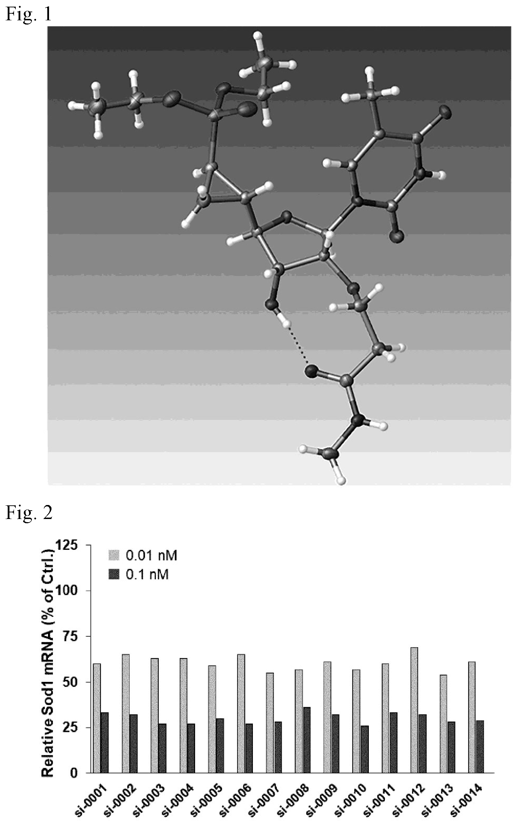

In order to determine the structure of the crystal, single crystal X-ray analysis was performed. Analysis of the obtained diffraction data was performed by integration and absorption correction using software CrysAlisPro and Rigaku, and then an initial structure was obtained by SHELX T (Acta Crystallogr. Sect. A 2015, 71, 3-8) and refined using SHELX L (Acta Crystallogr. Sect. C 2015, 71, 3-8). The results are shown in Table 1, and the ORTEP diagram is shown in Fig. 1.

**[Table 1]**

| Crystal system | Orthorhombic |
|---|---|
| Chemical Formula | C₂₀H₃₂N₃O₉P |
| a (Å) | 4.95490 (10) |
| b (Å) | 13.5250 (2) |
| c (Å) | 35.8549 (6) |
| α (°) | 90 |
| β (°) | 90 |
| γ (°) | 90 |
| V (Å³) | 2402.83 (7) |
| Measurement temperature (K) | 100 |
| Z | 4 |
| Space group | P2₁2₁2 |
| Rint | 0.0382 |
| R1 | 0.0438 |
| wR2 | 0.1211 |
| GoF | 1.129 |
| Flack parameter | 0.000 (15) |

From the above results, the structure of isomer A of Compound 13 was determined to be the following Formula (13A).

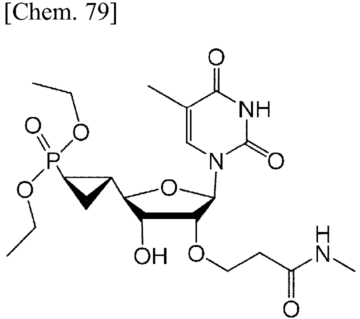

In addition, the structure of isomer B of Compound 13 was determined to be the following Formula (13B).

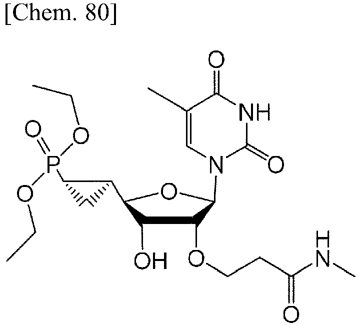

### Step (3'): Synthesis of Isomer B of Compound 13

Compound 12 (isomer B: 204.5 mg, 0.40 mmol) was dissolved in a mixed solution of tetrahydrofuran/water (3.2 mL, 1/1 (v/v)), OXONE (registered trademark), a monopersulfated compound (248.1 mg, 0.81 mmol), was added, and the mixture was stirred at room temperature for 5 hours. After ethanol was added to the reaction mixture, the mixture was filtered, and the resulting filtrate was concentrated under reduced pressure. The residue was purified by silica gel reverse phase column chromatography (0.1% formic acid-acetonitrile/0.1% aqueous formic acid) to obtain the desired isomer B of Compound 13 (76.60 mg, LC/MS (Condition 1) RT = 1.17 min, yield 39%) as a colorless transparent oil.
LC/MS: Condition 1
LC/MS(ESI+) m/z; 490.3 [M+H]⁺
¹H-NMR (CDCl₃) δ: 8.90 (1H, br s), 7.17-7.17 (1H, m), 6.07-6.06 (1H, m), 5.77 (1H, d, J = 3.7 Hz), 4.20-4.07 (5H, m), 4.02-3.97 (2H, m), 3.88-3.82 (1H, m), 3.42 (1H, dd, J = 8.4, 5.9 Hz), 2.82 (3H, d, J = 4.9 Hz), 2.59-2.54 (1H, m), 2.45-2.40 (1H, m), 1.95-1.95 (3H, m), 1.68-1.55 (1H, m), 1.35 (6H, td, J = 7.0, 2.0 Hz), 1.29-1.17 (1H, m), 1.08-0.92 (2H, m).
³¹P-NMR (162 MHz, CDCl₃) δ: 29.7

### Step (3'): Synthesis of Isomer B of Compound 13 (Another Method)

Another production example of isomer B of Compound 13 is shown. Compound 12 (isomer B: 1.83 g, 3.62 mmol) was dissolved in dichloromethane (124 mL), and 3-chloroperbenzoic acid (containing approximately 30 mass% water) (3.70 g, 5.61 mmol) was added at 0°C, and the mixture was stirred for 15 minutes. Thereafter, a mixed solution (1/1 (v/v)) of a 30% (w/v) aqueous sodium thiosulfate solution and a saturated aqueous sodium bicarbonate solution was added to the reaction mixture, and the mixture was stirred at room temperature for 5 minutes. The mixed solution was concentrated under reduced pressure, ethanol was added to the obtained residue, and the precipitated solid was filtered, and the filtrate was collected in a round-bottom flask. Silica gel was added to the filtrate, and the mixture was concentrated under reduced pressure. The mixture was concentrated, then purified by silica gel column chromatography (ethyl acetate/methanol) to obtain isomer B of the desired Compound 13 (1.99 g, crude purification) as a yellow oil.

### Step (4): Synthesis of Isomer A of Compound 14

Under an argon atmosphere, a methylene chloride solution (0.6 mL) of 4,5-dicyanoimidazole (10.8 mg, 0.092 mmol) and 2-cyanoethyl N,N,N',N'-tetraisopropylphosphorodiamidite (0.109 mL, 0.34 mmol) was added to a mixture of isomer A of Compound 13 (112.0 mg, 0.23 mmol) and dehydrated methylene chloride (1.4 mL), and the mixture was stirred at room temperature for 5 hours. The reaction mixture was purified by silica gel column chromatography (chloroform (added with 0.2% triethylamine)/methanol) to obtain the desired isomer A of Compound 14 (81.2 mg) as a white solid. Furthermore, the isomer A (50 mg, 0.10 mmol) of Compound 13 recovered by this column purification was dissolved in dehydrated methylene chloride (0.6 mL), and a methylene chloride solution (0.32 mL) of 4,5-dicyanoimidazole (7.2 mg, 0.061 mmol) and 2-cyanoethyl N,N,N',N'-tetraisopropylphosphorodiamidite (0.0486 mL, 0.15 mmol) was added, and then the mixture was stirred at room temperature for 5 hours. The reaction mixture was purified by silica gel column chromatography (chloroform (added with 0.2% triethylamine)/methanol) to obtain the desired isomer A of Compound 14 (50.8 mg) as a white solid. (Total yield: 132.0 mg, yield: 84%)
LC/MS: Condition 3
LC/MS(ESI-) m/z; 688.2, 688.2 [M-H]⁻
¹H-NMR (CDCl₃) δ: 7.25-7.24 (1H, m), 6.43-6.39 (1H, m), 5.79 (1H, dd, J = 20.4, 2.9 Hz), 4.30-4.02 (7H, m), 4.00-3.81 (3H, m), 3.78-3.41 (4H, m), 2.78 (3H, d, J = 4.9 Hz), 2.67-2.65 (2H, m), 2.61-2.39 (2H, m), 1.95-1.94 (3H, m), 1.34-1.28 (8H, m), 1.20-1.18 (12H, m), 1.01-0.95 (2H, m).
³¹P-NMR (162 MHz, CDCl₃) δ: 151.3, 149.9, 29.4, 29.2

### Step (4'): Synthesis of Isomer B of Compound 14

Dehydrated toluene was added to isomer B of Compound 13 (500 mg, 1.02 mmol), and the mixture was concentrated under reduced pressure. Subsequently, under an argon atmosphere, dehydrated methylene chloride (0.9 mL) was added thereto, and 4,5-dicyanoimidazole (120.6 mg, 1.02 mmol) and 2-cyanoethyl N,N,N',N'-tetraisopropylphosphorodiamidite (0.486 mL, 1.53 mmol) were added thereto, then the mixture was stirred at room temperature for 1.5 hours. The reaction mixture was purified by silica gel column chromatography (chloroform (added with 0.2% triethylamine)/methanol) to obtain the desired isomer B of Compound 14 (553 mg, yield 78%) as a colorless transparent oil.
LC/MS: Condition 3
LC/MS(ESI-) m/z; 688.70/688.75 [M-H]⁻
¹H-NMR (400MHz, CDCl₃) δ: 7.16 (1H, s), 6.31 (1H, s), 5.82 (1H, d, J = 16 Hz), 4.35-3.58 (14H, m), 2.84-2.59 (5H, m), 2.48 (2H, s), 1.96 (3H, s), 1.34 (6H, s), 1.20 (14H, s), 1.08-0.94 (2H, m).
³¹P-NMR (162 MHz, CDCl₃) δ: 150.6, 150.2, 29.8, 29.5

### [Production Example 3]

### Synthesis of Compound 16

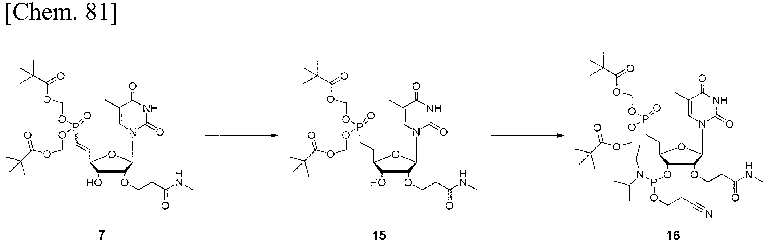

### Step (1): Synthesis of Compound 15

10% palladium-activated carbon (413 mg) was added to a mixture of Compound 7 (EZ mixture, 825.8 mg, 1.28 mmol) and methanol (16.5 mL) under an argon atmosphere. Subsequently, the mixture was stirred at room temperature for 9 hours under a hydrogen atmosphere. After the inside of the reaction system was substituted with argon, ethyl acetate was added, and palladium was removed by centrifugation. The obtained mixture was filtered and then concentrated under reduced pressure. The obtained residue was purified by silica gel column chromatography (chloroform/methanol) to obtain the desired Compound 15 (451.5 mg, yield 54%) as a white solid.
LC/MS: Condition 1
LC/MS(ESI+) m/z; 650.47 [M+H]⁺
¹H-NMR (400 MHz, CDCl₃) δ: 8.52-8.52 (m, 1H), 7.08-7.08 (m, 1H), 5.89-5.89 (m, 1H), 5.75 (d, 1H), 5.71-5.63 (m, 5H), 4.03-3.89 (m, 4H), 3.87-3.82 (m, 2H), 2.84-2.82 (m, 3H), 2.56-2.40 (m, 2H), 2.14-1.89 (m, 5H), 1.24-1.21 (m, 18H).
³¹P-NMR (162 MHz, CDCl₃) δ: 32.8.

### Step (2): Synthesis of Compound 16

4,5-Dicyanoimidazole (82.3 mg, 0.70 mmol), 2-cyanoethyl N,N,N',N'-tetraisopropylphosphorodiamidite (331.8 µL, 1.05 mmol) were added to a mixture of Compound 15 (451.5 mg, 0.70 mmol) and acetonitrile (6.8 mL), and the mixture was stirred at room temperature for 4 hours. Then, 2-cyanoethyl N,N,N',N'-tetraisopropylphosphorodiamidite (110.4 µL, 0.35 mmol) was added to the reaction mixture, and the mixture was stirred at room temperature for 3 hours. After the reaction mixture was concentrated under reduced pressure, the obtained residue was purified by silica gel column chromatography (chloroform (added with 0.2% triethylamine)/methanol) to obtain the desired Compound 16 (323.6 mg, yield 54%) as a white solid.
LC/MS: Condition 3
LC/MS(ESI-) m/z; 848.54, 848.61 [M-H]⁻
¹H-NMR (400 MHz, CDCl₃) δ: 7.11-7.08 (m, 1H), 6.35-6.30 (m, 1H), 5.71-5.64 (m, 4H), 4.08-3.57 (m, 7H), 2.79-2.77 (m, 3H), 2.75-2.60 (m, 2H), 2.55-2.41 (m, 2H), 2.12-2.01 (m, 2H), 1.97-1.95 (m, 3H), 1.24-1.18 (m, 34H).
³¹P-NMR (162 MHz, CDCl₃) δ: 151.0, 150.2, 32.6, 32.4.

### [Production Example 4]

### Synthesis of Compound 23

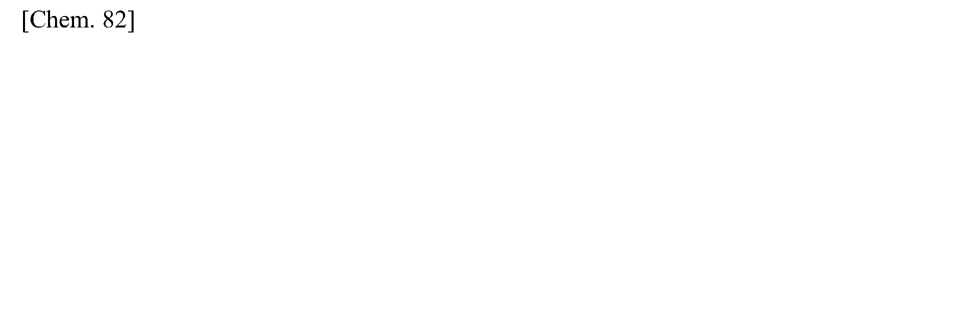

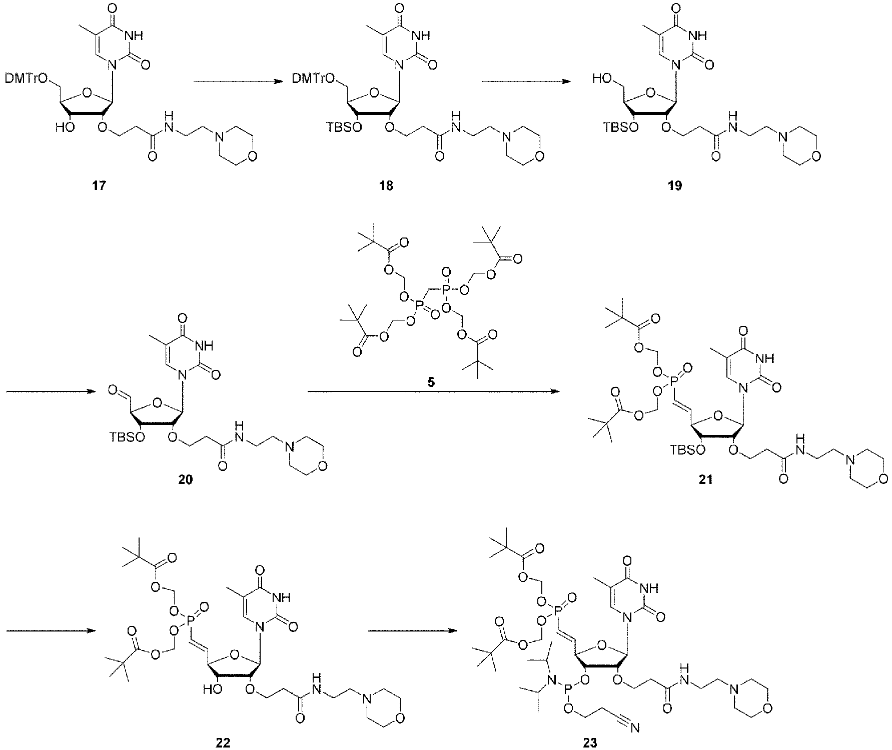

### Step (1): Synthesis of Compound 18

Imidazole (580.0 mg, 8.59 mmol) was added to a mixture of Compound 17 (3.20 g, 4.30 mmol; synthesized according to the method described in Organic & Biomolecular Chemistry (2019), 17(19), 4835-4842) and dehydrated N,N-dimethylformamide (32.0 mL), and the mixture was stirred at room temperature to effect dissolution. tert-butyldimethylchlorosilane (1.17 g, 7.73 mmol) was added to this mixed solution, and the mixture was stirred at room temperature overnight (approximately 16 to 20 hours) under an argon atmosphere. A saturated aqueous sodium bicarbonate solution was added to a reaction mixture, and the mixture was extracted with ethyl acetate. The obtained organic layer was washed with water, then dried over sodium sulfate, filtered, and then concentrated under reduced pressure. The obtained residue was purified by silica gel column chromatography (chloroform/methanol) to obtain the desired Compound 18 (3.1 g, yield 84%) as a white solid.
LC/MS: Condition 4
LC/MS(ESI-) m/z; 859.7 [M-H]⁻
¹H-NMR (CDCl₃) δ: 7.76 (1H, s), 7.40-7.39 (3H, m), 7.32-7.27 (4H, m), 7.18-7.16 (3H, m), 6.85-6.83 (4H, m), 6.02 (1H, d, J = 3.7 Hz), 4.35-4.29 (1H, m), 4.11-4.06 (1H, m), 3.98-3.86 (3H, m), 3.80 (6H, s), 3.74-3.65 (4H, m), 3.65-3.59 (1H, m), 3.41-3.33 (2H, m), 3.28-3.21 (1H, m), 2.53-2.42 (8H, m), 1.40-1.36 (3H, m), 0.82-0.76 (9H, m), 0.05-0.03 (2H, m), -0.06--0.08 (4H, m).

### Step (2): Synthesis of Compound 19

1-Dodecanethiol (0.15 mL, 0.64 mmol) was added to a mixture of Compound 18 (274.0 mg, 0.320 mmol) and methylene chloride (2.74 mL), then trifluoroacetic acid (0.04 mL, 0.48 mmol) was added thereto, and the mixture was stirred at room temperature for 15 minutes under an argon atmosphere. Further, after 2 hours and 10 minutes, 1-dodecanethiol (0.15 mL, 0.64 mmol) was added, then trifluoroacetic acid (0.04 mL, 0.48 mmol) was added, and the mixture was stirred at room temperature for 15 minutes under an argon atmosphere. The saturated aqueous sodium bicarbonate solution was added to the reaction mixture until the pH changed to 8 to 9, and the mixture was extracted with methylene chloride. The obtained organic layer was dried over sodium sulfate, filtered, and then concentrated under reduced pressure. The obtained residue was purified by silica gel column chromatography (chloroform/methanol) to obtain the desired Compound 19 (80.0 mg, yield 45%) as a white solid.
LC/MS: Condition 1
LC/MS(ESI+) m/z; 557.4 [M+H]⁺
¹H-NMR (CDCl₃) δ: 7.53 (1H, s), 5.73 (1H, d, J = 4.4 Hz), 4.37 (1H, t, J = 5.1 Hz), 4.11-4.06 (1H, m), 4.06-4.02 (1H, m), 4.02-3.95 (1H, m), 3.95-3.87 (1H, m), 3.87-3.79 (1H, m), 3.78-3.75 (1H, m), 3.74-3.68 (4H, m), 3.40-3.33 (2H, m), 2.52-2.43 (8H, m), 1.94-1.91 (3H, m), 0.91 (9H, s), 0.11 (6H, s).

### Step (3): Synthesis of Compound 21

To a mixture of Compound 19 (1.40 g, 2.51 mmol) and methylene chloride (70.0 mL) was added 1,1,1-triacetoxy-1,1-dihydro-1,2-benziodoxol-3-(1H)-one (Dess-Martin periodinane) (1.49 g, 3.51 mmol) at 0°C. The mixture was stirred at 0°C for 1 hour, and then further stirred at room temperature for 1 hour. After the reaction, a mixed solution of a 10% (w/v) aqueous sodium thiosulfate solution (18.0 mL) and a saturated aqueous sodium bicarbonate solution (18.0 mL) was added at 0°C, and the mixture was extracted with methylene chloride and ethyl acetate. The obtained organic layer was washed with water and a saturated aqueous sodium chloride solution, then dried over sodium sulfate, filtered, and then concentrated under reduced pressure to obtain a crude product containing the desired Compound 20. Subsequently, tetrahydrofuran (6.96 mL) was added to sodium hydride (55 wt% liquid paraffin, 430.0 mg, 9.79 mmol), and Compound 5 was added dropwise thereto at -78°C under an argon atmosphere, then the mixture was stirred at -78°C for 15 minutes. Subsequently, a tetrahydrofuran solution (13.9 mL) of a crude product containing Compound 20 was added dropwise to this reaction solution, and the mixture was stirred at -78°C for 3 hours, then heated to 0°C, and stirred for 45 minutes. After reaction, a saturated aqueous ammonium chloride solution was added, and the mixture was extracted with ethyl acetate. The obtained organic layer was dried over sodium sulfate, filtered, and then concentrated under reduced pressure. The obtained residue was purified by silica gel column chromatography (hexane/ethyl acetate → ethyl acetate/methanol) to obtain the desired Compound 21 (1.10 g, 2 step yield 51%) as a colorless and transparent oil.
LC/MS: Condition 1
LC/MS(ESI-) m/z; 861.58 [M+H]⁺
¹H-NMR (CDCl₃) δ: 10.66-10.63 (1H, br m), 8.11 (1H, br s), 7.25-6.80 (2H, m), 6.14-6.02 (1H, m), 5.81-5.78 (1H, m), 5.74-5.63 (4H, m), 4.49-4.43 (1H, m), 4.09-3.92 (7H, m), 3.81-3.50 (5H, m), 3.23-3.14 (2H, m), 2.89-2.78 (2H, m), 2.54-2.48 (2H, m), 1.93 (3H, s), 1.23-1.21 (18H, m), 0.92-0.89 (9H, m), 0.12-0.08 (6H, m).

### Step (4): Synthesis of Compound 22

A mixed solution of formic acid/water (135.0 mL, 1/1 (v/v)) was added to Compound 21 (3.0 g, 4.02 mmol), and the mixture was stirred at room temperature for 8 hours. Then, a mixed solution of formic acid/water (35.7 mL, 1/1 (v/v)) was added to the reaction solution, and the mixture was stirred at room temperature for 18 hours. Methanol was added to the reaction mixture, and the mixture was stirred and then concentrated under reduced pressure. The obtained residue was purified by silica gel column chromatography (chloroform/methanol) to obtain a crude product containing the desired Compound 7. The crude product was purified by supercritical fluid chromatography to obtain the desired compound 22(1.05 g, yield 35%) as a colorless transparent oil.
LC/MS: Condition 1
LC/MS(ESI+) m/z; 747.4 [M+H]⁺
¹H-NMR (CDCl₃) δ: 7.26-6.94 (2H, m), 6.17-6.05 (1H, m), 5.95-5.92 (1H, m), 5.75-5.64 (4H, m), 4.55-4.49 (1H, m), 4.15-4.09 (2H, m), 4.00-3.87 (7H, m), 3.79-3.68 (2H, m), 3.23-3.08 (6H, m), 2.65-2.40 (2H, m), 1.95 (3H, s), 1.33-1.16 (18H, m).

### Step (5): Synthesis of Compound 23

To a mixture of Compound 22(950.0 mg, 1.27 mmol) and dichloromethane (9.5 mL) were added diisopropylethylamine (0.865 mL, 5.09 mmol) and 2-cyanoethyl N,N-diisopropylchlorophosphoramidite (0.428 mL, 3.18 mmol), and the mixture was stirred at room temperature for 30 minutes. Furthermore, diisopropylethylamine (0.865 mL, 5.09 mmol) and 2-cyanoethyl N,N-diisopropylchlorophosphoramidite (0.428 mL, 3.18 mmol) were added to the reaction mixture, and the mixture was stirred for 1 hour. The reaction solution was purified by silica gel column chromatography (ethyl acetate containing 0.15% triethylamine/methanol) to obtain the desired compound 23 (781.0 mg, yield 65%) as a yellow oil.
LC/MS: Condition 4
LC/MS(ESI-) m/z; 947.7,947.8
¹H-NMR (CDCl₃) δ: 7.22-6.83 (2H, m), 6.63-6.52 (1H, m), 6.19-6.04 (1H, m), 6.04-5.93 (1H, m), 5.75-5.65 (4H, m), 4.69-4.56 (1H, m), 4.30-4.03 (3H, m), 4.00-3.88 (2H, m), 3.88-3.57 (7H, m), 3.47-3.26 (2H, m), 2.71-2.64 (1H, m), 2.59-2.39 (8H, m), 1.97-1.89 (3H, m), 1.24-1.21 (18H, m), 1.21-1.15 (12H, m).
³¹P-NMR (162 MHz, CDCl₃) δ:151.1, 150.4, 17.7, 17.1

### [Production Example 5]

### Synthesis of Compound 25

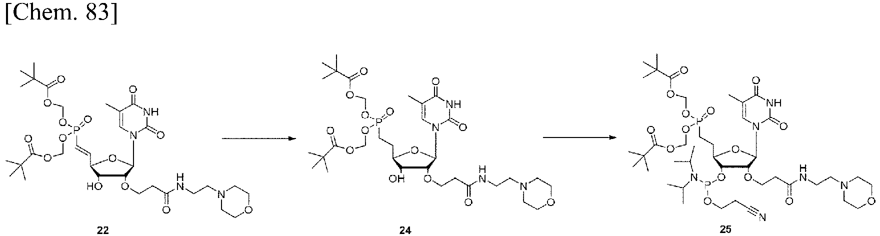

### Step (1): Synthesis of Compound 24

5% palladium-activated carbon (75.0 mg) was added to a mixture of Compound 22 (150.0 mg, 0.20 mmol) and methanol (1.5 mL), and after hydrogen substitution, the mixture was stirred at room temperature for 19 hours and 30 minutes. After argon substitution, the mixture was filtered with celite, washed with a mixed solvent of ethanol/water, and concentrated under reduced pressure. The obtained residue was purified by silica gel column chromatography (chloroform/methanol) to obtain the desired Compound 24 (106.0 mg, yield 70%) as a yellow oil.
LC/MS: Condition 3
LC/MS(ESI+) m/z; 749.6 [M+H]⁺

### Step (2): Synthesis of Compound 25

To a mixture of Compound 24 (450.0 mg, 0.60 mmol) and dichloromethane (4.5 mL) were added diisopropylethylamine (0.410 mL, 2.41 mmol) and 2-cyanoethyl N,N-diisopropylchlorophosphoramidite (0.203 mL, 1.51 mmol), and the mixture was stirred at room temperature for 1 hour. Furthermore, diisopropylethylamine (0.410 mL, 2.41 mmol) and 2-cyanoethyl N,N-diisopropylchlorophosphoramidite (0.203 mL, 1.51 mmol) were added to the reaction mixture, and the mixture was stirred for 20 minutes. Furthermore, diisopropylethylamine (0.820 mL, 4.81 mmol) and 2-cyanoethyl N,N-diisopropylchlorophosphoramidite (0.203 mL, 1.51 mmol) were added to the reaction mixture, and the mixture was stirred for 20 minutes. The reaction solution was purified by silica gel column chromatography (ethyl acetate containing 0.15% triethylamine/methanol) to obtain the desired compound 25 (290.0 mg, yield 51%) as a yellow oil.
LC/MS: Condition 4
LC/MS(ESI-) m/z; 949.8, 949.8 [M-H]⁻
¹H-NMR (CDCl₃) δ: 7.17-7.13 (1H, m), 5.89-5.80 (1H, m), 5.73-5.63 (4H, m), 4.13-3.84 (6H, m), 3.84-3.55 (7H, m), 3.49-3.26 (2H, m), 2.81-2.56 (2H, m), 2.56-2.38 (7H, m), 2.18-1.82 (7H, m), 1.25-1.21 (18H, m), 1.21-1.11 (12H, m).
³¹P-NMR (162 MHz, CDCl₃) δ:150.8, 150.2, 32.7, 32.3

### [Production Example 6] Method for Synthesizing siRNA

### 1-1) Synthesis of Oligonucleotides

The oligonucleotide used in Examples of the present specification was synthesized by a phosphoramidite method using an automatic nucleic acid synthesizer nS-8II (manufactured by GeneDesign Inc.), an automatic nucleic acid synthesizer M-8-SE (manufactured by Nippon Techno Service Co., Ltd.), or a polyspecimen nucleic acid synthesizer NTS T-48 A18R8. A monomer amidite was prepared as a 0.1 M acetonitrile solution. The coupling time was set to 30 seconds to 20 minutes, and 24 to 96 equivalents of the amidite form were used for condensation of one monomer. An acetonitrile solution of 10% (v/v) TBHP [tert-butyl hydroperoxide] (an acetonitrile solution was made using a 70 wt% aqueous TBHP solution) was used for the oxidation, and an acetonitrile/pyridine = 2/3 (v/v) solution of 50 mM DDTT [(dimethylaminomethylidene)amino-3H-1,2,4-dithiazoline-3-thione)] was used for the phosphorothioation. Activator Solution-3 (0.25 mol/L 5-Benzylthio-1H-tetrazole, Acetonitrile Solution) (Wako Pure Chemical Industries, Ltd.) was used as an activator, and Cap A Solution-1 [10 vol% Acetic anhydride/Tetrahydrofuran Solution] (Wako Pure Chemical Industries, Ltd.), and Cap B Solution-1 [Tetrahydrofuran/1-Methylimidazole/Pyridine (8/1/1) Solution] (Wako Pure Chemical Industries, Ltd.) were used as capping reagents. As a detritylation reagent, Deblocking Solution [Dichloroacetic Acid-Toluene (3/97 (v/v))] (Wako Pure Chemical Industries, Ltd.) was used.

### 1-2) Hydrolysis of Oligonucleotides Synthesized Using Compound 14

The oligonucleotide synthesized using Compound 14 was hydrolyzed by reacting a TMSI [iodotrimethylsilane]/pyridine/acetonitrile = 1/13/40 (v/v/v) solution on a solid phase using a synthesizer.

### 2) Cleavage from Solid Phase, Base, and Deprotection

Cleavage was carried out by allowing the mixture to stand at room temperature for 4 hours using 28 mass% aqueous ammonia or diethylamine/28 mass% aqueous ammonia = 3/97 (v/v). Subsequently, the filtrate was heated at 60°C to perform a deprotection reaction. Thereafter, the mixture was concentrated under reduced pressure at 50°C.

### 3) Purification

The oligonucleotide was purified by reverse phase HPLC (manufactured by SHIMADZU).
Conditions of reverse phase HPLC
Solvent
Liquid A: water (1.0% (v/v) hexafluoroisopropanol; 0.11% (v/v) triethylamine
Liquid B: methanol
Gradient condition: Liquid B was changed from 5% to 30%.
Column: WatersXBridge (registered trademark) BEH C18 OBD (registered trademark) Prep Column 130A, 5 µm (10 mm × 250 mm)
Flow rate: 1.0 ml/min
Column temperature: 60°C

### 4) Desalting and Lyophilization of Purified Oligonucleotides

The obtained oligonucleotide was concentrated under reduced pressure, and then freeze-dried to obtain a target oligonucleotide as a powder.

### 5) Confirmation of Structure of Oligonucleotides

The obtained oligonucleotide was subjected to mass spectrometry by ESI-MS under the following analysis conditions, and each was identified as a target product.

### Analysis condition:

Apparatus: Waters ACQUITY UPLC H CLASS/SQ Detector 2
Column: ACQUITY UPLC Oligonucleotide BEH C18 Column (130Å, 1.7 µm, 2.1x50 mm)
Column temperature: 60°C

### Solvent:

Liquid A: water (0.75% (v/v) hexafluoroisopropanol; 0.038% (v/v) triethylamine; 5 µM ethylenediaminetetraacetic acid)
Liquid B: 90/10 (v/v) mixed solution of methanol/water (0.75% (v/v) hexafluoroisopropanol; 0.038% (v/v) triethylamine; 5 µM ethylenediaminetetraacetic acid)

### Gradient conditions:

Measurement was started at a flow rate of 0.36 mL/min and a mixing ratio of the liquid A and the liquid B fixed at 95/5 (v/v), and after 0.56 minutes, the mixing ratio of the liquid A and the liquid B was linearly changed to 40/60 (v/v) in 5.5 minutes.

Detection wavelength: 260 nm

### Deconvolution:

The ion signal was analyzed using ProMass for MassLynx Software (manufactured by Waters).

### 6) Preparation of Double-Stranded Oligonucleotides

After aqueous solutions of the respective oligonucleotides of each sense strand (S) and antisense strand (AS) were mixed to be equimolar amounts, the mixture was heated at 95°C for 5 minutes and then allowed to stand at a constant temperature to obtain a double-stranded nucleic acid. The hybridization was confirmed by non-denaturing polyacrylamide gel electrophoresis.

In the chemical structural representation in Examples, unless otherwise specified, "(M)" means a 2'-OMe nucleotide, "(F)" means a 2'-fluoro nucleotide, "(V)" means a 2'-O-MCE nucleotide, "(Mo)" means a 2'-O-MorECE nucleotide, the lower case alphabet (a, t, g, c, u) (however, alphabets indicating "Galx3_glu_5A6_s#" and "#Gal_s_TRP_s" described later are excluded) means a deoxyribonucleotide, "^" means a phosphorothioate bond, and "5" in "5(V)" means that the nucleobase of the nucleotide is 5-methylcytosine.

"U(vM)" means a 5'-vinylphosphonate-modified 2'-OMe nucleoside represented by the following Formula (iii): 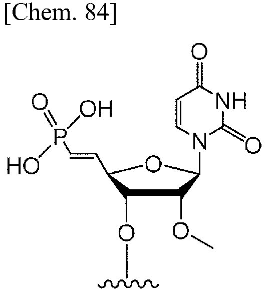
(in the formula, the wavy line represents an oxygen atom of an internucleoside bond such as a phosphodiester bond or a phosphorothioate bond)); "T(vV)" means a 5'-vinylphosphonate-modified 2'-O-MCE nucleoside represented by the following Formula (iv): 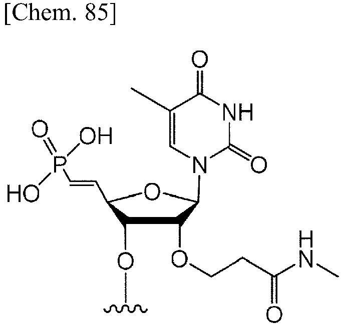
(in the formula, the wavy line represents an oxygen atom of an internucleoside bond such as a phosphodiester bond or a phosphorothioate bond)); "T(cV)" means a 5'-cyclopropanephosphonate-modified 2'-O-MCE nucleoside (isomer A) represented by the following Formula (v): 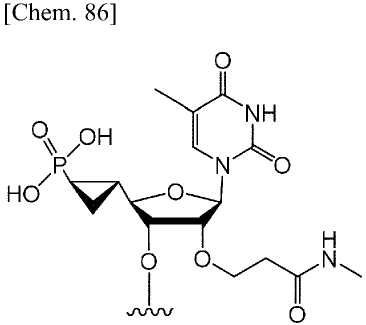
(in the formula, the wavy line represents an oxygen atom of an internucleoside bond such as a phosphodiester bond or a phosphorothioate bond)); "T(dcV)" means a 5'-cyclopropanephosphonate-modified 2'-O-MCE nucleoside (isomer B) represented by the following Formula (vi): 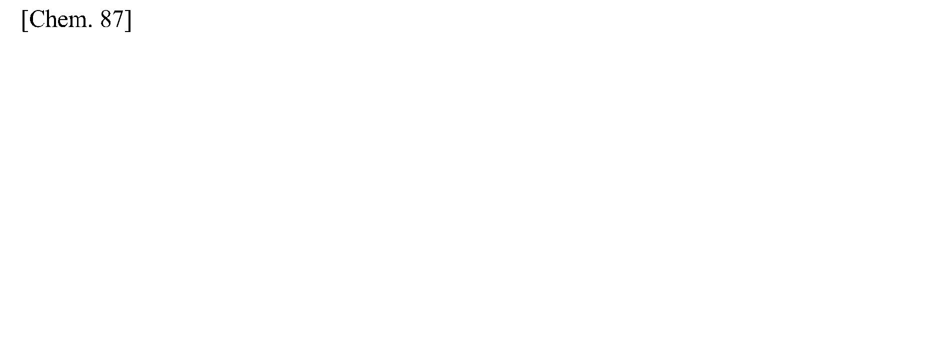 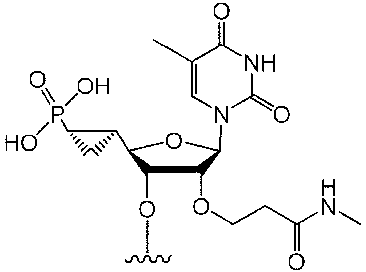
(in the formula, the wavy line represents an oxygen atom of an internucleoside bond such as a phosphodiester bond or a phosphorothioate bond)); "T(eV)" means a 5'-ethylphosphonate-modified 2'-O-MCE nucleoside represented by the following Formula (vii): 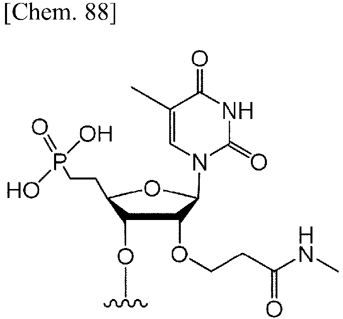
(in the formula, the wavy line represents an oxygen atom of an internucleoside bond such as a phosphodiester bond or a phosphorothioate bond)); "T(vMo)" means a 5'-vinylphosphonate-modified 2'-O-MorECE nucleoside represented by the following formula (viii): 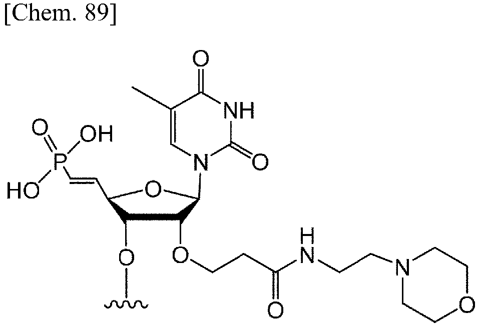
(in the formula, the wavy line represents an oxygen atom of an internucleoside bond such as a phosphodiester bond or a phosphorothioate bond)); and "T(eMo)" means a 5'-ethylphosphonate-modified 2'-O-MorECE nucleoside represented by the following Formula (ix): 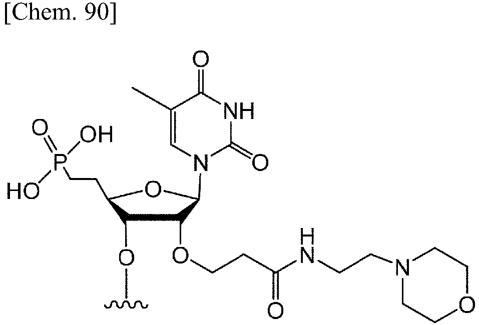
(in the formula, the wavy line represents an oxygen atom of an internucleoside bond such as a phosphodiester bond or a phosphorothioate bond)).

"U(H)" means a 2'-O-hexadecyl nucleoside represented by the following Formula (x): 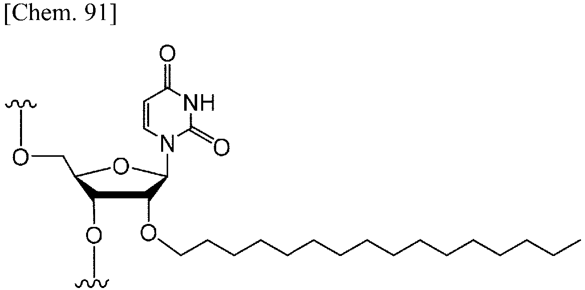 (in the formula, the wavy line represents an oxygen atom of an internucleoside bond such as a phosphodiester bond or a phosphorothioate bond)).

"Galx3_glu_5A6_s#" means a GalNAc ligand represented by the following formula (xi): 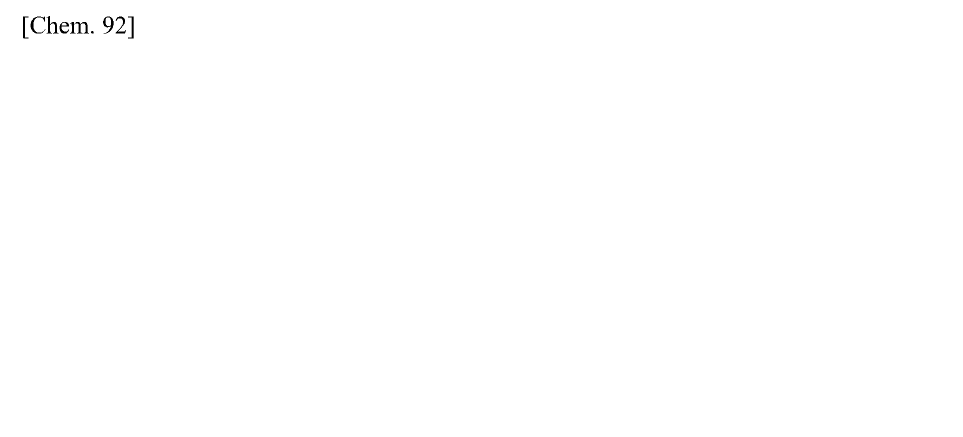 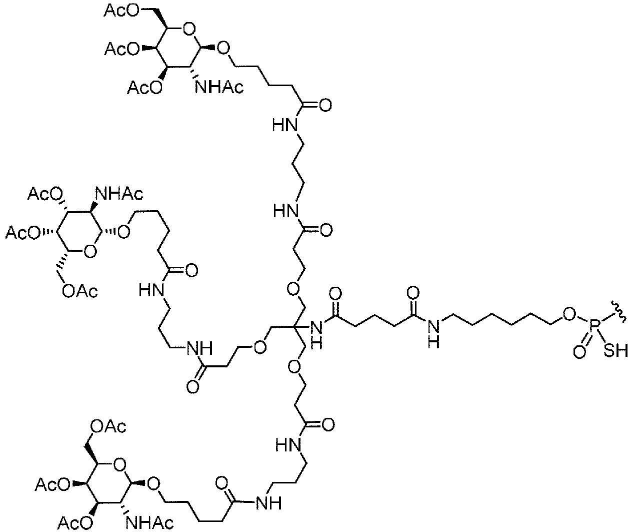
(in the formula, the wavy line represents a bonding position with a nucleoside oxygen atom in an oligonucleotide); and "#Gal_s_TRP_s" means a GalNAc ligand represented by the following formula (xii): 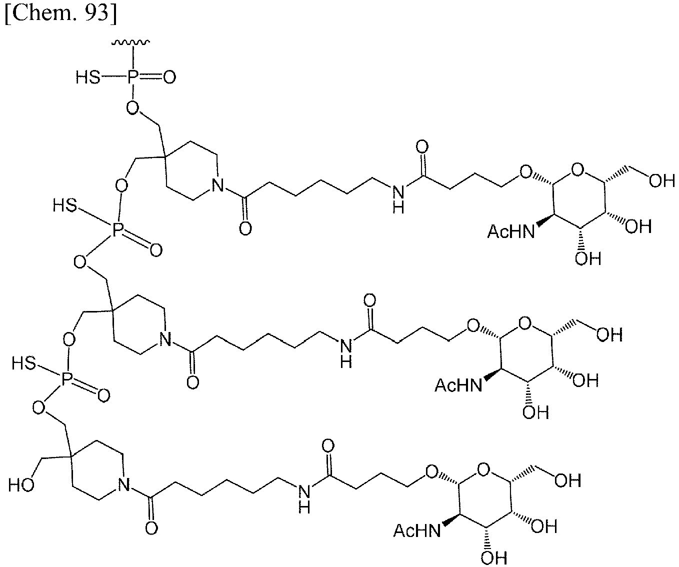
(in the formula, the wavy line represents a bonding position with a nucleoside oxygen atom in an oligonucleotide).

Note that in the notation of a chemical structure in Examples, when "^" is not described between two adjacent nucleotides, the internucleotide bond between the two nucleotides is a phosphodiester bond.

"V" (capital V) and "Mo" in the table are shown in bold for clarity. The bold "V" and the non-bold "V" have the same meaning, and the bold "Mo" and the non-bold "Mo" have the same meaning. The left side of both the sense strand (S) and the antisense strand (AS) is the 5' side, and the right side is the 3' side.

### [Production Example 7] Synthesis of siRNA

According to the method described in Production Example 6, siRNAs described in Tables 2 to 6 were synthesized. The target RNA of the siRNAs described in Tables 2 to 6 is Sod1 (mouse and rat).

Note that notations of each column in tables are as follows.

"Compd. No.": represents the compound number of each siRNA.

"SEQ ID": represents each SEQ ID NO.

"Chemical Structure": represents a chemical structure of each strand.

"Strand": represents whether each strand is a sense strand (S) or an antisense strand (AS).

"Remark": represents remarks.

Modifications to the model sequence are described in the remarks.

"parent" represents the siRNA used as a model.

Modifications from the model siRNA are represented by "α-β-γ(δ)".

"α" indicates whether the modified strand from the model siRNA is the sense strand (S) or the antisense strand (AS). "β" indicates the position number from the 5' end of the modification. "γ" indicates the base of the nucleotide of the modification. "δ" indicates the species of the modification.

For example, "S-1-C(V)" indicates that the base C of the first nucleotide as counted from the 5' end of the sense strand (S) of the model siRNA was modified to a 2'-O-MCE nucleotide, "S-1-5(V)" indicates that the base C of the first nucleotide as counted from the 5' end of the sense strand (S) of the model siRNA was modified to a 2'-O-MCE nucleotide having a 5-methylcytosine base, and "AS-1-U(V)" indicates that the base U of the first nucleotide as counted from the 5' end of the antisense strand (AS) of the model siRNA was modified to a 2'-O-MCE nucleotide.

si-0002 to si-0066 and si-0133 to si-0145 were designed based on si-0001. si-0068 to si-0132 were designed based on si-0067.

**[Table 2]**

| Compd. No. | Strand | SEQ No | Chemical Structure | Remarks |
|---|---|---|---|---|
| si-0001 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | parent |
| | AS | 2 | U(M)^U(**F**)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0002 | S | 3 | C(**V**)^A(M)^U(M)U(M)U(M)U(M)A(F)A(M)U(F)C(F)C(F)U(M)C(M)A(M)C(M)U(M)C(M)U(M)A(M)^A(M)^A(M) | S-1-C(**V**) |
| | AS | 2 | U(M)^U(**F**)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0003 | S | 4 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(M) | S_1_5(**V**) |
| | AS | 2 | U(M)^U(**F**)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0004 | S | 5 | C(M)^A(**V**)^U(M)U(M)U(M)U(M)A(F)A(M)U(F)C(F)C(F)U(M)C(M)A(M)C(M)U(M)C(M)U(M)A(M)^A(M)^A(M) | S_2_A(V) |
| | AS | 2 | U(M)^U(**F**)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0005 | S | 6 | C(M)^A(M)^U(**V**)U(M)U(M)U(M)A(F)A(M)U(F)C(F)C(F)U(M)C(M)A(M)C(M)U(M)C(M)U(M)A(M)^A(M)^A(M) | S-3-U(V) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0006 | S | 7 | C(M)^A(M)^T(**V**)U(M)U(M)U(M)A(F)A(M)U(F)C(F)C(F)U(M)C(M)A(M)C(M)U(M)C(M)U(M)A(M)^A(M)^A(M) | S-3-T(V) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0007 | S | 8 | C(M)^A(M)^U(M)U(**V**)U(M)U(M)A(F)A(M)U(F)C(F)C(F)U(M)C(M)A(M)C(M)U(M)C(M)U(M)A(M)^A(M)^A(M) | S-4-U(V) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0008 | S | 9 | C(M)^A(M)^U(M)T(**V**)U(M)U(M)A(F)A(M)U(F)C(F)C(F)U(M)C(M)A(M)C(M)U(M)C(M)U(M)A(M)^A(M)^A(M) | S-4-T(V) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0009 | S | 10 | C(M)^A(M)^U(M)U(M)U(**V**)U(M)A(F)A(M)U(F)C(F)C(F)U(M)C(M)A(M)C(M)U(M)C(M)U(M)A(M)^A(M)^A(M) | 5-5-U(V) |
| | AS | 2 | U(M)^U(**F**)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0010 | S | 11 | C(M)^A(M)^U(M)U(M)T(**V**)U(M)A(F)A(M)U(F)C(F)C(F)U(M)C(M)A(M)C(M)U(M)C(M)U(M)A(M)^A(M)^A(M) | S-5-T(V) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0011 | S | 12 | C(M)^A(M)^U(M)U(M)U(M)U(**V**)A(F)A(M)U(F)C(F)C(F)C(M)C(M)A(M)C(M)U(M)C(M)U(M)A(M)^A(M)^A(M) | S-6-U(V) |
| | AS | 2 | U(M)^U(**F**)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0012 | S | 13 | C(M)^A(M)^U(M)U(M)U(M)T(**V**)A(F)A(M)U(F)C(F)C(F)U(M)C(M)A(M)C(M)U(M)C(M)U(M)A(M)^A(M)^A(M) | S-6-T(V) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0013 | S | 14 | C(M)^A(M)^U(M)U(M)U(M)U(M)A(**V**)A(M)U(F)C(F)C(F)U(M)C(M)A(M)C(M)U(M)C(M)U(M)A(M)^A(M)^A(M) | S-7-A(V) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0014 | S | 15 | C(M)^A(M)^U(M)U(M)U(M)U(M)A(F)A(**V**)U(F)C(F)C(F)U(M)C(M)A(M)C(M)U(M)C(M)U(M)A(M)^A(M)^A(M) | S-8-A(V) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0015 | S | 16 | C(M)^A(M)^U(M)U(M)U(M)U(M)A(F)A(M)U(**V**)C(F)C(F)U(M)C(M)A(M)C(M)U(M)C(M)U(M)A(M)^A(M)^A(M) | S-9-U(V) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0016 | S | 17 | C(M)^A(M)^U(M)U(M)U(M)U(M)A(F)A(M)T(**V**)C(F)C(F)U(M)C(M)A(M)C(M)U(M)C(M)U(M)A(M)^A(M)^A(M) | S-9-T(V) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0017 | S | 18 | C(M)^A(M)^U(M)U(M)U(M)U(M)A(F)A(M)U(F)C(**V**)C(F)U(M)C(M)A(M)C(M)U(M)C(M)U(M)A(M)^A(M)^A(M) | S-10-C(V) |
| | AS | 2 | U(M)^U(**F**)^U(M)A(M)G(M)A(F)G(M)U(F)G(F)A(M)G(M)G(M)A(M)U(**F**)U(M)A(F)A(M)A(M)A(M)U(M)G(M)^A(M)^G(M) | |
| si-0018 | S | 19 | C(M)'A(M)'U(M)U(M)U(M) U(M)A(F)A(M)U(F)5(**V**)C(F)U(M)C(M)A(M)C(M)U(M)C(M)U(M)A(M)^A(M)^A(M) | S-10-5(**V**) |
| | AS | 2 | U(M)^U(**F**)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0019 | S | 20 | C(M)^A(M)^U(M)U(M)U(M)U(M)A(F)A(M)U(F)C(F)C(**V**)U(M)C(M)A(M)C(M)U(M)C(M)^(M)A(M)^A(M)^A(M) | S-11-C(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0020 | S | 21 | C(M)^A(M)^U(M)U(M)U(M)U(M)A(F)A(M)U(F)C(F)5(**V**)U(M)C(M)A(M)C(M)U(M)C(M)U(M)A(M)^A(M)^A(M) | S-11-5(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0021 | S | 22 | C(M)^A(M)^U(M)U(M)U(M)U(M)A(F)A(M)U(F)C(F)C(F)U(**V**)C(M)A(M)C(M)U(M)C(M)U(M)A(M)^A(M)^A(M) | S-12-U(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0022 | S | 23 | C(M)^A(M)^U(M)U(M)U(M)U(M)A(F)A(M)U(F)C(F)C(F)T(**V**)C(M)A(M)C(M)U(M)C(M)U(M)A(M)^A(M)^A(M) | S-12-T(**V**) |
| | AS | 2 | U(M)^U(**F**)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0023 | S | 24 | C(M)^A(M)^U(M)U(M)U(M)U(M)A(F)A(M)U(F)C(F)C(F)U(M)C(**V**)A(M)C(M)U(M)C(M)U(M)A(M)^A(M)^A(M) | S-13-C(**V**) |
| | AS | 2 | U(M)^U(**F**)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0024 | S | 25 | C(M)^A(M)^U(M)U(M)U(M)U(M)A(F)A(M)U(F)C(F)C(F)U(M)5(**V**)A(M)C(M)U(M)C(M)U(M)A(M)^A(M)^A(M) | S-13-5(**V**) |
| | AS | 2 | U(M)^U(**F**)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0025 | S | 26 | C(M)^A(M)^U(M)U(M)U(M)U(M)A(F)A(M)U(F)C(F)C(F)U(M)C(M)A(**V**)C(M)U(M)C(M)U(M)A(M)^A(M)^A(M) | S-14-A(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0026 | S | 27 | C(M)^A(M)^U(M)U(M)U(M)U(M)A(F)A(M)U(F)C(F)C(F)U(M)C(M)A(M)C(**V**)U(M)C(M)U(M)A(M)^A(M)^A(M) | S-15-C(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0027 | S | 28 | C(M)^A(M)^U(M)U(M)U(M)U(M)A(F)A(M)U(F)C(F)C(F)U(M)C(M)A(M)5(**V**)U(M)C(M)U(M)A(M)^A(M)^A(M) | S-15-5(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0028 | S | 29 | C(M)^A(M)^U(M)U(M)U(M)U(M)A(F)A(M)U(F)C(F)C(F)U(M)C(M)A(M)C(M)U(**V**)C(M)U(M)A(M)^A(M)^A(M) | S-16-U(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0029 | S | 30 | C(M)^A(M)^U(M)U(M)U(M)U(M)A(F)A(M)U(F)C(F)C(F)U(M)C(M)A(M)C(M)T(**V**)C(M)U(M)A(M)^A(M)^A(M) | S-16-T(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0030 | S | 31 | C(M)^A(M)^U(M)U(M)U(M)U(M)A(F)A(M)U(F)C(F)C(F)U(M)C(M)A(M)C(M)U(M)C(**V**)U(M)A(M)^A(M)^A(M) | S-17-C(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0031 | S | 32 | C(M)^A(M)^U(M)U(M)U(M)U(M)A(F)A(M)U(F)C(F)C(F)U(M)C(M)A(M)C(M)U(M)5(**V**)U(M)A(M)^A(M)^A(M) | S-17-5(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0032 | S | 33 | C(M)^A(M)^U(M)U(M)U(M)U(M)A(F)A(M)U(F)C(F)C(F)U(M)C(M)A(M)C(M)U(M)C(M)U(**V**)A(M)^A(M)^A(M) | S-18-U(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0033 | S | 34 | C(M)^A(M)^U(M)U(M)U(M)U(M)A(F)A(M)U(F)C(F)C(F)U(M)C(M)A(M)C(M)U(M)C(M)T(**V**)A(M)^A(M)^A(M) | S-18-T(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0034 | S | 35 | C(M)^A(M)^U(M)U(M)U(M)U(M)A(F)A(M)U(F)C(F)C(F)U(M)C(M)A(M)C(M)U(M)C(M)U(M)A(**V**)^A(M)^A(M) | S-19-A(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0035 | S | 36 | C(M)^A(M)^U(M)U(M)U(M)U(M)A(F)A(M)U(F)C(F)C(F)U(M)C(M)A(M)C(M)U(M)C(M)U(M)A(M)^A(**V**)^A(M) | S-20-A(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0036 | S | 37 | 5(M)^A(M)^U(M)U(M)U(M)U(M)A(F)A(M)U(F)C(F)C(F)U(M)C(M)A(M)C(M)U(M)C(M)U(M)A(M)^A(M)^A(**V**) | S-21-A(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |

**[Table 3]**

| | | | | |
|---|---|---|---|---|
| si-0037 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-1-U(**V**) |
| | AS | 38 | U(**V**)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0038 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-1-T(**V**) |
| | AS | 39 | T(**V**)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0039 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-2-U(**V**) |
| | AS | 40 | U(M)^U(**V**)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0040 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-2-T(**V**) |
| | AS | 41 | U(M)^T(**V**)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0041 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-3-U(**V**) |
| | AS | 42 | U(M)^U(F)^U(**V**)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0042 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-3-T(**V**) |
| | AS | 43 | U(M)^U(F)^T(**V**)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0043 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-4-A(**V**) |
| | AS | 44 | U(M)^U(F)^U(M)A(**V**)G(M)A(F)G(M)U(F)G(F)A(M)G(M)G(M)A(M)U(F)U(M)A(F)A(M)A(M)A(M)U(M)G(M)^A(M)^G(M) | |
| si-0044 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-5-G(**V**) |
| | AS | 45 | U(M)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) | |
| si-0045 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-6-A(**V**) |
| | AS | 46 | U(M)^U(**F**)^U(M)A(M)G(M)A(**V**)G(M)U(F)G(F)A(M)G(M)G(M)A(M)U(F)U(M)A(F)A(M)A(M)A(M)U(M)G(M)^A(M)^G(M) | |
| si-0046 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-7-G(**V**) |
| | AS | 47 | U(M)^U(F)^U(M)A(M)G(M)A(F)G(**V**)U(F)G(F)A(M)G(M)G(M)A(M)U(F)U(M)A(F)A(M)A(M)A(M)U(M)G(M)^A(M)^G(M) | |
| si-0047 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-8-U(**V**) |
| | AS | 48 | U(M)^U(F)^U(M)A(M)G(M)A(F)G(M)U(**V**)G(F)A(M)G(M)G(M)A(M)U(F)U(M)A(F)A(M)A(M)A(M)U(M)G(M)^A(M)^G(M) | |
| si-0048 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-8-T(**V**) |
| | AS | 49 | U(M)^U(F)^U(M)A(M)G(M)A(F)G(M)T(**V**)G(F)A(M)G(M)G(M)A(M)U(F)U(M)A(F)A(M)A(M)A(M)U(M)G(M)^A(M)^G(M) | |
| si-0049 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-9-G(**V**) |
| | AS | 50 | U(M)^U(F)^U(M)A(M)G(M)A(F)G(M)U(F)G(**V**)A(M)G(M)G(M)A(M)U(F)U(M)A(F)A(M)A(M)A(M)U(M)G(M)^A(M)^G(M) | |
| si-0050 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-10-A(**V**) |
| | AS | 51 | U(M)^U(F)^U(M)A(M)G(M)A(F)G(M)U(F)G(F)A(**V**)G(M)G(M)A(M)U(F)U(M)A(F)A(M)A(M)A(M)U(M)G(M)^A(M)^G(M) | |
| si-0051 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-11-G(**V**) |
| | AS | 52 | U(M)^U(F)^U(M)A(M)G(M)A(F)G(M)U(F)G(F)A(M)G(**V**)G(M)A(M)U(F)U(M)A(F)A(M)A(M)A(M)U(M)G(M)^A(M)^G(M) | |
| si-0052 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-12-G(**V**) |
| | AS | 53 | U(M)^U(F)^U(M)A(M)G(M)A(F)G(M)U(F)G(F)A(M)G(M)G(**V**)A(M)U(F)U(M)A(F)A(M)A(M)A(M)U(M)G(M)^A(M)^G(M) | |
| si-0053 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-13-A(**V**) |
| | AS | 54 | U(M)^U(F)^U(M)A(M)G(M)A(F)G(M)U(F)G(F)A(M)G(M)G(M)A(**V**)U(F)U(M)A(F)A(M)A(M)A(M)U(M)G(M)^A(M)^G(M) | |
| si-0054 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-14-U(**V**) |
| | AS | 55 | U(M)^U(F)^U(M)A(M)G(M)A(F)G(M)U(F)G(F)A(M)G(M)G(M)A(M)U(**V**)U(M)A(F)A(M)A(M)A(M)U(M)G(M)^A(M)^G(M) | |
| si-0055 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-14-T(**V**) |
| | AS | 56 | U(M)^U(F)^U(M)A(M)G(M)A(F)G(M)U(F)G(F)A(M)G(M)G(M)A(M)T(**V**)U(M)A(F)A(M)A(M)A(M)U(M)G(M)^A(M)^G(M) | |
| si-0056 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-15-U(**V**) |
| | AS | 57 | U(M)^U(F)^U(M)A(M)G(M)A(F)G(M)U(F)G(F)A(M)G(M)G(M)A(M)U(F)U(**V**)A(F)A(M)A(M)A(M)U(M)G(M)^A(M)^G(M) | |
| si-0057 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-15-T(**V**) |
| | AS | 58 | U(M)^U(F)^U(M)A(M)G(M)A(F)G(M)U(F)G(F)A(M)G(M)G(M)A(M)U(F)T(**V**)A(F)A(M)A(M)A(M)U(M)G(M)^A(M)^G(M) | |
| si-0058 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-16-A(**V**) |
| | AS | 59 | U(M)^U(F)^U(M)A(M)G(M)A(F)G(M)U(F)G(F)A(M)G(M)G(M)A(M)U(F)U(M)A(**V**)A(M)A(M)A(M)U(M)G(M)^A(M)^G(M) | |
| si-0059 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-17-A(**V**) |
| | AS | 60 | U(M)^U(F)^U(M)A(M)G(M)A(F)G(M)U(F)G(F)A(M)G(M)G(M)A(M)U(F)U(M)A(F)A(**V**)A(M)A(M)U(M)G(M)^A(M)^G(M) | |
| si-0060 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-18-A(**V**) |
| | AS | 61 | U(M)^U(F)^U(M)A(M)G(M)A(F)G(M)U(F)G(F)A(M)G(M)G(M)A(M)U(F)U(M)A(F)A(M)A(**V**)A(M)U(M)G(M)^A(M)^G(M) | |
| si-0061 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-19-A(**V**) |
| | AS | 62 | U(M)^U(F)^U(M)A(M)G(M)A(F)G(M)U(F)G(F)A(M)G(M)G(M)A(M)U(F)U(M)A(F)A(M)A(M)A(**V**)U(M)G(M)^A(M)^G(M) | |
| si-0062 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-20-U(**V**) |
| | AS | 63 | U(M)^U(F)^U(M)A(M)G(M)A(F)G(M)U(F)G(F)A(M)G(M)G(M)A(M)U(F)U(M)A(F)A(M)A(M)A(M)U(**V**)G(M)^A(M)^G(M) | |
| si-0063 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-20-T(**V**) |
| | AS | 64 | U(M)^U(F)^U(M)A(M)G(M)A(F)G(M)U(F)G(F)A(M)G(M)G(M)A(M)U(F)U(M)A(F)A(M)A(M)A(M)T(**V**)G(M)^A(M)^G(M) | |
| si-0064 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-21-G(**V**) |
| | AS | 65 | U(M)^U(F)^U(M)A(M)G(M)A(F)G(M)U(F)G(F)A(M)G(M)G(M)A(M)U(F)U(M)A(F)A(M)A(M)A(M)U(M)G(**V**)^A(M)^G(M) | |
| si-0065 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-22-A(**V**) |
| | AS | 66 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(**V**)^G(M) | |
| si-0066 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS-23-G(**V**) |
| | AS | 67 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(**V**) | |

**[Table 4]**

| Compd. No. | Strand | SEQ No | Chemical Structure | Remarks |
|---|---|---|---|---|
| si-0067 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | parent |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0068 | S | 70 | C(**V**)^A(M)^U(F)U(M)U(F)U(M)A(F)A(M)U(F)C(F)C(F)U(M)C(F)A(M)C(F)U(M)C(F)U(M)A(F)^A(M)^A(F) | S-1-C(V) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0069 | S | 71 | 5(**V**)^A(M)^U(F)U(M)U(F)U(M)A(F)A(M)U(F)C(F)C(F)U(M)C(F)A(M)C(F)U(M)C(F)U(M)A(F)^A(M)^A(F) | S_1_5(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0070 | S | 72 | C(F)^A(**V**)^U(F)U(M)U(F)U(M)A(F)A(M)U(F)C(F)C(F)U(M)C(F)A(M)C(F)U(M)C(F)U(M)A(F)^A(M)^A(F) | S_2_A(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0071 | S | 73 | C(F)^A(M)^U(**V**)U(M)U(F)U(M)A(F)A(M)U(F)C(F)C(F)U(M)C(F)A(M)C(F)U(M)C(F)U(M)A(F)^A(M)^A(F) | S-3-U(**V**) |
| | AS | 69 | U(M)^U(F)^ U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0072 | S | 74 | C(F)^A(M)^T(**V**)U(M)U(F)U(M)A(F)A(M)U(F)C(F)C(F)U(M)C(F)A(M)C(F)U(M)C(F)U(M)A(F)^A(M)^A(F) | S-3-T(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0073 | S | 75 | C(F)^A(M)^U(F)U(**V**)U(F)U(M)A(F)A(M)U(F)C(F)C(F)U(M)C(F)A(M)C(F)U(M)C(F)U(M)A(F)^A(M)^A(F) | S-4-U(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0074 | S | 76 | C(F)^A(M)^U(F)T(**V**)U(F)U(M)A(F)A(M)U(F)C(F)C(F)U(M)C(F)A(M)C(F)U(M)C(F)U(M)A(F)^A(M)^A(F) | S-4-T(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0075 | S | 77 | C(F)^A(M)^U(F)U(M)U(**V**)U(M)A(F)A(M)U(F)C(F)C(F)U(M)C(F)A(M)C(F)U(M)C(F)U(M)A(F)^A(M)^A(F) | 5-5-U(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0076 | S | 78 | C(F)^A(M)^U(F)U(M)T(**V**)U(M)A(F)A(M)U(F)C(F)C(F)U(M)C(F)A(M)C(F)U(M)C(F)U(M)A(F)^A(M)^A(F) | S-5-T(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0077 | S | 79 | C(F)^A(M)^U(F)U(M)U(F)U(**V**)A(F)A(M)U(F)C(F)C(F)U(M)C(F)A(M)C(F)U(M)C(F)U(M)A(F)^A(M)^A(F) | S-6-U(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0078 | S | 80 | C(F)^A(M)^U(F)U(M)U(F)T(**V**)A(F)A(M)U(F)C(F)C(F)U(M)C(F)A(M)C(F)U(M)C(F)U(M)A(F)^A(M)^A(F) | S-6-T(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0079 | S | 81 | C(F)^A(M)^U(F)U(M)U(F)U(M)A(**V**)A(M)U(F)C(F)C(F)U(M)C(F)A(M)C(F)U(M)C(F)U(M)A(F)^A(M)^A(F) | S-7-A(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0080 | S | 82 | C(F)^A(M)^U(F)U(M)U(F)U(M)A(F)A(**V**)U(F)C(F)C(F)U(M)C(F)A(M)C(F)U(M)C(F)U(M)A(F)^A(M)^A(F) | S-8-A(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0081 | S | 83 | C(F)^A(M)^U(F)U(M)U(F)U(M)A(F)A(M)U(**V**)C(F)C(F)U(M)C(F)A(M)C(F)U(M)C(F)U(M)A(F)^A(M)^A(F) | S-9-U(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0082 | S | 84 | C(F)^A(M)^U(F)U(M)U(F)U(M)A(F)A(M)T(**V**)C(F)C(F)U(M)C(F)A(M)C(F)U(M)C(F)U(M)A(F)^A(M)^A(F) | S-9-T(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0083 | S | 85 | C(F)^A(M)^U(F)U(M)U(F)U(M)A(F)A(M)U(F)C(**V**)C(F)U(M)C(F)A(M)C(F)U(M)C(F)U(M)A(F)^A(M)^A(F) | S-10-C(**V**) |
| | AS | 69 | U(M)^U(F)^U(M)A(F)G(M)A(F)G(M)U(**F**)G(M)A(F)G(M)G(M)A(M)U(F)U(M)A(F)A(M)A(F)A(M)U(F)G(M)^A(M)^G(M) | |
| si-0084 | S | 86 | C(F)^A(M)^ U(F)U(M)U(F)U(M)A(F)A(M)U(F)5(**V**)C(F)U(M)C(F)A(M)C(F)U(M)C(F)U(M)A(F)^A(M)^A(F) | S-10-5(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0085 | S | 87 | C(F)^A(M)^U(F)U(M)U(F)U(M)A(F)A(M)U(F)C(F)C(**V**)U(M)C(F)A(M)C(F)U(M)C(F)U(M)A(F)^A(M)^A(F) | S-11-C(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0086 | S | 88 | C(F)^A(M)^U(F)U(M)U(F)U(M)A(F)A(M)U(F)C(F)5(**V**)U(M)C(F)A(M)C(F)U(M)C(F)U(M)A(F)^A(M)^A(F) | S-11-5(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0087 | s | 89 | C(F)^A(M)^U(F)U(M)U(F)U(M)A(F)A(M)U(F)C(F)C(F)U(**V**)C(F)A(M)C(F)U(M)C(F)U(M)A(F)^A(M)^A(F) | S-12-U(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0088 | S | 90 | C(F)^A(M)^U(F)U(M)U(F)U(M)A(F)A(M)U(F)C(F)C(F)T(**V**)C(F)A(M)C(F)U(M)C(F)U(M)A(F)^A(M)^A(F) | S-12-T(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0089 | S | 91 | C(F)^A(M)^U(F)U(M)U(F)U(M)A(F)A(M)U(F)C(F)C(F)U(M)C(**V**)A(M)C(F)U(M)C(F)U(M)A(F)^A(M)^A(F) | S-13-C(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0090 | 5 | 92 | C(F)^A(M)^U(F)U(M)U(F)U(M)A(F)A(M)U(F)C(F)C(F)U(M)5(**V**)A(M)C(F)U(M)C(F)U(M)A(F)^A(M)^A(F) | S-13-5(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0091 | S | 93 | C(F)^A(M)^U(F)U(M)U(F)U(M)A(F)A(M)U(F)C(F)C(F)U(M)G(F)A(**V**)G(F)U(M)C(F)U(M)A(F)^A(M)^A(F) | S-14-A(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0092 | S | 94 | C(F)^A(M)^U(F)U(M)U(F)U(M)A(F)A(M)U(F)C(F)C(F)U(M)C(F)A(M)C(**V**)U(M)C(F)U(M)A(F)^A(M)^A(F) | S-15-C(**V**) |
| | AS | 69 | U(M)^U(F)^ U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0093 | S | 95 | C(F)^A(M)^U(F)U(M)U(F)U(M)A(F)A(M)U(F)C(F)C(F)U(M)C(F)A(M)5(**V**)U(M)C(F)U(M)A(F)^A(M)^A(F) | S-15-5(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0094 | S | 96 | C(F)^A(M)^U(F)U(M)U(F)U(M)A(F)A(M)U(F)C(F)C(F)U(M)C(F)A(M)C(F)U(**V**)C(F)U(M)A(F)^A(M)^A(F) | S-16-U(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0095 | S | 97 | C(F)^A(M)^U(F)U(M)U(F)U(M)A(F)A(M)U(F)C(F)C(F)U(M)C(F)A(M)C(F)T(**V**)C(F)U(M)A(F)^A(M)^A(F) | S-16-T(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0096 | S | 98 | C(F)^A(M)^U(F)U(M)U(F)U(M)A(F)A(M)U(F)C(F)C(F)U(M)C(F)A(M)C(F)U(M)C(**V**)U(M)A(F)^A(M)^A(F) | S-17-C(**V**) |
| | AS | 69 | U(M)^U(F)^ U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0097 | S | 99 | C(F)^A(M)^U(F)U(M)U(F)U(M)A(F)A(M)U(F)C(F)C(F)U(M)C(F)A(M)C(F)U(M)5(**V**)U(M)A(F)^A(M)^A(F) | S-17-5(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0098 | S | 100 | C(F)^A(M)^U(F)U(M)U(F)U(M)A(F)A(M)U(F)C(F)C(F)U(M)C(F)A(M)C(F)U(M)C(F)U(**V**)A(F)^A(M)^A(F) | S-18-U(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0099 | S | 101 | C(F)^A(M)^U(F)U(M)U(F)U(M)A(F)A(M)U(F)C(F)C(F)U(M)C(F)A(M)C(F)U(M)C(F)T(**V**)A(F)^A(M)^A(F) | S-18-T(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0100 | S | 102 | C(F)^A(M)^U(F)U(M)U(F)U(M)A(F)A(M)U(F)C(F)C(F)U(M)C(F)A(M)C(F)U(M)C(F)U(M)A(**V**)^A(M)^A(F) | S-19-A(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0101 | S | 103 | C(F)^A(M)^U(F)U(M)U(F)U(M)A(F)A(M)U(F)C(F)C(F)U(M)C(F)A(M)C(F)U(M)C(F)U(M)A(F)^A(**V**)^A(F) | S-20-A(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0102 | S | 104 | C(F)^A(M)^U(F)U(M)U(F)U(M)A(F)A(M)U(F)C(F)C(F)U(M)C(F)A(M)C(F)U(M)C(F)U(M)A(F)^A(M)^A(**V**) | S-21-A(**V**) |
| | AS | 69 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M) ^G(M) | |

**[Table 5]**

| | | | | |
|---|---|---|---|---|
| si-0103 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-1-U(**V**) |
| | AS | 105 | U(**V**)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0104 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-1-T(**V**) |
| | AS | 106 | T(**V**)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0105 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-2-U(**V**) |
| | AS | 107 | U(M)^U(**V**)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0106 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-2-T(**V**) |
| | AS | 108 | U(M)^T(**V**)^U(M)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0107 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-3-U(**V**) |
| | AS | 109 | U(M)^U(F)^U(**V**)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0108 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-3-T(**V**) |
| | AS | 110 | U(M)^U(F)^T(**V**)A(F)G(M)A(F)G(M)U(F)G(M)A(F)G(M)G(M)A(M)U(F)U(M)A(F)A(M)A(F)A(M)U(F)G(M)^A(M)^G(M) | |
| si-0109 | S | 68 | C(F)^A(M)^U(F)U(M)U(F)U(M)A(F)A(M)U(F)C(F)C(F)U(M)C(F)A(M)C(F)U(M)C(F)U(M)A(F)A(M)^A(F) | AS-4-A(**V**) |
| | AS | 111 | U(M)^U(F)^U(M)A(**V**)G(M)A(F)G(M)U(F)G(M)A(F)G(M)G(M)A(M)U(F)U(M)A(F)A(M)A(F)A(M)U(F)G(M)^A(M)^G(M) | |
| si-0110 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-5-G(**V**) |
| | AS | 112 | U(M)^U(F)^U(M)A(F)G(**V**)A(F)G(M)U(F)G(M)A(F)G(M)G(M)A(M)U(F)U(M)A(F)A(M)A(F)A(M)U(F)G(M)^A(M)^G(M) | |
| si-0111 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-6-A(**V**) |
| | AS | 113 | U(M)^U(F)^U(M)A(F)G(M)A(**V**)G(M)U(F)G(M)A(F)G(M)G(M)A(M)U(F)U(M)A(F)A(M)A(F)A(M)U(F)G(M)^A(M)^G(M) | |
| si-0112 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-7-G(**V**) |
| | AS | 114 | U(M)^U(F)^U(M)A(F)G(M)A(F)G(**V**)U(F)G(M)A(F)G(M)G(M)A(M)U(F)U(M)A(F)A(M)A(F)A(M)U(F)G(M)^A(M)^G(M) | |
| si-0113 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-8-U(**V**) |
| | AS | 115 | U(M)^U(F)^U(M)A(F)G(M)A(F)G(M)lJ(**V**)G(M)A(F)G(M)G(M)A(M)U(F)lJ(M)A(F)A(M)A(F)A(M)U(F)G(M)^A(M)^G(M) | |
| si-0114 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-8-T(**V**) |
| | AS | 116 | U(M)^U(F)^U(M)A(F)G(M)A(F)G(M)T(**V**)G(M)A(F)G(M)G(M)A(M)U(F)U(M)A(F)A(M)A(F)A(M)U(F)G(M)^A(M)^G(M) | |
| si-0115 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-9-G(**V**) |
| | AS | 117 | U(M)^U(F)^U(M)A(F)G(M)A(F)G(M)U(F)G(**V**)A(F)G(M)G(M)A(M)U(F)U(M)A(F)A(M)A(F)A(M)U(F)G(M)^A(M)^G(M) | |
| si-0116 | 5 | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-10-A(**V**) |
| | AS | 118 | U(M)^U(F)^U(M)A(F)G(M)A(F)G(M)U(F)G(M)A(**V**)G(M)G(M)A(M)U(F)U(M)A(F)A(M)A(F)A(M)U(F)G(M)^A(M)^G(M) | |
| si-0117 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-11-G(**V**) |
| | AS | 119 | U(M)^U(F)^U(M)A(F)G(M)A(F)G(M)U(F)G(M)A(F)G(**V**)G(M)A(M)U(F)U(M)A(F)A(M)A(F)A(M)U(F)G(M)^A(M)^G(M) | |
| si-0118 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-12-G(**V**) |
| | AS | 120 | U(M)^U(F)^U(M)A(F)G(M)A(F)G(M)U(F)G(M)A(F)G(M)G(**V**)A(M)U(F)U(M)A(F)A(M)A(F)A(M)U(F)G(M)^A(M)^G(M) | |
| si-0119 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-13-A(**V**) |
| | AS | 121 | U(M)^U(F)^U(M)A(F)G(M)A(F)G(M)U(F)G(M)A(F)G(M)G(M)A(**V**)U(F)U(M)A(F)A(M)A(F)A(M)U(F)G(M)^A(M)^G(M) | |
| si-0120 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-14-U(**V**) |
| | AS | 122 | U(M)^U(F)^U(M)A(F)G(M)A(F)G(M)U(F)G(M)A(F)G(M)G(M)A(M)U(**V**)U(M)A(F)A(M)A(F)A(M)U(F)G(M)^A(M)^G(M) | |
| si-0121 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-14-T(**V**) |
| | AS | 123 | U(M)^U(F)^U(M)A(F)G(M)A(F)G(M)U(F)G(M)A(F)G(M)G(M)A(M)T(**V**)U(M)A(F)A(M)A(F)A(M)U(F)G(M)^A(M)^G(M) | |
| si-0122 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-15-U(**V**) |
| | AS | 124 | U(M)^U(F)^U(M)A(F)G(M)A(F)G(M)U(F)G(M)A(F)G(M)G(M)A(M)U(F)U(**V**)A(F)A(M)A(F)A(M)U(F)G(M)^A(M)^G(M) | |
| si-0123 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-15-T(**V**) |
| | AS | 125 | U(M)^U(F)^U(M)A(F)G(M)A(F)G(M)U(F)G(M)A(F)G(M)G(M)A(M)U(F)T(**V**)A(F)A(M)A(F)A(M)U(F)G(M)^A(M)^G(M) | |
| si-0124 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-16-A(**V**) |
| | AS | 126 | U(M)^U(F)^U(M)A(F)G(M)A(F)G(M)U(F)G(M)A(F)G(M)G(M)A(M)U(F)U(M)A(**V**)A(M)A(F)A(M)U(F)G(M)^A(M)^G(M) | |
| si-0125 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-17-A(**V**) |
| | AS | 127 | U(M)^U(F)^U(M)A(F)G(M)A(F)G(M)U(F)G(M)A(F)G(M)G(M)A(M)U(F)U(M)A(F)A(**V**)A(F)A(M)U(F)G(M)^A(M)^G(M) | |
| si-0126 | S | 68 | C(F)^A(M)^U(F)U(M)U(F)(J(M)A(F)A(M)U(F)C(F)C(F)U(M)C(F)A(M)C(F)U(M)C(F)U(M)A(F)^A(M)^A(F) | AS-18-A(**V**) |
| | AS | 128 | U(M)^U(F)^U(M)A(F)G(M)A(F)G(M)U(F)G(M)A(F)G(M)G(M)A(M)U(F)U(M)A(F)A(M)A(**V**)A(M)U(F)G(M)^A(M)^G(M) | |
| si-0127 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-19-A(**V**) |
| | AS | 129 | U(M)^U(F)^U(M)A(F)G(M)A(F)G(M)U(F)G(M)A(F)G(M)G(M)A(M)U(F)U(M)A(F)A(M)A(F)A(**V**)U(F)G(M)^A(M)^G(M) | |
| si-0128 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-20-U(**V**) |
| | AS | 130 | U(M)^U(F)^U(M)A(F)G(M)A(F)G(M)U(F)G(M)A(F)G(M)G(M)A(M)U(F)U(M)A(F)A(M)A(F)A(M)U(**V**)G(M)^A(M)^G(M) | |
| si-0129 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-20-T(**V**) |
| | AS | 131 | U(M)^U(F)^U(M)A(F)G(M)A(F)G(M)U(F)G(M)A(F)G(M)G(M)A(M)U(F)U(M)A(F)A(M)A(F)A(M)T(**V**)G(M)^A(M)^G(M) | |
| si-0130 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-21-G(**V**) |
| | AS | 132 | U(M)^U(F)^U(M)A(F)G(M)A(F)G(M)U(F)G(M)A(F)G(M)G(M)A(M)U(F)U(M)A(F)A(M)A(F)A(M)U(F)G(V)^A(M)^G(M) | |
| si-0131 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-22-A(**V**) |
| | AS | 133 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(**V**)^G(M) | |
| si-0132 | S | 68 | C(F)^A(M)^U(F)U(M)U(F)U(M)A(F)A(M)U(F)C(F)C(F)U(M)C(F)A(M)C(F)U(M)C(F)U(M)A(F)^A(M)^A(F) | AS-23-G(**V**) |
| | AS | 134 | U(M)^U(F)^U(M)A(F)G(M)A(F)G(M)U(F)G(M)A(F)G(M)G(M)A(M)U(F)U(M)A(F)A(M)A(F)A(M)U(F)G(M)^A(M)^G(**V**) | |

**[Table 6]**

| Compd. No. | Strand | SEQ No | Chemical Structure | Remarks |
|---|---|---|---|---|
| si-0001 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | parent |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0133 | S | 1 | C(M)^A(M)^U(M)U(M)U(M)U(M)A(F)A(M)U(**F**)C(F)C(F)U(M)C(M)A(M)C(M)U(M)C(M)U(M)A(M)^A(M)^A(M) | AS: 1-T(**V**), 5-G(**V**), 6-A(**V**), 10-A(**V**) |
| | AS | 135 | T(**V**)^U(F)^U(M)A(M)G(**V**)A(**V**)G(M)U(F)G(F)A(**V**)G(M)G(M)A(M)U(F)A(M)A(F)A(M)A(M)A(M)U(M)G(M)^A(M)^C(M) | |
| si-0134 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS: 1-T(**V**), 5-G(**V**) |
| | AS | 136 | T(**V**)^U(F)^U(M)A(M)G(V)A(F)G(M)U(F)G(F)A(M)G(M)G(M)A(M)U(F)U(M)A(F)A(M)A(M)A(M)U(M)G(M)^A(M)^G(M) | |
| si-0135 | S | 1 | C(M)^A(M)^U(M)U(M)U(M)U(M)A(F)A(M)U(F)C(f)C(F)U(M)C(M)A(M)C(M)U(M)C(M)U(M)A(M)^A(M)^A(M) | AS: 1-T(**V**), 5-G(**V**), 6-A(**V**) |
| | AS | 137 | T(**V**)^U(F)^U(M)A(M)G(**V**)A(**V**)G(M)U(F)G(F)A(M)G(M)G(M)A(M)U(F)U(M)A(F)A(M)A(M)A(M)U(M)G(M)^A(M)^G(M) | |
| sí-0136 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS: 1-T(**V**), 10-A(**V**) |
| | AS | 138 | T(**V**)^U(F)^U(M)A(M)G(M)A(F)G(M)U(F)G(F)A(V)G(M)G(M)A(M)U(F)U(M)A(F)A(M)A(M)A(M)U(M)G(M)^A(M)^G(M) | |
| si-0137 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | **AS:** 1-T(**V**), 5-G(**V**), 10-A(**V**) |
| | AS | 139 | T(**V**)^U(F)^U(M)A(M)G(V)A(F)G(M)U(F)G(F)A(V)G(M)G(M)A(M)U(F)U(M)A(F)A(M)A(M)A(M)U(M)G(M)^A(M)^G(M) | |
| si-0138 | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) | AS: 1-T(**V**), 6-A(**V**), 10-A(**V**) |
| | AS | 140 | T(**V**)^U(F)^U(M)A(M)G(M)A(V)G(M)U(F)G(F)A(V)G(M)G(M)A(M)U(F)U(M)A(F)A(M)A(M)A(M)U(M)G(M)^A(M)^G(M) | |
| si-0139 | S | 141 | 5(**V**)^A(V)^U(M)U(M)U(M)U(M)A(F)A(M)U(F)C(F)C(F)U(M)C(M)A(M)C(M)U(M)C(M)U(M)A(M)^A(V)^A(V) | S: 1-5(**V**), 2-A(**V**), 20-A(**V**), 21-A(**V**) |
| | AS | 2 | U(M)^U(F)^U(M)A(M)G(M)A(F)G(M)U(F)G(F)A(M)G(M)G(M)A(M)U(F)A(M)A(F)A(M)A(M)A(M)U(M)G(M)^A(M)^G(M) | |
| si-0140 | 5 | 142 | 5(**V**)^A(**V**)^U(M)U(M)U(M)U(M)A(F)A(M)U(F)C(F)C(F)U(M)C(M)A(**V**)5(**V**)T(**V**)5(**V**)T(**V**)A(**V**)^A(**V**)^A(**V**) | S: 1-5(**V**), 2-A(**V**), 14-A(**V**), 15-5(**V**), 16-T(**V**),17-5(**V**), 18-T(**V**), 19-A(**V**), 20-A(**V**), 21-A(**V**) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0141 | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(V) | S: 1-5(V), 21-A(V) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0142 | S | 144 | 5(V)^A(V)^T(V)T(V)U(M)U(M)A(F)A(M)U(F)C(F)C(F)U(M)C(M)A(M)C(M)U(M)C(M)T(V)A(V)^A(V)^A(V) | S: 1-5(V), 2-A(V), 3-T(V), 4-T(V),18 T(V), 19-**A(V), 20-A(V), 21-A(V)** |
| | AS | 2 | U(M)^U(F)^U(M)A(M)G(M)A(F)G(M)U(F)G(F)A(M)G(M)G(M)A(M)U(F)A(M)A(F)A(M)A(M)A(M)U(M)G(M)^A(M)^G(M) | |
| si-0143 | S | 145 | 5(**V**)^A(M)^T(**V**)U(M)T(**V**)U(M)A(**V**)A(M)U(F)C(F)C(F)U(M)5(**V**)A(M)5(**V**)U(M)5(**V**)U(M)A(V)^A(M)^A(**V**) | S: 1-5(V), 3-T(V), S-T(V), 7-A(V),13-5(V), 15-5(V), 17-5(V), 19-A(V), 21-A(V) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) | |
| si-0144 | S | 141 | 5(**V**)^A(**V**)^U(M)U(M)U(M)U(M)A(F)A(M)U(F)C(F)C(F)U(M)C(M)A(M)C(M)U(M)C(M)U(M)A(M)^A(**V**)^A(**V**) | S: 1-5(V), 2-A(V), 20-A(V), 21-A(V), AS: 1-T(V), 5-G(V) |
| | AS | 136 | T(**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) | |
| si-0145 | S | 143 | 5(V)^A(M)^U(M)U(M)U(M)U(M)A(F)A(M)U(F)C(F)C(F)U(M)C(M)A(M)C(M)U(M)C(M)U(M)A(M)^A(M)^A(V) | s: 1-5(**V**), 21-A(V), AS: 1-T(V), S-G(**V**) |
| | AS | 136 | T(**V**)^U(F)^U(M)A(M)G(V)A(F)G(M)U(F)G(F)A(M)G(M)G(M)A(M)U(F)U(M)A(F)A(M)A(M)A(M)U(M)G(M)^A(M)^G(M) | |

### [Evaluation Example 1] Inhibition of expression of rat Sod1 in RPE-J cells (rat retinal pigment epithelial (RPE) cells)

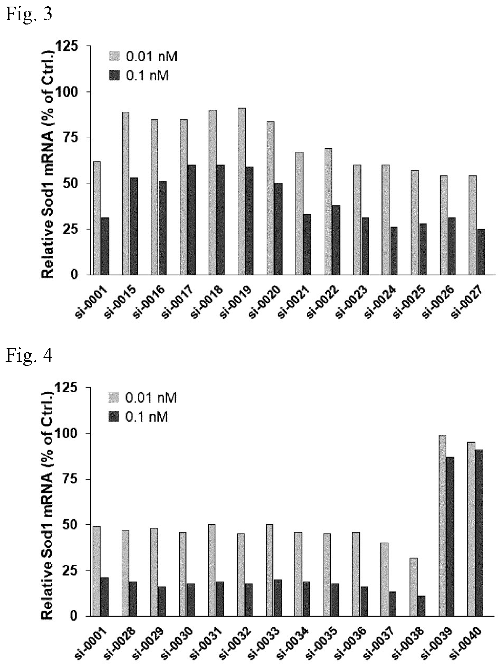

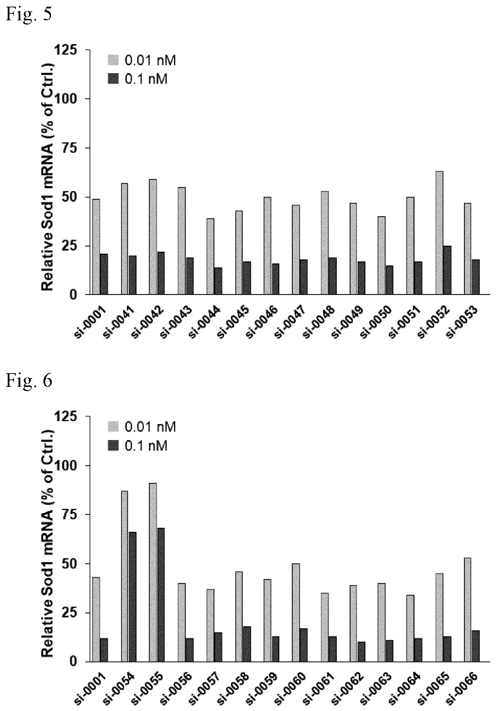

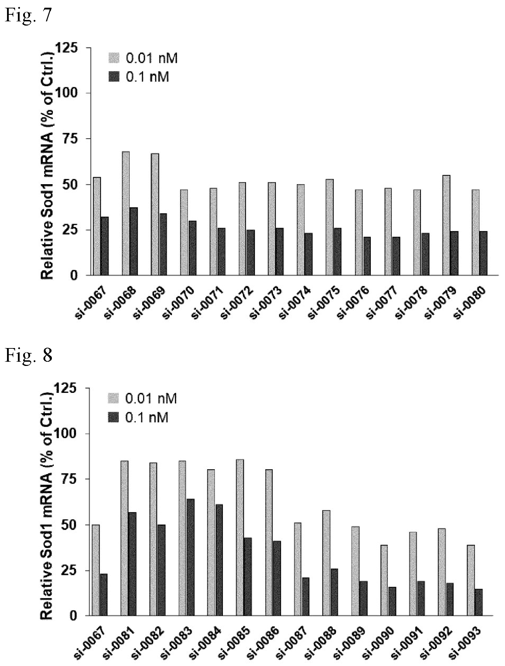

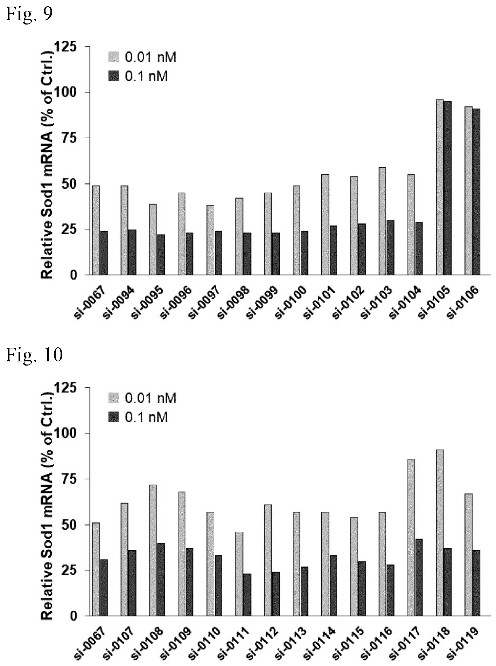

Each siRNA described in Tables 2 to 6 was mixed with Lipofectamine (registered trademark) RNAiMAX (Thermo Fisher Scientific) such that the final concentration was 0.01 or 0.1 nM, incubated for 15 minutes, and then dispensed into a 96 well plate. Then, each well was seeded with RPE-J cells at a density of 10,000 cells/well (Lipofection). After 20 hours, a cell lysate containing RNA was prepared using CellAmp (registered trademark) Direct RNA Prep Kit for RT-PCR (Real Time) (Takara Bio Inc.), and then the rat Sod1 (superoxide dismutase-1) gene expression level was measured by quantitative real-time PCR with TaqMan (registered trademark) Gene Expression Assays (Thermo Fisher Scientific) using One Step PrimeScript (registered trademark) III RT-qPCR Mix (Takara Bio Inc.). In the real-time PCR, the housekeeping gene Ppib mRNA level was also simultaneously quantified, and the Sod1 mRNA level with respect to the Ppib (Peptidylprolyl Isomerase B) mRNA level was evaluated as the Sod1 expression level. The results are shown in Figs. 2 to 13.

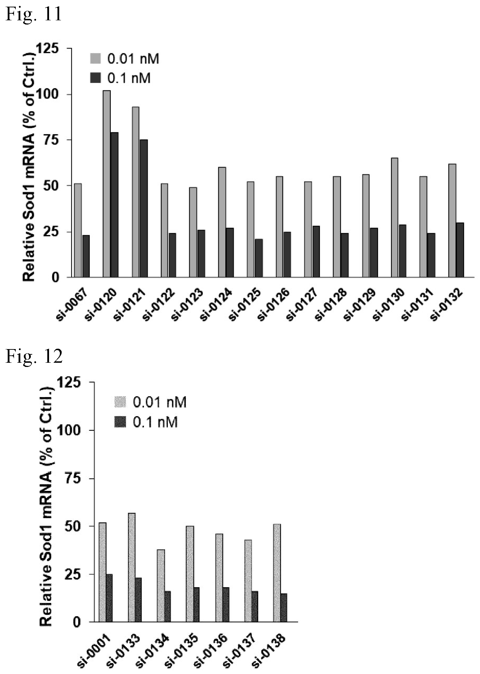

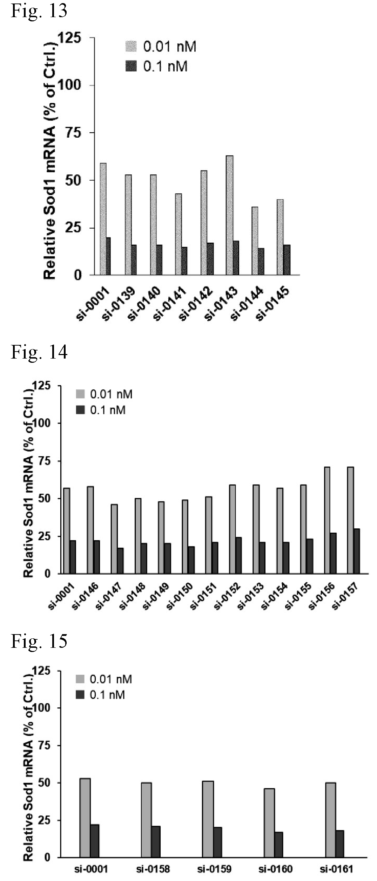

As is apparent from Figs. 2 to 11, the siRNA including 2'-O-MCE nucleotides at positions 1, 2, 3, 4, 5, 6, 7, 8, 12, 13, 14, 15, 16, 17, 18, 19, 20, and 21 as counted from the 5' end of the sense strand (that is, the 5' end of the sense strand hybridizing portion), or the siRNA including 2'-O-MCE nucleotides at positions 1, 3, 4, 5, 6, 7, 8, 9, 10, 11, 12, 13, 15, 16, 17, 18, 19, 20, 21, 22, and 23 as counted from the 5' end of the antisense strand (that is, the 5' end of the antisense strand hybridizing portion), exhibited the expression suppressing effect equivalent to that of the siRNA not including 2'-O-MCE nucleotides (si-0001 or si-0067, parent). In addition, as is apparent from Figs. 12 and 13, the siRNA including two or more 2'-O-MCE nucleotides in the sense strand and/or the antisense strand exhibited the expression suppressing effect equal to or superior to that of the siRNA not including 2'-O-MCE nucleotides (si-0001, parent). In particular, siRNA including 2'-O-MCE nucleotides at any one of positions 1 and 3 to 10 as counted from the 5' end of the antisense strand and siRNA including 2'-O-MCE nucleotides at positions 1 and 21 or positions 1 to 2 and 20 to 21 as counted from the 5' end of the sense strand (si-0134 to si-0138, si-0141, si-0144 to si-0145, and the like) exhibited the high expression suppressing effect.

### [Production Example 8] Synthesis of siRNA

According to the method described in Production Example 6, siRNAs described in Tables 7 to 10 were synthesized. The target RNA of the siRNAs described in Tables 7 to 10 is Sod1 (mouse and rat).

si-0146 to si-0208 are designed based on si-0001, and are siRNA including a plurality of 2'-O-MCE nucleotides.

**[Table 7]**

| Compd. No. | Strand | SEQ No | Chemical Structure |
|---|---|---|---|
| si-0001 | 5 | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| | AS | 2 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) |
| si-0146 | AS | 146 | T(**V**)^U(F)^U(M)A(M)G(V)A(**V**)G(M)U(F)G(F)A(**V**)G(M)G(M)A(M)U(F)U(M)A(F)A(M)A(M)A(M)U(M)G(M)^A(**V**)^G(M) |
| | S | 1 | C(M)^A(M)"U(M)U(M)U(M)U(M)A(F)A(M)U(F)C(F)C(F)U(M)C(M)A(M) C(M)U(M)C(M)U(M)A(M)^A(M)^A(M) |
| si-0147 | AS | 147 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(**V**)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0148 | AS | 148 | T(**V**)^U(F)^U(M)A(M)G(**V**)A(F)G(M)U(F)G(F)A(**V**)G(M)G(M)A(M)U(F)U(M)A(F)A(M)A(M)A(M)T(**V**)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0149 | AS | 149 | T(**V**)^U(F)^U(M)A(M)G(**V**)A(F)G(M)U(F)G(F)A(**V**)G(M)G(M)A(M)U(F)U(M)A(F)A(M)A(M)A(**V**)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0150 | AS | 150 | T(**V**)^U(F)^U(M)A(M)G(**V**)A(F)G(M)U(F)G(F)A(**V**)G(M)G(M)A(M)U(F)U(M)A(F)A(**V**)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0151 | AS | 151 | T(**V**)^U(F)^U(M)A(M)G(**V**)A(F)G(M)U(F)G(F)A(**V**)G(M)G(M)A(M)U(F)T(**V**)A(F)A(M)A(M)A(M)U(M)G(M)^A(M)^G(M) |
| | S | 1 | C(M)'A(M)^U(M)U(M)U(M)U(M)A(F)A(M)U(F)C(F)C(F)U(M)C(M)A(M)C(M)U(M)C(M)U(M)A(M)^A(M)^A(M) |
| si-0152 | AS | 152 | T(**V**)^U(F)^U(M)A(M)G(**V**)A(F)G(M)U(F)G(F)A(**V**)G(M)G(M)A(**V**)U(F)U(M)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0153 | AS | 153 | T(**V**)^U(F)^U(M)A(M)G(**V**)A(F)G(M)U(F)G(F)A(**V**)G(**V**)G(M)A(M)U(F)U(M)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0154 | AS | 154 | T(**V**)^U(F)^U(M)A(M)G(**V**)A(F)G(M)T(**V**)G(F)A(**V**)G(M)G(M)A(M)U(F)U(M)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0155 | AS | 155 | T(**V**)^U(F)^U(M)A(M)G(**V**)A(F)G(**V**)U(F)G(F)A(**V**)G(M)G(M)A(M)U(F)U(M)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0156 | AS | 156 | T(**V**)^U(F)^U(M)A(**V**)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0157 | AS | 157 | T(**V**)^U(F)^T(**V**)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0158 | AS | 158 | T(**V**)^U(F)^U(M)A(M)G(**V**)A(F)G(M)U(F)G(F)A(**V**)G(M)G(M)A(M)U(F)T(**V**)A(F)A(M)A(M)A(**V**)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0159 | AS | 159 | T(**V**)^U(F)^U(M)A(M)G(**V**)A(F)G(M)U(F)G(F)A(**V**)G(M)G(M)A(M)U(F)T(**V**)A(F)A(M)A(M)A(**V**)U(M)G(**V**)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0160 | AS | 160 | T(**V**)^U(F)^U(M)A(M)G(**V**)A(F)G(M)U(F)G(F)A(**V**)G(M)G(M)A(M)U(F)U(M)A(F)A(M)A(M)A(**V**)U(M)G(**V**)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0161 | AS | 161 | T(**V**)^U(F)^U(M)A(M)G(**V**)A(F)G(M)U(F)G(F)A(**V**)G(M)G(M)A(M)U(F)U(M)A(F)A(M)A(M)A(**V**)T(**V**)G(**V**)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0162 | AS | 162 | T(**V**)^U(F)^U(M)A(M)G(**V**)A(F)G(M)U(F)G(F)A(**V**)G(M)G(M)A(M)U(F)U(M)A(F)A(M)A(M)A(**V**)U(M)G(**V**)^A(**V**)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0163 | AS | 163 | T(**V**)^U(F)^U(M)A(M)G(**V**)A(F)G(M)U(F)G(F)A(**V**)G(M)G(M)A(M)U(F)U(M)A(F)A(M)A(M)A(**V**)T(**V**)G(M)^A(**V**)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0164 | AS | 164 | T(**V**)^U(F)^U(M)A(M)G(**V**)A(F)G(M)U(F)G(F)A(**V**)G(M)G(M)A(**V**)U(F)T(**V**)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0165 | AS | 165 | T(**V**)^U(F)^U(M)A(M)G(**V**)A(F)G(M)U(F)G(F)A(**V**)G(M)G(M)A(M)U(F)T(**V**)A(F)A(M)A(M)A(**V**)T(V)G(**V**)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0166 | AS | 166 | T(**V**)^U(F)^U(M)A(M)G(**V**)A(F)G(M)U(F)G(F)A(**V**)G(M)G(M)A(**V**)U(F)T(**V**)A(F)A(V)A(M)A(**V**)T(V)G(**V**)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0167 | AS | 167 | T(**V**)^U(F)^U(M)A(M)G(**V**)A(F)G(M)U(F)G(F)A(**V**)G(**V**)G(M)A(**V**)U(F)U(M)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)AíM)C(M)U(M)C(M)U(M)A(M)^A(M)^A(M) |
| si-0168 | AS | 168 | T(**V**)^U(F)^U(M)A(M)G(**V**)A(F)G(M)U(F)G(F)A(**V**)G(M)G(M)A(**V**)U(F)T(**V**)A(F)A(**V**)A(M)A(**V**)U(M)G(**V**)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0159 | AS | 169 | T(**V**)^U(F)^U(M)A(M)G(**V**)A(F)G(M)U(F)G(F)A(**V**)G(M)G(M)A(M)U(F)U(M)A(F)A(M)A(M)A(**V**)T(V)G(**V**)^A(**V**)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |

**[Table 8]**

| | | | |
|---|---|---|---|
| si-0170 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 170 | 5(**V**)^A(M)^U(M)U(M)T(**V**)U(M)A(F)A(M)U(F)C(F)C(F)U(M)C(M)A(M)C(M)U(M)C(M)U(M)A(M)^A(M)^A(**V**) |
| si-0171 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 171 | 5(**V**)^A(M)^U(M)U(M)U(M)U(M)A(V)A(M)U(F)C(F)C(F)U(M)C(M)A(M)C(M)U(M)C(M)U(M)A(M)^A(M)^A(**V**) |
| si-0172 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 172 | 5(**V**)^A(M)^U(M)U(M)U(M)U(M)A(F)A(M)U(F)C(F)C(F)U(M)5(**V**)A(M)C(M)U(M)C(M)U(M)A(M)^A(M)^A(**V**) |
| si-0173 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 173 | 5(**V**)^A(M)^U(M)U(M)U(M)U(M)A(F)A(M)U(F)C(F)C(F)U(M)C(M)A(V)C(M)U(M)C(M)U(M)A(M)^A(M)^A(**V**) |
| si-0174 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 174 | 5(**V**)^A(M)^U(M)U(M)U(M)U(M)A(F)A(M)U(F)C(F)C(F)U(M)C(M)A(M)5(**V**)U(M)C(M)U(M)A(M)^A(M)^A(**V**) |
| si-0175 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 175 | 5(**V**)^A(M)^U(M)U(M)U(M)U(M)A(F)A(M)U(F)C(F)C(F)U(M)C(M)A(**V**)5(**V**)U(M)C(M)U(M)A(M)^A(M)^A(**V**) |
| si-0176 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 176 | 5(**V**)^A(M)^U(M)U(M)U(M)U(M)A(**V**)A(M)U(F)C(F)C(F)U(M)C(M)A(M)5(**V**)U(M)C(M)U(M)A(M)^A(M)^A(**V**) |
| si-0177 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 177 | 5(**V**)^A(M)^U(M)U(M)T(**V**)U(M)A(F)A(M)U(F)C(F)C(F)U(M)C(M)A(M)5(**V**)U(M)C(M)U(M)A(M)^A(M)^A(**V**) |
| si-0178 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 178 | 5(**V**)^A(M)^U(M)U(M)T(**V**)U(M)A(**V**)A(M)U(F)C(F)C(F)U(M)C(M)A(V)5(V)U(M)C(M)U(M)A(M)^A(M)^A(**V**) |
| si-0179 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 179 | C(M)^A(V)^U(M)U(M)U(M)U(M)A(F)A(M)U(F)C(F)C(F)U(M)C(M)A(M)C(M)U(M)C(M)U(M)A(M)^A(M)^A(**V**) |
| si-0180 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 180 | C(M)^A(M)^U(M)U(M)T(**V**)U(M)A(F)A(M)U(F)C(F)C(F)U(M)C(M)A(M)T(**V**)U(M)C(M)U(M)A(M)^A(M)^A(**V**) |
| si-0181 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 181 | C(M)^A(M)^U(M)U(M)T(**V**)U(M)A(F)A(M)U(F)C(F)C(F)U(M)C(M)A(M)T(**V**)U(M)C(M)U(M)A(M)^A(M)^A(M) |
| si-0182 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 182 | C(M)^A(M)^U(M)U(M)T(**V**)U(M)A(V)A(M)U(F)C(F)C(F)U(M)C(M)A(M)T(**V**)U(M)C(M)U(M)A(M)^A(M)^A(M) |
| si-0183 | AS | 183 | T(**V**)^U(F)^U(M)A(M)G(**V**)A(F)G(M)U(F)G(F)A(M)G(M)G(M)A(M)U(F)U(M)A(F)A(M)A(M)A(**V**)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0184 | AS | 184 | T(**V**)^U(F)^U(M)A(M)G(**V**)A(F)G(M)U(F)G(F)A(M)G(M)G(M)A(M)U(F)U(M)A(F)A(M)A(M)A(M)T(**V**)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0185 | AS | 185 | T(**V**)^U(F)^U(M)A(M)G(**V**)A(F)G(M)U(F)G(F)A(M)G(M)G(M)Λ(M)U(F)U(M)A(F)A(M)A(M)A(M)U(M)G(**V**)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0186 | AS | 186 | T(**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(**V**)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0187 | AS | 187 | T(**V**)^U(F)^U(M)A(M)G(**V**)A(F)G(M)U(F)G(F)A(M)G(M)G(M)A(M)U(F)U(M)A(F)A(M)A(M)A(**V**)U(M)G(**V**)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0188 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 188 | C(M)^A(M)^U(M)U(M)U(M)U(M)A(F)A(M)C(F)C(F)U(M)C(M)A(M)C(M)U(M)C(M)U(M)A(M)^A(M)^A(M) |
| si-0189 | AS | 139 | T(**V**)^U(F)^U(M)A(M)G(**V**)A(F)G(M)U(F)G(F)A(**V**)G(N)G(M)A(M)U(F)U(M)A(F)A(M)A(M)A(M)U(M)G(M)^A(M)^G(M) |
| | S | 189 | C(M)^A(M)^U(M)U(M)U(M)U(M)A(F)A(M)U(F)cC(F)U(M)C(M)A(M)C(M)U(M)C(M)U(M)A(M)^A(M)^A(M) |
| si-0190 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 190 | C(M)^A(M)^U(M)U(M)U(M)U(M)A(F)A(M)U(F)C(F)cU(M)C(M)A(M)C(M)U(M)C(M)U(M)A(M)^A(M)^A(M) |

**[Table 9]**

| | | | |
|---|---|---|---|
| si-0191 | AS | 186 | T(**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(**V**)^G(M) |
| | S | 143 | 5(**V**)^A(M)^U(M)U(M)U(M)U(M)A(F)A(M)U(E)C(F)C(F)U(M)C(M)A(M)C(M)U(M)C(M)U(M)A(M)^A(M)^A(**V**) |
| si-0192 | AS | 185 | T(**V**)^U(F)^U(M)A(M)G(**V**)A(F)G(M)U(F)G(F)A(M)G(M)G(M)A(M)U(F)U(M)A(F)A(M)A(M)A(M)U(M)G(**V**)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0193 | AS | 184 | T(**V**)^U(F)^U(M)A(M)G(**V**)A(F)G(M)U(F)G(F)A(M)G(M)G(M)A(M)U(F)U(M)A(F)A(M)A(M)A(M)T(**V**)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0194 | AS | 146 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(**V**)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0195 | AS | 147 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(**V**)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0196 | AS | 148 | T(**V**)^U(F)^U(M)A(M)G(**V**)A(F)G(M)U(F)G(F)A(**V**)G(M)G(M)A(M)U(F)U(A(F)A(M)A(M)A(M)T(**V**)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0197 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 143 | 5(**V**)'A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0198 | AS | 186 | T(**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(**V**)^G(M) |
| | S | 141 | 5(**V**)^A(**V**)^U(M)U(M)U(M)U(M)A(F)A(M)U(F)C(F)C(F)U(M)C(M)A(M)C(M)U(M)C(M)U(M)A(M)^A(**V**)^A(**V**) |
| si-0199 | AS | 185 | T(**V**)^U(F)^U(M)A(M)G(**V**)A(F)G(M)U(F)G(F)A(M)G(M)G(M)A(M)U(F)U(M)A(F)A(M)A(M)A(M)U(M)G(**V**)^A(M)^G(M) |
| | S | 141 | 5(**V**)^A(**V**)U(M)U(M)U(M)U(M)A(F)A(M)U(F)C(F)C(F)U(M)C(M)A(M)C(M)A(M)C(M)U(M)A(M)^A(**V**)^A(**V**) |
| si-0200 | AS | 184 | T(**V**)^U(F)^U(M)A(M)G(V)A(F)G(M)U(F)G(F)A(M)G(M)G(M)A(M)U(F)U(M)A(F)A(M)A(M)A(M)T(**V**)G(M)^A(M)^G(M) |
| | S | 141 | 5(**V**)^A(**V**)^U(M)U(M)U(M)U(M)A(F)A(M)U(F)C(F)C(F)U(M)C(M)A(M)C(M)U(M)C(M)U(M)A(M)^A(**V**)^A(**V**) |
| si-0201 | AS | 146 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(**V**)^G(M) |
| | S | 141 | 5(**V**)^A(**V**)^U(M)U(M)U(M)U(M)A(F)A(M)U(F)C(F)C(F)U(M)C(M)A(M)C(M)U(M)C(M)U(M)A(M)^A(**V**)^A(**V**) |
| si-0202 | AS | 147 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(**V**)^A(M)^G(M) |
| | S | 141 | 5(**V**)^A(**V**)^U(M)U(M)U(M)U(M)A(F)A(M)U(F)C(F)C(F)U(M)C(M)A(M)C(M)U(M)C(M)U(M)A(M)^A(**V**)^A(**V**) |
| si-0203 | AS | 148 | T(**V**)^U(F)^U(M)A(M)G(**V**)A(F)G(M)U(F)G(F)A(**V**)G(M)G(M)A(M)U(F)U(M)A(F)A(M)A(M)A(M)T(**V**)G(M)^A(M)^G(M) |
| | S | 141 | 5(**V**)^A(**V**)^U(M)U(M)U(M)U(M)A(F)A(M)U(F)C(F)C(F)U(M)C(M)A(M)C(M)U(M)C(M)U(M)Λ(M)^A(**V**)^A(**V**) |
| si-0204 | AS | 139 | T(**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 141 | 5(**V**)^A(**V**)^U(M)U(M)U(M)U(M)A(F)A(M)U(F)C(F)C(F)U(M)C(M)A(M)C(M)U(M)C(M)U(M)A(M)^A(**V**)^A(**V**) |
| si-0205 | AS | 160 | T(**V**)^U(F)^U(M)A(M)G(**V**)A(F)G(M)U(F)G(F)A(**V**)G(M)G(M)A(M)U(F)U(M)A(F)A(M)A(M)A(**V**)U(M)G(**V**)^A(M)^G(M) |
| | s | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0206 | AS | 187 | 7(**V**)^U(F)^U(M)A(M)G(**V**)A(F)G(M)U(F)G(F)A(M)G(M)G(M)R(M)U(F)U(M)A(F)A(M)A(M)A(**V**)U(M)G(**V**)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0207 | AS | 191 | T(**V**)^U(F)^U(M)A(M)G(**V**)A(F)G(M)U(F)G(F)A(M)G(M)G(M)A(M)U(F)U(M)A(F)A(M)A(M)A(M)T(**V**)G(**V**)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0208 | AS | 192 | T(**V**)^U(F)^U(M)A(M)G(**V**)A(F)G(M)U(F)G(F)A(**V**)G(M)G(M)A(M)U(F)U(M)A(F)A(M)A(M)A(M)F(**V**)G(**V**)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |

### [Evaluation Example 2] Inhibition of expression of rat Sod1 in RPE-J cells (rat retinal pigment epithelial (RPE) cells)

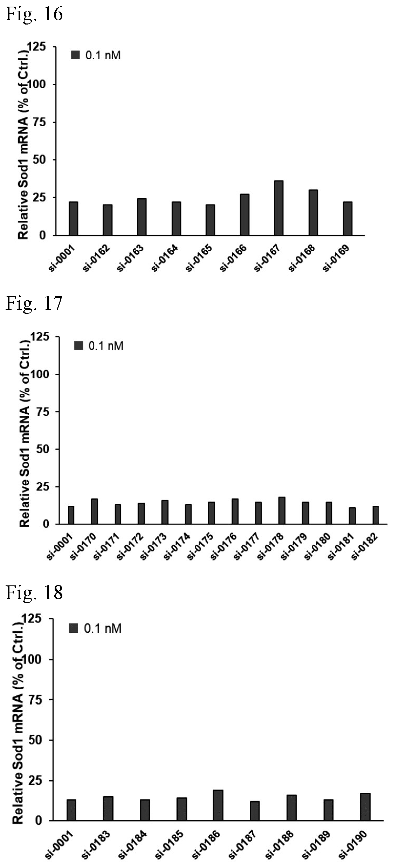

The siRNA described in Tables 7 to 9 was evaluated using the same evaluation method as in Evaluation Example 1. si-0146 to si-0161 were evaluated at 2 concentrations of 0.01 or 0.1 nM, and si-0162 to si-0208 were evaluated at 0.1 nM. The results are shown in Figs. 14 to 20.

As is apparent from Figs. 14 to 20, the siRNA including 2'-O-MCE nucleotides (si-0146 to si-0208) exhibited the expression suppressing effect equivalent to or superior to that of the siRNA not including 2'-O-MCE nucleotides (si-0001). In particular, siRNA (si-0147 to si-0151, si-0160 to si-0161, and the like) including 2'-O-MCE nucleotides at positions 1, 5, and 10 as counted from the 5' end of the antisense strand showed the high expression suppressing effect.

### [Production Example 9] Synthesis of siRNA

According to the method described in Production Example 6, siRNAs described in Table 10 were synthesized. The target RNA of the siRNA described in Table 10 is Sod1 (mouse and rat).

si-0210 to si-0223 are designed based on si-0001 or si-0209, and are siRNA including 2'-O-MCE nucleotides and further including 5'-vinylphosphonate-modified 2'-OMe nucleotides, 5'-vinylphosphonate-modified 2'-O-MCE nucleotides, or 5'-cyclopropanephosphonate-modified 2'-O-MCE nucleotides at position 1 as counted from the 5' end of the antisense strand.

**[Table 10]**

| Compd. No. | Strand | SEQ No | Chemical Structure |
|---|---|---|---|
| si-0001 | S | 2 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| | AS | 1 | U(M)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) |
| si-0209 | AS | 193 | U(vM)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0210 | AS | 194 | T(vV)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0211 | AS | 195 | T(cV)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0212 | AS | 196 | U(vM)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0213 | AS | 197 | U(vM)^U(F)^U(M)A(M)G(**V**)A(F)G(M)U(F)G(F)A(M)G(M)G(M)A(M)U(F)U(M)A(F)A(M)A(M)A(M)U(M)G(**V**)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0214 | AS | 198 | U(vM)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0215 | AS | 199 | U(vM)^U(F)^U(M)A(M)G(**V**)A(F)G(M)U(F)G(F)A(**V**)G(M)G(M)A(M)U(F)U(M)A(F)A(M)A(M)A(M)U(M)G(**V**)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0216 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0217 | AS | 201 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(**V**)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0218 | AS | 202 | T(v**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0219 | AS | 203 | T(v**V**)^U(F)^U(M)A(M)G(**V**)A(F)G(M)U(F)G(F)A(**V**)G(M)G(M)A(M)U(F)U(M)A(F)A(M)A(M)A(M)U(M)G(**V**)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)A(M)^A(**V**) |
| si-0220 | AS | 204 | T(c**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0221 | AS | 205 | T(c**V**)^U(F)^U(M)A(M)G(**V**)A(F)G(M)U(F)G(F)A(M)G(M)G(M)A(M)U(F)U(M)A(F)A(M)A(M)A(M)U(M)G(**V**)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0222 | AS | 206 | T(c**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0223 | AS | 207 | T(c**V**)^U(F)^U(M)A(M)G(**V**)A(F)G(M)U(F)G(F)A(**V**)G(M)G(M)A(M)U(F)U(M)A(F)A(M)A(M)A(M)U(M)G(**V**)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |

### [Evaluation Example 3] Inhibition of expression of rat Sod1 in RPE-J cells (rat retinal pigment epithelial (RPE) cells)

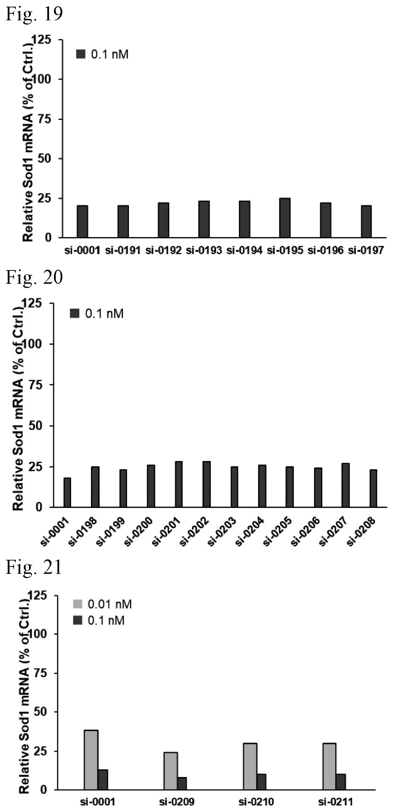

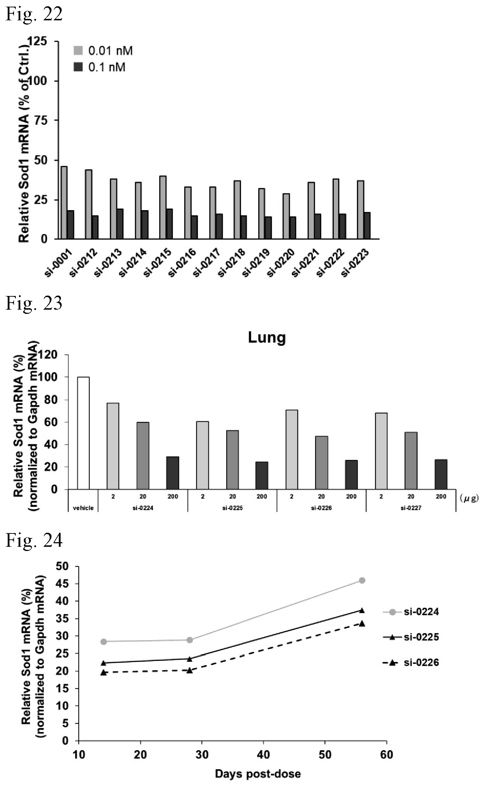

The siRNA described in Table 10 was evaluated at 2 concentrations of 0.01 or 0.1 nM using the same evaluation method as in Evaluation Example 1. The results are shown in Figs. 21 and 22.

As is apparent from Figs. 21 and 22, the siRNA including 2'-O-MCE nucleotides (si-0210 to si-0223) exhibited the expression suppressing effect equivalent to or superior to that of the siRNA not including 2'-O-MCE nucleotides (si-0001 or si-0209). It was confirmed that when 5'-vinylphosphonate-modified 2'-OMe nucleotides, 5'-vinylphosphonate-modified 2'-O-MCE nucleotides, or 5'-cyclopropanephosphonate-modified 2'-O-MCE nucleotides were included at position 1 as counted from the 5' end of the antisense strand, the high expression suppressing effect was exhibited.

### [Evaluation Example 4] Inhibition of expression of rat Sod1 in RPE-J cells (rat retinal pigment epithelial (RPE) cells)

siRNA was tested at various concentrations using the same evaluation method as in Evaluation Example 1. The results are calculated as a concentration (IC₅₀ value) at which an RNA transcript level was 50% with respect to untreated control cells and are shown in Table 11.

**[Table 11]**

| Compd. No. | IC50 (nM) |
|---|---|
| si-0001 | 14 |
| si-0145 | 8.8 |
| si-0197 | 11 |
| si-0209 | 6.9 |
| si-0216 | 5.2 |
| si-0220 | 5.0 |
| si-0217 | 5.3 |
| si-0221 | 5.7 |

As is apparent from Table 11, the siRNA including 2'-O-MCE nucleotides (si-0145, si-0197, si-0216, si-0220, si-0217, or si-0221) exhibited a higher expression suppressing effect than the siRNA not including 2'-O-MCE nucleotides (si-0001 or si-0209).

### [Evaluation Example 5] Inhibition of expression of mouse Sod1 in 3T3-L1 (mouse embryonic fibroblast) cells

Each siRNA was mixed with Lipofectamine (registered trademark) RNAiMAX (Thermo Fisher Scientific) to have various final concentrations, incubated for 15 minutes, and then dispensed into a 96 well plate. Then, 3T3-L1 cells were seeded to each well at a density of 5,000 cells/well (Lipofection). After 20 hours, a cell lysate containing RNA was prepared using CellAmp (registered trademark) Direct RNA Prep Kit for RT-PCR (Real Time) (Takara Bio Inc.), and then the mouse Sod1 gene expression level was measured by quantitative real-time PCR with TaqMan (registered trademark) Gene Expression Assays (Thermo Fisher Scientific) using One Step PrimeScript (registered trademark) III RT-qPCR Mix (Takara Bio Inc.). In the real-time PCR, the housekeeping gene Gapdh (Glyceraldehyde-3-phosphate dehydrogenase) mRNA level was also simultaneously quantified, and the Sod1 mRNA level with respect to the Gapdh mRNA level was evaluated as the Sod1 expression level. The results are calculated as a concentration (IC₅₀ value) at which an RNA transcript level was 50% with respect to untreated control cells and are shown in Table 12.

**[Table 12]**

| Compd. No. | IC50 (nM) |
|---|---|
| si-0001 | 105 |
| si-0144 | 79 |
| si-0145 | 55 |
| si-0197 | 82 |
| si-0209 | 24 |
| si-0216 | 11 |
| si-0220 | 8.2 |
| si-0217 | 20 |
| si-0221 | 23 |

As is apparent from Table 12, the siRNA including 2'-O-MCE nucleotides (si-0144, si-0145, si-0197, si-0216, si-0220, si-0217, or si-0221) exhibited a higher expression suppressing effect than the siRNA not including 2'-O-MCE nucleotides (si-0001 or si-0209).

### [Production Example 10] Synthesis of siRNA

According to the method described in Production Example 6, siRNAs described in Table 13 were synthesized. The target RNA of the siRNA described in Table 13 is Sod1 (mouse).

si-0225 to si-0228 are designed based on si-0224, and are siRNAs including 2'-O-MCE nucleotides and further including 2'-O-hexadecyl nucleotides. si-0224 is an siRNA in which the nucleotide at position 6 as counted from the 5' end of the sense strand of si-0209 is substituted with a 2'-O-hexadecyl nucleotide. Similarly, si-0225 is an siRNA in which a nucleotide of si-0216 is substituted with a 2'-O-hexadecyl nucleotide, si-0226 is an siRNA in which si-0220 is substituted with a 2'-O-hexadecyl nucleotide, si-0227 is an siRNA in which si-0221 is substituted with a 2'-O-hexadecyl nucleotide, and si-0228 is an siRNA in which si-0212 is substituted with a 2'-O-hexadecyl nucleotide.

**[Table 13]**

| Compd. No. | Strand | SEQ No | Chemical Structure |
|---|---|---|---|
| si-0224 | AS | 193 | U(vM)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) |
| | S | 208 | C(M)^A(M)^U(M)U(M)U(M)U(H)A(F)A(M)U(F)C(F)C(F)U(M)C(M)A(M)C(M)U(M)C(M)U(M)A(M)^A(M)^A(M) |
| si-0225 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 209 | 5(**V**)^A(M)^U(M)U(M)U(M)U(H)A(F)A(M)U(F)C(F)C(F)U(M)C(M)A(M)C(M)U(M)C(M)U(M)A(M)^A(M)^A(**V**) |
| si-0226 | AS | 204 | T(c**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 209 | 5(**V**)^A(M)^U(M)U(M)U(M)U(H)A(F)A(M)U(F)C(F)C(F)U(M)C(M)A(M)C(M)U(M)C(M)U(M)A(M)^A(M)^A(**V**) |
| si-0227 | AS | 205 | T(c**V**)^U(F)^U(M)A(M)G(**V**)A(F)G(M)U(F)G(F)A(M)G(M)G(M)A(M)U(F)U(M)A(F)A(M)A(M)A(M)U(M)G(**V**)^A(M)^G(M) |
| | S | 209 | 5(**V**)^A(M)^U(M)U(M)U(M)U(H)A(F)A(M)U(F)C(F)C(F)U(M)C(M)A(M)C(M)U(M)C(M)U(M)A(M)^A(M)^A(**V**) |
| si-0228 | AS | 196 | U(vM)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 209 | 5(**V**)^A(M)^U(M)U(M)U(M)U(H)A(F)A(M)U(F)C(F)C(F)U(M)C(M)A(M)C(M)U(M)C(M)U(M)A(M)^A(M)^A(**V**) |

### [Evaluation Example 6] Sod1 knockdown effect in mouse (lung)

si-0224 to si-0227 described in Table 13 were dissolved in physiological saline (Otsuka saline injection, Otsuka Pharmaceutical Factory) to prepare each siRNA agent. C57BL/6J mice (male, 6 weeks old, Jackson Laboratory Japan Co., Ltd.) were oropharyngeally administered 2, 20, or 200 µg of each siRNA agent. After 2 weeks, the mouse was dissected to excise the lung, RNA was isolated using MagMAX (registered trademark) mirVana (registered trademark) Total RNA Isolation Kit (Thermo Fisher Scientific), and then the mouse Sod1 gene expression level was measured by quantitative real-time PCR with TaqMan (registered trademark) Gene Expression Assays (Thermo Fisher Scientific) using One Step PrimeScript (registered trademark) III RT-qPCR Mix (Takara Bio Inc.). In the real-time PCR, the mRNA amount of Gapdh of the housekeeping gene was also simultaneously quantified, and the mRNA amount of Sod1 with respect to the mRNA amount of Gapdh was evaluated as the expression level of Sod1. The results are shown in Fig. 23 as percent expression of Sod1 relative to the negative control (vehicle).

As is apparent from Fig. 23, the siRNA including 2'-O-MCE nucleotides (si-0225 to si-0227) exhibited a higher expression suppressing effect than the siRNA not including 2'-O-MCE nucleotides (si-0224).

### [Evaluation Example 7] Sod1 knockdown effect in mouse (lung)

For the purpose of evaluating the long-term action of si-0224, si-0225, and si-0226 described in Table 13, the mRNA amount of Sod1 in the lung at 14, 28, and 56 days after administration was evaluated using the same evaluation method as in Evaluation Example 6. The results are shown in Fig. 24.

As is apparent from Fig. 24, it was confirmed that the siRNA including 2'-O-MCE nucleotides (si-0225 and si-0226) exhibited a higher expression suppressing effect at any time than that of the siRNA not including 2'-O-MCE nucleotides (si-0224), and had long-term persistence.

### [Evaluation Example 8] Sod1 knockdown action in mouse (central tissue)

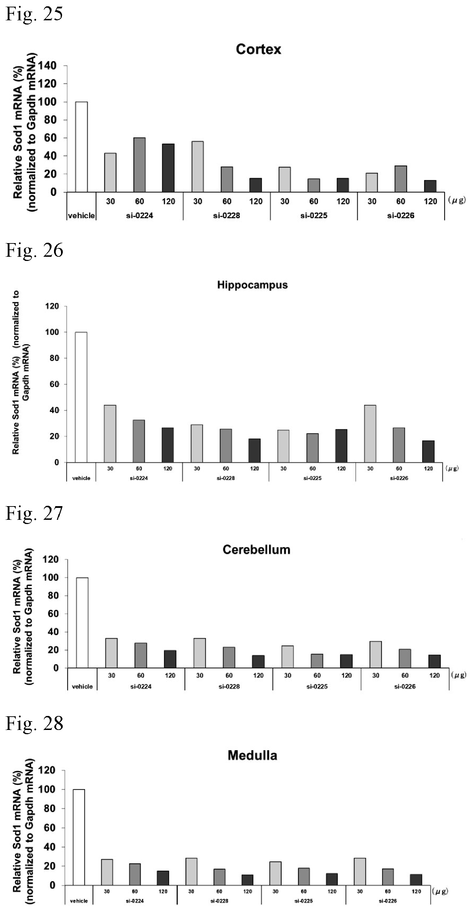

si-0224 to si-0226 and si-0228 described in Table 13 were dissolved in physiological saline (Otsuka saline injection, Otsuka Pharmaceutical Factory) to prepare each siRNA agent. C57BL/6J mice (male, 6 weeks old, Jackson Laboratory Japan Co., Ltd.) were fixed in a brain stereotaxic fixing apparatus under 2.5 to 4% isoflurane anesthesia. Thereafter, a 2 to 3 cm longitudinal skin incision was made between the ears, and a hole was made with a 1 mm diameter drill at 1 mm to the right and 0.5 mm posterior to the bregma. Each siRNA or physiological saline as a negative control was filled into a Hamilton syringe. A needle was inserted approximately 2 mm from the perforated part, and 30, 60, or 120 µg of each siRNA per mouse was administered right lateral ventricle at a rate of 10 µl/min, and skin suturing was performed with a nylon thread. After 2 weeks, the mouse was dissected to excise the cerebrum, the hippocampus, the cerebellum, and the medulla oblongata, RNA was isolated using MagMAX (registered trademark) mirVana (registered trademark) Total RNA Isolation Kit (Thermo Fisher Scientific), and then the mouse Sod1 gene expression level was measured by quantitative real-time PCR with TaqMan (registered trademark) Gene Expression Assays (Thermo Fisher Scientific) using One Step PrimeScript (registered trademark) III RT-qPCR Mix (Takara Bio Inc.). In the real-time PCR, the mRNA amount of Gapdh of the housekeeping gene was also simultaneously quantified, and the mRNA amount of Sod1 with respect to the mRNA amount of Gapdh was evaluated as the expression level of Sod1. The results are shown in Fig. 25 (cerebrum), Fig. 26 (hippocampus), Fig. 27 (cerebellum), and Fig. 28 (medulla oblongata) as percent expression of Sod1 relative to the negative control (vehicle).

As is apparent from Fig. 25 to 28, siRNA including 2'-O-MCE nucleotides (si-0225, si-0226, and si-0228) generally exhibited a higher expression suppressing effect than siRNA not including 2'-O-MCE nucleotides (si-0224) in any tissue.

### [Production Example 11] Synthesis of siRNA

According to the method described in Production Example X, siRNAs described in Tables 14 and 15 were synthesized. The target RNA of the siRNA described in Tables 14 and 15 is Ttr (mouse).

si-0230 to si-0250 are designed based on si-0229, and are siRNA including a plurality of 2'-O-MCE nucleotides. si-0252 to si-0259 are designed based on si-0229 or si-0251, and are siRNA including 2'-O-MCE nucleotides and further including 5'-vinylphosphonate-modified 2'-O-MCE nucleotides, or 5'-cyclopropanephosphonate-modified 2'-O-MCE nucleotides at position 1 as counted from the 5' end of the antisense strand.

**[Table 14]**

| Compd. No. | Strand | SEQ No | Chemical Structure |
|---|---|---|---|
| si-0229 | AS | 210 | J(M)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 211 | A(M)G(M)^U(M)G(M)U(M)U(M)C(F)U(M)U(F)G(F)C(F)U(M)C(M)U(M)A(M)U(M)A(M)A(M)A(M)^C(M)^A(M) |
| si-0230 | AS | 212 | T(**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0231 | AS | 214 | T(**V**)^G(F)^U(M)U(M)U(M)A(**V**)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M) U(F)G(F)C(F)U(M)C(M)U(M)A(M)U(M)A(M)A(M)A(M)^C(M)^A(**V**) |
| si-0232 | AS | 215 | T(**V**)^G(F)^U(M)U(M)T(**V**)A(**V**)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0233 | AS | 216 | T(**V**)^G(F)^U(M)U(M)U(M)A(M)U(M)A(M)G(M)A(**V**)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0234 | AS | 217 | T(**V**)^G(F)^U(M)U(M)T(**V**)A(M)U(M)A(M)G(M)A(**V**)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(**F**)G(F)C(F)U(M)C(M)U(M)A(M)U(M)A(M)A(M)A(M)^C(M)^A(**V**) |
| si-0235 | AS | 218 | T(**V**)^G(F)^U(M)U(M)U(M)A(**V**)U(M)A(M)G(M)A(**V**)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0236 | AS | 219 | T(**V**)^G(F)^U(M)U(M)T(**V**)A(**V**)U(M)A(M)G(M)A(**V**)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0237 | AS | 220 | T(**V**)^G(F)^U(M)U(M)U(M)A(M)U(M)A(**V**)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0238 | AS | 221 | T(**V**)^G(F)^U(M)U(M)U(M)A(M)U(M)A(M)G(**V**)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0239 | AS | 222 | T(**V**)^G(F)^U(M)U(M)T(**V**)A(M)U(M)A(**V**)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0240 | AS | 223 | T(**V**)^G(F)^U(M)U(M)T(**V**)A(M)U(M)A(M)G(**V**)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0241 | AS | 224 | T(**V**)^G(F)^U(M)U(M)U(M)A(**V**)U(M)A(**V**)G(**V**)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0242 | AS | 225 | T(**V**)^G(F)^U(M)U(M)U(M)A(M)U(M)A(**V**)G(M)A(**V**)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0243 | AS | 226 | T(**V**)^G(F)^U(M)U(M)U(M)A(M)U(M)A(M)G(**V**)A(**V**)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0244 | AS | 212 | T(**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 227 | A(**V**)^G(**V**)^U(M)G(M)U(M)U(M)C(F)U(M)U(F)G(F)C(F)U(M)C(M)U(M)A(M)U(M)A(M)A(M)A(M)^5(**V**)^A(**V**) |
| si-0245 | AS | 217 | T(**V**)^G(F)^U(M)U(M)T(**V**)A(M)U(M)A(M)G(M)A(**V**)G(M)C(M)A(M)A(F)G(M)A(F)A(M)C(M)A(M)C(M)U(M)^G(M)^U(M) |
| | S | 227 | A(**V**)^G(**V**)^U(M)G(M)U(M)U(M)C(F)U(M)U(F)G(F)C(F)U(M)C(M)U(M)A(M)U(M)A(M)A(M)A(M)^5(**V**)^A(**V**) |
| si-0246 | AS | 212 | T(**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 211 | A(M)^G(M)^U(M)G(M)U(M)U(M)C(F)U(M)U(F)G(F)C(F)U(M)C(M)U(M)A(M)U(M)A(M)A(M)A(M)^C(M)^A(M) |
| si-0247 | AS | 217 | T(**V**)^G(F)^U(M)U(M)T(**V**)A(M)U(M)A(M)G(M)A(**V**)G(M)C(M)A(M)A(F)G(M)A(F)A(M)C(M)A(M)C(M)U(M)^G(M)^U(M) |
| | S | 211 | A(M)^G(M)^U(M)G(M)U(M)U(M)C(F)U(M)U(F)G(F)C(F)U(M)C(M)U(M)A(M)U(M)A(M)A(M)A(M)^C(M)^A(M) |
| si-0248 | AS | 228 | T(**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(**V**)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0249 | AS | 229 | T(**V**)^G(F)^U(M)U(M)T(**V**)A(M)U(M)A(M)G(M)A(M)G(M)C(M)A(M)A(F)G(M)A(F)A(M)C(M)A(M)C(M)T(**V**)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0250 | AS | 230 | T(**V**)^G(F)^U(M)U(M)T(**V**)A(M)U(M)A(M)G(M)A(M)G(M)C(M)A(M)A(F)G(M)A(F)A(M)C(M)A(M)5(**V**)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |

**[Table 15]**

| | | | |
|---|---|---|---|
| si-0251 | AS | 231 | U(vM)^G(F)^U(M)U(M)U(M)A(M)U(M)A(M)G(M)A(M)G(M)C(M)A(M)A(F)G(M)A(F)A(M)C(M)A(M) C(M)U(M)^G(M)^U(M) |
| | S | 211 | A(M)^G(M)^U(M)G(M)U(M)U(M)C(F)U(M)U(F)G(F)C(F)U(M)C(M)U(M)A(M)U(M)A(M)A(M)A(M)^C(M)^A(M) |
| si-0252 | AS | 232 | T(v**V**)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | 5 | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0253 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0254 | AS | 234 | T(v**V**)^G(F)^U(M)U(M)U(M)A(M)U(M)A(**V**)G(M)A(M)G(M)C(M)A(M)A(F)G(M)A(F)A(M)C(M)A(M)C(M)U(M)^G(M)'U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0255 | AS | 235 | T(v**V**)^G(F)^U(M)U(M)U(M)A(M)U(M)A(M)G(V)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0256 | AS | 236 | T(v**V**)^G(F)^U(M)U(M)U(M)A(M)U(M)A(M)G(M)A(**V**)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0257 | AS | 237 | T(v**V**)^G(F)^U(M)U(M)U(M)A(M)U(M)A(M)G(M)A(M)G(M)C(M)A(M)A(F)G(M)A(F)A(M)C(M)A(M)C(M)U(M)^G**(**V)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0258 | AS | 238 | T(v**V**)^G(F)^U(M)U(M)T(**V**)A(M)U(M)A(M)G(M)A(**V**)G(M)C(M)A(M)A(F)G(M)A(F)A(M)C(M)A(M)C(M)lJ(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0259 | AS | 239 | T(c**V**)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |

### [Evaluation Example 9] Inhibition of expression of mouse Ttr in mouse primary hepatocytes

Mouse primary hepatocytes were seeded at a density of 20,000 cells/well in a 96 well plate, and each siRNA described in Tables 14 and 15 was added using Lipofectamine RNAiMAX Transfection Reagent (Thermo Fisher Scientific) such that the set final concentration was at each concentration, followed by incubation at 37°C for 20 hours. RNA was isolated using MagMAX (registered trademark) mirVana (registered trademark) Total RNA Isolation Kit (Thermo Fisher Scientific), and then the mouse Ttr (Transthyretin) gene expression level was measured by quantitative real-time PCR with TaqMan (registered trademark) Gene Expression Assays (Thermo Fisher Scientific) using One Step PrimeScript (registered trademark) III RT-qPCR Mix (Takara Bio Inc.). In the real-time PCR, the mRNA level of Gapdh, a housekeeping gene, was also simultaneously quantified, and the mRNA level of Ttr relative to that of Gapdh was evaluated as the expression level of Ttr.

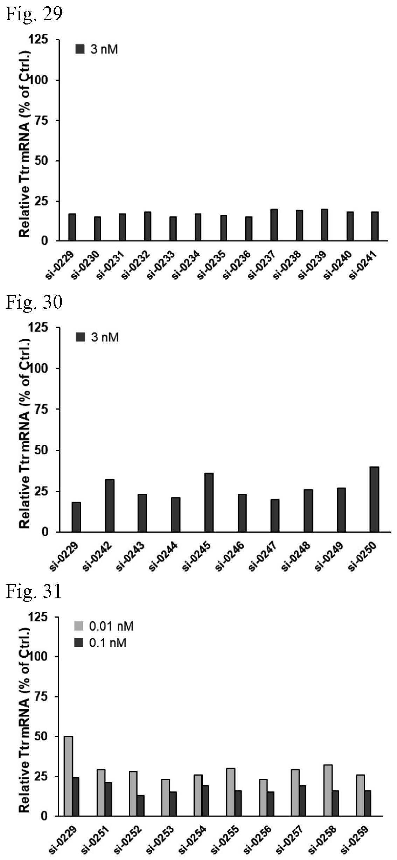

si-0230 to si-0250 was evaluated at 3 nM, and si-0252 to si-0259 was evaluated at 2 concentrations of 0.01 or 0.1 nM. The results are shown in Figs. 29 to 31.

As is apparent from Figs. 29 to 31, the siRNA including 2'-O-MCE nucleotides (si-0230 to si-0250 and si-0252 to si-0259) exhibited the expression suppressing effect equivalent to or superior to that of the siRNA not including 2'-O-MCE nucleotides (si-0229 or si-0251). In particular, siRNA (si-0252 to si-0259) including 5'-vinylphosphonate-modified 2'-O-MCE nucleotides or 5'-cyclopropanephosphonate-modified 2'-O-MCE nucleotides at position 1 as counted from the 5' end of the antisense strand showed a high expression suppressing effect.

### [Production Example 12] Synthesis of siRNA

According to the method described in Production Example 6, siRNAs described in Tables 16 and 17 were synthesized. Target RNAs of the siRNAs described in Tables 16 and 17 are FXII (mouse).

si-0261 to si-0281 are designed based on si-0260, and are siRNA including a plurality of 2'-O-MCE nucleotides. si-0283 to si-0289 are designed based on si-0260 or si-0282, and are siRNA including 2'-O-MCE nucleotides and further including 5'-vinylphosphonate-modified 2'-O-MCE nucleotides, or 5'-cyclopropanephosphonate-modified 2'-O-MCE nucleotides.

**[Table 16]**

| Compd. No. | Strand | SEQ No | Chemical Structure |
|---|---|---|---|
| si-0260 | AS | 240 | U(M)^A(F)^A(M)A(M)G(M)C(F)A(M)C(M)U(M)U(M)U(M)A(M)U(M)U(F)G(M)A(F)G(M)U(M)U(M)U(M)C(M)^U(M)^G(M) |
| | S | 241 | G(M)^A(M)^A(M)A(M)C(M)U(M)C(F)A(M)A(F)U(F)A(F)A(M)A(M)G(M)U(M)G(M)C(M)U(M)U(M)^U(M)^A(M) |
| si-0261 | AS | 242 | T(**V**)^A(F)^A(M)A(M)G(**V**)C(F)A(M)C(M)U(M)U(M)U(M)A(M)U(M)U(F)G(M)A(F)G(M)U(M)U(M)U(M)C(M)^U(M)^G(M) |
| | S | 243 | G(**V**)^A(M)^A(M)A(M)C(M)U(M)C(F)A(M)A(F)U(F)A(F)A(M)A(M)G(M)U(M)G(M)C(M)U(M)U(M)^U(M)^A(**V**) |
| si-0262 | AS | 244 | T(**V**)^A(F)^A(M)A(M)G(M)5(**V**)A(M)C(M)U(M)U(M)U(M)A(M)U(M)U(F)G(M)A(F)G(M)U(M)U(M)U(M)C(M)^U(M)^G(M) |
| | S | 243 | G(**V**)^A(M)^A(M)A(M)C(M)U(M)C(F)A(M)A(F)U(F)A(F)A(M)A(M)G(M)U(M)G(M)C(M)U(M)U(M)^U(M)^A(**V**) |
| si-0263 | AS | 245 | T(**V**)^A(F)^A(M)A(M)G(M)C(F)A(M)C(M)U(M)T(**V**)U(M)A(M)U(M)U(F)G(M)A(F)G(M)U(M)U(M)U(M)C(M)^U(M)^G(M) |
| | S | 243 | G(**V**)^A(M)^A(M)A(M)C(M)U(M)C(F)A(M)A(F)U(F)A(F)A(M)A(M)G(M)U(M)G(M)C(M)U(M)U(M)^U(M)^A(**V**) |
| si-0264 | AS | 246 | T(**V**)^A(F)^A(M)A(M)G(**V**)C(F)A(M)C(M)U(M)T(**V**)U(M)A(M)U(M)U(F)G(M)A(F)G(M)U(M)U(M)U(M)C(M)^U(M)^G(M) |
| | S | 243 | G(**V**)^A(M)^A(M)A(M)C(M)U(M)C(F)A(M)A(F)U(F)A(F)A(M)A(M)G(M)U(M)G(M)C(M)U(M)U(M)^U(M)^A(**V**) |
| si-0265 | AS | 247 | T(**V**)^A(F)^A(M)A(M)G(M)5(**V**)A(M)C(M)U(M)T(**V**)U(M)A(M)U(M)U(F)G(M)A(F)G(M)U(M)U(M)U(M)C(M)^U(M)^G(M) |
| | S | 243 | G(**V**)^A(M)^A(M)A(M)C(M)U(M)C(F)A(M)A(F)U(F)A(F)A(M)A(M)G(M)U(M)G(M)C(M)U(M)U(M)^U(M)^A(**V**) |
| si-0266 | AS | 248 | T(**V**)^A(F)^A(M)A(M)G(M)C(F)A(M)5(V)U(M)U(M)U(M)A(M)U(M)U(F)G(M)A(F)G(M)U(M)U(M)U(M)C(M)^U(M)^G(M) |
| | S | 243 | G(**V**)^A(M)^A(M)A(M)C(M)U(M)C(F)A(M)A(F)U(F)A(F)A(M)A(M)G(M)U(M)G(M)C(M)U(M)U(M)^U(M)^A(**V**) |
| si-0267 | AS | 249 | T(**V**)^A(F)^A(M)A(M)G(M)C(F)A(M)C(M)T(**V**)U(M)U(M)A(M)U(M)U(F)G(M)A(F)G(M)U(M)U(M)U(M)C(M)^U(M)^G(M) |
| | S | 243 | G(**V**)^A(M)^A(M)A(M)C(M)U(M)C(F)A(M)A(F)U(F)A(F)A(M)A(M)G(M)U(M)G(M)C(M)U(M)U(M)^U(M)^A(**V**) |
| si-0268 | AS | 250 | T(**V**)^A(F)^A(M)A(M)G(**V**)C(F)A(M)5(**V**)U(M)U(M)U(M)A(M)U(M)U(F)G(M)A(F)G(M)U(M)U(M)U(M)C(M)^U(M)^G(M) |
| | S | 243 | G(**V**)^A(M)^A(M)A(M)C(M)U(M)C(F)A(M)A(F)U(F)A(F)A(M)A(M)G(M)U(M)G(M)C(M)U(M)U(M)^U(M)^A(**V**) |
| si-0269 | AS | 251 | T(**V**)^A(F)^A(M)A(M)G(**V**)C(F)A(M)C(M)T(**V**)U(M)U(M)A(M)U(M)U(F)G(M)A(F)G(M)U(M)U(M)U(M)C(M)^U(M)^G(M) |
| | S | 243 | G(**V**)^A(M)^A(M)A(M)C(M)U(M)C(F)A(M)A(F)U(F)A(F)A(M)A(M)G(M)U(M)G(M)C(M)lJ(M)U(M)^U(M)^A(**V**) |
| si-0270 | AS | 252 | T(**V**)^A(F)^A(M)A(M)G(M)5(**V**)A(M)5(**V**)T(**V**)U(M)U(M)A(M)U(M)U(F)G(M)A(F)G(M)U(M)U(M)U(M)C(M)^U(M)^G(M) |
| | S | 243 | G(**V**)^A(M)^A(M)A(M)C(M)U(M)C(F)A(M)A(F)U(F)A(F)A(M)A(M)G(M)U(M)G(M)C(M)U(M)U(M)^U(M)^A(**V**) |
| si-0271 | AS | 253 | T(**V**)^A(F)^A(M)A(M)G(M)C(F)A(M)5(**V**)U(M)T**(**V)U(M)A(M)U(M)U(F)G(M)A(F)G(M)U(M)U(M)U(M)C(M)^U(M)^G(M) |
| | S | 243 | G(**V**)^A(M)^A(M)A(M)C(M)U(M)C(F)A(M)A(F)U(F)A(F)A(M)A(M)G(M)U(M)G(M)C(M)U(M)U(M)^U(M)^A(**V**) |
| si-0272 | AS | 254 | T(**V**)^A(F)^A(M)A(M)G(M)C(F)A(M)C(M)T(**V**)T(**V**)U(M)A(M)U(M)U(F)G(M)A(F)G(M)U(M)U(M)U(M)C(M)^U(M)^G(M) |
| | S | 243 | G(**V**)^A(M)^A(M)A(M)C(M)U(M)C(F)A(M)A(F)U(F)A(F)A(M)A(M)G(M)U(M)G(M)C(M)U(M)U(M)^U(M)^A(**V**) |
| si-0273 | AS | 242 | T(**V**)^A(F)^A(M)A(M)G(**V**)C(F)A(M)C(M)U(M)U(M)U(M)A(M)U(M)U(F)G(M)A(F)G(M)U(M)U(M)U(M)C(M)^U(M)^G(M) |
| | S | 255 | G(**V**)^A(**V**)^A(M)A(M)C(M)U(M)C(F)A(M)A(F)U(F)A(F)A(M)A(M)G(M)U(M)G(M)C(M)U(M)U(M)^T(**V**)^A(**V**) |
| si-0274 | AS | 246 | T(**V**)^A(F)^A(M)A(M)G(**V**)C(F)A(M)C(M)U(M)T(V)U(M)A(M)U(M)U(F)G(M)A(F)G(M)U(M)U(M)U(M)C(M)^U(M)^G(M) |
| | S | 255 | G(**V**)^A(**V**)^A(M)A(M)C(M)U(M)C(F)A(M)A(F)U(F)A(F)A(M)A(M)G(M)U(M)G(M)C(M)U(M)U(M)^T(**V**)^A(**V**) |
| si-0275 | AS | 242 | T(**V**)^A(F)^A(M)A(M)G(**V**)C(F)A(M)C(M)U(M)U(M)U(M)A(M)U(M)U(F)G(M)A(F)G(M)U(M)U(M)U(M)C(M)^U(M)^G(M) |
| | S | 241 | G(M)^A(M)^A(M)A(M)C(M)U(M)C(F)A(M)A(F)U(F)A(F)A(M)A(M)G(M)U(M)G(M)C(M)U(M)U(M)^U(M)^A(M) |
| si-0276 | AS | 246 | T(**V**)^A(F)^A(M)A(M)G(**V**)C(F)A(M)C(M)U(M)T(V)U(M)A(M)U(M)U(F)G(M)A(F)G(M)U(M)U(M)U(M)C(M)^U(M)^G(M) |
| | S | 241 | G(M)^A(M)^A(M)A(M)C(M)U(M)C(F)A(M)A(F)U(F)A(F)A(M)A(M)G(M)U(M)G(M)C(M)U(M)U(M)^U(M)^A(M) |
| si-0277 | AS | 256 | T(**V**)^A(F)^A(M)A(M)G(**V**)C(F)A(M)C(M)U(M)U(M)U(M)A(M)U(M)U(F)G(M)A(F)G(M)U(M)U(M)U(M)C(M)^T(V)^G(M) |
| | S | 243 | G(V)^A(M)^A(M)A(M)C(M)U(M)C(F)A(M)A(F)U(F)A(F)A(M)A(M)G(M)U(M)G(M)C(M)U(M)U(M)^U(M)^A(V) |
| si-0278 | AS | 257 | T(V)^A(F)^A(M)A(M)G(V)C(F)A(M)C(M)U(M)U(M)U(M)A(M)U(M)U(F)G(M)A(F)G(M)U(M)U(M)U(M)5(V)^U(M)^G(M) |
| | S | 243 | G(V)^A(M)^A(M)A(M)C(M)U(M)C(F)A(M)A(F)U(F)A(F)A(M)A(M)G(M)U(M)G(M)C(M)U(M)U(M)^U(M)^A(V) |
| si-0279 | AS | 258 | T(V)^A(F)^A(M)A(M)G(V)C(F)A(M)C(M)U(M)U(M)U(M)A(M)U(M)U(F)G(M)A(F)G(M)U(M)U(M)T(V)C(M)^U(M)^G(M) |
| | S | 243 | G(V)^A(M)^A(M)A(M)C(M)U(M)C(F)A(M)A(F)U(F)A(F)A(M)A(M)G(M)U(M)G(M)C(M)U(M)U(M)^U(M)^A(V) |
| si-0280 | AS | 259 | T(V)^A(F)^A(M)A(M)G(V)C(F)A(M)C(M)U(M)U(M)U(M)A(M)U(M)U(F)G(M)A(F)G(M)U(M)U(M)U(M)5(V)^T(V)^G(M) |
| | S | 243 | G(V)^A(M)^A(M)A(M)C(M)U(M)C(F)A(M)A(F)U(F)A(F)A(M)A(M)G(M)U(M)G(M)C(M)U(M)U(M)^U(M)^A(V) |
| si-0281 | AS | 260 | T(V)^A(F)^A(M)A(M)G(V)C(F)A(M)C(M)U(M)U(M)U(M)A(M)U(M)U(F)G(M)A(F)G(M)U(M)U(M)T(V)5(V)^U(M)^G(M) |
| | S | 243 | G(V)^A(M)^A(M)A(M)C(M)U(M)C(F)A(M)A(F)U(F)A(F)A(M)A(M)G(M)U(M)G(M)C(M)U(M)U(M)^U(M)^A(V) |

**[Table 17]**

| Compd. No. | Strand | SEQ No | Chemical Structure |
|---|---|---|---|
| si-0282 | AS | 261 | U(vM)^A(F)^A(M)A(M)G(M)C(F)A(M)C(M)U(M)U(M)U(M)A(M)U(M)U(F)G(M)A(F)G(M)U(M)U(M)U(M)C(M)^U(M)^G(M) |
| | S | 241 | G(M)^A(M)^A(M)A(M)C(M)U(M)C(F)A(M)A(F)U(F)A(F)A(M)A(M)G(M)U(M)G(M)C(M)U(M)U(M)^U(M)^A(M) |
| si-0283 | AS | 262 | T(vV)^A(F)^A(M)A(M)G(M)C(F)A(M)C(M)U(M)U(M)U(M)A(M)U(M)U(F)G(M)A(F)G(M)U(M)U(M)U(M)C(M)^U(M)^G(M) |
| | S | 243 | G(V)^A(M)^A(M)A(M)C(M)U(M)C(F)A(M)A(F)U(F)A(F)A(M)A(MìG(M)U(M)G(M)C(M)U(M)U(M)^U(M)^A(V) |
| si-0284 | AS | 263 | T(vV)^A(F)^A(M)A(M)G(V)C(F)A(M)C(M)U(M)U(M)U(M)A(M)U(M)U(F)G(M)A(F)G(M)U(M)U(M)U(M)C(M)^U(M)^G(M) |
| | S | 243 | G(V)^A(M)^A(M)A(M)C(M)U(M)C(F)A(M)A(F)U(F)A(F)A(M)A(M)G(M)U(M)G(M)C(M)U(M)U(M)^U(M)^A(V) |
| si-0285 | AS | 264 | T(vV)^A(F)^A(M)A(M)G(M)C(F)A(M)5(V)U(M)U(M)U(M)A(M)U(M)U(F)G(M)A(F)G(M)U(M)U(M)U(M)C(M)^U(M)^G(M) |
| | S | 243 | G(V)^A(M)^A(M)A(M)C(M)U(M)C(F)A(M)A(F)U(F)A(F)A(M)A(M)G(M)U(M)G(M)C(M)U(M)U(M)^U(M)^A(V) |
| si-0286 | AS | 265 | T(vV)^A(F)^A(M)A(M)G(M)C(F)A(M)C(M)U(M)T(V)U(M)A(M)U(M)U(F)G(M)A(F)G(M)U(M)U(M)U(M)C(M)^U(M)^G(M) |
| | S | 243 | G(V)^A(M)^A(M)A(M)C(M)U(M)C(F)A(M)A(F)U(F)A(F)A(M)A(M)G(M)U(M)G(M)C(M)U(M)U(M)^U(M)^A(V) |
| si-0287 | AS | 266 | T(vV)^A(F)^A(M)A(M)G(V)C(F)A(M)C(M)U(M)T(V)U(M)A(M)U(M)U(F)G(M)A(F)G(M)U(M)U(M)U(M)C(M)^U(M)^G(M) |
| | S | 243 | G(V)^A(M)^A(M)A(M)C(M)U(M)C(F)A(M)A(F)U(F)A(F)A(M)A(M)G(M)U(M)G(M)C(M)U(M)U(M)^U(M)^A(V) |
| si-0288 | AS | 267 | T(vV)^A(F)^A(M)A(M)G(M)C(F)A(M)C(M)U(M)U(M)U(M)A(M)U(M)U(F)G(M)A(F)G(M)U(M)U(M)U(M)C(M)^T(V)^G(M) |
| | S | 243 | G(V)^A(M)^A(M)A(M)C(M)U(M)C(F)A(M)A(F)U(F)A(F)A(M)A(M)G(M)U(M)G(M)C(M)U(M)U(M)^U(M)^A(V) |
| si-0289 | AS | 268 | T(cV)^A(F)^A(M)A(M)G(M)C(F)A(M)C(M)U(M)U(M)U(M)A(M)U(M)U(F)G(M)A(F)G(M)U(M)U(M)U(M)C(M)^U(M)^G(M) |
| | S | 243 | G(V)^A(M)^A(M)A(M)C(M)U(M)C(F)A(M)A(F)U(F)A(F)A(M)A(M)G(M)U(M)G(M)C(M)U(M)U(M)^U(M)^A(V) |

### [Evaluation Example 10] Inhibition of expression of mouse FXII in mouse primary hepatocytes

Mouse primary hepatocytes were seeded at a density of 20,000 cells/well in a 96 well plate, and each siRNA described in Tables 16 and 17 was added using Lipofectamine RNAiMAX Transfection Reagent (Thermo Fisher Scientific) such that the set final concentration was at each concentration, followed by incubation at 37°C for 20 hours. RNA was isolated using MagMAX (registered trademark) mirVana (registered trademark) Total RNA Isolation Kit (Thermo Fisher Scientific), and then the mouse FXII (FactorXII) gene expression level was measured by quantitative real-time PCR with TaqMan (registered trademark) Gene Expression Assays (Thermo Fisher Scientific) using One Step PrimeScript (registered trademark) III RT-qPCR Mix (Takara Bio Inc.). In the real-time PCR, the mRNA amount of Gapdh of the housekeeping gene was also simultaneously quantified, and the mRNA amount of FXII with respect to the mRNA amount of Gapdh was evaluated as the expression level of FXII.

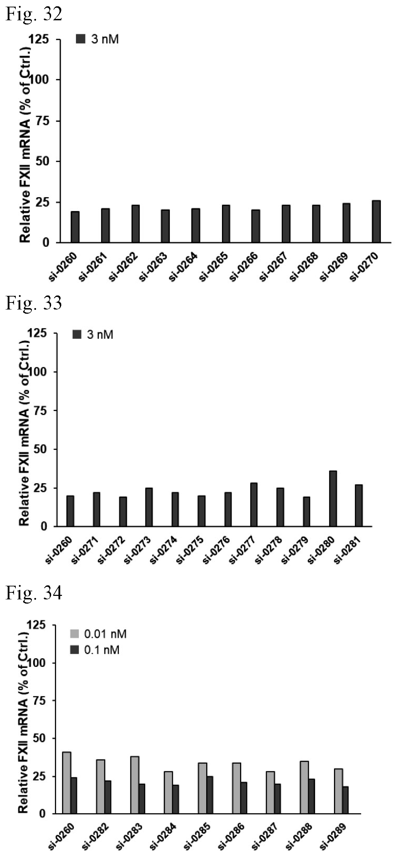

si-0261 to si-0281 were evaluated at 1 concentration of 3 nM, and si-0283 to 0289 were evaluated at 2 concentrations of 0.01 or 0.1 nM. The results are shown in Figs. 32 to 34.

As is apparent from Figs. 32 to 34, the siRNA including 2'-O-MCE nucleotides exhibited the expression suppressing effect equivalent to or superior to that of the siRNA not including 2'-O-MCE nucleotides (si-0260 or si-0282). In particular, siRNA (si-0284, si-0286, si-0287, and si-0289) including 5'-vinylphosphonate-modified 2'-O-MCE nucleotides or 5'-cyclopropanephosphonate-modified 2'-O-MCE nucleotides at position 1 as counted from the 5' end of the antisense strand showed a high expression suppressing effect.

### [Production Example 13] Synthesis of siRNA

According to the method described in Production Example 6, siRNAs described in Table 18 were synthesized. The target RNA of the siRNA described in Table 18 is Ttr (mouse).

A conjugate with a GalNAc ligand was synthesized with reference to Bioconjugate Chem. 2015, 26, 8, 1451-1455.

si-0292 to si-0297 are siRNAs including 2'-O-MCE nucleotides and further including a GalNAc ligand, designed based on si-0290 or si-0291. si-0290 is an siRNA in which a GalNAc ligand is conjugated to the 5' end of the sense strand of si-0229. Similarly, si-0291 is an siRNA in which a GalNAc ligand is conjugated to the 5' end of a sense strand of si-0251, si-0292 is an siRNA in which a GalNAc ligand is conjugated to the 5' end of a sense strand of si-0252, si-0293 is an siRNA in which a GalNAc ligand is conjugated to the 5' end of a sense strand of si-0253, si-0294 is an siRNA in which a GalNAc ligand is conjugated to the 5' end of a sense strand of si-0255, si-0295 is an siRNA in which a GalNAc ligand is conjugated to the 5' end of a sense strand of si-0258, and si-0296 is an siRNA in which a GalNAc ligand is conjugated to the 5' end of a sense strand of si-0259.

**[Table 18]**

| Compd. No. | Strand | SEQ No | Chemical Structure |
|---|---|---|---|
| si-0290 | AS | 210 | U(M)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 269 | Galx3_glu_5A6_s#A(M)^G(M)^U(M)G(M)U(M)U(M)C(F)U(M)U(F)G(F)C(F)U(V)C(M)U(M)A(M)U(M)A(M)A(M)A(M)^C(M)^A(M) |
| si-0291 | AS | 231 | U(vM)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 269 | Galx3_glu_5A6_s#A(M)^G(M)^U(M)G(M)U(M)U(M)C(F)U(M)U(F)G(F)C(F)U(M)C(M)U(M)A(M)U(M)A(M)A(M)A(M)^C(M)^A(M) |
| si-0292 | AS | 232 | T(v**V**)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 270 | Galx3_glu_5A6_s#A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0293 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 270 | Galx3_glu_5A6_s#A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0294 | AS | 235 | T(v**V**)^G(F)^U(M)U(M)U(M)A(M)U(M)A(M)G(**V**)A(M)G(M)C(M)A(M)A(F)G(M)A(F)A(M)C(M)A(M)C(M)U(M)^G(M)^U(M) |
| | S | 270 | Galx3)glu)5A6)s#A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0295 | AS | 238 | T(v**V**)^G(F)^U(M)U(M)T(**V**)A(M)U(M)A(M)G(M)A(**V**)G(M)C(M)A(M)A(F)G(M)A(F)A(M)C(M)A(M)C(M)U(M)^G(M)^U(M) |
| | S | 270 | Galx3_glu_5A6_s#A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0296 | AS | 239 | T(c**V**)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 270 | Galx3_glu_5A6_s#A(**V**)^G(M)^U(M)G(M)U (M)U(M)C(F)U(M)U(F)G(F)C(F)U(M)C(M)U(M)A(M)U(M)A(M)A(M) A(M)^C(M)^A(**V**) |
| si-0297 | AS | 271 | T(c**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 270 | Galx3_glu_5A6_s#A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |

### [Evaluation Example 11] Ttr knockdown action in mice

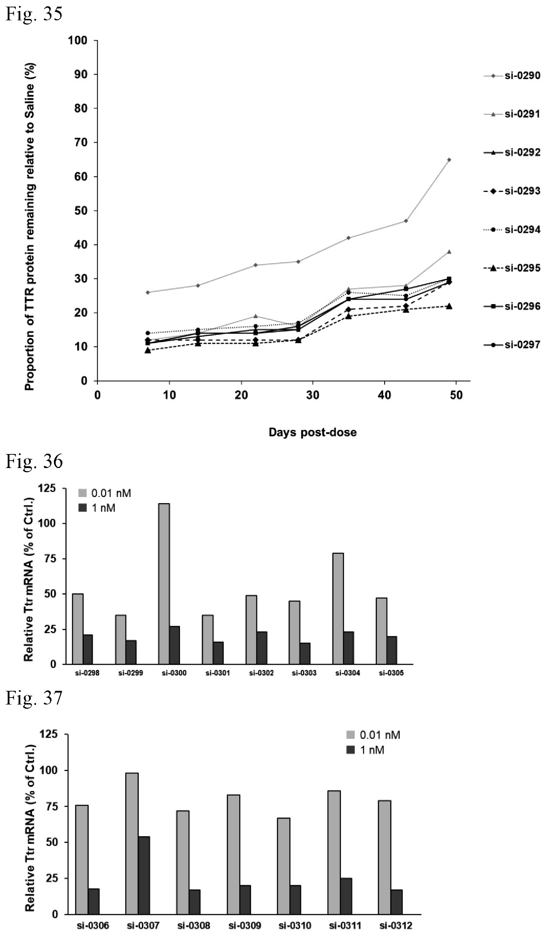

siRNA described in Table 18 was dissolved in physiological saline (Otsuka saline injection, Otsuka Pharmaceutical Factory) to prepare each siRNA agent. Each siRNA agent was subcutaneously administered to C57BL/6J mice (male, 6 weeks old, Jackson Laboratory Japan Co., Ltd.) at 1 mg/kg, and blood was collected and treated into serum on Days 7, 14, 22, 28, 35, 43, and 49 after administration. TTR levels were determined by ELISA and expressed as percent expression of TTR relative to a negative control (vehicle). The results are shown in Fig. 35.

As is apparent from Fig. 35, on Day 49, it was confirmed that the siRNA including 2'-O-MCE nucleotides (si-0292 to si-0297) exhibited a higher expression suppressing effect than that of the siRNAs not including 2'-O-MCE nucleotides (si-0290 and si-0291), and had long-term persistence.

### [Production Example 14] Synthesis of siRNA

According to the method described in Production Example 6, siRNAs described in Tables 19 and 20 were synthesized. The target RNA of the siRNA described in Tables 19 and 20 is Ttr (mouse).

si-0299 to si-0305 are siRNAs including 2'-O-MCE nucleotides, designed based on si-0298 including deoxyribonucleotides. si-0307 to si-0312 are siRNAs including 2'-O-MCE nucleotides, designed based on si-0306 including deoxyribonucleotides.

**[Table 19]**

| Compd. No. | Strand | SEQ No | Chemical Structure |
|---|---|---|---|
| si-0298 | AS | 272 | U(vM)^g^G(M)U(M)tU(M)aU(M)A(M)G(M)A(M)gC(M)A(F)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0299 | AS | 274 | T(v**V**)^g^G(M)U(M)tU(M)aU(M)A(M)G(M)A(M)gC(M)A(F)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0300 | AS | 275 | T(v**V**)^G(**V**)^G(M)U(M)tU(M)aU(M)A(M)G(M)A(M)gC(M)A(F)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0301 | AS | 276 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0302 | AS | 277 | T(v**V**)^g^G(M)U(M)tU(M)A(V)U(M)A(M)G(M)A(M)gC(M)A(F)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0303 | AS | 278 | T(v**V**)^g^G(M)U(M)tU(M)aU(M)A(M)G(M)A(M)G(**V**)C(M)A(F)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0304 | AS | 279 | T(v**V**)^g^G(M)U(M)tU(M)aU(M)A(M)G(M)A(M)gC(M)A(**V**)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0305 | AS | 280 | T(v**V**)^g^G(M)U(M)T(**V**)U(M)aU(M)A(M)G(M)A(M)gC(M)A(F)A(M)G(M)A(M)A(M)C(M)A(M)C(M)^T(**V**)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |

**[Table 20]**

| | | | |
|---|---|---|---|
| si-0306 | AS | 281 | U(M)^g^G(M)U(M)tU(M)aU(M)A(M)G(M)A(M)gC(M)A(F)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0307 | AS | 282 | U(M)^G(**V**)^G(M)U(M)tU(M)aU(M)A(M)G(M)A(M)gC(M)A(F)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0308 | AS | 283 | U(M)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0309 | AS | 284 | U(M)^g^G(M)U(M)tU(M)A(**V**)U(M)A(M)G(M)A(M)gC(M)A(F)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0310 | AS | 285 | U(M)^g^G(M)U(M)tU(M)aU(M)A(M)G(M)A(M)G(**V**)C(M)A(F)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0311 | AS | 286 | U(M)^g^G(M)U(M)tU(M)aU(M)A(M)G(M)A(M)gC(M)A(**V**)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0312 | AS | 287 | U(M)^g^G(M)U(M)T(**V**)U(M)aU(M)A(M)G(M)A(M)gC(M)A(F)A(M)G(M)A(M)A(M)C(M)A(M)C(M)^T(**V**)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |

### [Evaluation Example 12] Inhibition of expression of mouse Ttr in mouse primary hepatocytes

The siRNA described in Tables 19 and 20 was evaluated at 2 concentrations of 0.01 or 1 nM using the same evaluation method as in Evaluation Example 9. The results are shown in Figs. 36 and 37.

As is apparent from Figs. 36 and 37, the siRNA including 2'-O-MCE nucleotides, excluding si-0300 and si-0307 including 2'-O-MCE nucleotides at position 2 as counted from the 5' end of the antisense strand, exhibited the expression suppressing effect equivalent to or superior to that of the siRNA not including 2'-O-MCE nucleotides (si-0298 or si-0306). In particular, siRNA including a 5'-vinylphosphonate-modified 2'-O-MCE nucleotide at position 1 as counted from the 5' end of the antisense strand (si-0299 and the like) or siRNA further including 2'-O-MCE nucleotides at position 5 (si-0301 and the like) exhibited the high expression suppressing effect.

### [Production Example 15] Synthesis of siRNA

According to the method described in Production Example 6, siRNAs described in Tables 21 and 22 were synthesized. The target RNA of the siRNA described in Tables 21 and 22 is Ttr (mouse).

si-0314 to si-0321 are siRNAs including 2'-O-MCE nucleotides, designed based on si-0313 including deoxyribonucleotides. si-0323 to si-0329 are siRNAs including 2'-O-MCE nucleotides, designed based on si-0322 including deoxyribonucleotides.

**[Table 21]**

| Compd. No. | Strand | SEQ No | Chemical Structure |
|---|---|---|---|
| si-0313 | AS | 288 | U(vM)^g^G(M)U(M)tU(M)aU(M)A(M)G(M)A(M)gC(M)aA(M)gA(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0314 | AS | 289 | T(v**V**)^g^G(M)U(M)tU(M)aU(M)A(M)G(M)A(M)gC(M)aA(M)gA(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0315 | AS | 290 | T(v**V**)^G(**V**)^G(M)U(M)tU(M)aU(M)A(M)G(M)A(M)gC(M)aA(M)gA(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0316 | AS | 291 | T(v**V**)^g^G(M)U(M)T(**V**)U(M)aU(M)A(M)G(M)A(M)gC(M)aA(M)gA(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0317 | AS | 292 | T(v**V**)^g^G(M)U(M)tU(M)A(**V**)U(M)A(M)G(M)A(M)gC(M)aA(M)gA(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0318 | AS | 293 | T(v**V**)^g^G(M)U(M)tU(M)aU(M)A(M)G(M)A(M)G(**V**)C(M)aA(M)gA(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0319 | AS | 294 | T(v**V**)^g^G(M)U(M)tU(M)aU(M)A(M)G(M)A(M)gC(M)A(**V**)A(M)gA(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0320 | AS | 295 | T(v**V**)^g^G(M)U(M)tU(M)aU(M)A(M)G(M)A(M)gC(M)aA(M)G(**V**)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0321 | AS | 296 | T(v**V**)^g^G(M)U(M)T(**V**)U(M)aU(M)A(M)G(M)A(M)gC(M)aA(M)gA(M)A(M)C(M)A(M)C(M)^T(**V**)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |

**[Table 22]**

| | | | |
|---|---|---|---|
| si-0322 | AS | 297 | U(M)^g^G(M)U(M)tU(M)aU(M)A(M)G(M)A(M)gC(M)aA(M)gA(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0323 | AS | 298 | U(M)^G(**V**)^G(M)U(M)tU(M)aU(M)A(M)G(M)A(M)gC(M)aA(M)gA(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0324 | AS | 299 | U(M)^g^G(M)U(M)T(**V**)U(M)aU(M)A(M)G(M)A(M)gC(M)aA(M)gA(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0325 | AS | 300 | U(M)^g^G(M)U(M)tU(M)A(**V**)U(M)A(M)G(M)A(M)gC(M)aA(M)gA(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0326 | AS | 301 | U(M)^g^G(M)U(M)tU(M)aU(M)A(M)G(M)A(M)G(**V**)C(M)aA(M)gA(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0327 | AS | 302 | U(M)^g^G(M)U(M)tU(M)aU(M)A(M)G(M)A(M)gC(M)A(**V**)A(M)gA(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0328 | AS | 303 | U(M)^g^G(M)U(M)tU(M)aU(M)A(M)G(M)A(M)gC(M)aA(M)G(**V**)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0329 | AS | 304 | U(M)^g^G(M)U(M)T(**V**)U(M)aU(M)A(M)G(M)A(M)gC(M)aA(M)gA(M)A(M)C(M)A(M)C(M)^T(**V**)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |

### [Evaluation Example 13] Inhibition of expression of mouse Ttr in mouse primary hepatocytes

The siRNAs described in Tables 21 and 22 were evaluated using the same evaluation method as in Evaluation Example 9.

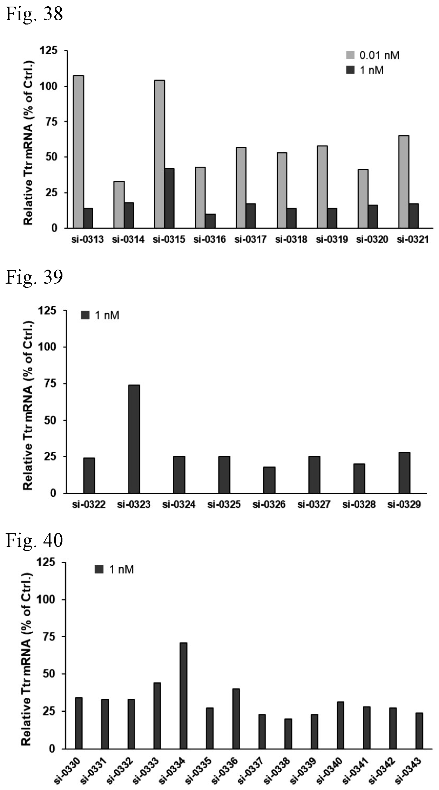

si-0314 to si-0321 were evaluated at 2 concentrations of 0.01 or 1 nM, and si-0323 to si-0329 were evaluated at 1 concentration of 1 nM. The results are shown in Figs. 38 and 39.

As is apparent from Figs. 38 and 39, the siRNA including 2'-O-MCE nucleotides (si-0314, si-0316 to si-0321, and si-0324 to si-0329) excluding si-0315 and si-0323 including 2'-O-MCE nucleotides at position 2 as counted from the 5' end of the antisense strand exhibited the expression suppressing effect equivalent to or superior to that of the siRNA not including 2'-O-MCE nucleotides (si-0313 or si-0322). In particular, siRNA including a 5'-vinylphosphonate-modified 2'-O-MCE nucleotide at position 1 as counted from the 5' end of the antisense strand (si-0314, si-0316 to si-0321, and the like) exhibited the high expression suppressing effect.

### [Production Example 16] Synthesis of siRNA

According to the method described in Production Example 6, siRNAs described in Tables 23 and 24 were synthesized. The target RNA of the siRNA described in Tables 23 and 24 is Ttr (mouse).

si-0331 to si-0336 are siRNAs including 2'-O-MCE nucleotides, designed based on si-0330 including deoxyribonucleotides. si-0338 to si-0343 are siRNAs including 2'-O-MCE nucleotides, designed based on si-0337 including deoxyribonucleotides.

**[Table 23]**

| Compd. No. | Strand | SEQ No | Chemical Structure |
|---|---|---|---|
| si-0330 | AS | 305 | U(M)^G(F)^G(M)U(M)tU(M)aU(M)A(M)G(M)A(M)G(M)C(M)A(F)A(M)G(F)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0331 | AS | 306 | T(**V**)^G(F)^G(M)U(M)tU(M)aU(M)A(M)G(M)A(M)G(M)C(M)A(F)A(M)G(F)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0332 | AS | 307 | U(M)^G(F)^G(M)U(M)T(**V**)U(M)aU(M)A(M)G(M)A(M)G(M)C(M)A(F)A(M)G(F)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0333 | AS | 308 | U(M)^G(F)^G(M)U(M)tU(M)A(**V**)U(M)A(M)G(M)A(M)G(M)C(M)A(F)A(M)G(F)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0334 | AS | 309 | U(M)^G(F)^G(M)U(M)tU(M)aU(M)A(M)G(M)A(M)G(M)C(M)A(**V**)A(M)G(F)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0335 | AS | 310 | U(M)^G(F)^G(M)U(M)tU(M)aU(M)A(M)G(M)A(M)G(M)C(M)A(F)A(M)G(**V**)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0336 | AS | 311 | U(M)^G(F)^G(M)U(M)T(**V**)U(M)aU(M)A(M)G(M)A(M)G(M)C(M)A(F)A(M)G(F)A(M)A(M)C(M)A(M)C(M)^T(**V**)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |

**[Table 24]**

| | | | |
|---|---|---|---|
| si-0337 | AS | 312 | U(vM)^G(F)^G(M)U(M)tU(M)aU(M)A(M)G(M)A(M)G(M)C(M)A(F)A(M)G(F)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0338 | AS | 313 | T(v**V**)^G(F)^G(M)U(M)tU(M)aU(M)A(M)G(M)A(M)G(M)C(M)A(F)A(M)G(F)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0339 | AS | 314 | T(v**V**)^G(F)^G(M)U(M)T(**V**)U(M)aU(M)A(M)G(M)A(M)G(M)C(M)A(F)A(M)G(F)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0340 | AS | 315 | T(v**V**)^G(F)^G(M)U(M)tU(M)A(**V**)U(M)A(M)G(M)A(M)G(M)C(M)A(F)A(M)G(F)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0341 | AS | 316 | T(v**V**)^G(F)^G(M)U(M)tU(M)aU(M)A(M)G(M)A(M)G(M)C(M)A(**V**)A(M)G(F)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0342 | AS | 317 | T(v**V**)^G(F)^G(M)U(M)tU(M)aU(M)A(M)G(M)A(M)G(M)C(M)A(F)A(M)G(**V**)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0343 | AS | 318 | T(v**V**)^G(F)^G(M)U(M)T(**V**)U(M)aU(M)A(M)G(M)A(M)G(M)C(M)A(F)A(M)G(F)A(M)A(M)C(M)A(M)C(M)^T(**V**)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |

### [Evaluation Example 14] Inhibition of expression of mouse Ttr in mouse primary hepatocytes

The siRNAs described in Tables 23 and 24 were evaluated at 1 nM using the same evaluation method as in Evaluation Example 9. The results are shown in Fig. 40.

As is apparent from Fig. 40, the siRNA including 2'-O-MCE nucleotides (si-0331 to si-0333, si-0335 to si-0336, and si-0338 to si-0343) excluding si-0334 including 2'-O-MCE nucleotides at position 14 as counted from the 5' end of the antisense strand exhibited the expression suppressing effect equivalent to or superior to that of the siRNA not including 2'-O-MCE nucleotides (si-0330 or si-0337).

### [Production Example 17] Synthesis of siRNA

According to the method described in Production Example 6, siRNAs described in Table 25 were synthesized. The target RNA of the siRNA described in Table 25 is Ttr (mouse).

si-0334 to si-0348 are siRNAs including 2'-O-MCE nucleotides, designed based on si-0298 including deoxyribonucleotides. si-0349 is an siRNA including a plurality of 2'-O-MCE nucleotides, designed based on si-0313 including deoxyribonucleotides.

**[Table 25]**

| Compd. No. | Strand | SEQ No | Chemical Structure |
|---|---|---|---|
| si-0298 | AS | 272 | U(vM)^g^G(M)U(M)tU(M)aU(M)A(M)G(M)A(M)gC(M)A(F)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0344 | AS | 319 | T(v**V**)^G(F)^G(M)U(M)T(**V**)U(F)A(M)U(M)A(M)G(M)A(M)G(M)C(M)A(F)A(M)G(F)A(M)A(M)C(M)A(M)C(M)^U(M)^G(M) |
| | S | 320 | G(**V**)^U(M)^G(M)U(M)U(M)C(M)U(F)U(M)G(F)C(F)U(F)C(M)U(M)A(M)U(M)A(M)A(M)A(M)C(M)^C(M)^A(**V**) |
| si-0345 | AS | 321 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(**V**) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0346 | AS | 321 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(**V**) |
| | S | 322 | G(**V**)^U(M)^G(M)U(M)U(M)C(M)U(M)U(M)G(F)C(F)U(F)C(M)U(M)A(M)U(M)A(M)A(M)A(M)C(M)^C(M)^A(**V**) |
| si-0347 | AS | 276 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 322 | G(**V**)^U(M)^G(M)U(M)U(M)C(M)U(M)U(M)G(F)C(F)U(F)C(M)U(M)A(M)U(M)A(M)A(M)A(M)C(M)^C(M)^A(**V**) |
| si-0348 | AS | 278 | T(v**V**)^g^G(M)U(M)tU(M)aU(M)A(M)G(M)A(M)G(**V**)C(M)A(F)A(M)G(M)A(M)A(M)C(M)A(M)C(M)^U(M)^G(M) |
| | S | 322 | G(**V**)^U(M)^G(M)U(M)U(M)C(M)U(M)U(M)G(F)C(F)U(F)C(M)U(M)A(M)U(M)A(M)A(M)A(M)C(M)^C(M)^A(**V**) |
| si-0313 | AS | 288 | U(vM)^g^G(M)U(M)tU(M)aU(M)A(M)G(M)A(M)gC(M)aA(M)gA(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0349 | AS | 291 | T(v**V**)^g^G(M)U(M)T(**V**)U(M)aU(M)A(M)G(M)A(M)gC(M)aA(M)gA(M)A(M)C(M)A(M)C(M)^U(M)^G(M) |
| | S | 322 | G(**V**)^U(M)^G(M)U(M)U(M)C(M)U(M)U(M)G(F)C(F)U(F)C(M)U(M)A(M)U(M)A(M)A(M)A(M)C(M)^C(M)^A(**V**) |

### [Evaluation Example 15] Inhibition of expression of mouse Ttr in mouse primary hepatocytes

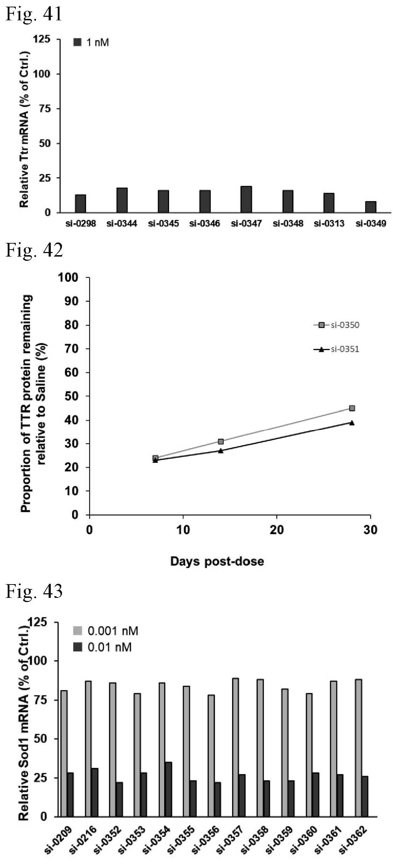

The siRNA described in Table 24 was evaluated at 1 nM using the same evaluation method as in Evaluation Example 9. Evaluation was performed. The results are shown in Fig. 41.

As is apparent from Fig. 41, the siRNAs including 2'-O-MCE nucleotides (si-0344 to si-0348 and si-0349) exhibited the expression suppressing effect equivalent to or superior to that of the siRNA not including 2'-O-MCE nucleotides (si-0298 or si-0313).

### [Production Example 18] Synthesis of siRNA

According to the method described in Production Example 6, siRNAs described in Table 26 were synthesized. The target RNA of the siRNA described in Table 25 is Ttr (mouse).

A conjugate with a GalNAc ligand was synthesized with reference to Bioconjugate Chem. 2015, 26, 8, 1451-1455.

si-0351 is an siRNA including 2'-O-MCE nucleotides and further including a GalNAc ligand, designed based on si-0350. si-0350 is an siRNA in which a GalNAc ligand is conjugated to the 5' end of the sense strand of si-0298, and si-0351 is an siRNA in which a GalNAc ligand is conjugated to the 5' end of the sense strand of si-0299.

**[Table 26]**

| Compd. No. | Strand | SEQ No | Chemical Structure |
|---|---|---|---|
| si-0350 | AS | 272 | U(vM)^g^G(M)U(M)tU(M)aU(M)A(M)G(M)A(M)gC(M)A(F)A(M)G(M)A(M)A(M)C(M)A(M)C(M)^U(M)^G(M) |
| | S | 323 | Galx3_glu_5A6 _s#G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0351 | AS | 274 | T(vV)^g^G(M)U(M)tU(M)aU(M)A(M)G(M)A(M)gC(M)A(F)A(M)G(M)A(M)A(M)C(M)A(M)C(M)^U(M)^G(M) |
| | S | 323 | Galx3_glu_5A6_s#G(M)^U(M)^G(M)U(M)U(M)C(M)U(M)U(M)G(F)C(F)U(F)C(M)U(M)A(M)U(M)A(M)A(M)A(M)C(M)^C(M)^A(M) |

### [Evaluation Example 16] Ttr knockdown action in mice

The siRNA described in Table 26 was evaluated using the same evaluation method as in Evaluation Example 11. Each siRNA agent was subcutaneously administered at 0.5 mg/kg, and blood was collected and treated to obtain serum on Days 7, 14, and 28 after administration. TTR levels were determined by ELISA and expressed as percent expression of TTR relative to a negative control (vehicle). The results are shown in Fig. 42.

As is apparent from Fig. 42, on Day 28, it was confirmed that the siRNA including 2'-O-MCE nucleotides (si-0351) exhibited a higher expression suppressing effect than that of the siRNA not including 2'-O-MCE nucleotides (si-0350), and had long-term persistence.

### [Production Example 19] Synthesis of siRNA

According to the method described in Production Example 6, siRNAs described in Table 27 were synthesized. The target RNA of the siRNA described in Table 27 is Sod1 (mouse and rat).

si-0216 and si-0352 to si-0373 are siRNAs including 2'-O-MCE nucleotides and further including 2'-O-Me nucleotides or deoxyribonucleotides at positions 9 to 11 as counted from the 5' end of the sense strand, designed based on si-0209.

**[Table 27]**

| Compd. No. | Strand | SEQ No | Chemical Structure |
|---|---|---|---|
| si-0209 | AS | 193 | U(vM)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0216 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0352 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 324 | 5(**V**)^A(M)^U(M)U(M)U(M)U(M)A(F)A(M)U(M)C(F)C(F)U(M)C(M)A(M)C(M)U(M)C(M)U(M)A(M)^A(M)^A(**V**) |
| si-0353 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 325 | 5(**V**)^A(M)^U(M)U(M)U(M)U(M)A(F)A(M)U(F)C(M)C(F)U(M)C(M)A(M)C(M)U(M)C(M)U(M)A(M)^A(M)^A(**V**) |
| si-0354 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 326 | 5(**V**)^A(M)^U(M)U(M)U(M)U(M)A(F)A(M)U(F)C(F)C(M)U(M)C(M)A(M)C(M)U(M)C(M)U(M)A(M)^A(M)^A(**V**) |
| si-0355 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | 5 | 327 | C(M)^A(M)^U(M)U(M)U(M)U(M)A(F)A(M)U(F)C(M)C(F)U(M)C(M)A(M)C(M)U(M)C(M)U(M)A(M)^A(M)^A(M) |
| si-0356 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 328 | 5(**V**)^A(M)^U(M)U(M)U(M)U(M)A(F)A(M)U(M)C(F)C(F)U(M)C(F)A(M)C(M)U(M)C(M)U(M)A(M)^A(M)^A(**V**) |
| si-0357 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 329 | 5(**V**)^A(M)^U(M)U(M)U(M)U(M)A(F)A(M)U(F)C(F)C(M)U(M)C(F)A(M)C(M)U(M)C(M)U(M)A(M)^A(M)^A(**V**) |
| si-0358 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 330 | 5(**V**)^A(M)^U(M)U(M)U(M)U(M)A(F)A(M)U(F)C(M)C(F)U(M)C(F)A(M)C(M)U(M)C(M)U(M)A(M)^A(M)^A(**V**) |
| si-0359 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 331 | C(M)^A(M)^U(M)U(M)U(M)U(M)A(F)A(M)U(F)C(M)C(F)U(M)C(F)A(M)C(M)U(M)C(M)U(M)A(M)^A(M)^A(M) |
| si-0360 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 332 | 5(**V**)^A(M)^U(M)U(M)U(M)U(M)A(M)A(M)U(F)C(M)C(F)U(M)C(F)A(M)C(M)U(M)C(M)U(M)A(M)^A(M)^A(**V**) |
| si-0361 | AS | 333 | T(v**V**)^U(F)^U(M)A(M)G(**V**)A(M)G(M)U(M)G(F)A(M)G(M)G(F)A(M)U(F)U(M)A(F)A(M)A(M)A(M)U(M)G(M)^A(M)^G(M) |
| | S | 330 | 5(**V**)^A(M)^U(M)U(M)U(M)U(M)A(F)A(M)U(F)C(M)C(F)U(M)C(F)A(M)C(M)U(M)C(M)U(M)A(M)^A(M)^A(V) |
| si-0362 | AS | 333 | T(v**V**)^U(F)^U(M)A(M)G(**V**)A(M)G(M)U(M)G(F)A(M)G(M)G(F)A(M)U(F)U(M)A(F)A(M)A(M)A(M)U(M)G(M)^A(M)^G(M) |
| | S | 332 | 5(**V**)^A(M)^U(M)U(M)U(M)U(M)A(M)A(M)U(F)C(M)C(F)U(M)C(F)A(M)C(M)U(M)C(M)U(M)A(M)^A(M)^A(**V**) |
| si-0363 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 188 | C(M)^A(M)^U(M)U(M)U(M)U(M)A(F)A(M)tC(F)C(F)U(M)C(M)A(M)C(M)U(M)C(M)U(M)A(M)^A(M)^A(M) |
| si-0364 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 189 | C(M)^A(M)^U(M)U(M)U(M)U(M)A(F)A(M)U(F)cC(F)U(M)C(M)A(M)C(M)U(M)C(M)U(M)A(M)^A(M)^A(M) |
| si-0365 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 190 | C(M)^A(M)^U(M)U(M)U(M)U(M)A(F)A(M)U(F)C(F)cU(M)C(M)A(M)C(M)U(M)C(M)U(M)A(M)^A(M)^A(M) |
| si-0366 | AS | 202 | T(v**V**)^U(F)^U(M)A(M)G(**V**)A(F)G(M)U(F)G(F)A(**V**)G(M)G(M)A(M)U(F)U(M)A(F)A(M)A(M)A(M)U(M)G(M)^A(M)^G(M) |
| | S | 189 | C(M)^A(M)^U(M)U(M)U(M)U(M)A(F)A(M)U(F)cC(F)U(M)C(M)A(M)C(M)U(M)C(M)U(M)A(M)^A(M)^A(M) |
| si-0367 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 334 | 5(**V**)^A(M)^U(M)U(M)U(M)U(M)A(F)A(M)U(F)cC(F)U(M)C(M)A(M)C(M)U(M)C(M)U(M)A(M)^A(M)^A(M) |
| si-0368 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 335 | C(M)^A(M)^U(M)U(M)U(M)U(M)A(F)A(M)U(F)cC(F)U(M)C(M)A(M)C(M)U(M)C(M)U(M)A(M)^A(M)^A(**V**) |
| si-0369 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 336 | 5(**V**)^A(M)^U(M)U(M)U(M)U(M)A(F)A(M)U(F)cC(F)U(M)C(M)A(M)C(M)U(M)C(M)U(M)A(M)^A(M)^A(**V**) |
| si-0370 | AS | 337 | T(v**V**)^U(F)^U(M)A(M)G(**V**)A(F)G(M)U(F)G(F)A(M)G(M)G(F)A(M)U(F)U(M)A(F)A(M)A(M)A(M)U(M)G(M)^A(M)^G(M) |
| | S | 189 | C(M)^A(M)^U(M)U(M)U(M)U(M)A(F)A(M)U(F)cC(F)U(M)C(M)A(M)C(M)U(M)C(M)U(M)A(M)^A(M)^A(M) |
| si-0371 | AS | 337 | T(v**V**)^U(F)^U(M)A(M)G(**V**)A(F)G(M)U(F)G(F)A(M)G(M)G(F)A(M)U(F)U(M)A(F)A(M)A(M)A(M)U(M)G(M)^A(M)^G(M) |
| | S | 338 | C(M)^A(M)^U(M)U(M)U(M)U(M)A(M)A(M)U(F)cC(F)U(M)C(F)A(M)C(M)U(M)C(M)U(M)A(M)^A(M)^A(M) |
| si-0372 | AS | 333 | T(v**V**)^U(F)^U(M)A(M)G(**V**)A(M)G(M)U(M)G(F)A(M)G(M)G(F)A(M)U(F)U(M)A(F)A(M)A(M)A(M)U(M)G(M)^A(M)^G(M) |
| | S | 338 | C(M)^A(M)^U(M)U(M)U(M)U(M)A(M)A(M)U(F)cC(F)U(M)C(F)A(M)C(M)U(M)C(M)U(M)A(M)^A(M)^A(M) |
| si-0373 | AS | 337 | T(v**V**)^U(F)^U(M)A(M)G(**V**)A(F)G(M)U(F)G(F)A(M)G(M)G(F)A(M)U(F)U(M)A(F)A(M)A(M)A(M)U(M)G(M)^A(M)^G(M) |
| | 5 | 339 | 5(**V**)^A(M)^U(M)U(M)U(M)U(M)A(M)A(M)U(F)cC(F)U(M)C(F)A(M)C(M)U(M)C(M)U(M)A(M)^A(M)^A(**V**) |

### [Evaluation Example 17] Inhibition of expression of rat Sod1 in RPE-J cells (rat retinal pigment epithelial (RPE) cells)

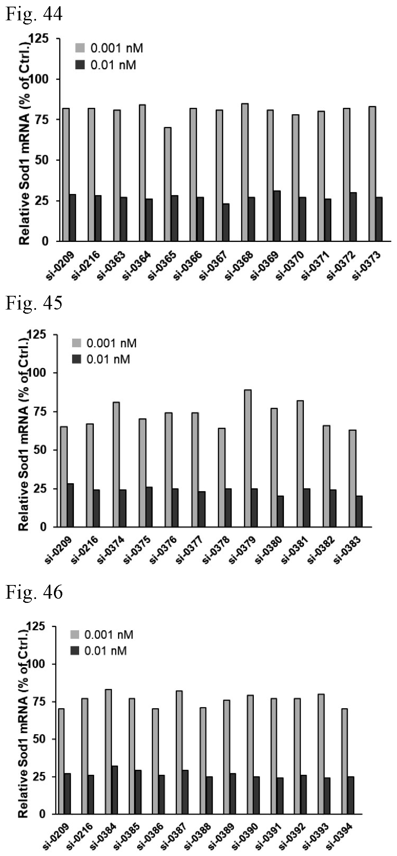

The siRNA described in Table 26 was evaluated at 2 concentrations of 0.001 or 0.01 nM using the same evaluation method as in Evaluation Example 1. The results are shown in Figs. 43 and 44.

As is apparent from Figs. 43 and 44, the siRNA including 2'-O-MCE nucleotides (si-0216, and si-0352 to si-0373) exhibited the expression suppressing effect equivalent to or superior to that of the siRNA not including 2'-O-MCE nucleotides (si-0209). In addition, it was confirmed that the expression suppressing effect was maintained even when the siRNA including 2'-O-MCE nucleotides included 2'-O-Me nucleotides or deoxyribonucleotides at positions 9 to 11 as counted from the 5' end of the sense strand.

### [Production Example 20] Synthesis of siRNA

According to the method described in Production Example 6, siRNAs described in Table 28 were synthesized. The target RNA of the siRNA described in Table 28 is Sod1 (mouse and rat).

si-0216 and si-0374 to si-0377 are siRNAs including 2'-O-MCE nucleotides, designed based on si-0209. si-0379 and si-0380 are siRNAs including 2'-O-MCE nucleotides, designed based on si-0378 in which the sense strand has a length of 20 nucleotides. si-0382 and si-0383 are siRNAs including 2'-O-MCE nucleotides, designed based on si-0381 in which the sense strand has a length of 19 nucleotides. si-0385 and si-0386 are siRNAs including 2'-O-MCE nucleotides, designed based on si-0384 in which the sense strand has a length of 19 nucleotides and the antisense strand has a length of 21 nucleotides. si-0388 and si-0389 are siRNAs including 2'-O-MCE nucleotides, designed based on si-0387 in which the sense strand has a length of 20 nucleotides and the antisense strand has a length of 22 nucleotides. si-0390 to si-0394 are siRNAs including 2'-O-MCE nucleotides, in which the sense strand has a length of 21 nucleotides and the antisense strand has a length of 21 nucleotides.

**[Table 28]**

| Compd. No. | Strand | SEQ No | Chemical Structure |
|---|---|---|---|
| si-0209 | AS | 193 | U(vM)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0216 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | 5 | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0374 | AS | 340 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(**V**) |
| | S | 37 | C(M)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0375 | AS | 340 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(**V**) |
| | S | 4 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0376 | AS | 340 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(**V**) |
| | S | 179 | C(M)^A(**V**)^U(M)U(M)U(M)U(M)A(F)A(M)U(F)C(F)C(F)U(M)C(M)A(M)C(M)U(M)C(M)U(M)A(M)^A(M)^A(**V**) |
| si-0377 | AS | 340 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(**V**) |
| | 5 | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0378 | AS | 193 | U(vM)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) |
| | S | 341 | A(M)^U(M)^U(M)U(M)U(M)A(F)A(M)U(F)C(F)C(F)U(M)C(M)A(M)C(M)U(M)C(M)U(M)A(M)^A(M)^A(M) |
| si-0379 | AS | 340 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(**V**) |
| | S | 342 | A(**V**)^U(M)^U(M)U(M)U(M)A(F)A(M)U(F)C(F)C(F)U(M)C(M)A(M)C(M)U(M)C(M)U(M)A(M)^A(M)^A(**V**) |
| si-0380 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 342 | A(**V**)^U(M)^U(M)U(M)U(M)A(F)A(M)U(F)C(F)C(F)U(M)C(M)A(M)C(M)U(M)C(M)U(M)A(M)^A(M)^A(**V**) |
| si-0381 | AS | 193 | U(vM)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) |
| | 5 | 343 | U(M)^U(M)^U(M)U(M)A(F)A(M)U(F)C(F)C(F)U(M)C(M)A(M)C(M)U(M)C(M)U(M)A(M)^A(M)^A(M) |
| si-0382 | AS | 340 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(**V**) |
| | S | 344 | T(**V**)^U(M)^U(M)U(M)A(F)A(M)U(F)C(F)C(F)U(M)C(M)A(M)C(M)U(M)C(M)U(M)A(M)^A(M)^A(**V**) |
| si-0383 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 344 | T(**V**)^U(M)^U(M)U(M)A(F)A(M)U(F)C(F)C(F)U(M)C(M)A(M)C(M)U(M)C(M)U(M)A(M)^A(M)^A(**V**) |
| si-0384 | AS | 345 | U(vM)^U(F)^U(M)A(M)G(M)A(F)G(M)U(F)G(F)A(M)G(M)G(M)A(M)U(F)U(M)A(F)A(M)A(M)A(M)^U(M)^G(M) |
| | S | 343 | U(M)^U(M)^U(M)U(M)A(F)A(M)U(F)C(F)C(F)U(M)C(M)A(M)C(M)U(M)C(M)U(M)A(M)^A(M)^A(M) |
| si-0385 | AS | 346 | T(v**V**)^U(F)^U(M)A(M)G(V)A(F)G(M)U(F)G(F)A(M)G(M)G(M)A(M)U(F)U(M)A(F)A(M)A(M)A(M)^U(M)^G(**V**) |
| | S | 344 | T(**V**)^U(M)^U(M)U(M)A(F)A(M)U(F)C(F)C(F)U(M)C(M)A(M)C(M)U(M)C(M)U(M)A(M)^A(M)^A(**V**) |
| si-0386 | AS | 347 | T(v**V**)^U(F)^U(M)A(M)G(**V**)A(F)G(M)U(F)G(F)A(M)G(M)G(M)A(M)U(F)U(M)A(F)A(M)A(M)A(M)^U(M)^G(M) |
| | S | 344 | T(**V**)^U(M)^U(M)U(M)A(F)A(M)U(F)C(F)C(F)U(M)C(M)A(M)C(M)U(M)C(M)U(M)A(M)^A(M)^A(**V**) |
| si-0387 | AS | 348 | U(vM)^U(F)^U(M)A(M)G(M)A(F)G(M)U(F)G(F)A(M)G(M)G(M)A(M)U(F)U(M)A(F)A(M)A(M)A(M)U(M)^G(M)^A(M) |
| | S | 341 | A(M)^U(M)^U(M)U(M)U(M)A(F)A(M)U(F)C(F)C(F)U(M)C(M)A(M)C(M)U(M)C(M)U(M)A(M)^A(M)^A(M) |
| si-0388 | AS | 349 | T(v**V**)^U(F)^U(M)A(M)G(**V**)A(F)G(M)U(F)G(F)A(M)G(M)G(M)A(M)U(F)U(M)A(F)A(M)A(M)A(M)U(M)^G(M)^A(**V**) |
| | S | 342 | A(**V**)^U(M)^U(M)U(M)U(M)A(F)A(M)U(F)C(F)C(F)U(M)C(M)A(M)C(M)U(M)C(M)U(M)A(M)^A(M)^A(**V**) |
| si-0389 | AS | 350 | T(v**V**)^U(F)^U(M)A(M)G(**V**)A(F)G(M)U(F)G(F)A(M)G(M)G(M)A(M)U(F)U(M)A(F)A(M)A(M)A(M)U(M)^G(M)^A(M) |
| | S | 342 | A(**V**)^U(M)^U(M)U(M)U(M)A(F)A(M)U(F)C(F)C(F)U(M)C(M)A(M)C(M)U(M)C(M)U(M)A(M)^A(M)^A(**V**) |
| si-0390 | AS | 351 | T(v**V**)^U(F)^T(**V**)A(M)G(M)A(F)G(M)U(F)G(F)A(M)G(M)G(M)A(M)U(F)U(M)A(F)A(M)A(M)A(M)^U(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0391 | AS | 352 | T(v**V**)^U(F)^U(M)A(**V**)G(M)A(F)G(M)U(F)G(F)A(M)G(M)G(M)A(M)U(F)U(M)A(F)A(M)A(M)A(M)^U(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0392 | AS | 353 | T(v**V**)^U(F)^U(M)A(M)G(M)A(**V**)G(M)U(F)G(F)A(M)G(M)G(M)A(M)U(F)U(M)A(F)A(M)A(M)A(M)^U(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0393 | AS | 354 | T(v**V**)^U(F)^U(M)A(M)G(M)A(F)G(**V**)U(F)G(F)A(M)G(M)G(M)A(M)U(F)U(M)A(F)A(M)A(M)A(M)^U(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0394 | AS | 355 | T(v**V**)^U(F)^U(M)A(M)G(M)A(F)G(M)U(F)G(F)A(**V**)G(M)G(M)A(M)U(F)U(M)A(F)A(M)A(M)A(M)^U(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |

### [Evaluation Example 18] Inhibition of expression of rat Sod1 in RPE-J cells (rat retinal pigment epithelial (RPE) cells)

The siRNA described in Table 28 was evaluated using the same evaluation method as in Evaluation Example 1. The results are shown in Figs. 45 and 46.

As is apparent from Figs. 45 and 46, the siRNA including 2'-O-MCE nucleotides (si-0216, si-0374 to si-0377, si-0379 to si-0380, si-0382 to si-0383, si-0385 to si-0386, si-0388 to si-0389, and si-0390 to si-0394) exhibited the expression suppressing effect equivalent to or superior to that of the siRNA not including 2'-O-MCE nucleotides (si-0209, si-0378, si-0381, si-0384, or si-0387). In addition, it was confirmed that the expression suppressing effect of siRNA including 2'-O-MCE nucleotides was maintained even when the nucleotide length of each strand was shortened.

### [Production Example 21] Synthesis of siRNA

According to the method described in Production Example 6, siRNAs described in Table 29 were synthesized. The target RNA of the siRNA described in Table 29 is Ttr (mouse).

si-0253, si-0395 to si-0410, and si-0413 to si-0416 are siRNAs including 2'-O-MCE nucleotides, designed based on si-0251. si-0412 is an siRNA including 2'-O-MCE nucleotides, designed based on si-0411 in which the sense strand has a length of 19 nucleotides. Among these, si-0395 to si-0410 are siRNAs including 2'-O-Me nucleotides or deoxyribonucleotides at positions 9 to 11 as counted from the 5' end of the sense strand.

**[Table 29]**

| Compd. No. | Strand | SEQ No | Chemical Structure |
|---|---|---|---|
| si-0251 | AS | 231 | U(vM)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 211 | A(M)^G(M)^U(M)G(M)U(M)U(M)C(F)U(M)U(F)G(F)C(F)U(M)C(M)U(M)A(M)U(M)A(M)A(M)A(M)^C(M)^A(M) |
| si-0253 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0395 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 221 | A(**V**)^G(**V**)^U(M)G(M)U(M)U(M)C(F)U(M)U(F)G(F)C(F)U(M)C(M)U(M)A(M)U(M)A(M)A(M)A(M)^5(**V**)^A(**V**) |
| si-0396 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 356 | A(**V**)^G(M)^U(M)G(M)U(M)U(M)C(F)U(M)U(M)G(F)C(F)U(M)C(M)U(M)A(M)U(M)A(M)A(M)A(M)^C(M)^A(**V**) |
| si-0397 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | 5 | 357 | A(**V**)^G(M)^U(M)G(M)U(M)U(M)C(F)U(M)U(F)G(M)C(F)U(M)C(M)U(M)A(M)U(M)A(M)A(M)A(M)^C(M)^A(**V**) |
| si-0398 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 358 | A(**V**)^G(M)^U(M)G(M)U(M)U(M)C(F)U(M)U(F)G(F)C(M)U(M)C(M)U(M)A(M)U(M)A(M)A(M)A(M)^C(M)^A(**V**) |
| si-0399 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 359 | A(**V**)^G(**V**)^U(M)G(M)U(M)U(M)C(F)U(M)U(F)G(M)C(F)U(M)C(M)U(M)A(M)U(M)A(M)A(M)A(M)^5(**V**)^A(**V**) |
| si-0400 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | 5 | 360 | A(**V**)^G(M)^U(M)G(M)U(M)U(M)C(F)U(M)U(M)G(F)C(F)U(M)C(F)U(M)A(M)U(M)A(M)A(M)A(M)^C(M)^A(**V**) |
| si-0401 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 361 | A(**V**)^G(M)^U(M)G(M)U(M)U(M)C(F)U(M)U(F)G(M)C(F)U(M)C(F)U(M)A(M)U(M)A(M)A(M)A(M)^C(M)^A(**V**) |
| si-0402 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 362 | A(**V**)^G(M)^U(M)G(M)U(M)U(M)C(F)U(M)U(F)G(F)C(M)U(M)C(F)U(M)A(M)U(M)A(M)A(M)A(M)^C(M)^A(**V**) |
| si-0403 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 363 | A(**V**)^G(M)^U(M)G(M)U(M)U(M)C(M)U(M)U(F)G(M)C(F)U(M)C(F)U(M)A(M)U(M)A(M)A(M)A(M)^C(M)^A(**V**) |
| si-0404 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 364 | A(**V**)^G(M)^U(M)G(M)U(M)U(M)C(F)U(M)tG(F)C(F)U(M)C(M)U(M)A(M)U(M)A(M)A(M)A(M)^C(M)^A(**V**) |
| si-0405 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 365 | A(**V**)^G(M)^U(M)G(M)U(M)U(M)C(F)U(M)U(F)gC(F)U(M)C(M)U(M)A(M)U(M)A(M)A(M)A(M)^C(M)^A(**V**) |
| si-0406 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 366 | A(**V**)^G(M)^U(M)G(M)U(M)U(M)C(F)U(M)U(F)G(F)cU(M)C(M)U(M)A(M)U(M)A(M)A(M)A(M)^C(M)^A(**V**) |
| si-0401 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 367 | A(**V**)^G(**V**)^U(M)G(M)U(M)U(M)C(F)U(M)U(F)gC(F)U(M)C(M)U(M)A(M)U(M)A(M)A(M)A(M)^5(**V**)^A(**V**) |
| si-0408 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 368 | A(**V**)^G(M)^U(M)G(M)U(M)U(M)C(M)U(M)tG(F)C(F)U(M)C(F)U(M)A(M)U(M)A(M)A(M)A(M)^C(M)^A(**V**) |
| si-0409 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 369 | A(**V**)^G(M)^U(M)G(M)U(M)U(M)C(M)U(M)U(F)gC(F)U(M)C(F)U(M)A(M)U(M)A(M)A(M)A(M)^C(M)^A(**V**) |
| si-0410 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 370 | A(**V**)^G(M)^U(M)G(M)U(M)U(M)C(M)U(M)U(F)G(F)cU(M)C(F)U(M)A(M)U(M)A(M)A(M)A(M)^C(M)^A(**V**) |
| si-0411 | AS | 231 | U(vM)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 371 | U(M)^G(M)^U(M)U(M)C(F)U(M)U(F)G(F)C(F)U(M)C(M)U(M)A(M)U(M)A(M)A(M)A(M)^C(M)^A(M) |
| si-0412 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 372 | T(**V**)^G(M)^U(M)U(M)C(F)U(M)U(F)G(F)C(F)U(M)C(M)U(M)A(M)U(M)A(M)A(M)A(M)^C(M)^A(**V**) |
| si-0413 | AS | 373 | T(v**V**)^G(F)^T(**V**)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0414 | AS | 374 | T(v**V**)^G(F)^U(M)T(**V**)U(M)A(M)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0415 | AS | 375 | T(v**V**)^G(F)^U(M)U(M)U(M)A(**V**)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0416 | AS | 376 | T(v**V**)^G(F)^U(M)U(M)U(M)A(M)T(**V**)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |

### [Evaluation Example 19] Inhibition of expression of mouse Ttr in mouse primary hepatocytes

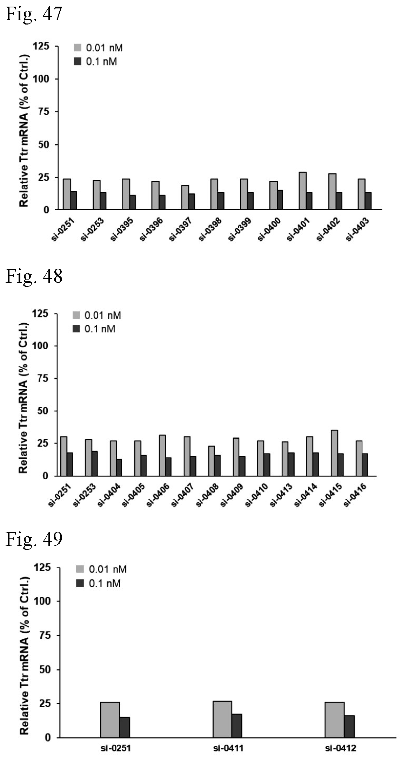

The siRNA described in Table 29 was evaluated at 2 concentrations of 0.01 or 0.1 nM using the same evaluation method as in Evaluation Example 9. The results are shown in Figs. 47 to 49.

As is apparent from Figs. 47 to 49, the siRNA including 2'-O-MCE nucleotides (si-0253, si-0395 to si-0410, and si-0412 to si-0416) exhibited the expression suppressing effect equivalent to or superior to that of the siRNA not including 2'-O-MCE nucleotides (si-0251 or si-0411). In addition, it was confirmed that the expression suppressing effect was maintained even when the siRNA including 2'-O-MCE nucleotides included 2'-O-Me nucleotides or deoxyribonucleotides at positions 9 to 11 as counted from the 5' end of the sense strand. Furthermore, it was confirmed that the expression suppressing effect of siRNA including 2'-O-MCE nucleotides was maintained even when the nucleotide length of the sense strand was shortened.

### [Production Example 22] Synthesis of siRNA

According to the method described in Production Example 6, siRNAs described in Table 30 were synthesized. The target RNA of the siRNA described in Table 30 is FXII (mouse).

si-0284 and si-0417 to si-0422 are siRNAs including 2'-O-MCE nucleotides, designed based on si-0282. Among these, si-0417 to si-0422 are siRNAs including 2'-O-Me nucleotides or deoxyribonucleotides at positions 9 to 11 as counted from the 5' end of the sense strand. si-0424 and si-0425 are siRNAs including 2'-O-MCE nucleotides, designed based on si-0423 in which the sense strand has a length of 19 nucleotides.

**[Table 30]**

| Compd. No. | Strand | SEQ No | Chemical Structure |
|---|---|---|---|
| si-0282 | AS | 261 | U(vM)^A(F)^A(M)A(M)G(M)C(F)A(M)C(M)U(M)U(M)U(M)A(M)U(M)U(F)G(M)A(F)G(M)U(M)U(M)U(M)C(M)^U(M)^G(M) |
| | S | 241 | G(M)^A(M)^A(M)A(M)C(M)U(M)C(F)A(M)A(F)U(F)A(F)A(M)A(M)G(M)U(M)G(M)C(M)U(M)U(M)^U(M)^A(M) |
| si-0284 | AS | 263 | T(v**V**)^A(F)^A(M)A(M)G(**V**)C(F)A(M)C(M)U(M)U(M)U(M)A(M)U(M)U(F)G(M)A(F)G(M)U(M)U(M)U(M)C(M)^U(M)^G(M) |
| | S | 243 | G(**V**)^A(M)^A(M)A(M)C(M)U(M)C(F)A(M)A(F)U(F)A(F)A(M)A(M)G(M)U(M)G(M)C(M)U(M)U(M)^U(M)^A(**V**) |
| si-0417 | AS | 263 | T(v**V**)^A(F)^A(M)A(M)G(**V**)C(F)A(M)C(M)U(M)U(M)U(M)A(M)U(M)U(F)G(M)A(F)G(M)U(M)U(M)U(M)C(M)^U(M)^G(M) |
| | 5 | 377 | G(**V**)^A(M)^A(M)A(M)C(M)U(M)C(F)A(M)A(M)U(F)A(F)A(M)A(M)G(M)U(M)G(M)C(M)U(M)U(M)^U(M)^A(**V**) |
| si-0418 | AS | 263 | T(v**V**)^A(F)^A(M)A(M)G(**V**)C(F)A(M)C(M)U(M)U(M)U(M)A(M)U(M)U(F)G(M)A(F)G(M)U(M)U(M)U(M)C(M)^U(M)^G(M) |
| | S | 378 | G(**V**)^A(M)^A(M)A(M)C(M)U(M)C(F)A(M)A(F)U(M)A(F)A(M)A(M)G(M)U(M)G(M)C(M)U(M)U(M)^U(M)^A(**V**) |
| si-0419 | AS | 263 | T(v**V**)^A(F)^A(M)A(M)G(**V**)C(F)A(M)C(M)U(M)U(M)U(M)A(M)U(M)U(F)G(M)A(F)G(M)U(M)U(M)U(M)C(M)^U(M)^G(M) |
| | S | 379 | G(**V**)^A(M)^A(M)A(M)C(M)U(M)C(F)A(M)A(F)U(F)A(M)A(M)A(M)G(M)U(M)G(M)C(M)U(M)U(M)^U(M)^A(**V**) |
| si-0420 | AS | 263 | T(v**V**)^A(F)^A(M)A(M)G(**V**)C(F)A(M)C(M)U(M)U(M)U(M)A(M)U(M)U(F)G(M)A(F)G(M)U(M)U(M)U(M)C(M)^U(M)^G(M) |
| | S | 380 | G(**V**)^A(M)^A(M)A(M)C(M)U(M)C(F)A(M)aU(F)A(F)A(M)A(M)G(M)U(M)G(M)C(M)U(M)U(M)^U(M)^A(**V**) |
| si-0421 | AS | 263 | T(v**V**)^A(F)^A(M)A(M)G(**V**)C(F)A(M)C(M)U(M)U(M)U(M)A(M)U(M)U(F)G(M)A(F)G(M)U(M)U(M)U(M)C(M)^U(M)^G(M) |
| | S | 381 | G(**V**)^A(M)^A(M)A(M)C(M)U(M)C(F)A(M)A(F)tA(F)A(M)A(M)G(M)U(M)G(M)C(M)U(M)U(M)^U(M)^A(**V**) |
| si-0422 | AS | 263 | T(v**V**)^A(F)^A(M)A(M)G(**V**)C(F)A(M)C(M)U(M)U(M)U(M)A(M)U(M)U(F)G(M)A(F)G(M)U(M)U(M)U(M)C(M)^U(M)^G(M) |
| | S | 382 | G(**V**)^A(M)^A(M)A(M)C(M)U(M)C(F)A(M)A(F)U(F)aA(M)A(M)G(M)U(M)G(M)C(M)U(M)U(M)^U(M)^A(**V**) |
| si-0423 | AS | 261 | U(vM)^A(F)^A(M)A(M)G(M)C(F)A(M)C(M)U(M)U(M)U(M)A(M)U(M)U(F)G(M)A(F)G(M)U(M)U(M)U(M)C(M)^U(M)^G(M) |
| | S | 383 | A(M)^A(M)^C(M)U(M)C(F)A(M)A(F)U(F)A(F)A(M)A(M)G(M)U(M)G(M)C(M)U(M)U(M)^U(M)^A(M) |
| si-0424 | AS | 263 | T(v**V**)^A(F)^A(M)A(M)G(**V**)C(F)A(M)C(M)U(M)U(M)U(M)A(M)U(M)U(F)G(M)A(F)G(M)U(M)U(M)U(M)C(M)^U(M)^G(M) |
| | S | 384 | A(**V**)^A(M)^C(M)U(M)C(F)A(M)A(F)U(F)A(F)A(M)A(M)G(M)U(M)G(M)C(M)U(M)U(M)^U(M)^A(**V**) |
| si-0425 | AS | 385 | T(v**V**)^A(F)^A(M)A(M)G(**V**)C(F)A(M)C(M)U(M)U(M)U(M)A(M)U(M)U(F)G(M)A(F)G(M)U(M)U(M)U(M)C(M)^U(M)^G(**V**) |
| | S | 384 | A(**V**)^A(M)^C(M)U(M)C(F)A(M)A(F)U(F)A(F)A(M)A(M)G(M)U(M)G(M)C(M)U(M)U(M)^U(M)^A(**V**) |

### [Evaluation Example 20] Inhibition of expression of mouse FXII in mouse primary hepatocytes

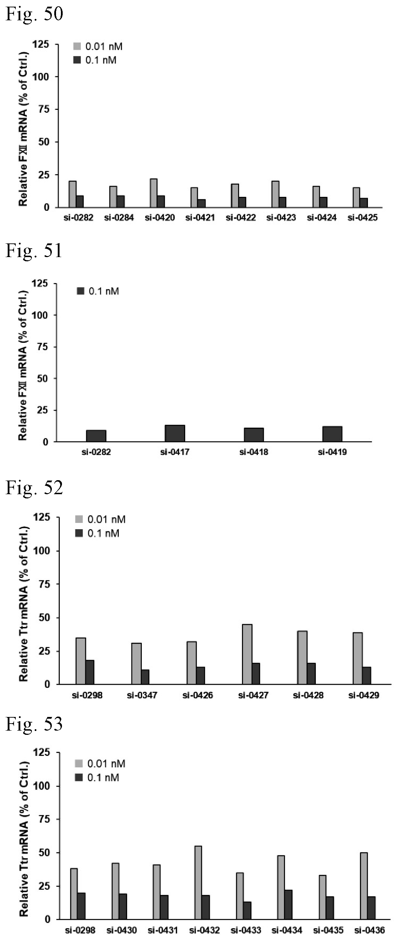

The siRNA described in Table 29 was evaluated using the same evaluation method as in Evaluation Example 9. si-0417 to si-0419 were evaluated at 1 concentration of 0.1 nM, and si-0284 and si-0420 to si-0425 were evaluated at 2 concentrations of 0.01 or 0.1 nM. The results are shown in Figs. 50 and 51.

As is apparent from Figs. 50 and 51, the siRNA including 2'-O-MCE nucleotides (si-0284, si-0417 to si-0422, and si-0424 to si-0425) exhibited the expression suppressing effect equivalent to or superior to that of the siRNA not including 2'-O-MCE nucleotides (si-0251 or si-0423). In addition, it was confirmed that the expression suppressing effect was maintained even when the siRNA including 2'-O-MCE nucleotides included 2'-O-Me nucleotides or deoxyribonucleotides at positions 9 to 11 as counted from the 5' end of the sense strand. Furthermore, it was confirmed that the expression suppressing effect of siRNA including 2'-O-MCE nucleotides was maintained even when the nucleotide length of the sense strand was shortened.

### [Production Example 23] Synthesis of siRNA

According to the method described in Production Example 6, siRNAs described in Table 31 were synthesized. The target RNA of the siRNA described in Table 31 is Ttr (mouse).

si-0347 and si-0426 to si-0436 are siRNAs including 2'-O-MCE nucleotides, designed based on si-0298 including deoxyribonucleotides. Among these, si-0427 to si-0436 are siRNAs including 2'-O-Me nucleotides or deoxyribonucleotides at positions 9 to 11 as counted from the 5' end of the sense strand.

**[Table 31]**

| Compd. No. | Strand | SEQ No | Chemical Structure |
|---|---|---|---|
| si-0298 | AS | 272 | U(vM)^g^G(M)U(M)tU(M)aU(M)A(M)G(M)A(M)gC(M)A(F)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0347 | AS | 276 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 322 | G(**V**)^U(M)^G(M)U(M)U(M)C(M)U(M)U(M)G(F)C(F)U(F)C(M)U(M)A(M)U(M)A(M)A(M)A(M)C(M)^C(M)^A(**V**) |
| si-0426 | AS | 276 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 386 | G(**V**)^T(**V**)^G(M)U(M)U(M)C(M)U(M)U(M)G(F)C(F)U(F)C(M)U(M)A(M)U(M)A(M)A(M)A(M)C(M)^5(**V**)^A(**V**) |
| si-0427 | AS | 276 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 387 | G(**V**)^U(M)^G(M)U(M)U(M)C(M)U(M)U(M)G(F)C(M)U(F)C(M)U(M)A(M)U(M)A(M)A(M)A(M)C(M)^C(M)^A(**V**) |
| si-0428 | AS | 276 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 388 | G(**V**)^T(**V**)^G(M)U(M)U(M)C(M)U(M)U(M)G(F)C(M)U(F)C(M)U(M)A(M)U(M)A(M)A(M)A(M)C(M)^5(**V**)^A(**V**) |
| si-0429 | AS | 276 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 389 | G(**V**)^U(M)^G(M)U(M)U(M)C(M)U(M)U(M)G(F)C(M)U(F)C(M)U(F)A(M)U(M)A(M)A(M)A(M)C(M)^C(M)^A(**V**) |
| si-0430 | AS | 276 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 390 | G(**V**)^U(M)^G(M)U(M)U(M)C(M)U(M)U(M)gC(F)U(**F**)C(M)U(M)A(M)U(M)A(M)A(M)A(M)C(M)^C(M)^A(**V**) |
| si-0431 | AS | 276 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 391 | G(**V**)^U(M)^G(M)U(M)U(M)C(M)U(M)U(M)G(F)cU(F)C(M)U(M)A(M)U(M)A(M)A(M)A(M)C(M)^C(M)^A(**V**) |
| si-0432 | AS | 276 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 392 | G(**V**)^U(M)^G(M)U(M)U(M)C(M)U(M)U(M)G(F)C(F)tC(M)U(M)A(M)U(M)A(M)A(M)A(M)C(M)^C(M)^A(**V**) |
| si-0433 | AS | 276 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 393 | G(**V**)^T(**V**)^G(M)U(M)U(M)C(M)U(M)U(M)G(F)cU(F)C(M)U(M)A(M)U(M)A(M)A(M)A(M)C(M)^5(**V**)^A(**V**) |
| si-0434 | AS | 276 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 394 | G(**V**)^U(M)^G(M)U(M)U(M)C(M)U(M)U(M)gC(F)U(F)C(M)U(F)A(M)U(M)A(M)A(M)A(M)C(M)^C(M)^A(**V**) |
| si-0435 | AS | 276 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 395 | G(**V**)^U(M)^G(M)U(M)U(M)C(M)U(M)U(M)G(F)cU(F)C(M)U(F)A(M)U(M)A(M)A(M)A(M)C(M)^C(M)^A(**V**) |
| si-0436 | AS | 276 | T(v**V**)^g^G(M)U(M)T(**V**)U(M)aU(M)A(M)G(M)A(M)gC(M)A(F)A(M)G(M)A(M)A(M)C(M)A(M)C(M)^U(M)^G(M) |
| | S | 396 | G(**V**)^U(M)^G(M)U(M)U(M)C(M)U(M)U(M)G(F)C(F)tC(M)U(F)A(M)U(M)A(M)A(M)A(M)C(M)^C(M)^A(**V**) |

### [Evaluation Example 21] Inhibition of expression of mouse Ttr in mouse primary hepatocytes

The siRNA described in Table 31 was evaluated at 2 concentrations of 0.01 or 0.1 nM using the same evaluation method as in Evaluation Example 9. The results are shown in Figs. 52 and 53.

As is apparent from Figs. 52 and 53, the siRNA including 2'-O-MCE nucleotides (si-0347, and si-0426 to si-0436) exhibited the expression suppressing effect equivalent to or superior to that of the siRNA not including 2'-O-MCE nucleotides (si-0298). In addition, it was confirmed that the expression suppressing effect was maintained even when the siRNA including 2'-O-MCE nucleotides included 2'-O-Me nucleotides or deoxyribonucleotides at positions 9 to 11 as counted from the 5' end of the sense strand.

### [Production Example 24] Synthesis of siRNA

According to the method described in Production Example 6, siRNAs described in Table 32 were synthesized. The target RNA of the siRNA described in Table 32 is Ttr (mouse).

si-0437 to si-0448 are siRNAs including 2'-O-MCE or 2'-O-MorECE nucleotides, designed based on si-0251, and further including 5'-vinylphosphonate-modified 2'-O-MorECE nucleotides, 5'-ethylphosphonate-modified 2'-O-MCE nucleotides or 5'-ethylphosphonate-modified 2'-O-MorECE nucleotides.

**[Table 32]**

| Compd. No. | Strand | SEQ No | Chemical Structure |
|---|---|---|---|
| si-0251 | AS | 231 | U(vM)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 211 | A(M)^G(M)^U(M)G(M)U(M)U(M)C(F)U(M)U(F)G(F)C(F)U(M)C(M)U(M)A(M)U(M)A(M)A(M)A(M)^C(M)^A(M) |
| si-0437 | AS | 397 | T(v**Mo**)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0438 | AS | 398 | T(v**Mo**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0439 | AS | 399 | T(v**Mo**)^G(F)^U(M)U(M)T(**V**)A(M)U(M)A(M)G(M)A(V)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0440 | AS | 400 | T(v**Mo**)^G(F)^U(M)U(M)T(**Mo**)A(M)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0441 | AS | 401 | T(v**Mo**)^G(F)^U(M)U(M)T(**Mo**)A(M)U(M)A(M)G(M)A(**Mo**)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0442 | AS | 400 | T(v**Mo**)^G(F)^U(M)U(M)T(**Mo**)A(M)U(M)A(M)G(M)A(M)G(M)C(M)A(M)A(F)G(M)A(F)A(M)C(M)A(M)C(M)U(M)^G(M)^U(M) |
| | S | 402 | A(**Mo**)^G(M)^U(M)G(M)U(M)U(M)C(F)U(M)U(F)G(F)C(F)U(M)C(M)U(M)A(M)U(M)A(M)A(M)A(M)^C(M)^A(**Mo**) |
| si-0443 | AS | 403 | T(e**V**)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0444 | AS | 404 | T(e**V**)^G(F)^U(M)U(M)T(**V**)A(M)U(M)A(M)G(M)A(M)G(M)C(M)A(M)A(F)G(M)A(F)A(M)C(M'A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0445 | AS | 405 | T(e**V**)^G(F)^U(M)U(M)T(**V**)A(M)U(M)A(M)G(M)A(V)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0446 | AS | 406 | T(e**Mo**)^G(F)AU(M)U(M)U(M)A(M)U(M)A(M)G(M)A(M)G(M)C(M)A(M)A(F)G(M)A(F)A(M)C(M)A(M)C(M)U(M)AG(M)AU(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0447 | AS | 407 | T(e**Mo**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0448 | AS | 408 | T(e**Mo**)^G(F)^U(M)U(M)T(V)A(M)U(M)A(M)G(M)A(**V**)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |

### [Evaluation Example 22] Inhibition of expression of mouse Ttr in mouse primary hepatocytes

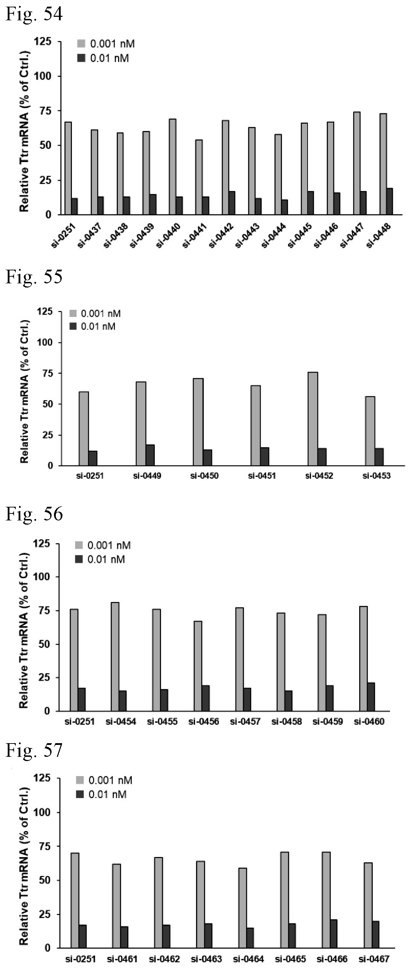

The siRNA described in Table 32 was evaluated at 2 concentrations of 0.001 or 0.01 nM using the same evaluation method as in Evaluation Example 9. Evaluation was performed. The results are shown in Fig. 54.

As apparent from Fig. 54, siRNAs including 2'-O-MCE nucleotides or 2'-O-MorECE nucleotides, and further including 5'-vinylphosphonate-modified 2'-O-MorECE nucleotides, 5'-ethylphosphonate-modified 2'-O-MCE nucleotides or 5'-ethylphosphonate-modified 2'-O-MorECE nucleotides (si-0437 to si-0448) exhibited the expression suppressing effect equivalent to or superior to that of the siRNA not including these nucleotides (si-0251).

### [Production Example 25] Synthesis of siRNA

According to the method described in Production Example 6, siRNAs described in Table 33 were synthesized. The target RNA of the siRNA described in Table 33 is Ttr (mouse).

si-0449 to si-0453 are siRNAs including 2'-O-MCE or 2'-O-MorECE nucleotides and further including 5'-vinylphosphonate-modified 2'-O-MCE nucleotides, designed based on si-0251.

**[Table 33]**

| Compd. No. | Strand | SEQ No | Chemical Structure |
|---|---|---|---|
| si-0251 | AS | 231 | U(vM)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 211 | A(M)^G(M)^U(M)G(M)U(M)U(M)C(F)U(M)U(F)G(F)C(F)U(M)C(M)U(M)A(M)U(M)A(M)A(M)A(M)^C(M)^A(M) |
| si-0449 | AS | 409 | T(v**V**)^G(F)^U(M)U(M)T(**Mo**)A(M)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0450 | AS | 410 | T(v**V**)^G(F)^U(M)U(M)T(**Mo**)A(M)U(M)A(M)G(M)A(V)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0451 | AS | 411 | T(v**V**)^G(F)^U(M)U(M)T(**V**)A(M)U(M)A(M)G(M)A(**Mo**)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0452 | AS | 412 | T(v**V**)^G(F)^U(M)U(M)T(**Mo**)A(M)U(M)A(M)G(M)A(**Mo**)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0453 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 402 | A(**Mo**)^G(M)^U(M)G(M)U(M)U(M)C(F)U(M)U(F)G(F)C(F)U(M)C(M)U(M)A(M)U(M)A(M)A(M)A(M)^C(M)^A(**Mo**) |

### [Evaluation Example 23] Inhibition of expression of mouse Ttr in mouse primary hepatocytes

The siRNA described in Table 33 was evaluated at 2 concentrations of 0.001 or 0.01 nM using the same evaluation method as in Evaluation Example 9. The results are shown in Fig. 55.

As is apparent from Fig. 55, the siRNA including 2'-O-MCE nucleotides or 2'-O-MorECE nucleotides, and further containing a 5'-vinylphosphonate-modified 2'-O-MCE nucleotide (si-0449 to si-0453) exhibited the expression suppressing effect equivalent to or superior to that of the siRNA (si-0251) not containing these nucleotides.

### [Production Example 26] Synthesis of siRNA

According to the method described in Production Example 6, siRNAs described in Table 34 were synthesized. The target RNA of the siRNA described in Table 34 is Ttr (mouse).

si-0454 to si-0460 are siRNAs including 2'-O-MCE nucleotides and further including 5'-vinylphosphonate-modified 2'-O-MCE nucleotides, designed based on si-0251. Among these, si-0454 to si-0459 are siRNAs including 2'-O-Me nucleotides or deoxyribonucleotides at positions 9 to 11 as counted from the 5' end of the sense strand. In si-0460, the sense strand has a length of 19 nucleotides.

**[Table 34]**

| Compd. No. | Strand | SEQ No | Chemical Structure |
|---|---|---|---|
| si-0251 | AS | 231 | U(vM)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 211 | A(M)^G(M)^U(M)G(M)U(M)U(M)C(F)U(M)U(F)G(F)C (F)U(M)C(M)U(M)A(M)U(M)A(M)A(M)A(M)^C(M)^A(M) |
| si-0454 | AS | 238 | T(v**V**)^G(F)^U(M)U(M)T(**V**)A(M)U(M)A(M)G(M)A(V)G(M)C(M)A(M)A(F)G(M)A(F)A(M)C(M)A(M)C(M)U(M)^G(M)^U(M) |
| | S | 356 | A(**V**)^G(M)^U(M)G(M)U(M)U(M)C(F)U(M)U(M)G(F)C(F)U(M)C(M)U(M)A(M)U(M)A(M)A(M)A(M)^C(M)^A(**V**) |
| si-0455 | AS | 238 | T(v**V**)^G(F)^U(M)U(M)T(**V**)A(M)U(M)A(M)G(M)A(**V**)G(M)C(M)A(M)A(F)G(M)A(F)A(M)C(M)A(M)C(M)U(M)^G(M)^U(M) |
| | S | 357 | A(**V**)^G(M)^U(M)G(M)U(M)U(M)C(F)U(M)U(F)G(M)C(F)U(M)C(M)U(M)A(M)U(M)A(M)A(M)A(M)^C(M)^A(**V**) |
| si-0456 | AS | 238 | T(v**V**)^G(F)^U(M)U(M)T(**V**)A(M)U(M)A(M)G(M)A(V)G(M)C(M)A(M)A(F)G(M)A(F)A(M)C(M)A(M)C(M)U(M)^G(M)^U(M) |
| | S | 358 | A(V)^G(M)^U(M)G(M)U(M)U(M)C(F)U(M)U(F)G(F)C(M)U(M)C(M)U(M)A(M)U(M)A(M)A(M)A(M)^C(M)^A(V) |
| si-0457 | AS | 238 | T(v**V**)^G(F)^U(M)U(M)T(**V**)A(M)U(M)A(M)G(M)A(V)G(M)C(M)A(M)A(F)G(M)A(F)A(M)C(M)A(M)C(M)U(M)^G(M)^U(M) |
| | S | 364 | A(**V**)^G(M)^U(M)G(M)U(M)U(M)C(F)U(M)tG(F)C(F)U(M)C(M)U(M)A(M)U(M)A(M)A(M)A(M)^C(M)^A(**V**) |
| si-0458 | AS | 238 | T(v**V**)^G(F)^U(M)U(M)T(**V**)A(M)U(M)A(M)G(M)A(V)G(M)C(M)A(M)A(F)G(M)A(F)A(M)C(M)A(M)C(M)U(M)^G(M)^U(M) |
| | S | 365 | A(**V**)^G(M)^U(M)G(M)U(M)U(M)C(F)U(M)U(F)gC(F)U(M)C(M)U(M)A(M)U(M)A(M)A(M)A(M)^C(M)^A(**V**) |
| si-0459 | AS | 238 | T(v**V**)^G(F)^U(M)U(M)T(**V**)A(M)U(M)A(M)G(M)A(**V**)G(M)C(M)A(M)A(F)G(M)A(F)A(M)C(M)A(M)C(M)U(M)^G(M)^U(M) |
| | S | 366 | A(**V**)^G(M)^U(M)G(M)U(M)U(M)C(F)U(M)U(F)G(F)cU(M)C(M)U(M)A(M)U(M)A(M)A(M)A(M)^C(M)^A(**V**) |
| si-0460 | AS | 238 | T(v**V**)^G(F)^U(M)U(M)T(**V**)A(M)U(M)A(M)G(M)A(**V**)G(M)C(M)A(M)A(F)G(M)A(F)A(M)C(M)A(M)C(M)U(M)^G(M)^U(M) |
| | S | 372 | T(**V**)^G(M)^U(M)U(M)C(F)U(M)U(F)G(F)C(F)U(M)C(M)U(M)A(M)U(M)A(M)A(M)A(M)^C(M)^A(**V**) |

### [Evaluation Example 24] Inhibition of expression of mouse Ttr in mouse primary hepatocytes

The siRNA described in Table 34 was evaluated at 2 concentrations of 0.001 or 0.01 nM using the same evaluation method as in Evaluation Example 9. The results are shown in Fig. 56.

As is apparent from Fig. 56, the siRNA including 2'-O-MCE nucleotides (si-0454 to si-0460) exhibited the expression suppressing effect equivalent to or superior to that of the siRNA not including these nucleotides (si-0251). In addition, it was confirmed that the expression suppressing effect was maintained even when the siRNA including 2'-O-MCE nucleotides included 2'-O-Me nucleotides or deoxyribonucleotides at positions 9 to 11 as counted from the 5' end of the sense strand. Furthermore, it was confirmed that the expression suppressing effect of siRNA including 2'-O-MCE nucleotides was maintained even when the nucleotide length of the sense strand was shortened.

### [Production Example 27] Synthesis of siRNA

According to the method described in Production Example 6, siRNAs described in Table 35 were synthesized. The target RNA of the siRNA described in Table 35 is Ttr (mouse).

si-0461 to si-0467 are siRNAs including 2'-O-MCE or 2'-O-MorECE nucleotides and further including 5'-vinylphosphonate-modified 2'-O-MCE nucleotides, designed to model si-0251.

**[Table 35]**

| Compd. No. | Strand | SEQ No | Chemical Structure |
|---|---|---|---|
| si-0251 | AS | 231 | U(vM)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 211 | A(M)^G(M)^U(M)G(M)U(M)U(M)C(F)U(M)U(F)G(F)C(F)U(M)C(M)U(M)A(M)U(M)A(M)A(M)A(M)^C(M)^A(M) |
| si-0461 | AS | 413 | T(v**V**)^G(F)^T(**Mo**)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0462 | AS | 414 | T(v**V**)^G(F)^U(M)T(**Mo**)U(M)A(M)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0463 | AS | 415 | T(v**V**)^G(F)^U(M)U(M)U(M)A(**Mo**)U(M)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0464 | AS | 416 | T(v**V**)^G(F)^U(M)U(M)U(M)A(M)T(**Mo**)A(M)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0465 | AS | 417 | T(v**V**)^G(F)^U(M)U(M)U(M)A(M)U(M)A(**Mo**)G(M)A(M)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0466 | AS | 418 | T(v**V**)^G(F)^U(M)U(M)U(M)A(M)U(M)A(M)G(**Mo**)A(M)G(M)C(M)A(M)A(F)G(M)A(F)A(M)C(Mg(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |
| si-0467 | AS | 419 | T(v**V**)^G(F)^U(M)U(M)U(M)A(M)U(M)A(M)G(M)A(**Mo**)G(M)C(M)A(M)A(F)G(M)A(F)A(M)C(M)A(M)C(M)U(M)^G(M)^U(M) |
| | S | 213 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(**V**) |

### [Evaluation Example 25] Inhibition of expression of mouse Ttr in mouse primary hepatocytes

The siRNA described in Table 35 was evaluated at 2 concentrations of 0.001 or 0.01 nM using the same evaluation method as in Evaluation Example 9. The results are shown in Fig. 57.

As is apparent from Fig. 57, the siRNA including 2'-O-MCE nucleotides or 2'-O-MorECE nucleotides, and further including a 5'-vinylphosphonate-modified 2'-O-MCE nucleotide (si-0461 to si-0467) exhibited the expression suppressing effect equivalent to or superior to that of the siRNA (si-0251) not containing these nucleotides. In addition, it was confirmed that the siRNA including 2'-O-MorECE nucleotides at positions 3 to 10 as counted from the 5' end of the antisense strand exhibited an expression suppressing effect equivalent to or superior to that of the control.

### [Production Example 28] Synthesis of siRNA

According to the method described in Production Example 6, siRNAs described in Table 36 were synthesized. The target RNA of the siRNA described in Table 36 is Ttr (mouse).

si-0468 to si-0470 are siRNAs including 2'-O-MCE nucleotides or 2'-O-MorECE nucleotides and further including 5'-vinylphosphonate-modified 2'-O-MCE nucleotides, designed based on si-0298 including deoxyribonucleotides. In si-0470, the sense strand has a length of 19 nucleotides.

**[Table 36]**

| Compd. No. | Strand | SEQ No | Chemical Structure |
|---|---|---|---|
| si-0298 | AS | 272 | U(vM)^g^G(M)U(M)tU(M)aU(M)A(M)G(M)A(M)gC(M)A(F)A(M)G(M)A(M)A(M)C(M)A(M)C(M)^U(M)^G(M) |
| | S | 273 | G(M)^U(M)^G(M)U(M)U(M)C(M)U(M)U(M)G(F)C(F)U(F)C(M)U(M)A(M)U(M)A(M)A(M)A(M)C(M)^C(M)^A(M) |
| si-0468 | AS | 420 | T(v**V**)^g^G(M)U(M)tU(M)aU(M)A(M)G(M)A(M)G(**Mo**)C(M)A(F)A(M)G(M)A(M)A(M)C(M)A(M)C(M)^U(M)^G(M) |
| | S | 322 | G(**V**)^U(M)^G(M)U(M)U(M)C(M)U(M)U(M)G(F)C(F)U(F)C(M)U(M)A(M)U(M)A(M)A(M)A(M)C(M)^C(M)^A(**V**) |
| si-0469 | AS | 278 | T(v**V**)^g^G(M)U(M)tU(M)aU(M)A(M)G(M)A(M)G(**V**)C(M)A(F)A(M)G(M)A(M)A(M)C(M)A(M)C(M)^U(M)^G(M) |
| | S | 421 | G(**Mo**)^U(M)^G(M)U(M)U(M)C(M)U(M)U(M)G(F)C(F)U(F)C(M)U(M)A(M)U(M)A(M)A(M)A(M)C(M)^C(M)^A(**Mo**) |
| si-0470 | AS | 420 | T(v**V**)^g^G(M)U(M)tU(M)aU(M)A(M)G(M)A(M)G(**Mo**)C(M)A(F)A(M)G(M)A(M)A(M)C(M)A(M)C(M)^U(M)^G(M) |
| | S | 422 | G(**V**)^U(M)^U(M)C(M)U(M)U(M)G(F)C(F)U(F)C(M)U(M)A(M)U(M)A(M)A(M)A(M)C(M)^C(M)^A(**V**) |

### [Evaluation Example 26] Inhibition of expression of mouse Ttr in mouse primary hepatocytes

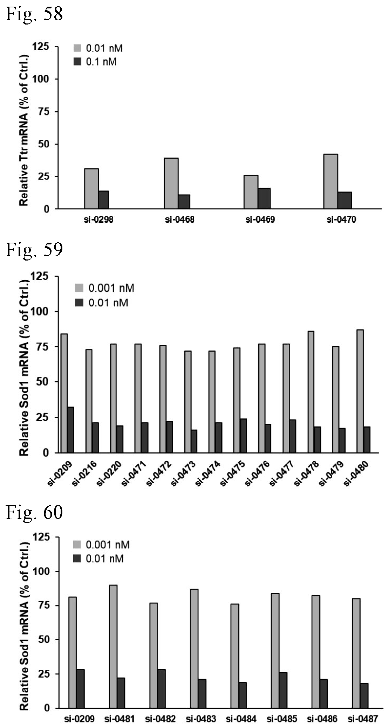

The siRNA described in Table 36 was evaluated at 2 concentrations of 0.01 or 0.1 nM using the same evaluation method as in Evaluation Example 9. The results are shown in Fig. 58.

As is apparent from Fig. 58, the siRNA including 2'-O-MCE nucleotides or 2'-O-MorECE nucleotides, and further including 5'-vinylphosphonate-modified 2'-O-MCE nucleotides (si-0468 to si-0470) exhibited the expression suppressing effect equivalent to or superior to that of the siRNA (si-0298) not including these nucleotides. In particular, siRNA including 2'-O-MorECE nucleotides at positions 1 and 21 as counted from the 5' end of the sense strand showed the high expression suppressing effect. Furthermore, it was confirmed that the expression suppressing effect of siRNA including 2'-O-MorECE nucleotides was maintained even when the nucleotide length of the sense strand was shortened.

### [Production Example 29] Synthesis of siRNA

According to the method described in Production Example 6, siRNAs described in Table 37 were synthesized. The target RNA of the siRNA described in Table 37 is Sodl (mouse and rat).

si-0216, si-0220, and si-0471 to si-0480 are siRNAs including 2'-O-MCE nucleotides, designed based on si-0209. si-0216 and si-0471 to si-0477 further include 5'-vinylphosphonate-modified 2'-O-MCE nucleotides, si-0220 further includes 5'-cyclopropanephosphonate-modified 2'-O-MCE nucleotides (isomer A), and si-0478 to si-0480 further include 5'-cyclopropanephosphonate-modified 2'-O-MCE nucleotides (isomer B).

**[Table 37]**

| Compd. No. | Strand | SEQ No | Chemical Structure |
|---|---|---|---|
| si-0209 | AS | 193 | U(vM)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0216 | AS | 200 | T(v**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0220 | AS | 204 | T(c**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0471 | AS | 423 | T(v**V**)^U(F)^T(**V**)A(M)G(M)A(F)G(M)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0472 | AS | 424 | T(v**V**)^U(F)^U(M)A(**V**)G(M)A(F)G(M)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0473 | AS | 425 | T(v**V**)^U(F)^U(M)A(M)G(M)A(**V**)G(M)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0474 | AS | 426 | T(v**V**)^U(F)^U(M)A(M)G(M)A(F)G(**V**)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0475 | AS | 427 | T(v**V**)^U(F)^U(M)A(M)G(M)A(F)G(M)T(V)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0476 | AS | 428 | T(v**V**)^U(F)^U(M)A(M)G(M)A(F)G(M)U(F)G(**V**)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0477 | AS | 429 | T(v**V**)^U(F)^U(M)A(M)G(M)A(F)G(M)U(F)G(F)A(**V**)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0478 | AS | 430 | T(dc**V**)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0479 | AS | 431 | T(dc**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0480 | AS | 432 | T(dc**V**)^U(F)^U(M)A(M)G(V)A(F)G(M)U(F)G(F)A(**V**)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |

### [Evaluation Example 27] Inhibition of expression of rat Sodl in RPE-J cells (rat retinal pigment epithelial (RPE) cells)

The siRNA described in Table 37 was evaluated at 2 concentrations of 0.001 or 0.01 nM using the same evaluation method as in Evaluation Example 1. The results are shown in Fig. 59.

As is apparent from Fig. 59, siRNAs including 2'-O-MCE nucleotides, and further containing 5'-vinylphosphonate-modified 2'-O-MCE nucleotides, 5'-cyclopropanephosphonate-modified 2'-O-MCE nucleotides (isomer A), or 5'-cyclopropanephosphonate-modified 2'-O-MCE nucleotides (isomer B) exhibited the expression suppressing effect equivalent to or superior to that of the siRNA not including 2'-O-MCE nucleotides (si-0209). In particular, siRNA (si-0216 and si-0471 to si-0477) including 2'-O-MCE nucleotides at positions 3 to 10 as counted from the 5' end of the antisense strand showed the high expression suppressing effect. In addition, all of the siRNA including either isomer A or isomer B of the 5'-cyclopropanephosphonate-modified 2'-O-MCE nucleotide (si-0220 or si-0478 to si-0480) showed the high expression suppressing effect.

### [Evaluation Example 28] Inhibition of expression of rat Sodl in RPE-J cells (rat retinal pigment epithelial (RPE) cells)

siRNA was tested at various concentrations using the same evaluation method as in Evaluation Example 1. The results are calculated as a concentration (IC₅₀ value) at which an RNA transcript level was 50% with respect to untreated control cells and are shown in Table 38.

**[Table 38]**

| Compd. No. | IC50 (nM) |
|---|---|
| si-0209 | 5.4 |
| si-0216 | 3.6 |
| si-0471 | 3.1 |
| si-0472 | 2.7 |
| si-0473 | 2.7 |
| si-0474 | 2.8 |
| si-0475 | 2.6 |
| si-0476 | 2.7 |
| si-0477 | 1.7 |

As is apparent from Table 38, the siRNA including the 5'-vinylphosphonate-modified 2'-O-MCE nucleotide and further including the 2'-O-MCE nucleotide at positions 3 to 10 as counted from the 5' end of the antisense strand exhibited the higher expression suppressing effect than the siRNA not including 2'-O-MCE nucleotides (si-0209).

### [Production Example 30] Synthesis of siRNA

According to the method described in Production Example 6, siRNAs described in Table 39 were synthesized. The target RNA of the siRNA described in Table 39 is Sodl (mouse and rat).

si-0481 to si-0487 are siRNAs including 2'-O-MorECE nucleotides and further including 5'-vinylphosphonate-modified 2'-O-MCE nucleotides, designed based on si-0209.

**[Table 39]**

| Compd. No. | Strand | SEQ No | Chemical Structure |
|---|---|---|---|
| si-0209 | AS | 193 | U(vM)^U(F)^U(M)A(M)G(M)A(F)G(M)U(F)G(F)A(M)G(M)G(M)A(M)U(F)U(M)A(F)A(M)A(M)A(M)U(M)G(M)^A(M)^G(M) |
| | S | 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| si-0481 | AS | 433 | T(v**V**)^U(F)^T(**Mo**)A(M)G(M)A(F)G(M)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0482 | AS | 434 | T(v**V**)^U(F)^U(M)A(**Mo**)G(M)A(F)G(M)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0483 | AS | 435 | T(v**V**)^U(F)^U(M)A(M)G(M)A(**Mo**)G(M)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0484 | AS | 436 | T(v**V**)^U(F)^U(M)A(M)G(M)A(F)G(**Mo**)U(F)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0485 | AS | 437 | T(v**V**)^U(F)^U(M)A(M)G(M)A(F)G(M)T(**Mo**)G(F)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0486 | AS | 438 | T(v**V**)^U(F)^U(M)A(M)G(M)A(F)G(M)U(F)G(**Mo**)A(M)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |
| si-0487 | AS | 439 | T(v**V**)^U(F)^U(M)A(M)G(M)A(F)G(M)U(F)G(F)A(**Mo**)G(M)G(M)A(M)U(F)U(M)A(F)A(M)A(M)A(M)U(M)G(M)^A(M)^G(M) |
| | S | 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |

### [Evaluation Example 29] Inhibition of expression of rat Sod1 in RPE-J cells (rat retinal pigment epithelial (RPE) cells)

The siRNA described in Table 39 was evaluated at 2 concentrations of 0.001 or 0.01 nM using the same evaluation method as in Evaluation Example 1. The results are shown in Fig. 60.

As is apparent from Fig. 60, the siRNA including 2'-O-MorECE nucleotides, and further including 5'-vinylphosphonate-modified 2'-O-MCE nucleotides (si-0481 to si-0487) exhibited the expression suppressing effect equivalent to or superior to that of the siRNA (si-0209) not including these nucleotides.

### [Production Example 31] Synthesis of siRNA

According to the method described in Production Example 6, siRNAs described in Table 40 were synthesized. The target RNA of the siRNA described in Table 40 is Ttr (mouse).

si-0489 to si-0500 are siRNAs including 2'-O-MCE nucleotides and 5'-vinylphosphonate-modified 2'-O-MCE nucleotides, and further including a GalNAc ligand, designed based on si-0488. si-0488 is an siRNA in which a GalNAc ligand is conjugated to the 3' end of the sense strand of si-0251. Similarly, si-0489 is an siRNA in which a GalNAc ligand is conjugated to the 3' end of a sense strand of si-0253, si-0490 is an siRNA in which a GalNAc ligand is conjugated to the 3' end of a sense strand of si-0396, si-0491 is an siRNA in which a GalNAc ligand is conjugated to the 3' end of a sense strand of si-0397, si-0492 is an siRNA in which a GalNAc ligand is conjugated to the 3' end of a sense strand of si-0398, si-0493 is an siRNA in which a GalNAc ligand is conjugated to the 3' end of a sense strand of si-0404, si-0494 is an siRNA in which a GalNAc ligand is conjugated to the 3' end of a sense strand of si-0405, si-0495 is an siRNA in which a GalNAc ligand is conjugated to the 3' end of a sense strand of si-0406, si-0496 is an siRNA in which a GalNAc ligand is conjugated to the 3' end of a sense strand of si-0399, si-0497 is an siRNA in which a GalNAc ligand is conjugated to the 3' end of a sense strand of si-0401, si-0498 is an siRNA in which a GalNAc ligand is conjugated to the 3' end of a sense strand of si-0456, si-0499 is an siRNA in which a GalNAc ligand is conjugated to the 3' end of a sense strand of si-0459, and si-0500 is an siRNA in which a GalNAc ligand is conjugated to the 3' end of a sense strand of si-0407.

A conjugate with a GalNAc ligand was synthesized by a standard method provided by Glen Research using a corresponding GalNAc-loaded solid phase and a GalNAc phosphoramidite (both from Glen Research). It can also be synthesized with reference to WO 2023014938 A and the like.

**[Table 40]**

| Compd. No. | Strand | SEQ No | Chemical Structure |
|---|---|---|---|
| si-0488 | AS | 231 | U(vM)^G(F)^U(M)U(M)U(M)A(M)U(M)A(M)G(M)A(M)G(M)C(M)A(M)A(F)G(M)A(F)A(M)C(M)A(M)C(M)U(M)^G(M)^U(M) |
| | S | 440 | A(M)^G(M)^U(M)G(M)U(M)U(M)C(F)U(M)U(F)G(F)C(F)U(M)C(M)U(M)A(M)U(M)A(M)A(M)^A(M)^C(M)^A(M)#Gal_s_TRP_s |
| si-0489 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 441 | A(**V**)^G(M)^U(M)G(M)U(M)U(M)C(F)U(M)U(F)G(F)C(F)U(M)C(M)U(M)A(M)U(M)A(M)A(M)A(M)^C(M)^A(V)#Gal_s_TRP_s |
| si-0490 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 442 | A(**V**)^G(M)^U(M)G(M)U(M)U(M)C(F)U(M)U(M)G(F)C(F)U(M)C(M)U(M)A(M)U(M)A(M)A(M)A(M)^C(M)^A(**V**)#Gal_s_TRP_s |
| si-0491 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 443 | A(V)^G(M)^U(M)G(M)U(M)U(M)C(F)U(M)U(F)G(M)C(F)U(M)C(M)U(M)A(M)U(M)A(M)A(M)A(M)^C(M)^A(V)#Gal_s_TRP_s |
| si-0492 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 444 | A(**V**)^G(M)^U(M)G(M)U(M)U(M)C(F)U(M)U(F)G(F)C(M)U(M)C(M)U(M)A(M)U(M)A(M)A(M)A(M)^C(M)^A(**V**)#Gal_s_TRP_s |
| si-0493 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 445 | A(**V**)^G(M)^U(M)G(M)U(M)U(M)C(F)U(M)tG(F)C(F)U(M)C(M)U(M)A(M)U(M)A(M)A(M)A(M)^C(M)^A(**V**)#Gal_s_TRP_s |
| si-0494 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 446 | A(**V**)^G(M)^U(M)G(M)U(M)U(M)C(F)U(M)U(F)gC(F)U(M)C(M)U(M)A(M)U(M)A(M)A(M)A(M)^C(M)^A(**V**)#Gal_s_TRP_s |
| si-0495 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 447 | A(**V**)^G(M)^U(M)G(M)U(M)U(M)C(F)U(M)U(F)G(F)cU(M)C(M)U(M)A(M)U(M)A(M)A(M)A(M)^C(M)^A(**V**)#Gal_s_TRP_s |
| si-0496 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 448 | A(**V**)^G(**V**)^U(M)G(M)U(M)U(M)C(F)U(M)U(F)G(M)C(F)U(M)C(M)U(M)A(M)U(M)A(M)A(M)^5(**V**)^A(**V**)#Gal_s_TRP_s |
| si-0497 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 449 | A(**V**)^G(M)^U(M)G(M)U(M)U(M)C(F)U(M)U(F)G(M)C(F)U(M)C(F)U(M)A(M)U(M)A(M)A(M)A(M)^C(M)^A(**V**)#Gal_s_TRP_s |
| si-0498 | AS | 238 | T(v**V**)^G(F)^U(M)U(M)T(**V**)A(M)U(M)A(M)G(M)A(V)G(M)C(M)A(M)A(F)G(M)A(F)A(M)C(M)A(M)C(M)U(M)^G(M)^U(M) |
| | S | 444 | A(V)^G(M)^U(M)G(M)U(M)U(M)C(F)U(M)U(F)G(F)C(M)U(M)C(M)U(M)A(M)U(M)A(M)A(M)A(M)^C(M)^A(V)#Gal_s_TRP_s |
| si-0499 | AS | 238 | T(v**V**)^G(F)^U(M)U(M)T(**V**)A(M)U(M)A(M)G(M)A(**V**)G(M)C(M)A(M)A(F)G(M)A(F)A(M)C(M)A(M)C(M)U(M)^G(M)^U(M) |
| | S | 447 | A(**V**)^G(M)^U(M)G(M)U(M)U(M)C(F)U(M)U(F)G(F)cU(M)C(M)U(M)A(M)U(M)A(M)A(M)A(M)^C(M)^A(**V**)#Gal_s_TRP_s |
| si-0500 | AS | 233 | T(v**V**)^G(F)^U(M)U(M)T(**V**)A(M)U(M)A(M)G(M)A(M)G(M)C(M)A(M)A(F)G(M)A(F)A(M)C(M)A(M)C(M)U(M)^G(M)^U(M) |
| | S | 450 | A(**V**)^G(**V**)^U(M)G(M)U(M)U(M)C(F)U(M)U(F)gC(F)U(M)C(M)U(M)A(M)U(M)A(M)A(M)A(M)^5(V)^A(**V**)#Gal_s_TRP_s |

### [Evaluation Example 30] Ttr knockdown action in mice

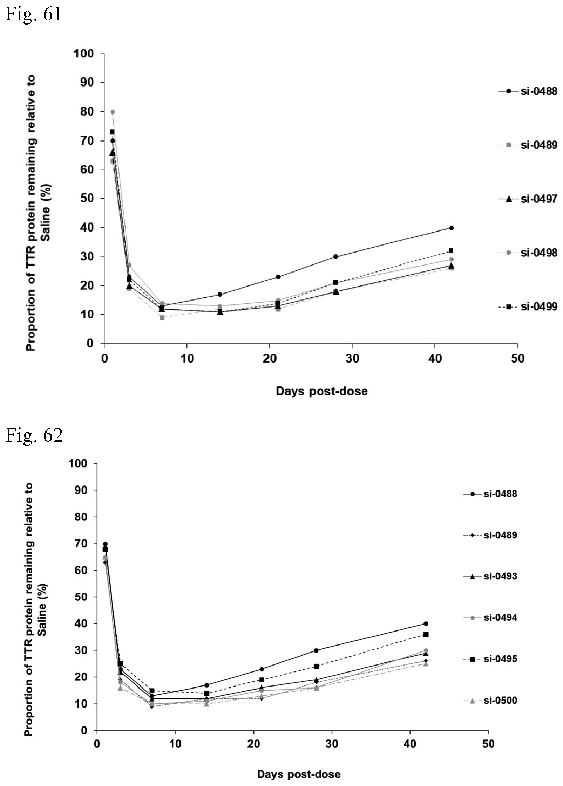

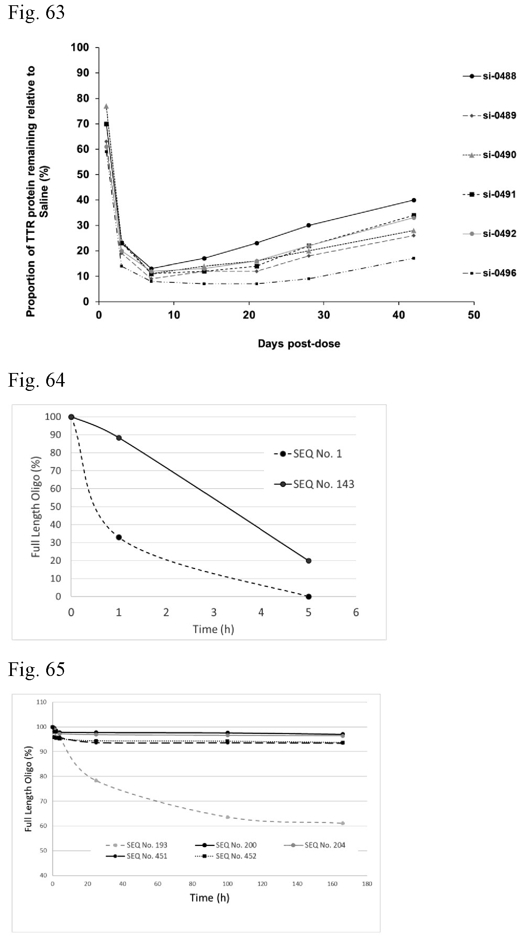

The siRNA described in Table 40 was evaluated using the same evaluation method as in Evaluation Example 11. Each siRNA agent was subcutaneously administered at 1 mg/kg, and blood was collected and treated to obtain serum on Days 1, 3, 7, 14, 21, 28, and 42 after administration. TTR levels were determined by ELISA and expressed as percent expression of TTR relative to a negative control (vehicle). The results are shown in Fig. 61 (si-0488, si-0489, si-0497, si-0498, and si-0499), Fig. 62 (si-0488, si-0489, si-0493, si-0494, si-0495, and si-0500), and Fig. 63 (si-0488, si-0489, si-0490, si-0491, si-0492, and si-0496).

As is apparent from Figs. 61 to 63, it was confirmed that the siRNA including 2'-O-MCE nucleotides (si-0489 to si-0500) exhibited a higher expression suppressing effect at any time on and after Day 14 than the siRNA not including 2'-O-MCE nucleotides (si-0488), and had long-term persistence.

### [Evaluation Example 31] Evaluation of nuclease resistance of oligonucleotide including XCE nucleotide

Resistance to nucleases was examined for the sense strand (SEQ No. 143) including 2'-O-MCE nucleotides at positions 1 and 21, which is commonly used in siRNAs including 2'-O-XCE nucleotides in the above Examples, and the sense strand (SEQ No. 1) including 2'-O-Me nucleotides at positions 1 and 21, which is commonly used in siRNAs not including 2'-O-XCE nucleotides. Oligonucleotides described in Table 41 were used as representative sense oligonucleotide strands, respectively.

**[Table 41]**

| SEQ No | Chemical Structure |
|---|---|
| 1 | C(M)^A(M)^U(M)U(M)U(M)U(M)A(F)A(M)U(F)C(F)C(F)U(M)C(M)A(M)C(M)U(M)C(M)U(M)A(M)^A(M)^A(M) |
| 143 | 5(**V**)^A(M)^U(M)U(M)U(M)U(M)A(F)A(M)U(F)C(F)C(F)U(M)C(M)A(M)C(M)U(M)C(M)U(M)A(M)^A(M)^A(**V**) |

To a buffer solution (75 µL) including 7.5 nmol of oligonucleotide, a buffer solution (960 µL) and SVPDE1 [snake venom phosphodiesterase 1] (Worthington Biochemical Corporation) (40 µg/40 µL buffer solution) were added, and the mixture was held at 37°C. 100 µl was withdrawn at each time and heated at 95°C for 5 min (enzyme deactivation). Thereafter, the mixture was cooled to room temperature, and the residual amount was measured by HPLC.

The composition (final concentration) of the buffer used was an aqueous solution containing 50 mM of Tris·HCl (tris(hydroxymethyl)aminomethane hydrochloride, pH 8.0) and 10 mM of MgCl₂, and was sufficiently degassed before measurement. Quantitative conditions by HPLC are as follows.

### (HPLC Analysis Conditions)

Eluent: aqueous solution including 0.1 M hexafluoroisopropyl alcohol and 8 mM triethylamine/methanol = 95/5 (1 min) → (14 min) → 70/30 (3 min)
Flow rate: 1.0 mL/min
Column: WatersXBridge (trademark) C18 2.5 µm,4.6 mm × 75 mm
Column temperature: 60°C
Detection: UV (260 nm)

The results are shown in Fig. 64. In Fig. 64, "Full Length Oligo (%)" indicates the residual ratio of the undegraded oligonucleotide (21 mer) at the measurement time point with respect to the undegraded oligonucleotide (21 mer) at time zero. In addition, "Time [h]" indicates the measurement time (the unit is hours).

As is apparent from Fig. 64, it was found that the sense oligonucleotide strand (SEQ No. 143) commonly used in siRNA including 2'-O-XCE nucleotides had higher enzyme resistance than the sense oligonucleotide strand (SEQ No. 1) not including 2'-O-XCE nucleotides.

### [Production Example 32] Synthesis of Oligonucleotides

According to the method described in Production Example 6, oligonucleotides described in Table 41 were synthesized. The oligonucleotides described in Table 41 include 5'-phosphonate-modified 2'-O-XCE nucleotides.

**[Table 42]**

| SEQ No | Chemical Structure |
|---|---|
| 193 | U(vM)^U(F)^U(M)A(M)G(M)A(F)G(M)U(F)G(F)A(M)G(M)G(M)A(M)U(F) U(M)A(F)A(M)A(M)A(M)U(M)G(M)^A(M)^G(M) |
| 200 | T(v**V**)^U(F)^U(M)A(M)G(**V**)A(F)G(M)U(F)G(F)A(M)G(M)G(M)A(M)U(F)U(M)A(F)A(M)A(M)A(M) U(M)G(M)^A(M)^G(M) |
| 204 | T(c**V**)^U(F)^U(M)A(M)G(**V**)A(F)G(M)U(F)G(F)A(M)C(M)G(M)A(M)U(F)U(MjA(F)A(M)A(M)A(M)U(M)G(M)^A(M)^G(M) |
| 451 | T(e**V**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |
| 452 | T(v**Mo**)^U(F)^U(M)A(M)G(**V**)A(F)G(M)U(F)G(F)A(M)G(M)G(M)A(M)U(F)U(M)A(F)A(M)A(M)A(M)U(M)G(M)^A(M)^G(M) |

### [Evaluation Example 32] Evaluation of nuclease resistance of oligonucleotide including 5'-phosphonate-modified 2'-O-XCE nucleotide

Resistance to nucleases was examined for an antisense strand including a 2'-O-MCE nucleotide at position 5 and further including a 5'-phosphonate-modified 2'-O-XCE nucleotide at position 1, which is commonly used in siRNAs including 2'-O-XCE nucleotides in the above Examples, and an antisense strand including a 5'-vinylphosphonate-modified 2'-O-Me nucleotide at position 1, which is commonly used in siRNAs not including 2'-O-XCE nucleotides. Oligonucleotides described in Table 41 were used as representative sense oligonucleotide strands, respectively.

A buffer solution (45 µL) including 150 pmol of oligonucleotide was mixed with a buffer solution (15 µL) including 5'-Phosphate-Dependent Exonuclease (Lucigen), and the mixture was held at 30°C. The sample was immediately heated at 95°C for 5 minutes at each time (enzyme deactivation), then cooled to room temperature, and the remaining amount was measured by HPLC.

Buffer A enclosed in 5'-Phosphate-Dependent Exonuclease (Lucigen) was used as the buffer used. Quantitative conditions by HPLC are as follows.

### (HPLC Analysis Conditions)

Eluent: aqueous solution including 0.1 M hexafluoroisopropyl alcohol and 8 mM triethylamine/methanol = 95/5 (1 min) → (14 min) → 70/30 (3 min)
Flow rate: 1.0 mL/min
Column: WatersXBridge (trademark) C18 2.5 µm,4.6 mm × 75 mm
Column temperature: 60°C
Detection: UV (260 nm)

The results are shown in Fig. 65. In Fig. 65, "Full Length Oligo (%)" indicates the residual ratio of the undegraded oligonucleotide (23 mer) at the measurement time point with respect to the undegraded oligonucleotide (23 mer) at time zero. In addition, "Time [h]" indicates the measurement time (the unit is hours).

As is apparent from Fig. 65, antisense oligonucleotide strands including 5'-phosphonate-modified 2'-O-XCE nucleotides that are commonly used in siRNAs including 2'-O-XCE nucleotides were found to have higher enzyme resistance than antisense oligonucleotide strands including 5'-vinylphosphonate-modified 2'-O-Me nucleotides not including 2'-O-XCE nucleotides.

### Industrial Applicability

The siRNA including carbamoylethyl modification (2'-O-XCE nucleotide) provides a chemically modified siRNA that further enhances metabolic stability while maintaining activity. Therefore, the double-stranded RNA of the present invention can be expected to effectively control the expression of the target RNA, and is useful as a nucleic acid drug.

## Claims

1. A double-stranded RNA capable of inhibiting expression of a target RNA, wherein
the double-stranded RNA comprises a sense strand and an antisense strand,
the antisense strand has 14 to 40 nucleotides and has complementarity to the target RNA sufficient to mediate RNA interference,
the sense strand has 14 to 40 nucleotides and has complementarity to the antisense strand, and
the double-stranded RNA comprises at least one 2'-O-XCE nucleotide.

2. The double-stranded RNA according to claim 1, wherein the antisense strand comprises at least one 2'-O-XCE nucleotide, or the sense strand comprises at least one 2'-O-XCE nucleotide.

3. The double-stranded RNA according to claim 1 or 2, wherein the double-stranded RNA comprises at least one 2'-O-XCE nucleotide and at least one 2'-fluoro nucleotide.

4. The double-stranded RNA according to any one of claims 1 to 3, wherein the double-stranded RNA comprises at least one 2'-O-XCE nucleotide and at least one 2'-O-Me nucleotide.

5. The double-stranded RNA according to any one of claims 1 to 4, wherein the double-stranded RNA comprises at least one 2'-O-XCE nucleotide and at least one deoxyribonucleotide.

6. The double-stranded RNA according to any one of claims 1 to 5, wherein the antisense strand comprises a 2'-O-XCE nucleotide at least one of positions 1, 3, 4, 5, 6, 7, 8, 9, 10, 11, 12, 13, 15, 16, 17, 18, 19, 20, 21, 22, and 23 as counted from a 5' end of the antisense strand hybridizing portion.

7. The double-stranded RNA according to any one of claims 1 to 6, wherein the sense strand comprises a 2'-O-XCE nucleotide at least one of positions 1, 2, 3, 4, 5, 6, 7, 8, 9, 10, 14, 15, 16, 17, 18, 19, 20, 21, 22, and 23 as counted from a 3' end of the sense strand hybridizing portion.

8. The double-stranded RNA according to any one of claims 1 to 7, wherein the 2'-O-XCE nucleotide is a nucleotide comprising a partial structure represented by Formula (I) below: 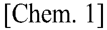 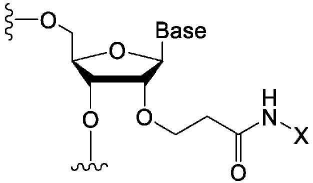
{in which Base is a purin-9-yl group, a 2-oxo-pyrimidin-1-yl group, or a 2-thioxo-pyrimidin-1-yl group (the purin-9-yl group, the 2-oxo-pyrimidin-1-yl group, and the 2-thioxo-pyrimidin-1-yl group are each independently unsubstituted or substituted with one or more substituents solely or differently selected from the group consisting of a halogen atom, a C1-6 alkyl group, an amino group, a protected amino group, a hydroxy group, a protected hydroxy group, a sulfanyl group, and a protected sulfanyl group),
X is a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group), or
a group represented by Formula (Ia) below: 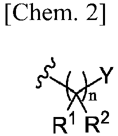
[in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group) or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with the nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
n is an integer of 1 to 3, and when n is 2 or 3, two or three R¹ and R² may be the same or different, respectively]}.

9. The double-stranded RNA according to claim 8, wherein the X is a C1-6 alkyl group or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group).

10. The double-stranded RNA according to claim 8 or 9, wherein the X is a methyl group.

11. The double-stranded RNA according to claim 8, wherein the X is a group represented by the following Formula (Ia): 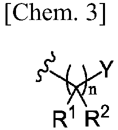
[in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with a nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
n is an integer of 1 to 3, and when n is 2 or 3, two or three R¹ and R² may be the same or different, respectively].

12. The double-stranded RNA according to claim 11, wherein the R¹ and R² are hydrogen atoms.

13. The double-stranded RNA according to claim 11 or 12, wherein
the Y is NR³R⁴, and the R³ and R⁴ together with a nitrogen atom, to which they are bonded, form a ring comprising 4 to 6 methylene groups among 4- to 8-membered nitrogen-containing non-aromatic heterocyclic rings, or
Y is a C2-9 aromatic heterocyclic group.

14. The double-stranded RNA according to any one of claims 11 to 13, wherein the Y is NR³R⁴, and the R³ and R⁴ together with a nitrogen atom, to which they are bonded, form morpholine.

15. The double-stranded RNA according to any one of claims 11 to 13, wherein the Y is a pyridyl group, an imidazolyl group, or a benzimidazolyl group.

16. The double-stranded RNA according to any one of claims 11 to 15, wherein n is 2.

17. The double-stranded RNA according to any one of claims 1 to 16, wherein the antisense strand comprises one, two, three, four, five, or six 2'-fluoro nucleotides.

18. The double-stranded RNA according to any one of claims 1 to 17, wherein the sense strand comprises one, two, three, four, or five 2'-fluoro nucleotides.

19. The double-stranded RNA according to any one of claims 1 to 18, wherein the sense strand comprises seven to eighteen 2'-O-Me nucleotides.

20. The double-stranded RNA according to any one of claims 1 to 19, wherein the antisense strand comprises thirteen to twenty 2'-O-Me nucleotides.

21. The double-stranded RNA according to any one of claims 1 to 20, wherein the sense strand comprises 0 to 6 deoxyribonucleotides.

22. The double-stranded RNA according to any one of claims 1 to 21, wherein the antisense strand comprises 0 to 6 deoxyribonucleotides.

23. The double-stranded RNA according to any one of claims 1 to 22, wherein the double-stranded RNA comprises at least one phosphorothioate bond.

24. The double-stranded RNA according to any one of claims 1 to 23, wherein the sense strand comprises 1, 2, 3, or 4 phosphorothioate bonds.

25. The double-stranded RNA according to any one of claims 1 to 24, wherein the sense strand comprises a phosphorothioate bond at least one position selected from the group consisting of between nucleotide positions 1 and 2 and between nucleotide positions 2 and 3 as counted from a 5' end of the sense strand, and between nucleotide positions 1 and 2, and between nucleotide positions 2 and 3 as counted from a 3' end.

26. The double-stranded RNA according to any one of claims 1 to 25, wherein the antisense strand comprises 1, 2, 3, or 4 phosphorothioate bonds.

27. The double-stranded RNA according to any one of claims 1 to 26, wherein the antisense strand comprises phosphorothioate bonds between nucleotide positions 1 and 2 and between nucleotide positions 2 and 3 as counted from a 5' end of the antisense strand, and between nucleotide positions 1 and 2 and between nucleotide positions 2 and 3 as counted from a 3' end.

28. The double-stranded RNA according to any one of claims 1 to 27, wherein the antisense strand comprises a 5'-phosphonate group at a 5' end of the antisense strand.

29. The double-stranded RNA according to claim 28, wherein the 5'-phosphonate group is a 5'-vinylphosphonate group.

30. The double-stranded RNA according to claim 28 or 29, wherein the antisense strand comprises a 5'-vinylphosphonate-modified 2'-O-Me nucleotide at position 1 as counted from a 5' end of the antisense strand.

31. The double-stranded RNA according to claim 28 or 29, wherein the antisense strand comprises a 5'-vinylphosphonate-modified 2'-O-XCE nucleotide at position 1 as counted from a 5' end of the antisense strand.

32. The double-stranded RNA according to claim 28, wherein the 5'-phosphonate group is a 5'-cyclopropylphosphonate group.

33. The double-stranded RNA according to claim 28 or 32, wherein the antisense strand comprises a 5'-cyclopropylphosphonated 2'-O-Me nucleotide at position 1 as counted from a 5' end of the antisense strand.

34. The double-stranded RNA according to claim 28 or 32, wherein the antisense strand comprises a 5'-cyclopropylphosphonated 2'-O-XCE nucleotide at position 1 as counted from a 5' end of the antisense strand.

35. The double-stranded RNA according to claim 28, wherein the 5'-phosphonate group is a 5'-ethylphosphonate group.

36. The double-stranded RNA according to claim 28 or 35, wherein the antisense strand comprises a 5'-ethylphosphonate-modified 2'-O-Me nucleotide at position 1 as counted from a 5' end of the antisense strand.

37. The double-stranded RNA according to claim 28 or 35, wherein the antisense strand comprises a 5'-ethylphosphonate-modified 2'-O-XCE nucleotide at position 1 as counted from a 5' end of the antisense strand.

38. The double-stranded RNA according to any one of claims 1 to 37, wherein the sense strand and the antisense strand independently have a length of 19 to 25 nucleotides.

39. The double-stranded RNA according to any one of claims 1 to 38, wherein the sense strand has a length of 21 nucleotides.

40. The double-stranded RNA according to any one of claims 1 to 39, wherein the antisense strand has a length of 23 nucleotides.

41. The double-stranded RNA according to any one of claims 1 to 40, wherein the sense strand has a length of 21 nucleotides, and the antisense strand has a length of 23 nucleotides.

42. The double-stranded RNA according to any one of claims 1 to 41, wherein the antisense strand comprises an overhang at a 3' end of the antisense strand.

43. The double-stranded RNA according to any one of claims 1 to 42, wherein the antisense strand comprises a blunt end at a 5' end of the antisense strand.

44. The double-stranded RNA according to any one of claims 1 to 43, further comprising a group derived from a functional molecule having at least one function selected from the group consisting of a labeling function, a purification function, and a function of delivery to a target site.

45. The double-stranded RNA according to claim 44, wherein the functional molecule is selected from the group consisting of a sugar, a lipid, a peptide, and a protein, and derivatives thereof.

46. The double-stranded RNA according to claim 44 or 45, wherein the functional molecule is selected from the group consisting of cholesterol, vitamins, steroids, C5-30 saturated fatty acids, C5-30 unsaturated fatty acids, C5-30 alkyl groups, and C5-30 alkenyl groups.

47. The double-stranded RNA according to claim 44 or 45, wherein the functional molecule is a peptide or protein selected from the group consisting of a ligand of a receptor and an antibody.

48. The double-stranded RNA according to claim 44 or 45, wherein the functional molecule is a sugar derivative that interacts with an asialoglycoprotein receptor.

49. A pharmaceutical composition comprising the double-stranded RNA according to any one of claims 1 to 48 and a pharmacologically acceptable carrier.

50. A method for controlling a function of a target RNA, comprising a step of bringing the double-stranded RNA according to any one of claims 1 to 48 into contact with a cell.

51. A method for controlling a function of a target RNA in a mammal, comprising a step of administering the pharmaceutical composition according to claim 49 to the mammal.

52. A method for producing the double-stranded RNA according to any one of claims 1 to 48 using a 2'-O-XCE nucleotide.

53. A compound represented by Formula (II) below, or a salt thereof: 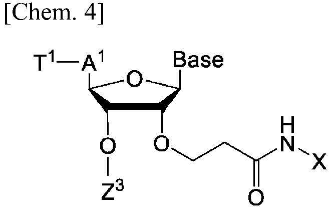
{in which Base is a purin-9-yl group, a 2-oxo-pyrimidin-1-yl group, or a 2-thioxo-pyrimidin-1-yl group (the purin-9-yl group, the 2-oxo-pyrimidin-1-yl group, and the 2-thioxo-pyrimidin-1-yl group are each independently unsubstituted or substituted with one or more substituents solely or differently selected from the group consisting of a halogen atom, a C1-6 alkyl group, an amino group, a protected amino group, a hydroxy group, a protected hydroxy group, a sulfanyl group, and a protected sulfanyl group),
Z³ is a hydrogen atom, a hydroxy-protecting group, or a phosphorus-containing group,
X is a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group), or
a group represented by Formula (IIa) below: 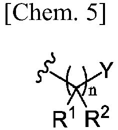
[in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group) or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with the nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
n is an integer of 1 to 3, and when n is 2 or 3, two or three R¹ and R² may be the same or different, respectively],
T¹ is a group represented by Formula (IIb) below: 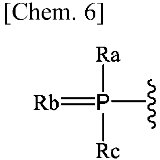
[Ra and Rc are each independently selected from a hydroxy group, a protected hydroxy group, a thiol group, a protected thiol group, an amino group, a protected amino group, a C1-6 alkyl group, a C2-6 alkenyl group, and a C1-6 alkoxy group (the hydroxy group, the thiol group, the amino group, the C1-6 alkyl group, the C2-6 alkenyl group, and the C1-6 alkoxy group are each independently unsubstituted or substituted with one or more substituents solely or differently selected from the group consisting of a halogen atom, a C1-6 alkyl group, a C1-6 alkoxy group, and a cyano group), and
Rb is an oxygen atom or a sulfur atom], and
A¹ is a group selected from Formula (IIc) below: 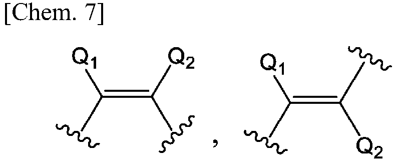
[Q₁ and Q₂ are each independently a hydrogen atom, a halogen atom, a C1-C6 alkyl group, a C1-6 alkoxy group, a C2-6 alkenyl group, a C2-6 alkynyl group, or an amino group (the C1-C6 alkyl group, the C1-6 alkoxy group, the C2-6 alkenyl group, the C2-6 alkynyl group, and the amino group are each independently unsubstituted or substituted with one or more substituents solely or differently selected from the group consisting of a halogen atom, a C1-6 alkyl group, a C1-6 alkoxy group, and a cyano group)]}.

54. The compound according to claim 53, or a salt thereof, wherein the X is a methyl group, and Q₁ and Q₂ are hydrogen atoms.

55. The compound according to claim 53 or 54, or a salt thereof, wherein the R¹ and R² are hydrogen atoms, the Y is NR³R⁴, the R³ and R⁴ together with the nitrogen atom, to which they are bonded, form morpholine, and Q₁ and Q₂ are hydrogen atoms.

56. The compound according to any one of claims 53 to 55, or a salt thereof, wherein the phosphorus-containing group is a cyanoethoxy(diisopropylamino)phosphino group or a hydroxyphosphinyl group.

57. A compound represented by Formula (III) below, or a salt thereof: 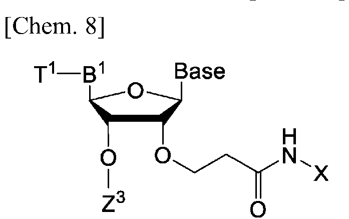
{in which Base is a purin-9-yl group, a 2-oxo-pyrimidin-1-yl group, or a 2-thioxo-pyrimidin-1-yl group (the purin-9-yl group, the 2-oxo-pyrimidin-1-yl group, and the 2-thioxo-pyrimidin-1-yl group are each independently unsubstituted or substituted with one or more substituents solely or differently selected from the group consisting of a halogen atom, a C1-6 alkyl group, an amino group, a protected amino group, a hydroxy group, a protected hydroxy group, a sulfanyl group, and a protected sulfanyl group),
Z³ is a hydrogen atom, a hydroxy-protecting group, or a phosphorus-containing group,
X is a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group), or
a group represented by Formula (IIIa) below: 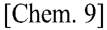 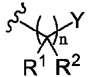
[in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group) or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with the nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
n is an integer of 1 to 3, and when n is 2 or 3, two or three R¹ and R² may be the same or different, respectively],
T¹ is a group represented by Formula (IIIb) below: 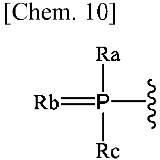
[Ra and Rc are each independently selected from a hydroxy group, a protected hydroxy group, a thiol group, a protected thiol group, an amino group, a protected amino group, a C1-6 alkyl group, a C2-6 alkenyl group, and a C1-6 alkoxy group (the hydroxy group, the thiol group, the amino group, the C1-6 alkyl group, the C2-6 alkenyl group, and the C1-6 alkoxy group are each independently unsubstituted or substituted with one or more substituents solely or differently selected from the group consisting of a halogen atom, a C1-6 alkyl group, a C1-6 alkoxy group, and a cyano group), and
Rb is an oxygen atom or a sulfur atom], and
B¹ is a group represented by Formula (IIIc) below: 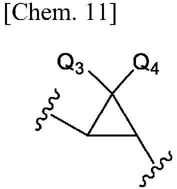
[Q₃ and Q₄ are each independently a hydrogen atom, a halogen atom, a C1-C6 alkyl group, a C1-6 alkoxy group, a C2-6 alkenyl group, a C2-6 alkynyl group, or an amino group (the C1-C6 alkyl group, the C1-6 alkoxy group, the C2-6 alkenyl group, the C2-6 alkynyl group, and the amino group are each independently unsubstituted or substituted with one or more substituents solely or differently selected from the group consisting of a halogen atom, a C1-6 alkyl group, a C1-6 alkoxy group, and a cyano group)]}.

58. The compound according to claim 57, or a salt thereof, wherein the X is a methyl group, and Q₃ and Q₄ are hydrogen atoms.

59. The compound according to claim 57 or 58, or a salt thereof, wherein the R¹ and R² are hydrogen atoms, the Y is NR³R⁴, the R³ and R⁴ together with the nitrogen atom, to which they are bonded, form morpholine, and Q₃ and Q₄ are hydrogen atoms.

60. The compound according to any one of claims 57 to 59, or a salt thereof, wherein the phosphorus-containing group is a cyanoethoxy(diisopropylamino)phosphino group or a hydroxyphosphinyl group.

61. A compound represented by Formula (IV) below, or a salt thereof: 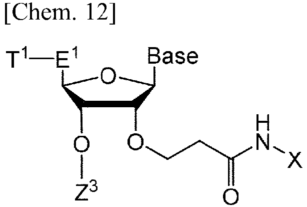
{in which Base is a purin-9-yl group, a 2-oxo-pyrimidin-1-yl group, or a 2-thioxo-pyrimidin-1-yl group (the purin-9-yl group, the 2-oxo-pyrimidin-1-yl group, and the 2-thioxo-pyrimidin-1-yl group are each independently unsubstituted or substituted with one or more substituents solely or differently selected from the group consisting of a halogen atom, a C1-6 alkyl group, an amino group, a protected amino group, a hydroxy group, a protected hydroxy group, a sulfanyl group, and a protected sulfanyl group),
Z³ is a hydrogen atom, a hydroxy-protecting group, or a phosphorus-containing group,
X is a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group), or
a group represented by Formula (IVa) below: 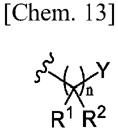
[in which R¹ and R² are each independently a hydrogen atom, a halogen atom, a cyano group, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a C1-6 alkoxy group, and a cyano group),
Y is NR³R⁴ (the R³ and R⁴ are each independently a hydrogen atom, a C1-6 alkyl group, or a C2-6 alkenyl group (the C1-6 alkyl group and the C2-6 alkenyl group are each independently unsubstituted or substituted with one or more substituents solely or differently selected from the group consisting of a halogen atom, a cyano group, a nitro group, an amino group, a hydroxy group, a carboxy group, a carbamoyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group) or a C7-10 aralkyl group (the C7-10 aralkyl group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), or the R³ and R⁴ together with the nitrogen atom, to which they are bonded, form a 3- to 11-membered nitrogen-containing non-aromatic heterocyclic ring (the 3- to 11-membered nitrogen-containing non-aromatic heterocyclic ring is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group)), or
a C2-9 aromatic heterocyclic group (the C2-9 aromatic heterocyclic group is unsubstituted or substituted with one or more substituents solely or differently selected from the group consisting of a halogen atom, a cyano group, a nitro group, an amino group, a hydroxy group, a carboxy group, a carbamoyl group, a C1-6 alkyl group, a C2-6 alkenyl group, a C1-6 alkoxy group, a C2-6 alkenyloxy group, a C1-6 alkoxycarbonyl group, a C2-6 alkenyloxycarbonyl group, a C1-6 alkylcarbonyl group, a C1-6 haloalkyl group, a C1-6 alkylamino group, a C1-6 alkylaminocarbonyl group, a C1-6 alkylcarbonyloxy group, a C1-6 alkylcarbonylamino group, and a C1-6 alkoxycarbonylamino group), and
n is an integer of 1 to 3, and when n is 2 or 3, two or three R¹ and R² may be the same or different, respectively],
T¹ is a group represented by Formula (IVb) below: 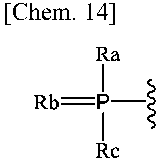
[Ra and Rc are each independently selected from a hydroxy group, a protected hydroxy group, a thiol group, a protected thiol group, an amino group, a protected amino group, a C1-6 alkyl group, a C2-6 alkenyl group, and a C1-6 alkoxy group (the hydroxy group, the thiol group, the amino group, the C1-6 alkyl group, the C2-6 alkenyl group, and the C1-6 alkoxy group are each independently unsubstituted or substituted with one or more substituents solely or differently selected from the group consisting of a halogen atom, a C1-6 alkyl group, a C1-6 alkoxy group, and a cyano group), and
Rb is an oxygen atom or a sulfur atom], and
E¹ is a group represented by Formula (IVc) below: 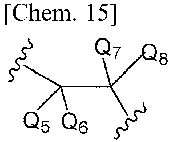
[Q₅ to Q₈ are each independently a hydrogen atom, a halogen atom, a C1-C6 alkyl group, a C1-6 alkoxy group, a C2-6 alkenyl group, a C2-6 alkynyl group, or an amino group (the C1-C6 alkyl group, the C1-6 alkoxy group, the C2-6 alkenyl group, the C2-6 alkynyl group, and the amino group are each independently unsubstituted or substituted with one or more substituents solely or differently selected from the group consisting of a halogen atom, a C1-6 alkyl group, a C1-6 alkoxy group, and a cyano group)]}.

62. The compound according to claim 61, or a salt thereof, wherein the X is a methyl group, and Q₅ to Q₈ are hydrogen atoms.

63. The compound according to claim 61 or 62, or a salt thereof, wherein the R¹ and R² are hydrogen atoms, the Y is NR³R⁴, the R³ and R⁴ together with the nitrogen atom, to which they are bonded, form morpholine, and Q₅ to Q₈ are hydrogen atoms.

64. The compound according to any one of claims 61 to 63, or a salt thereof, wherein the phosphorus-containing group is a cyanoethoxy(diisopropylamino)phosphino group or a hydroxyphosphinyl group.
